# EUROPEAN PATENT APPLICATION

(11) **EP 2 390 256 A1**
(43) Date of publication of application: **30.11.2011**
(21) Application number: 10179915.3
(22) Date of filing: 30.05.2002
(51) Int. Cl.: C07H 19/00, C07H 21/00, C07H 21/04, C12N 15/00, C12N 15/05, C12N 15/09, C12N 15/10, C12N 15/11, C12N 15/12, C12N 15/63, C12N 15/65, C12N 15/82

(54) **Plant artificial chromosomes, uses thereof and methods of preparing plant artificial chromosomes**

(30) Priority: 30.05.2001 US 294687 P; 04.06.2001 US 296329 P
(62) Divisional of application: 02739627.4
(71) Applicant: Agrisoma, Inc., Burnaby, B.C. V5A 1W9 (CA); Calyx Bio-Ventures Inc., North Vancouver, BC V7P 3N4 (CA)
(72) Inventor: Perez, Carl, Vancouver British Columbia V5Y 1S6 (CA); Fabijanski, Steven, Ottawa Ontario K1C 5N4 (CA); Perkins, Edward, Savannah GA 31404 (US)
(74) Representative: Spencer, Matthew Peter

(57) **Abstract**

Methods for preparing cell lines that contain plant artificial chromosomes, methods for preparation of plant artificial chromosomes, methods for targeted insertion of heterologous DNA into plant artificial chromosomes, and methods for delivery of plant chromosomes to selected cells and tissues are provided. In particular, plant artificial chromosomes that are substantially composed of repeated nucleic acid units of varying amounts of heterochromatin and euchromatin are provided. Also provided are methods of using plant and animal artificial chromosomes in the production of valuable transgenic plants. Methods for identifying plant genes encoding particular traits using artificial chromosomes and for producing an acrocentric plant chromosome are also provided.

## Description

### RELATED APPLICATIONS

Benefit of priority is claimed to U.S. Provisional Application No. 60/294,687, filed May 30, 2001, by CARL PEREZ AND STEVEN FABIJANSKl entitled *PLANT ARTlFlCIAL CHROMOSOMES, USES THEREOF AND METHODS FOR PREPARING PLANT ARTIFICIAL CHROMOSOMES* and to U.S. Provisional Application No. 60/296,329, filed June 4, 2001, by CARL PEREZ AND STEVEN FABIJANSKI entitled *PLANT ARTIFlClAL CHROMOSOMES, USES THEREOF AND METHODS FOR PREPARING PLANT ARTIFICIAL CHROMOSOMES.* This application is related to U.S. Provisional Application No. 60/294,758, filed May 30, 2001, by EDWARD PERKINS et al.*.* entitled *CHROMOSOME-BASED PLATFORMS* and to U.S. Provisional Application No. 60/366,891, filed March 21, 2002, by by EDWARD PERKINS et al.. entitled *CHROMOSOME-BASED PLATFORMS.* This application is also related to U.S. Provisional Application Attorney Docket No. 24601-420, filed May 30, 2002, by EDWARD PERKINS *et al..* entitled *CHROMOSOME-BASED PLATFORMS* and to PCT International Patent Application Attorney Docket No. 24601-420PC, filed May 30, 2002, by EDWARD PERKINS *et al..* entitled *CHROMOSOME-BASED PLATFORMS.* This application is related to U.S. application Serial No. 08/695,191, filed August 7, 1996 by GYULA HADLACZKY and ALADAR SZALAY, entitled *ARTIFlClAL CHROMOSOMES, USES THEREOF AND METHODS FOR PREPARING ARTIFICIAL CHROMOSOMES,* now U.S. Patent No. 6,025,155. This application is also related to U.S. application Serial No. 08/682,080, filed July 15, 1996 by GYULA HADLACZKY and ALADAR SZALAY, entitled *ARTlFlCIAL CHROMOSOMES, USES THEREOF AND METHODS FOR PREPARING ARTIFICIAL CHROMOSOMES,* now U.S. Patent No. 6,077,697. This application is also related U.S. application Serial No. 08/629,822, filed April 10, 1996 by GYULA HADLACZKY and ALADAR SZALAY, entitled *ARTIFICIAL CHROMOSOMES, USES THEREOF AND METHODS FOR PREPARING ARTIFICIAL CHROMOSOMES* (now abandoned), and is also related to copending U.S. application Serial No. 09/096,648, filed June 12, 1998, by GYULA HADLACZKY and ALADAR SZALAY, entitled *ARTlFlCIAL CHROMOSOMES, USES THEREOF AND METHODS FOR PREPARING ARTIFlClAL CHROMOSOMES* and to U.S. application Serial No. 09/835,682, April 10, 1997 by GYULA HADLACZKY and ALADAR SZALAY, entitled *ARTIFICIAL CHROMOSOMES, USES THEREOF AND METHODS FOR PREPARING ARTIFICIAL CHROMOSOMES* (now abandoned). This application is also related to copending U.S. application Serial No. 09/724,726, filed November 28, 2000, U.S. application Serial No. 09/724,872, filed November 28, 2000, U.S. application Serial No. 09/724,693, filed November 28, 2000, U.S. application Serial No. 09/799,462, filed March 5, 2001, U.S. application Serial No. 09/836,911, filed April 17, 2001, and U.S. application Serial No. 10/125,767, filed April 17, 2002, each of which is by GYULA HADLACZKY and ALADAR SZALAY, and is entitled *ARTIFICIAL CHROMOSOMES, USES THEREOF AND METHODS FOR PREPARING ARTIFICIAL CHROMOSOMES.* This application is also related to International PCT application No. WO 97/40183. Where permitted the subject matter of each of these applications is incorporated by reference in its entirety.

### FIELD OF THE INVENTION

Artificial chromosomes and methods of producing artificial chromosomes, particularly for use in delivery of nucleic acids and expression thereof in plants are provided. Also provided are methods of use of artificial chromosomes in the delivery of nucleic acids to host cells, including plant cells, and the expression of the nucleic acids therein. The resulting plant cells, tissues, organs and whole plants containing the artificial chromosomes, plant cell-based methods for production of heterologous proteins and methods of producing transgenic organisms, particularly plants, using the artificial chromosomes are provided.

### BACKGROUND OF THE INVENTION

The stable transfer of nucleic acids into plant cells and the expression of the nucleic acids therein poses many challenges. Many efforts at the stable introduction of nucleic acids into plant cells have utilized *Agrobacterium*-mediated transformation. *Agrobacterium* is a free-living Gram-negative soil bacterium. Virulent strains of this bacterium are able to infect plant tissue and induce the production of a neoplastic growth commonly referred to as a crowngall. Virulent strains of *Agrobacterium* contain a large plasmid DNA known as a Ti-plasmid that contains genes required for DNA transfer *(vir* genes) and replication as well as a region of DNA that is transferred to plant cells called T-DNA. The T-DNA region is bordered by T-DNA border sequences that are crucial to the DNA transfer process. These T-DNA border sequences are recognized by the *vir* genes encoded on the Ti-plasmid and the vir genes are responsible for the DNA transfer process.

Most wild-type *Agrobacterium* have a relatively broad dicot plant host range and are capable of transferring T-DNA regions up to 25 kilobases of DNA (e.g., nopaline strains) or more (e.g., octopine strains). Accordingly, numerous methods of using *Agrobacterium* to transfer DNA into plant cells have been developed based on the engineering of the Ti-plasmid to no longer contain the genes responsible for altered morphology and replacing these genes with a recombinant gene encoding a trait of interest. There are two primary types of *Agrobacterium-based* plant transformation systems, binary [see, e.g., U.S. Patent No. 4,940,838] and co-integrate [see, e.g., Fraley et a/. (1985) Biotechnology 3:629-635] methods. The T-DNA border repeats are maintained in both systems and the natural DNA transfer process is used to transfer the portion of DNA located between the T-DNA borders into the plant cell.

Another plant cell transformation system, termed biolistics, involves the bombardment of plant cells with microscopic particles coated with DNA encoding a new trait. The particles are rapidly accelerated, typically by gas or electrical discharge, through the cell wall and membranes, whereby the DNA is released into the cell and is incorporated into the genome of the cell. This method is used for transformation of many crops, including corn, wheat, barley, rice, woody tree species and others.

A significant number of crop species of commercial interest have been transformed using either *Agrobacterium-*mediated or biolistic systems. However, these methods have many limitations that limit their utility. For example, there are limits to the size of the heterologous DNA that can be transferred using these methods; typically, only one to two genes may be transferred. Thus, although these methods may have utility in producing crop products modified to contain a single new trait, such as insect or herbicide tolerance, they may not be sufficient to transfer DNA that will provide for multiple traits, or very large DNA segments encoding a multiplicity of traits.

In addition, the genetically modified plant cells produced by these methods tend to contain the transferred DNA in euchromatic regions of the genomic DNA. Typically, a large number of independent transgenic insertion events must be screened before a suitable event (such as insertion of a gene into the host genomic DNA such that it provides a sufficient level of gene expression within temporal and spatial expectations and without evidence of gene rearrangement) is identified.

Another limitation of these methods is the effort required to utilize them in the genetic modification of many commercially important crops. For example, transformation efficiency can vary with the crop and can be low, notably in cereal crops such as corn and wheat. Often the inserted genes are rearranged and unstable over generations.

Furthermore *Agrobacterium tumefaciens* relies on host-parasite interaction in order to be successful. This has the effect that *Agrobacterium* has a preference for some dicots, while other dicots, monocots and conifers are resistant to transformation via *Agrobacterium.* Self-replicating vectors have also been used in the transfer of nucleic acids into plant cells. Such episomal vectors contain DNA sequences that are required for DNA replication and sustainability of the vector in a living cell. In higher plants, very few episomal vectors have been developed. These episomal vectors have the drawback of having a very limited capacity for carrying genetic information and are unstable. One example of an episomal plant vector is the Cauliflower Mosaic Virus [Brisson et al. (1984) Nature 310:511].

Limitations of these gene delivery technologies necessitate the development of alternative vector systems suitable for transferring large (up to Mb size or larger) genes, gene complexes, and multiple genes together with regulatory elements for safe, controlled, and persistent expression of the desired genetic material in higher organisms, particularly plants, without rearrangement caused by insertion or mutagenesis. Therefore, it is an object herein to provide artificial chromosomes for the introduction of large nucleic acids into eukaryotic cells and methods using the artificial chromosomes, particularly for the introduction and expression of nucleic acids in plants.

### SUMMARY OF THE INVENTION

Provided herein are plant artificial chromosomes and methods for producing plant artificial chromosomes. The artificial chromosomes are fully functional stable chromosomes. Plant artificial chromosomes provided herein have a particular composition that makes them ideal vectors for stable, controlled, high-level expression of heterologous nucleic acids in plant cells. The artificial chromosomes are capable of independent, extra-genomic maintenance, replication and segregation within cells and can carry multiple, large heterologous genes.

Artificial plant chromosomes provided herein are non-natural chromosomes that exhibit an ordered segmentation that distinguishes them from naturally occurring chromosomes. The segmented appearance can be visualized using a variety of chromosome analysis techniques and correlates with the unique structure of these artificial chromosomes, which, in particular methods of producing these chromosomes, can arise through amplification of chromosomal segments (i.e., amplification-based artificial chromosomes). The artificial chromosomes, throughout the region or regions of segmentation, are predominantly made up of one or more nucleic acid units that is (are) repeated in the region (referred to as the repeat region) and that have a similar gross structure. Repeats of a nucleic acid unit tend to be of similar size and share some common nucleic acid sequences, for example, a replication site involved in amplification of chromosome segments and/or some heterologous nucleic acid. Although the size of a repeating nucleic acid unit can vary, typically they tend to be greater than about 100 kb, greater than about 500 kb, greater than about 1 Mb, greater than about 5 Mb or greater than about 10 Mb. Typically, repeats of a nucleic acid unit are substantially similar in nucleic acid composition and can be nearly identical. The common nucleic acid sequences can contain sequences that represent euchromatic and heterochromatic nucleic acid. The composition of the amplification-based artificial chromosomes can be such that substantially the entire chromosome exhibits a segmented appearance or such that only one or more portions that make-up less than the entire chromosome appear segmented.

The composition of the plant artificial chromosomes provided herein can vary. For example, in some of the artificial chromosomes provided herein, the repeat region or regions can be made up predominantly of heterochromatic DNA (i.e., the repeat region or regions contain more heterochromatic DNA than other types of DNA, e.g., euchromatic DNA). In other artificial chromosomes provided herein, the repeat region or regions can be made up predominantly of euchromatic DNA (i.e., the repeat region or regions contain more euchromatic DNA than other types of DNA, *e.g.,* heterochromatic DNA) or can be made up of substantially equivalent amounts of heterochromatic and euchromatic DNA, e.g., about 40% to about 50% of one type of nucleic acid and about 50% to about 60% of the other type of nucleic acid. The repeat region or regions thus can be entirely heterochromatic (while still containing one or more heterologous genes), or can contain increasing amounts of euchromatic DNA, such that, for example, the region contains about 10%, 20%, 30%, 40%, 50%, 60%, 70%, 80%, 90% or greater than 90% euchromatic DNA. Common nucleic acid sequences within repeated nucleic acid units in a repeat region can contain DNA that represents euchromatic nucleic acid and DNA that represents heterochromatic nucleic acid. Because the entire artificial chromosome can be made up predominantly of a repeat region or regions (e.g., the composition of the chromosome is such that the repeat region or regions make up greater than about 50% or greater than about 60% of the chromosome), it is thus possible for the artificial chromosome to be made up predominantly of heterochromatin or euchromatin, or to be made up of substantially equivalent amounts of heterochromatin and euchromatin, e.g., about 40% to about 50% of one type of nucleic acid and about 50% to about 60% of the other type of nucleic acid. Plant artificial chromosomes provided herein can be isolated or contained within cells or vesicles.

Also provided herein are cells containing plant artificial chromosomes as described herein, including plant cells and animal cells. Included among the cells containing the plant artificial chromosomes are any cells that include one or more plant chromosomes. Included, for example, are plant cells, including plant protoplasts, in culture and within plant tissues, organs, seeds, pollen or whole plants. Plant cells containing the plant artificial chromosomes can be from any type of plant, including monocots and dicots. For example, the plant cells can be from *Arabidopsis, Nicotiana, Solanum, Lycopersicon, Daucus, Hordeum, Zea mays, Brassica, Triticum, Helianthus, Oryza,* Glycine (soybean), gossypium (cotton). Also contemplated are mammalian and other animal cells that contain plant ACs

Plant cells containing artificial chromosomes of any species are also provided herein. Thus, for example, such plant cells can contain an artificial chromosome containing an animal, *e.g.,* mammalian, centromere or an insect or avian centromere. Included among the artificial chromosomes contained within plant cells as provided herein are predominantly heterochromatic [formerly referred to as satellite artificial chromosomes (SATACs); see, *e.g.,* U.S. Patent Nos. 6,077,697 and 6,025,155 and published International PCT application No. WO 97/40183], minichromosomes which contain a *de novo* centromere, artificial chromosomes containing one or more regions of repeating nucleic acid units wherein the repeat region(s) contain substantially equivalent amounts of euchromatic and heterochromatic nucleic acid and *in vitro* assembled artificial chromosomes, each from any species. An exemplary artificial chromosome is a mammalian satellite artificial chromosome containing a mouse centromere. Included among the plant cells containing artificial chromosomes of any species are plant cells, including plant protoplasts, in culture and within plant tissues, organs, seeds, pollen or whole plants. Plant cells containing the artificial chromosomes can be from any type of plant, including monocots and dicots. For example, the plant cells can be from *Arabidopsis, Nicotiana, Solanum, L ycopersicon, Daucus, Hordeum, Zea mays, Brassica, Triticum, Helianthus* and *Oryza.*

Further provided herein are methods of producing plant artificial chromosomes. One embodiment of these methods includes the steps of introducing nucleic acid into a cell containing plant chromosomes and selecting a cell containing an artificial chromosome that contains one or more repeat regions in which one or more nucleic acid units is (are) repeated. The repeats of a nucleic acid unit in a repeat region can contain common nucleic acid sequences and can be substantially identical. In some embodiments of this method, the repeat region(s) of the artificial chromosome contain substantially equivalent amounts of euchromatic and heterochromatic nucleic acid. The artificial chromosome can be predominantly made up of one or more repeat regions. In further embodiments of this method, the artificial chromosome is made up of substantially equivalent amounts of euchromatic and heterochromatic nucleic acid. In further embodiments of this method, the repeats of a nucleic acid unit have common nucleic acid sequences which contain sequences that represent euchromatic and heterochromatic nucleic acid.

Any cell containing plant chromosomes can be used in these embodiments of methods of producing plant artificial chromosomes described herein. For example, the cell can be any cell that contains chromosomes from *Arabidopsis,* tobacco, *Solanum, Lycopersicon, Daucus, Hordeum, Zea mays, Brassica, Triticum, Oryza, Capsicum,* lentil and/or *Helianthus,* including cells or protoplasts of *Arabidopsis,* tobacco and/or *Helianthus.*

The nucleic acid that is introduced into a cell containing plant chromosomes in methods of producing a plant artificial chromosome as provided herein can be any nucleic acid, including, but not limited to, satellite DNA, rDNA and lambda phage DNA. Satellite DNA and rDNA includes such DNA from plants, such as, for example, *Arabidopsis, Nicotiana, Solanum, Lycopersicon, Daucus, Hordeum, Zea mays, Brassica, Triticum* and *Oryza,* and from animals, such as mammals. The rDNA can contain sequences of an intergenic spacer region, such as can be obtained, for example, from DNA of *Arabidopsis, Solanum, Lycopersicon, Hordeum, Zea, Oryza,* rye, wheat, radish and mung bean. In some embodiments of the method, the nucleic acid contains a nucleic acid sequence that facilitates amplification of a region of a plant chromosome or targets it to an amplifiable region of a plant chromosome.

In further embodiments of methods of producing plant artificial chromosomes provided herein, the nucleic acid that is introduced into a cell containing one or more plant chromosomes includes nucleic acid that for identification of cells containing the nucleic acid. Such nucleic acids include nucleic acid encoding a fluorescent protein, such as a green, blue or red fluorescent protein, and nucleic acid encoding a selectable marker, such as, for example, proteins that confer resistance to phosphinothricin, ammonium glufosinate, glyphosate, kanamycin, hydromycin, dihydrofolate or sulfonylurea.

In embodiments of methods of producing plant artificial chromosomes in which nucleic acid is introduced into a cell containing one or more plant chromosomes, the cell can be cultured through two or more cell doublings, and typically from about 5 to about 60, or about 5 to about 55, or about 10 to about 55, or about 25 to about 55, or about 35 to about 55 cell doublings following introduction of nucleic acid into a cell. The step of selecting a cell containing a plant artificial chromosome can include sorting of cells into which nucleic acid was introduced. For example, cells can be sorted on the basis of the presence of a selectable marker, such as a reporter protein, or by growing (culturing) the cells under selective conditions. The selection step can include fluorescent *in situ* hybridization (FISH) analysis of cells into which nucleic acid is introduced.

Also provided are methods of producing a transgenic plant using artificial chromosomes that function in plants and transgenic plants containing artificial chromosomes. Artificial chromosomes used in the methods of producing transgenic plants can be of any species. For example, the artificial chromosomes can contain a centromere from species such as animals, e.g., mammals, birds, plants, or insects, that functions to segregate nucleic acids to daughter cells through cell division. In some embodiments of the methods for producing a transgenic plant, the artificial chromosomes contain repeat regions predominantly made up of repeats of one or more nucleic acid units. Repeats of a nucleic acid unit can share some common nucleic acid sequences, for example, a replication site involved in amplification of chromosome segments and/or some heterologous nucleic acid. Repeats of a nucleic acid unit can be substantially identical. Common nucleic acid sequences of repeats of a nucleic acid unit can contain sequences that represent euchromatic and heterochromatic nucleic acid.

Repeat regions of artificial chromosomes that can be used in the methods of producing a transgenic plant can be made up of substantially equivalent amounts of heterochromatic and euchromatic DNA or can be made up predominantly of heterochromatic DNA or can be made up predominantly of euchromatic DNA. The artificial chromosome can be made up predominantly of heterochromatic or euchromatic DNA or can be made up of substantially equivalent amounts of heterochromatin and euchromatin. Such artificial chromosomes that contain plant centromeres can contain a plant centromere from any species of plant, including monocots and dicots. For example, the centromere can be from *Arabidopsis,* tobacco, *Helianthus, Solanum, Lycopersicon, Daucus, Hordeum, Zea, Brassica, Triticum,* rye, wheat, radish, mung bean or O*ryza.* The artificial chromosomes can be made using methods described herein.

In a method of producing a transgenic plant provided herein, an artificial chromosome, such as those described above and elsewhere herein, is introduced into a plant cell. The artificial chromosome can contain heterologous nucleic acid encoding a gene product such as, for example, an enzyme, antisense RNA, tRNA, rDNA, a structural protein, a marker or reporter protein, a ligand, a receptor, a ribozyme, a therapeutic protein, a biopharmaceutical protein, a vaccine, a blood factor, an antigen, a hormone, a cytokine, a growth factor or an antibody. The product can be one that provides for resistance to diseases, insects, herbicides or stress in the plant. The product can be one that provides for an agronomically important trait in the plant and/or that alters the nutrient utilization and/or improves the nutrient quality of the plant. Heterologous nucleic acid of an artificial chromosome can be contained within a bacterial artificial chromosome (BAC) or a yeast artificial chromosome (YAC).

The plant cell into which such artificial chromosomes can be introduced in methods of producing a transgenic plant provided herein can be any species of plant cell, including, but not limited to, *Arabidopsis,* tobacco, *Helianthus, Solanum, Lycopersicon, Daucus, Hordeum, Zea, Brassica Triticum,* rye, wheat, radish, mung bean, *Capsicum,* lentil and *Oryza.* Any cell that can develop into a plant can be used, including plant cells and protoplasts of plant embryos, calli, tissues, meristem, organs, seeds, seedlings, pollen, pollen tubes or whole plants.

Artificial chromosomes can be introduced into plant cells in the methods of producing a transgenic plant using any process for transfer of nucleic acids into plant cells, including, but not limited to chemical, physical and electrical processes and combinations thereof. For example, the artificial chromosomes can be transferred into plant cells via direct contact in the absence or presence of a fusogen, *e.g.,* polyethylene glycol (PEG), calcium phosphate and/or lipid or they can be encapsulated in a lipid structure *(e.g.,* a liposome) or contained within a protoplast or microcell which is then allowed to fuse (in the presence or absence of a fusogen such as PEG) with a plant cell for introduction of the artificial chromosome into the cell in a method of producing a transgenic plant. Artificial chromosomes can be transferred to plant cells that are subjected to electrical pulses *(e.g.,* electroporation) and/or ultrasound *(e.g.,* sonoporation) before, during and/or after exposure of the cells to the artificial chromosomes. Use of electrical pulses and/or ultrasound can be in combination with any other agents, *e.g.,* PEG and/or lipids, used in transferring nucleic acids into plant cells. Artificial chromosomes can also be physically injected into plant cells through a micropipette or needle or introduced into plant cells through bombardment of the cells with microprojectiles coated with the chromosomes. To facilitate transfer of nucleic acids into plant cells, the recipient cells or tissue can be subjected to mechanical wounding.

Plant cells into which artificial chromosomes have been introduced for purposes of producing a transgenic plant are cultured under conditions that permit generation of a whole plant therefrom. The transformed cells can be analyzed prior to use in the generation of whole plants to determine suitability. For example, the cells can be analyzed for the presence of artificial chromosomes and/or regenerative capacity. Plant regeneration techniques, many of which are known to those of skill in the art, can be used to generate whole plants from, for example, cells, embryos and calli containing artificial chromosomes. For example, plants can be regenerated from cells containing artificial chromosomes by the planting of transformed roots, plantlets, seed, seedlings, and any structure capable of growing into a whole plant.

Further provided herein are methods for producing an acrocentric plant chromosome and methods for producing plant chromosomes containing adjacent regions of rDNA and heterochromatin, in particular, pericentric and/or satellite heterochromatin. Also provided herein are methods for generating acrocentric plant chromosomes containing adjacent regions of heterochromatin, such as pericentric heterochromatin and/or satellite DNA, and rDNA on the short arm of the chromosome.

One embodiment of these methods includes steps of introducing nucleic acid containing two site-specific recombination sites into a cell containing one or more plant chromosomes, recombining nucleic acids of the two site-specific recombination sites, and selecting a cell containing an acrocentric plant chromosome and/or a plant chromosome containing adjacent regions of rDNA and heterochromatin. The two site-specific recombination sites can be contained on separate nucleic acid fragments which are introduced into the cell simultaneously or sequentially.

Other embodiments of the methods of producing an acrocentric plant chromosome and/or a plant chromosome that contains adjacent regions of rDNA and heterochromatin include steps of introducing a first nucleic acid containing a site-specific recombination site into a first plant chromosome, introducing a second nucleic acid containing a site-specific recombination site into a second plant chromosome, recombining nucleic acids of the first and second chromosomes and selecting a plant chromosome that is acrocentric or that contains adjacent regions of rDNA and heterochromatin. For example, to produce an acrocentric plant chromosome, the first nucleic acid can be introduced into or adjacent to the pericentric heterochromatin of the first chromosome and/or the second nucleic acid can be introduced into the distal end of the arm of the second chromosome. To produce an acrocentric plant chromosome containing adjacent regions of rDNA and heterochromatin, for example, the first nucleic acid can be introduced into or adjacent the pericentric heterochromatin on the short arm of an acrocentric plant chromosome and the second nucleic acid can be introduced into or adjacent to rDNA. To produce a plant chromosome containing adjacent regions of rDNA and heterochromatin, for example, the first nucleic acid can be introduced into or adjacent to heterochromatin, such as pericentric heterochromatin or satellite DNA, and the second nucleic acid can be introduced into or adjacent to rDNA. When the chromosomes are located within a cell, the method can include selecting a cell containing a plant chromosome that is acrocentric and/or that contains adjacent regions of rDNA and heterochromatin.

Another embodiment of the methods of producing an acrocentric plant chromosome includes steps of introducing a first nucleic acid containing a site-specific recombination site into the pericentric heterochromatin of a plant chromosome, introducing a second nucleic acid containing a site-specific recombination site into the distal end of the chromosome in which the first and second recombination sites are located on the same arm of the chromosome, recombining nucleic acids of the first and second recombination sites in the chromosome and selecting a plant chromosome that is acrocentric.

Another method of producing an acrocentric plant chromosome or a plant chromosome containing adjacent regions of rDNA and heterochromatin includes steps of introducing nucleic acid containing a recombination site adjacent to or sufficiently near nucleic acid encoding a selectable marker into a first plant cell for recombination and introduction of the marker into the chromosome, generating a first transgenic plant from the first plant cell, introducing nucleic acid containing a promoter functional in a plant cell and a recombination site in operative linkage into a second plant cell, generating a second transgenic plant from the second plant cell, crossing the first and second plants, obtaining plants resistant to an agent that selects for cells containing the nucleic acid encoding the selectable marker, and selecting a resistant plant that contains cells containing an acrocentric plant chromosome or a plant chromosome containing adjacent regions of rDNA and heterochromatin. Methods of this embodiment can optionally include steps of selecting first and second transgenic plants such that one of the plants contains a chromosome containing a recombination site in a region within or adjacent to the pericentric heterochromatin and the other plant contains a chromosome containing a recombination site located within or adjacent to rDNA of the chromosome. These methods can further include the steps of selecting first and second transgenic plants where one of the plants contains a chromosome containing a recombination site located on a short arm of the chromosome in a region adjacent to the pericentric heterochromatin; and the other plant contains a chromosome containing a recombination site located in rDNA of the chromosome. In one embodiment, the recombination sites on the two chromosomes are in the same orientation.

In methods of producing an acrocentric plant chromosome, one or both of these recombination sites is located on a short arm of the chromosome. For example, one of the one of the plants contains a chromosome containing a recombination site in region within or adjacent to the pericentric heterochromatin located on the short arm of the chromosome. The selecting steps can further include selecting first and second transgenic plants such that the recombination sites on the two chromosomes are in the same orientation.

In any of these methods of producing an acrocentric plant chromosome or a plant chromosome containing adjacent regions of rDNA and heterochromatin (in particular, pericentric heterochromatin and/or satellite DNA), recombination between the first and second site-specific recombination sites can be provided for in a number of ways. For example, a recombinase activity can be introduced into a cell containing one or more chromosomes containing the sites which catalyzes the recombination reaction. The recombinase activity can be encoded by nucleic acid that is introduced into the cell simultaneously with nucleic acid containing a site-specific recombination site or that is introduced into the cell at a different time. Recombinase activity occurs within the cell upon expression of the nucleic acid encoding a recombinase activity, which can be operatively linked to a promoter functional in the cell. The recombinase activity can be constitutively expressed or can be induced, for example, by linking the nucleic acid encoding the recombinase to an inducible promoter. It is also possible that a cell into which nucleic acid containing site-specific recombination sites is introduced contains a recombinase enzyme which can be constitutively or inducibly expressed. Alternatively, a transgenic plant can be generated from cells containing the recombination sites and crossed with a transgenic plant containing nucleic acid encoding a recombinase.

Any site-specific recombinase system known to those of skill in the art is contemplated for use herein. It is contemplated that one or a plurality of sites that direct the recombination by the recombinase are introduced into the ACes (or other ACs) and then heterologous genes linked to the cognate site are introduced into an ACes to produce platform ACes. The resulting ACes are introduced into cells with nucleic acid encoding the cognate recombinase, typically on a vector, and nucleic acid encoding heterologous nucleic acid of interest linked to the appropriate recombination site for insertion into the ACes chromosome. The recombinase encoding nucleic acid may be introduced into the AC, includes ACes or on the same or a difference vector from the heterologous nucleic acid.

For the methods herein any recombinase enzyme that catalyzes site-specific recombination can be used to facilitate recombination between the first and second site-specific recombination sites. A variety of recombinases and attachment/recombination sites therefor are available and/or known to those of skill in the art. These include, but not limited to: the Cre//ox recombination system using CRE recombinase from the Escherichia coli phage P1 , the FLP/FRT system of yeast using the FLP recombinase from the 2*µ* episome of Saccharomyces cerevisiae, the resolvases, including Gin recombinase of phage Mu, Cin, Hin, *αδ* Tn3; the Pin recombinase of *E. coli,* the R/RS system of the pSR1 plasmid of Zygosaccharomyces rouxii site specific recombinases from Kluyveromyces drosophilarium and Kluyveromyces waltii and other systems are Also contempalted is the *E. coli* phage lambda integrase system, the phage lambda integrase and the cognate *att* sites (see, also copending application U.S. application Serial No. (attorney docket No. 24601-420, filed on the same day herewith)).

In any of these methods of producing acrocentric plant chromosomes, nucleic acid containing a site-specific recombination site can also contain nucleic acid encoding a selectable marker. The nucleic acids used in the methods can be designed such that expression of the selectable marker occurs only upon the desired recombination event.

Acrocentric plant chromosomes produced by the methods provided herein can be of any composition. For example the DNA of the short arm of the acrocentric chromosome can contain less than 5% or less than 1 % euchromatic DNA or can contain no euchromatic DNA. Acrocentric plant artificial chromosomes in which the short arm of the acrocentric chromosome does not contain euchromatic DNA are provided.

In another embodiment a method of producing a plant artificial chromosome, that includes the steps of introducing nucleic acid into a plant cell acrocentric chromosome in which the short arm does not contain euchromatic DNA; culturing the cell through at least one cell division; and selecting a cell containing an artificial chromosome, such as one that is predominantly heterochromatic, is provided. The acrocentric chromosome is produced by the method of any the methods described herein or other suitable methods.

In another embodiment, a method for producing an artificial chromosome, that includes the steps of introducing nucleic acid into a plant cell; and selecting a plant cell that includes an artificial chromosome that contains one or more repeat regions is provided. In this AC, one or more nucleic acid units is (are) repeated in a repeat region; repeats of a nucleic acid unit have common nucleic acid sequences; and the common sequences of nucleotides include sequences that represent euchromatic and heterochromatic nucleic acid. The nucleic acid can include plant rDNA from a dicot plant species or plant rDNA from a monocot plant species. The intergenic spacer region can be from DNA from a *Nicotiana* plant or other suitable source of such DNA. The rDNA can be plant rDNA, and the plant can be a dicot or a monocot.

Also provided are isolated plant artificial chromosomes that contain one or more repeat regions. In these ACs one or more nucleic acid units is (are) repeated in a repeat region; repeats of a nucleic acid unit have common nucleic acid sequences; and the common sequences of nucleotides include sequences that represent euchromatic and heterochromatic nucleic acid. The artificial chromosome can be produced by a method that includes the steps of: introducing nucleic acid into a plant cell; and selecting a plant cell containing an artificial chromosome that contains one or more repeat regions. The repeats of a nucleic acid unit have common nucleic acid sequences; and the common nucleic acid sequences contain sequences that represent euchromatic and heterochromatic nucleic acid.

In another embodiment, another method for producing an acrocentric plant chromosome is provided. The method includes the steps of: introducing nucleic acid containing two site-specific recombination sites into a cell containing one or more plant chromosomes; introducing into the cell a recombinase activity that catalyzes recombination between the two recombination sites to produce a plant acrocentric chromosome. In the embodiment, the two site-specific recombination sites can be on separate nucleic acid fragments, which optionally can be introduced into the cell simultaneously or sequentially. The resulting artificial chromosome can be one that is predominantly heterochromatic.

In another embodiment, a method of producing a plant artificial chromosome is provided. The method includes the steps of: introducing nucleic acid into a plant chromosome, such as but not limited to, an acrocentric chromosome, in a cell that contains adjacent regions of rDNA and heterochromatic DNA; culturing the cell through at least one cell division; and selecting a cell containing an artificial chromosome. The resulting artificial chromosome can be predominantly heterochromatic. The acrocentric chromosome can be one where the short arm of the chromosome contains adjacent regions of rDNA and heterochromatic DNA, such as, but not limited to, pericentric heterochromatin.

Also provided are a variety of vectors. Among these are vectors containing nucleic acid encoding a selectable marker that is not operably associated with any promoter, wherein the selectable marker permits growth of animal cells in the presence of an agent normally toxic to the animal cells; and wherein the agent is not toxic to plant cells; a recognition site for recombination; and a sequence of nucleotides that facilitates amplification of a region of a plant chromosome or targets the vector to an amplifiable region of a plant chromosome. Exemplary of such vectors is pAglla and pAgllb.

Another vector provided herein contains nucleic acid encoding a selectable marker that is not operably associated with any promoter, wherein the selectable marker permits growth of animal cells in the presence of an agent normally toxic to the animal cells; and wherein the agent is not toxic to plant cells; a recognition site for recombination; and nucleic acid encoding a protein operably linked to a plant promoter. Exemplary of these vectors is pAg1 and pAg2.

Another vector that is provided contains: nucleic acid encoding a selectable marker that is not operably associated with any promoter, where the selectable marker permits growth of plant cells in the presence of an agent normally toxic to the plant cells but not toxic to animal cells; a recognition site for recombination; and nucleic acid encoding a protein operably linked to a plant promoter.

Another vector is a plant transformation vector that contains nucleic acid encoding a recognition site for recombination; a sequence of nucleotides that facilitates or causes amplification of a region of a plant chromosome; one or more selectable markers that are expressed in plant cells to permit the selection of cells containing the vector, and *Agrobacterium* nucleic acid. The vector is for *Agrobacterium-*mediated transformation of plants.

Another vector that is provided contains a recognition site for recombination; and a sequence of nucleotides that facilitates amplification of a region of a plant chromosome or targets the vector to an amplifiable region of a plant chromosome, wherein the plant is selected from the group consisting of *Arabidopsis, Nicotiana, Solanum, Lycopersicon, Daucus, Hordeum, Zea mays, Brassica, Triticum, Helianthus,* soybean, cotton and *Oryza.*

In these vectors, the amplifiable region can contain heterochromatic nucleic acid; the amplifiable region can contain rDNA. Exemplary sequences of nucleotides that facilitates amplification of a region of a plant chromosome or targets the vector to an amplifiable region of a plant chromosome are any that contain a sufficient portion of an intergenic spacer region of rDNA to facilitate amplification or effect the targeting. Such sufficient portion can be at least 14, 20, 30, 50, 100, 150, 300, 500, 1 kB, 2 kB, 3 kB, 5 kB, 10 kB or more contiguous nucleotides from an intergenic spacer region and/or other rDNA region. An exemplary selectable marker encodes a product confers resistance to zeomycin. The protein in the vectors include a protein that is a selectable marker that permits growth of plant cells in the presence of an agent normally toxic to the plant cells, such as, for example, resistance to hygromycin or to phosphothricin. Other such protein markers include, but are not limited to, fluorescent proteins, such as, for example, green, blue and red fluorescent proteins. An exemplary recognition site contains an *att* site. Exemplary promoters for inclusion in the vectors, include, but are not limited to, nopaline synthase (NOS) or CaMV35S.

Cell, containing any of the vectors or mixtures thereof are provided. The cells include any cells that have at least one plant chromosome, such as a plant cell. The cells can be protoplasts.

Methods using these vectors are provided. The methods includes a step of introducing one of the vectors into a cell, such as a cell that contains at least one plant chromosome. Such vector is for example, a vector that contains nucleic acid encoding a selectable marker that is not operably associated with any promoter, where the selectable marker permits growth of animal cells in the presence of an agent normally toxic to the animal cells but is not toxic to plant cells; a recognition site for recombination; and nucleic acid encoding a protein operably linked to a plant promoter. In this method, the cell contains an animal, such as a mammal, platform ACes that contains a recognition site, such as, for example, an *att* site, that recombines with the recognition site in the vector in the presences of the recombinase therefor, thereby incorporating the selectable marker that is not operably associated with any promoter and the nucleic acid encoding a protein operably linked to a plant promoter into the platform ACes to produce a resulting platform ACes. The platform ACes can contain a promoter that, upon recombination, is operably linked to the selectable marker that in the vector is not operably associated with a promoter. The method can further include transferring the resulting platform ACes into a plant cell to produce a plant cell that contains the platform Aces. The method optionally further includes culturing the plant cell that contains the platform Aces under conditions whereby the protein encoded by the nucleic acid that is operably linked to a plant promoter is expressed.

The resulting platform ACes optionally is isolated prior to transfer. The Aces can be introduced into a plant cell by any suitable method, such as one selected from among protoplast transfection, lipid-mediated delivery, liposomes, electroporation, sonoporation, microinjection, particle bombardment, silicon carbide whisker-mediated transformation, polyethylene glycol (PEG)-mediated DNA uptake, lipofection and lipid-mediated carrier systems. The resulting platform ACes can be transferred by fusion of the cells, which, for example, are plant protoplasts. In another embodiment, the cell can be an animal cell, such as a mammalian, including human, cell.

In another, method a vector is introduced into plant cells. Such vector, for example, can be a vector that includes nucleic acid encoding a selectable marker that is not operably associated with any promoter, where the selectable marker permits growth of animal cells in the presence of an agent normally toxic to the animal cells but is not toxic to plant cells; a recognition site for recombination; and a sequence of nucleotides that facilitates amplification of a region of a plant chromosome or targets the vector to an amplifiable region of a plant chromosome. The plant cells are cultured and a plant cell(s) containing an artificial chromosome that contains one or more repeat regions is selected. In this method, a sufficient portion of the vector can integrates into a chromosome in the plant cell to result in amplification of chromosomal DNA. The resulting selected artificial chromosome can be on in which one or more nucleic acid units is (are) repeated in a repeat region; repeats of a nucleic acid unit have common nucleic acid sequences; and the repeat region(s) contain substantially equivalent amounts of euchromatic and heterochromatic nucleic acid. The resulting artificial chromosome produced in the method optionally can be isolated.

Anther method is also provided. This method includes the steps of introducing a vector into a cell, and culturing the resulting cell under conditions, whereby the protein encoded by nucleic acid operably linked to an animal promoter is expressed. In the method the vector can contains: nucleic acid encoding a selectable marker that is not operably associated with any promoter, where the selectable marker permits growth of animal cells in the presence of an agent normally toxic to the animal cells but is not toxic to plant cells; a recognition site for recombination; and nucleic acid encoding a protein operably linked to an animal promoter. The cell can contain a platform plant artificial chromosome (PAC) that contains a recombination site and an animal promoter that upon recombination is operably linked to the selectable marker that in the vector is not operably associated with a promoter. Introduction can be effected under conditions whereby the vector recombines with the PAC to produce a plant platform PAC that contains the selectable marker operably linked to the promoter. In this method, the artificial chromosome can be an ACes. In addition, the plant platform PAC can be an ACes.

The vectors, such as those that contain nucleic acid encoding a selectable marker that is not operably associated with any promoter, where the selectable marker permits growth of animal cells in the presence of an agent normally toxic to the animal cells but is not toxic to plant cells; a recognition site for recombination; and a sequence of nucleotides that facilitates amplification of a region of a plant chromosome or targets the vector to an amplifiable region of a plant chromosome, and the plant transformation vectors that contain nucleic acid for *Agrobacterium-*mediated transformation of plants, can be used to produce artificial chromosomes. In one exemplary method, such vector is introduced into a cell containing one or more plant chromosomes; and a cell containing an artificial chromosome that contains one or more repeat regions is selected. The artificial chromosome contains one or more nucleic acid units that is (are) repeated in a repeat region; the repeats of a nucleic acid unit have common nucleic acid sequences; and the common nucleic acid sequences contain sequences that represent euchromatic and heterochromatic nucleic acid. In another method, a cell containing an artificial chromosome that contains one or more repeat regions is selected. The artificial chromosome contains one or more nucleic units that is (are) repeated in a repeat region; repeats of a nucleic acid unit have common nucleic acid sequences; and the repeat region(s) contain substantially equivalent amounts of euchromatic and heterochromatic nucleic acid.

### DESCRIPTION OF THE DRAWINGS

Figure 1 provides a map of plasmid pAg1.
Figure 2 provides a schematic representation of the construction of plasmid pAg1.
Figure 3 provides a map of plasmid pAg2.
Figure 4 provides a schematic representation of the construction of plasmid pAg2.
Figure 5 provides a schematic representation of the construction of plasmids pAglla and pAgllb.
Figure 6A-6B provide restriction maps of the DNA inserted into pAg1 to form plasmids pAglla and pAgllb.
Figure 7 provides a map of plasmid pSV40193attPsensePUR.

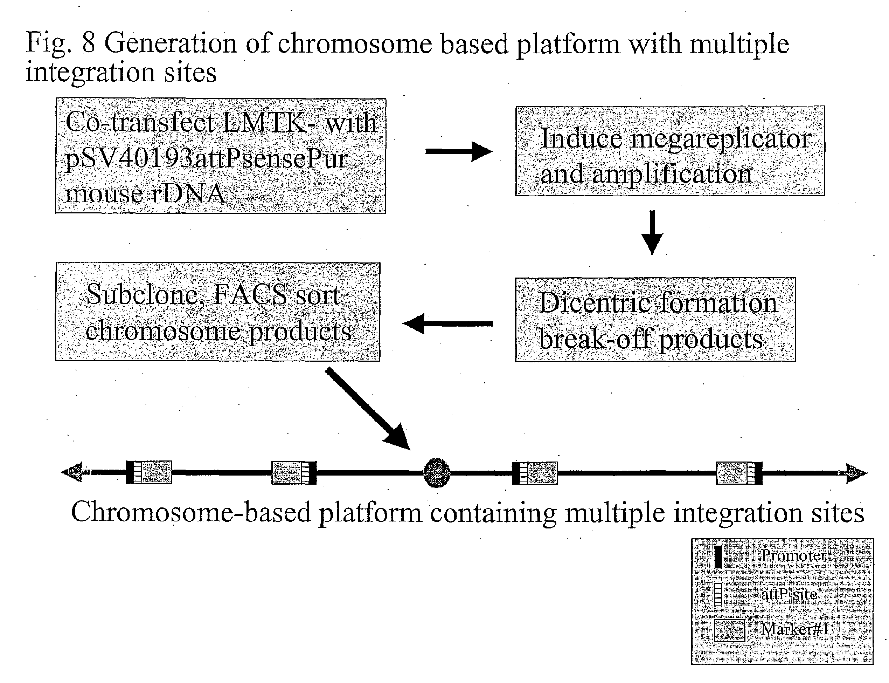

Figure 8 depicts a method for formation of a chromosome platform with multiple recombination integration sites, such as attP sites.
Figure 9 diagrammatically summarizes the platform technology; marker 1 permits selection of the artificial chromosomes containing the integration site; marker 2, which is promoterless in the donor vector permits selection of recombinants. Upon recombination with the platform marker 2 is expressed under the control of a promoter resident on the platform.

### DETAILED DESCRIPTION OF PREFERRED EMBODIMENTS

### Definitions

Unless defined otherwise, all technical and scientific terms used herein have the same meaning as is commonly understood by one of skill in the art to which this invention belongs. All patents, patent applications, published applications and other publications and published nucleotide and amino acid sequences (e.g., sequences available in GenBank or other databases) referred to herein are incorporated by reference in their entirety. Where reference is made to a URL or other such identifier or address, it is understood that such identifiers can change and particular information on the internet can come and go, but equivalent information can be found by searching the internet. Reference thereto evidences the availability and public dissemination of such information.

As used herein, a chromosome is a defined composition of nucleic acid that is capable of replication and segregation within a cell upon cell division. Typically, a chromosome may contain a centromeric region, telomeric regions and a region of nucleic acid between the centromeric and telomeric regions.

As used herein, a centromere is a molecular composition that includes a nucleic acid sequence that confers an ability to segregate to daughter cells through cell division. A centromere may confer stable segregation of a nucleic acid sequence, including an artificial chromosome containing the centromere, through mitotic and/or meiotic divisions. A plant centromere is not necessarily derived from plants, but has the ability to promote DNA segregation in plant cells.

As used herein, euchromatin and heterochromatin have their recognized meanings. Euchromatin refers to chromatin that stains diffusely and that typically contains genes, and heterochromatin refers to chromatin that remains unusually condensed and that has been thought to be transcriptionally inactive or has low transcriptional activity relative to euchromatin. Highly repetitive DNA sequences (satellite DNA) are usually located in regions of the heterochromatin surrounding the centromere (pericentric or pericentromeric heterochromatin). Constitutive heterochromatin refers to heterochromatin that contains the highly repetitive DNA which is constitutively condensed and genetically inactive.

As used herein, an acrocentric chromosome refers to a chromosome with arms of unequal length.

As used herein, endogenous chromosomes refer to genomic chromosomes as found in the cell prior to generation or introduction of an artificial chromosome.

As used herein, artificial chromosomes are nucleic acid molecules, typically DNA, that stably replicate and segregate alongside endogenous chromosomes in cells and have the capacity to accommodate and express heterologous genes contained therein. A mammalian artificial chromosome (MAC) refers to a chromosome that has an active mammalian centromere(s). Plant artificial chromosomes (PAC), insect artificial chromosomes and avian artificial chromosomes refer to chromosomes that include centromeres that function in plant, insect and avian cells, respe ctively. Human artificial chromosomes (HAC) refers to chromosomes that include centromeres that function in human cells. For exemplary artificial chromosomes, see, *e.g.,* U.S. Patent Nos. 6,025,155; 6,077,697; 5,288,625; 5,712,134; 5,695,967; 5,869,294; 5,891,691 and 5,721,118 and published International PCT application Nos, WO 97/40183 and WO 98/08964.

As used herein, amplification, with reference to DNA, is a process in which segments of DNA are duplicated to yield two or multiple copies of substantially similar or identical or nearly identical DNA segments that are typically joined as substantially tandem or successive repeats or inverted repeats.

As used herein, amplification-based artificial chromosomes are artificial chromosomes derived from natural or endogenous chromosomes by virtue of an amplification event, such as one that may be initiated by introduction of heterologous nucleic acid into heterochromatin, for example, pericentric heterochromatin, in a chromosome. As a result of such an event, chromosomes and/or fragments thereof exhibiting segmented or repeating patterns arise. Artificial chromosomes can be formed from these chromosomes and fragments. Hence, amplification-based artificial chromosomes refer to non-natural or isolated chromosomes that exhibit an ordered segmentation that is not typically observed in naturally occurring chromosomes and that can be a basis for distinguishing them from naturally occurring chromosomes. Amplification-based artificial chromosomes can also be distinguished from naturally occurring chromosomes by virtue of their typically smaller size and often segmented appearance when visualized. The segmented appearance, which can be visualized using a variety of chromosome analysis techniques as described herein and known to those of skill in the art, correlates with the unique structure of these artificial chromosomes. In addition to containing one or more centromeres, the amplification-based artificial chromosomes, throughout the region or regions of segmentation, are predominantly made up of one or more nucleic acid units, also referred to as "amplicons", that is (are) repeated in the region and that have a similar gross structure. Thus, a region of segmentation may be referred to as a repeat region. Repeats of an amplicon tend to be of similar size and share some common nucleic acid sequences. For example, each repeat of an amplicon may contain a replication site involved in amplification of chromosome segments and/or some heterologous nucleic acid that was utilized in the initial production of the artificial chromosome. Typically, the repeating units are substantially similar in nucleic acid composition and may be nearly identical. The common nucleic acid sequences may contain sequences that represent euchromatic and heterochromatic nucleic acid. Amplicon sizes vary but typically tend to be greater than about 100 kb, greater than about 500 kb, greater than about 1 Mb, greater than about 5 Mb or greater than about 10 Mb. The composition of the amplification-based artificial chromosomes may be such that substantially the entire chromosome exhibits a segmented appearance or such that only one or more portions that make-up less than the entire chromosome appear segmented. The amplification-based artificial chromosomes can also differ depending on the chromosomal region that has undergone amplification in the process of artificial chromosome formation. The structures of the resulting chromosomes can vary depending upon the initiating event and/or the conditions under which the heterologous nucleic acid is introduced, including modification to the endogenous chromosomes. For example, in some of the artificial chromosomes provided herein, the region or regions of segmentation may be made up predominantly of heterochromatic DNA. In other artificial chromosomes provided herein, the region or regions of segmentation may be made up predominantly of euchromatic DNA or may be made up of similar amounts of heterochromatic and euchromatic DNA. The region or regions of segmentation thus may be entirely heterochromatic (while still containing one or more heterologous nucleic acid sequences), or may contain increasing amounts of euchromatic DNA, such that, for example, the region contains about 10%, 20%, 30%, 40%, 50%, 60%, 70%, 80%, 90% or greater than 90% euchromatic DNA. Because the entire artificial chromosome can be made up predominantly of a region or regions of segmentation, it is thus possible for the artificial chromosome to be made up predominantly of heterochromatin or euchromatin, or to be made up of substantially equivalent amounts of heterochromatin and euchromatin, e.g., about 40% to about 50% of one type of nucleic acid and about 50% to about 60% of the other type of nucleic acid.

As used herein the term "predominantly" with respect to a composition generally refers to a state of the composition in which it can be characterized as being or having more of the predominant feature than other features which are not predominant. The predominant feature may represent more than about 50%, more than about 60%, more than about 70%, more than about 80%, more than about 90%, more than about 95% or essentially 100% of the composition. Thus, for example, a repeat region that is predominantly made up of heterochromatic DNA contains more heterochromatic DNA than other types, *e.g.,* euchromatic, of DNA. The repeat region may be more than about 50%, more than about 60%, more than about 70%, more than about 80%, more than about 90% or more than about 95% heterochromatic DNA or may be essentially 100% heterochromatic DNA. An artificial chromosome predominantly made up of heterochromatin contains more heterochromatic DNA than other types, e.g., euchromatic, of DNA and may be more than about 50%, more than about 60%, more than about 70%, more than about 80%, more than about 90% or more than about 95% heterochromatic DNA or may be essentially 100% heterochromatic DNA.

As used herein an amplicon is a repeated nucleic acid unit. In some of the artificial chromosomes described herein, an amplicon may contain a set of inverted repeats of a megareplicon. A megareplicon represents a higher order replication unit. For example, with reference to some of the predominantly heterochromatic artificial chromosomes, particularly eukaryotic chromosomes, described herein, the megareplicon may contain a set of tandem DNA blocks (e.g., ∼7.5 Mb DNA blocks) each containing satellite DNA flanked by non-satellite DNA or may substantially be made up of rDNA. Contained within the megareplicon is a primary replication site, referred to as the megareplicator, which may be involved in organizing and facilitating replication of segments of chromosomes, including, for example, heterochromatin, pericentric heterochromatin, rDNA and/or possibly the centromeres. Within the megareplicon there may be smaller (e.g., 50-300 kb) secondary replicons. As used herein, amplifiable, when used in reference to a chromosome, particularly the method of generating artificial chromosomes provided herein, refers to a region of a chromosome that is prone to amplification. Amplification typically occurs during replication and other cellular events involving recombination (e.g., DNA repair). Included among such regions are regions of the chromosome that contain tandem repeats, such as satellite DNA, rDNA, and other such sequences.

Among the artificial chromosome systems provided herein are those that are predominantly heterochromatic [formerly referred to as satellite artificial chromosomes (SATACs); see, e.g., U.S. Patent Nos. 6,077,697 and 6,025,155 and published International PCT application No. WO 97/40183], minichromosomes which contain a *de novo* centromere, artificial chromosomes containing one or more regions of repeating nucleic acid units wherein the repeat region(s) contain substantially equivalent amounts of euchromatic and heterochromatic nucleic acid and *in vitro* assembled artificial chromosomes. Of particular interest herein are artificial chromosomes that introduce and express heterologous nucleic acids in plants. These include artificial chromosomes that have a centromere derived from a plant, and, also, artificial chromosomes that have centromeres that may be derived from other organisms but that function in plants. Methods for the construction, isolation, and delivery to target cells of each type of artificial chromosome are provided herein.

As used herein, to target nucleic acid to a locus on a chromosome means that the nucleic acid integrates at or near the targeted locus. Any method or means for effecting such integration, including, but not limited to, homologous recombination, is contemplated.

As used herein, a dicentric chromosome is a chromosome that contains two centromeres. A multicentric chromosome contains more than two centromeres.

As used herein, a formerly dicentric chromosome is a chromosome that is produced when a dicentric chromosome fragments and acquires new telomeres so that two chromosomes, each having one of the centromeres, are produced. Each of the fragments are replicable chromosomes. If one of the chromosomes undergoes amplification of primarily euchromatic DNA to produce a fully functional chromosome that is predominantly (more than about 50%, more than about 70% or more than about 90% euchromatin) euchromatin, it is a minichromosome. The remaining chromosome is a formerly dicentric chromosome. If one of the chromosomes undergoes amplification, whereby heterochromatin (such as, for example, satellite DNA) is amplified and a euchromatic portion (such as, for example, an arm) remains, it is referred to as a sausage chromosome. A chromosome that is substantially all heterochromatin, except for portions of heterologous DNA, is called a predominantly heterochromatic artificial chromosome. Predominantly heterochromatic artificial chromosomes can be produced from other partially heterochromatic artificial chromosomes by culturing the cell containing such chromosomes under conditions that destabilize the chromosome and/or under selective conditions so that a predominantly heterochromatic artificial chromosome is produced. For purposes herein, it is understood that the artificial chromosomes may not necessarily be produced in multiple steps, but may appear after the initial introduction of the heterologous DNA. Typically, artificial chromosomes appear after about 5 to about 60, or about 5 to about 55, or about 10 to about 55 or about 25 to about 55 or about 35 to about 55 cell divisions following introduction of nucleic acid into a cell. Artificial chromosomes may, however, appear after only about 5 to about 15 or about 10 to about 15 cell divisions.

As used herein, the term "satellite DNA-based artificial chromosome (SATAC)" is interchangable with the term "artificial chromosome expression system (ACes)". These artificial chromosomes (ACes) include those that are substantially all neutral non-coding sequences (heterochromatin) except for foreign heterologous, typically gene or protein-encoding, nucleic acid, that may be interspersed within the heterochromatin for the expression therein (see U.S. Patent Nos. 6,025,155 and 6,077,697 and International PCT application No. WO 97/40183), or that is in a single locus as provided herein. The delineating structural feature is the presence of repeating units, which are generally predominantly heterochromatin. The precise structure of the ACes will depend upon the structure of the chromosome in which the initial amplification event occurs; all share the common feature of including a defined pattern of repeating units. Generally ACes have more heterochromatin than euchromatin. Foreign nucleic acid molecules (heterologous genes) contained in these artificial chromosome expression systems can include any nucleic acid whose expression is of interest in a particular host cell.

As used herein, an artificial chromosome that is predominantly heterochromatic *(i.e.,* containing more heterochromatin than euchromatin, typically more than about 50%, more than about 60%, more than about 70%, more than about 80% or more than about 90% heterochromatin) may be produced by introducing nucleic acid molecules into cells, particularly plant cells, and selecting cells that contain a predominantly heterochromatic artificial chromosome. Any nucleic acid may be introduced into cells in the methods of producing the artificial chromosomes. For example, the nucleic acid may contain a selectable marker and/or a sequence that targets nucleic acid to a heterochromatic region of a chromosome, particularly a plant chromosome, such as in the pericentric heterochromatin, in the short arm of acrocentric chromosomes, rDNA or nucleolar organizing regions. Targeting sequences include, but are not limited to, lambda phage DNA and rDNA *(e.g.,* a sequence of an intergenic spacer of rDNA), particularly plant rDNA, for production of predominantly heterochromatic artificial chromosomes in plant cells.

After introducing the nucleic acid into cells, a cell containing a predominantly heterochromatic artificial chromosome is selected. Such cells may be identified using a variety of procedures. For example, repeating units of heterochromatic DNA of these chromosomes may be discerned by G-and/or C-banding and/or fluorescence in situ hybridization (FISH) techniques. Prior to such analyses, the cells to be analyzed may be enriched with artificial chromosome-containing cells by sorting the cells on the basis of the presence of a selectable marker, such as a reporter protein, or by growing (culturing) the cells under selective conditions. Selection of cells containing amplified nucleic acids may also be facilitated by use of techniques such as PCR and Southern blotting to identify cell lines with amplified regions. It is also possible, after introduction of nucleic acids into cells, to select cells that have a multicentric, typically dicentric, chromosome, a formerly multicentric (typically dicentric) chromosome and/or various heterochromatic structures and to treat them such that desired artificial chromosomes are produced. Conditions for generation of a desired structure include, but are not limited to, further growth under selective conditions, introduction of additional nucleic acid molecules and/or growth under selective conditions and treatment with destabilizing agents, and other such methods (see International PCT application No. WO 97/40183 and U.S. Patent Nos. 6,025,155 and 6,077,697).

As used herein, heterologous and foreign are used interchangeably with respect to nucleic acid and refer to any nucleic acid, including DNA and RNA, that does not occur naturally as part of the genome in which it is present or which is found in a location or locations in the genome that differ from that in which it occurs in nature. Thus, heterologous or foreign nucleic acid that is not normally found in the host genome in an identical context. It is nucleic acid that is not endogenous to the cell and has been exogenously introduced into the cell. Examples of heterologous DNA include, but are not limited to, DNA that encodes a gene product or gene product(s) of interest, introduced for purposes of modification of the endogenous genes or for production of an encoded protein. For example, a heterologous or foreign gene may be isolated from a different species than that of the host genome, or alternatively, may be isolated from the host genome but operably linked to one or more regulatory regions which differ from those found in the unaltered, native gene. Other examples of heterologous DNA include, but are not limited to, DNA that encodes traceable marker proteins, and DNA that encodes a protein that confers an input trait including, but not limited to, herbicide, insect, or disease resistance or an output trait, including, but not limited to, oil quality or carbohydrate composition. Antibodies that are encoded by heterologous DNA may be secreted, sequestered, stored in an organ or tissue, accumulate in the cytoplasm or cellular organelles or expressed on the surface of the cell in which the heterologous DNA has been introduced.

As used herein, a "selectable marker" is a composition that can be used to distinguish one cell from another cell. For example, a selectable marker may be a nucleic acid encoding a readily detected protein that has been introduced into some cells but not others. Detection of the expressed protein in cells facilitates identification of cells containing the marker nucleic acid by distinguishing them from cells that do not contain the nucleic acid. Thus, for example, a selectable marker may be a fluorescent protein, such as green fluorescent protein (GFP), or *β*-galactosidase (or a nucleic acid encoding either of these proteins). Selectable markers such as these, which are not required for cell survival and/or proliferation in the presence of a selection agent, may also be referred to as reporter molecules. Other selectable markers, e.g., the neomycin phosphotransferase gene, provide for isolation and identification of cells containing them by conferring properties on the cells that make them resistant to an agent, *e.g.,* a drug such as an antibiotic, that inhibits proliferation of cells that do not contain the marker.

As used herein, growth under selective conditions means growth of a cell under conditions that require expression of a selectable marker for survival.

As used herein, an agent that destabilizes a chromosome is any agent known by those of skill in the art to enhance amplification events, and/or mutations. Such agents, which include BrdU, are well known to those of skill in the art.

In order to generate an artificial chromosome containing a particular heterologous nucleic acid of interest, it is possible to include the nucleic acid of interest in the nucleic acid that is being introduced into cells to initiate production of the artificial chromosome. Thus, for example, a nucleic acid of interest could be introduced into a cell along with nucleic acid encoding a selectable marker and/or a nucleic acid that targets to a heterochromatic region of a chromosome. For example, the nucleic acid of interest can be linked to targeting nucleic acid(s). Alternatively, heterologous nucleic acid of interest can be introduced into an artificial chromosome at a later time after the initial generation of the artificial chromosome.

As used herein, the minichromosome refers to a chromosome derived from a multicentric, typically dicentric, chromosome that contains more euchromatic than heterochromatic DNA. For purposes herein, the minichromosome contains a *de novo* centromere, preferably a centromere that replicates in plants, more preferably a plant centromere.

As used herein, *de novo* with reference to a centromere, refers to generation of an excess centromere in a chromosome as a result of incorporation of a heterologous nucleic acid fragment using the methods herein.

As used herein, *in vitro* assembled artificial chromosomes or synthetic chromosomes are artificial chromosomes produced by joining essential components of a chromosome *in vitro.* These components include at least a centromere, a telomere and an origin of replication. An *in vitro* assembled artificial chromosome may include one or more megareplicators. In particular embodiments, the megareplicator contains sequences of rDNA, particularly plant rDNA.

As used herein, *in vitro* assembled plant artificial chromosomes are produced by joining components (e.g., the centromere, telomere(s) megareplicator and an origin of replication) that function in plants, and preferably, one or more of which is derived from a plant. *ln vitro* assembled artificial chromosomes may contain any amount of heterochromatic and/or euchromatic nucleic acid. For example, an *in vitro* assembled artificial chromosome may be substantially all heterochromatin, or may contain increasing amounts of euchromatic DNA, such that, for example, it contains about 10%, 20%, 30%, 40%, 50%, 60%, 70%, 80%, 90% or greater than about 90% euchromatic DNA. *In vitro* assembled artificial chromosomes may contain one or more regions of segmentation as described with reference to amplification-based artificial chromosomes.

As used herein, an artificial chromosome platform refers to an artificial chromosome that has been engineered to include one or more sites for site specific recombination-directed integration. Included within the artificial chromosome platforms are ACes, particularly plant ACes, that are so-engineered. Any sites, including but not limited to any described herein, that are suitable for such integration are contemplated. Among the ACes contemplated herein are those that are predominantly heterochromatic (formerly referred to as satellite artificial chromosomes (SATACs); see, e.g., U.S. Patent Nos. 6,077,697 and 6,025,155 and published International PCT application No. WO 97/40183), artificial chromosomes predominantly made up of repeating nucleic acid units and that contain substantially equivalent amounts of euchromatic and heterochromatic DNA or wherein the repeat regions of the chromosomes contain substantially equivalent amounts of euchromatic and heterochromatic nucleic acid. Included among the ACes for use in generating platforms are artificial chromosomes that introduce and express heterologous nucleic acids in plants as described herein. These include artificial chromosomes that have a centromere derived from a plant, and, also, artificial chromosomes that have centromeres that may be derived from other organisms but that function in plants.

As used herein, recognition sequences are particular sequences of nucleotides that a protein, DNA, or **R**NA molecule, or combinations thereof, (such as, but not limited to, a restriction endonuclease, a modification methylase and a recombinase) recognizes and binds. For example, a recognition sequence for Cre recombinase (see, e.g., SEQ ID No. 30) is a 34 base pair sequence containing two 13 base pair inverted repeats (serving as the recombinase binding sites) flanking an 8 base pair core and designated IoxP (see, *e.g.,* Sauer (1994) Current Opinion in Biotechnology 5:521-527). Other examples of recognition sequences, include, but are not limited to, attB and attP, a*ttR* and *att*L and others (see, e.g., SEQ ID Nos. 32-48), that are recognized by the recombinase enzyme Integrase (see, SEQ ID Nos. 49 and 50) for the nucleotide and encoded amino acid sequences of an exemplary lambda phage integrase).

The recombination site designated *att*B is an approximately 33 base pair sequence containing two 9 base pair core-type Int binding sites and a 7 base pair overlap region; *att*P (SEQ ID No. 48) is an approximately 240 base pair sequence containing core-type Int binding sites and arm-type Int binding sites as well as sites for auxiliary proteins **I**HF, FIS, and Xis (see, e.g., Landy (1993) Current Opinion in Biotechnology 3:699-7071 see, e.g., SEQ ID Nos. 32 and 48).

As used herein, a recombinase is an enzyme that catalyzes the exchange of DNA segments at specific recombination sites. An integrase herein refers to a recombinase that is a member of the lambda (λ) integrase family.

As used herein, recombination proteins include excisive proteins, integrative proteins, enzymes, co-factors and associated proteins that are involved in recombination reactions using one or more recombination sites (see, Landy (1993) Current Opinion in Biotechnology 3:699-707).

As used herein the expression "lox site" means a sequence of nucleotides at which the gene product of the cre gene, referred to herein as Cre, can catalyze a site-specific recombination event. A LoxP site is a 34 base pair nucleotide sequence from bacteriophage P1 (see, *e.g.,* Hoess et al. (1982) Proc. Natl. Acad Sci. U.S.A. 79:3398-3402). The LoxP site contains two 13 base pair inverted repeats separated by an 8 base pair spacer region as follows: (SEQ ID NO. 51):

### ATAACTTCGTATA ATGTATGC TATACGAAGTTAT

*E*. *coli*DH5ΔLac and yeast strain BSY23 transformed with plasmid pBS44 carrying two loxP sites connected with a LEU2 gene are available from the American Type Culture Collection (ATCC) under accession numbers ATCC 53254 and ATCC 20773, respectively. The lox sites can be isolated from plasmid pBS44 with restriction enzymes *Eco*Rl and Sa*l*l, or *Xh*ol and *Bam*Hl*.* In addition, a preselected DNA segment can be inserted into pBS44 at either the *Sal*l or *Bam*Hl restriction enzyme sites. Other lox sites include, but are not limited to, LoxB, LoxL, LoxC2 and LoxR sites, which are nucleotide sequences isolated from *E. coli* (see, e.g., Hoess et al. (1982) Proc. Natl. Acad. Sci. U.S.A. 79:3398). Lox sites can also be produced by a variety of synthetic techniques (see, ***e**.g.,* Ito et al. (1982) Nuc. Acid Res. 10: 1755 and Ogilvie et al. (1981) Science 270:270).

As used herein, the expression "cre gene" means a sequence of nucleotides that encodes a gene product that effects site-specific recombination of DNA in eukaryotic cells at lox sites. One cre gene can be isolated from bacteriophage P1 (see, e.g., Abremski et al. (1983) Ce// 32:1301-1311). *E. coli* DH1 and yeast strain BSY90 transformed with plasmid pBS39 carrying a cre gene isolated from bacteriophage P1 and a GAL1 regulatory nucleotide sequence are available from the American Type Culture Collection (ATCC) under accession numbers ATCC 53255 and ATCC 20772, respectively. The cre gene can be isolated from plasmid pBS39 with restriction enzymes *Xhol* and *Sal*l*.*

As used herein, site-specific recombination refers to site-specific recombination that is effected between two specific sites on a single nucleic acid molecule or between two different molecules that requires the presence of an exogenous protein, such as an integrase or recombinase.

For example, Cre-lox site-specific recombination can include the following three events:
a. deletion of a pre-selected DNA segment flanked by lox sites;
b. inversion of the nucleotide sequence of a pre-selected DNA segment flanked by lox sites; and
c. reciprocal exchange of DNA segments proximate to lox sites located on different DNA molecules.

This reciprocal exchange of DNA segments can result in an integration event if one or both of the DNA molecules are circular. DNA segment refers to a linear fragment of single- or double-stranded deoxyribonucleic acid (DNA), which can be derived from any source. Since the lox site is an asymmetrical nucleotide sequence, two lox sites on the same DNA molecule can have the same or opposite orientations with respect to each other. Recombination between lox sites in the same orientation results in a deletion of the DNA segment located between the two lox sites and a connection between the resulting ends of the original DNA molecule. The deleted DNA segment forms a circular molecule of DNA. The original DNA molecule and the resulting circular molecule each contain a single lox site. Recombination between lox sites in opposite orientations on the same DNA molecule result in an inversion of the nucleotide sequence of the DNA segment located between the two lox sites. In addition, reciprocal exchange of DNA segments proximate to lox sites located on two different DNA molecules can occur. All of these recombination events are catalyzed by the gene product of the cre gene. Thus, the Cre-lox system can be used to specifically delete, invert, or insert DNA. The precise event is controlled by the orientation of lox DNA sequences, in *cis* the lox sequences direct the *Cre* recombinase to either delete (lox sequences in direct orientation) or invert (lox sequences in inverted orientation) DNA flanked by the sequences, while *in trans* the lox sequences can direct a homologous recombination event resulting in the insertion of a recombinant DNA.

As used herein, a plant refers to an organism that is taxonomically classifed as being in the kingdom Plantae. Such organisms include eukaryotic organisms that contain chloroplasts capable of carrying out photosynthesis. A plant can be unicellular or multicellular and can contain multiple tissues and/or organs. Plants can reproduce sexually and/or asexually and include species that are perennial or annual in growth habit. A plants can be found to exist in a variety of habitats, including terrestrial and aquatic environments. The term "plant" includes a whole plant, plant cell, plant protoplast, plant calli, plant seed, plant organ, plant tissue, and other parts of a whole plant.

As used herein, reproductive mode with reference to a plant refers to any and all methods by which a plant produces progeny. Reproductive modes include, but are not limited to, sexual and asexual reproduction. Plants may produce progeny by one or multiple reproductive modes. Sexual reproduction can include union of cells derived from haploid gametophytes (e.g., eggs produced from ovules and sperm produced from pollen in seed plants) to form diploid zygotes. Zygotes may be formed from gametophytes from different plants or from gametophytes of the same plant (e.g., through self-fertilization). Asexual reproduction can occur when offspring are produced through modifications of the sexual life cycle that do not include meiosis and syngamy. For example, when vascular plants reproduce asexually, they may do so by vegetative reproduction, such as budding, branching, and tillering, or by producing spores or seed genetically identical to the sporophytes that produced them.

As used herein, stable maintenance of chromosomes occurs when at least about 85%, preferably 90%, more preferably 95%, of the cells retain the chromosome. Stability is measured in the presence of a selective agent. Preferably these chromosomes are also maintained in the absence of a selective agent. Stable chromosomes also retain their structure during cell culturing, suffering no unintended intrachromosomal nor interchromosomal rearrangements.

As used herein, BrdU refers to 5-bromodeoxyuridine, which during replication is inserted in place of thymidine. BrdU is used as a mutagen; it also inhibits condensation of metaphase chromosomes during cell division.

As used herein, ribosomal RNA (rRNA) is the specialized RNA that forms part of the structure of a ribosome and participates in the synthesis of proteins. Ribosomal RNA is produced by transcription of genes which, in eukaryotic cells, are present in multiple copies. In human cells, the approximately 250 copies of rRNA genes (i.e., genes which encode rRNA) per haploid genome are spread out in clusters on at least five different chromosomes (chromosomes 13, 14, 15, 21 and 22). In mouse cells, the presence of ribosomal DNA (rDNA, which is DNA containing sequences that encode rRNA) has been verified on at least 11 pairs out of 20 mouse chromosomes (chromosomes 5, 6, 7, 9, 11, 12, 15, 16, 17, 18, and 19) [see e.g., Rowe et al. (1996) Mamm. Genome 7:886-889 and Johnson et al. (1993) Mamm. Genome 4:49-52]. In *Arabidopsis thaliana* the presence of rDNA has been verified on chromosomes 2 and 4 (18S, 5.8S, and 25S rDNA) and on chromosomes 3,4, and 5 (5S rDNA)[see The Arabidopsis Genome Initiative (2000) Nature 408:796-815]. In eukaryotic cells, the multiple copies of the highly conserved rRNA genes are located in a tandemly arranged series of rDNA units, which are generally about 40-45 kb in length and contain a transcribed region and a nontranscribed region known as spacer (i.e., intergenic spacer) DNA which can vary in length and sequence. In the human and mouse, these tandem arrays of rDNA units are located adjacent to the pericentric satellite DNA sequences (heterochromatin). The regions of these chromosomes in which the rDNA is located are referred to as nucleolar organizing regions (NOR) which loop into the nucleolus, the site of ribosome production within the cell nucleus. In higher plants, the rDNA is arragened in long tandem repeating units, similar to those of other higher eukaroytes. The 18S, 5.8S and 25S rRNA genes are clustered and are transcribed as one unit, while the 5S genes are located elsewhere in the genome. Between the 3' end of the 25S gene and the 5' end of the 18S gene is located a DNA spacer that ranges from 1 kb to greater than 12 kb in length for different species. Therefore, the rDNA repeat ranges from about 4 kb to about 15 kb for different plant species [see, e.g., Rogers and Bendich (1987) *Plant Mol. Biol. 9*:509-520].

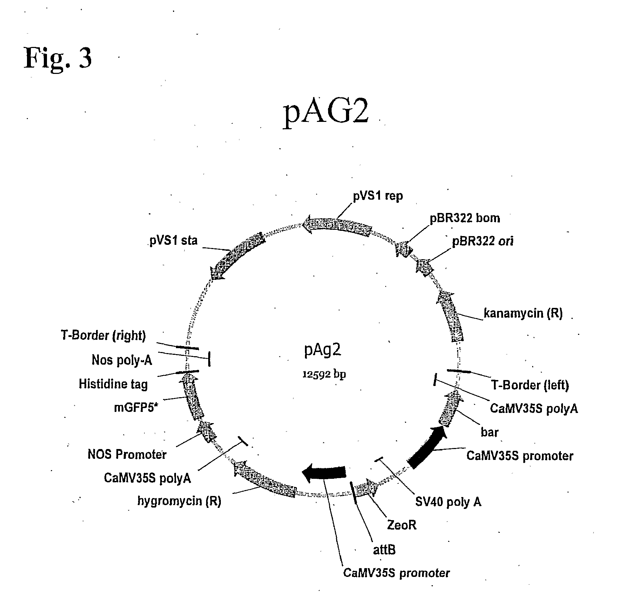

As used herein, a megachromosome refers to a chromosome that, except for introduced heterologous DNA, is substantially composed of heterochromatin. Megachromosomes are made up of an array of repeated amplicons that contain two inverted megareplicons bordered by introduced heterologous DNA [see, *e.g.,* Figure 3 of U.S. Patent No. 6,077,697 for a schematic drawing of a megachromosome]. For purposes herein, a megachromosome is about 50 to 400 Mb, generally about 250-400 Mb. Shorter variants are also referred to as truncated megachromosomes [about 90 to 120 or 150 Mb], dwarf megachromosomes [∼150-200 Mb] and cell lines, and a micro-megachromosome [∼50-90 Mb, typically 50-60 Mb]. For purposes herein, the term megachromosome refers to the overall repeated structure based on an array of repeated chromosomal segments (amplicons) that contain two inverted megareplicons bordered by any inserted heterologous DNA.

As used herein, transformation and transfection are used interchangeably to refer to the process of introducing nucleic acid introduced into cells. The terms transfection and transformation refer to the taking up of exogenous nucleic acid, e.g., an expression vector, by a host cell whether or not any coding sequences are in fact expressed. Numerous methods of introducing nucleic acids into cells are known to the ordinarily skilled artisan, for example, by *Agrobaeterium-*mediated transformation, protoplast transfection (including polyethylene glycol (PEG)-mediated transfection, electroporation, protoplast fusion, and microcell fusion), lipid-mediated delivery, liposomes, electroporation, microinjection, particle bombardment and silicon carbide whisker-mediated transformation (see, *e.g.,* Paszkowski et al. (1984) EMBO J. 3:2717-2722; Potrykus et al. (1985) Mol. Gen. Genet. 199:169-177; Reich et al. (1986) Biotechnology 4:1001-1004; Klein et al. (1987) Nature 327:70-73; U.S. Patent No. 6,143,949; Paszkowski et al. (1989) in Cell Culture and Somatic Cell Genetics of Plants, Vo/. 6, Molecular Biology of Plant Nuclear Genes, eds. Schell, J and Vasil, L.K. Academic Publishers, San Diego, California, p. 52-68; and Frame et al. (1994) Plant J. 6:941-948), direct uptake using calcium phosphate [CaP04; see,e.g., Wigler et al. (1979) Proc. Natl. Acad. Sci. U.S.A. 76:1373-1376], polyethylene glycol [PEG]-mediated DNA uptake, lipofection [see, e.g., Strauss (1996) Meth. Mol. Bio/. 54:307-327], microcell fusion [see Lambert (1991) Proc. Natl. Acad. Sci. U.S.A. 88:5907-5911; U.S. Patent No. 5,396,767, Sawford et al. (1987) Somatic Cell Mol. Genet. 13:279-284; Dhar et al. (1984) Somatic Cell Mol. Genet. 10:547-559; and McNeill-Killary *et al.* (1995) *Meth. Enzymol. 254*:133-152], lipid-mediated carrier systems [see, *e.g.,* Teifel et al. (1995) Biotechniques 19:79-80; Albrecht et al. (1996) Ann. Hematol. 72:73-79; Holmen et al. (1995) /n Vitro Cell Dev. Biol. Anim. 31:347-351; Remy et al. (1994) Bioconjug. Chem. 5:647-654; Le Bolch et al. (1995) Tetrahedron Lett. 36:6681-6684; Loeffler et al. (1993) Meth. Enzymol. 217:599-618] or other suitable method. Successful transfection is generally recognized by detection of the presence of the heterologous nucleic acid within the transfected cell, such as, for example, any visualization of the heterologous nucleic acid or any indication of the operation of a vector within the host cell.

As used herein, injected refers to the microinjection (use of a small syringe, needle, or pipette) of nucleic acid into a cell.

As used herein, gene therapy involves the transfer or insertion of nucleic acid molecules into certain cells, which are also referred to as target cells, to produce products that are involved in preventing, curing, correcting, controlling or modulating diseases, disorders and/or deleterious conditions. The nucleic acid is introduced into the selected target cells in a manner such that the nucleic acid is expressed and a product encoded thereby is produced. Alternatively, the nucleic acid may in some manner mediate expression of DNA that encodes a therapeutic product. This product may be a therapeutic compound, which is produced in therapeutically effective amounts or at a therapeutically useful time. It may also encode a product, such as a peptide or RNA, that in some manner mediates, directly or indirectly, expression of a therapeutic product. Expression of the nucleic acid by the target cells within an organism afflicted with a disease or disorder thereby enables modulation of the disease or disorder. The nucleic acid encoding the therapeutic product may be modified prior to introduction into the cells of the afflicted host in order to enhance or otherwise alter the product or expression thereof.

For use in gene therapy, cells can be transfected *in vitro,* followed by introduction of the transfected cells into an organism. This is often referred to as *ex vivo* gene therapy. Alternatively, the cells can be transfected directly *in vivo* within an organism.

As used herein, a therapeutically effective product is a product that effectively ameliorates or eliminates the symptoms or manifestations of an inherited or acquired disease or disorder or that cures said disease or disorder in an organism. For example, therapeutically effective products include a product that is encoded by heterologous DNA expressed in a diseased organism and a product produced from heterologous DNA in a host cell and to which a diseased organism is exposed.

As used herein, a transgenic plant refers to a plant (e.g., a plant cell, tissue, organ or whole plant) containing heterologous or foreign nucleic acid or in which the expression of a gene naturally present in the plant has been altered. Heterologous nucleic acid within a transgenic plant may be transiently or stably maintained within the plant. Stable maintenance of heterologous nucleic acid may be maintenance of the nucleic acid through one or more, or two or more, or five or more, or ten or more, or 25 or more, or 50 or more or 60 or more cell divisions. A transgenic plant may contain heterologous nucleic acid in one cell, multiple cells or all cells. A transgenic plant may produce progeny that contain or do not contain the heterologous nucleic acid.

As used herein, a promoter, with respect to a region of DNA, refers to a sequence of DNA that contains a sequence of bases that signals RNA polymerase to associate with the DNA and initiate transcription of messenger RNA (mRNA) from a template strand of the DNA. A promoter thus generally regulates transcription of DNA into mRNA.

As used herein, operative linkage of heterologous DNA to regulatory and effector sequences of nucleotides, such as promoters, enhancers, transcriptional and translational stop sites, and other signal sequences refers to the relationship between such DNA and such sequences of nucleotides. For example, operative linkage of heterologous DNA to a promoter refers to the physical relationship between the DNA and the promoter such that the transcription of such DNA is initiated from the promoter by an RNA polymerase that specifically recognizes, binds to and transcribes the DNA in reading frame.

As used herein, isolated, substantially pure nucleic acid, such as, for example, DNA, refers to nucleic acid fragments purified according to standard techniques employed by those skilled in the art, such as that found in Maniatis et al. [( 1982) Molecular Cloning: A Laboratory Manual, Cold Spring Harbor Laboratory Press, Cold Spring Harbor, NY].

As used herein, expression refers to the transcription and/or translation of nucleic acid. For example, expression can be the transcription of a gene into an RNA molecule, such as a messenger RNA (mRNA) molecule. Expression may further include translation of an RNA molecule into peptides, polypeptides, or proteins. If the nucleic acid is derived from genomic DNA, expression may, if an appropriate eukaryotic host cell or organism is selected, include splicing of the mRNA. With respect to an antisense construct, expression may refer to the transcription of the antisense DNA.

As used herein, vector or plasmid refers to discrete elements that are used to introduce heterologous nucleic acids into cells for either expression of the heterologous nucleic acid or for replication of the heterologous nucleic acid. Selection and use of such vectors and plasmids are well within the level of skill of the art.

As used herein, substantially homologous DNA refers to DNA that includes a sequence of nucleotides that is sufficiently similar to another such sequence to form stable hybrids under specified conditions.

It is well known to those of skill in this art that nucleic acid fragments with different sequences may, under the same conditions, hybridize detectably to the same "target" nucleic acid. Two nucleic acid fragments hybridize detectably, under stringent conditions over a sufficiently long hybridization period, because one fragment contains a segment of at least about 14 nucleotides in a sequence which is complementary (or nearly complementary) to the sequence of at least one segment in the other nucleic acid fragment. If the time during which hybridization is allowed to occur is held constant, at a value during which, under preselected stringency conditions, two nucleic acid fragments with exactly complementary base-pairing segments hybridize detectably to each other, departures from exact complementarity can be introduced into the base-pairing segments, and base-pairing will nonetheless occur to an extent sufficient to make hybridization detectable. As the departure from complementarity between the base-pairing segments of two nucleic acids becomes larger, and as conditions of the hybridization become more stringent, the probability decreases that the two segments will hybridize detectably to each other.

Two single-stranded nucleic acid segments have "substantially the same sequence," within the meaning of the present specification, if (a) both form a base-paired duplex with the same segment, and (b) the melting temperatures of said two duplexes in a solution of 0.5 X SSPE differ by less than 10_{°}C. If the segments being compared have the same number of bases, then to have "substantially the same sequence", they will typically differ in their sequences at fewer than 1 base in 10. Methods for determining melting temperatures of nucleic acid duplexes are well known [see, e.g., Meinkoth and Wahl (1984) Anal. Biochem. 138:267-284 and references cited therein].

As used herein, a nucleic acid probe is a DNA or RNA fragment that includes a sufficient number of nucleotides to specifically hybridize to DNA or RNA that includes identical or closely related sequences of nucleotides. A probe may contain any number of nucleotides, from as few as about 10 and as many as hundreds of thousands of nucleotides. The conditions and protocols for such hybridization reactions are well known to those of skill in the art as are the effects of probe size, temperature, degree of mismatch, salt concentration and other parameters on the hybridization reaction. For example, the lower the temparature and higher the salt concentration at which the hybridization reaction is carried out, the greater the degree of mismatch that may be present in the hybrid molecules.

To be used as a hybridization probe, the nucleic acid is generally rendered detectable by labelling it with a detectable moiety or label, such as ³²P, ³H and ¹⁴C, or by other means, including chemical labelling, such as by nick-translation in the presence of deoxyuridylate biotinylated at the 5'-position of the uracil moiety. The resulting probe includes the biotinylated uridylate in place of thymidylate residues and can be detected (via the biotin moieties) by any of a number of commercially available detection systems based on binding of streptavidin to the biotin. Such commercially available detection systems can be obtained, for example, from Enzo Biochemicals, Inc. (New York, NY). Any other label known to those of skill in the art, including non-radioactive labels, may be used as long as it renders the probes sufficiently detectable, which is a function of the sensitivity of the assay, the time available (for culturing cells, extracting DNA, and hybridization assays), the quantity of DNA or RNA available as a source of the probe, the particular label and the means used to detect the label.

Once sequences with a sufficiently high degree of homology to the probe are identified, they can readily be isolated by standard techniques, which are described, for example, by Maniatis et al. [( 1982) Molecular Cloning: A Laboratory Manual, Cold Spring Harbor Laboratory Press, Cold Spring Harbor, NY].

As used herein, conditions under which DNA molecules form stable hybrids and are considered substantially homologous are such that DNA molecules with at least about 60% complementarity form stable hybrids. Such DNA fragments are herein considered to be "substantially homologous". For example, DNA that encodes a particular protein is substantially homologous to another DNA fragment if the DNA forms stable hybrids such that the sequences of the fragments are at least about 60% complementary and if a protein encoded by the DNA retains its activity.

For purposes herein, the following stringency conditions are defined:
1) high stringency: 0.1 x SSPE, 0.1% SDS, 65°C
2) medium stringency: 0.2 x SSPE, 0.1 % SDS, 50°C
3) low stringency: 1.0 x SSPE, 0.1 % SDS, 50°C
or any combination of salt and temperature and other reagents that result in selection of the same degree of mismatch or matching.

As used herein, all assays and procedures, such as hybridization reactions and antibody-antigen reactions, unless otherwise specified, are conducted under conditions recognized by those of skill in the art as standard conditions.

### A. Amplificafiion of Chromosomal Segments and Use Thereof in the Generation of Artificial Chromosomes

The methods, cells and artificial chromosomes provided herein are produced by virtue of the discovery of the existence of a higher-order replication unit (megareplicon) of the centromeric region, including the pericentric DNA, of a chromosome. This megareplicon is delimited by a primary replication initiation site (megareplicator), and appears to facilitate replication of the centromeric heterochromatin, and, most likely, centromeres. Integration of heterologous nucleic acid into the megareplicator region, or in close proximity thereto, initiates a large-scale amplification of megabase-size chromosomal segments. Products of such amplification may be used as artificial chromosomes or in the generation of artificial chromosomes as described herein.

Included among the DNA sequences that may provide a megareplicator are the rDNA units that give rise to ribosomal RNA (rRNA). In plants and animals, particularly mammals such as mice and humans, these rDNA units can contain specialized elements, such as the origin of replication (or origin of bidirectional replication, *i.e.,* OBR, in mouse) and amplification promoting sequences (APS) and amplification control elements (ACE) [see, *e.g.,* with respect to plant rDNA, U.S. Patent Nos. 6,096,546 (to Raskin) and 6,100,092 (to Borysyuk et al.*);* PCT International Application Publication No. W099/66058; Genbank Accession no. Y08422 (containing the central AT-rich region of a tobacco rDNA intergenic spacer); Borysyuk et al. (1997) Plant Mol. Biol. 35:655-660); Borysyuk et al.. (2000) Nature Biotechnology 18:1303-1306; Hernandez et al. (1993) EMBO J. 12:1475-1485; Van't Hof and Lamm (1992) Plant Mol. Biol. 20:377-382; Hernandez et al. (1988) Plant Mol. Bio/. 10:413-322; and with respect to mammalian rDNA, Gogel et al. (1996) Chromosoma 104:511-518; Coffman et al. (1993) Exp. Cell. Res. 209:123-132; Little et al. (1993) Mol. Cell. Biol. 13:6600-6613; Yoon et al. (1995) Mol. Cell. Biol. 15:2482-2489; Gonzalez and Sylvester (1995) Genomics 27:320-328; Miesfeld and Arnheim (1982) Nuc. Acids Res. 10:3933-3949; Maden et al. (1987) Biochem. J. 246:519-527].

As described herein, without being bound by any theory, specialized elements such as these may facilitate replication and/or amplification of megabase-size chromosomal segments in the *de novo* formation of chromosomes, such as the artificial chromosomes described herein, in cells. These specialized elements are typically located in the nontranscribed intergenic spacer region upstream of the transcribed region of rDNA. The intergenic spacer region may itself contain internally repeated sequences which can be classified as tandemly repeated blocks and nontandem blocks (see *e.g.,* Gonzalez and Sylvester (1995) Genomics 27:320-328). In mouse rDNA, an origin of bidirectional replication may be found within a 3-kb initiation zone centered approximately 1.6 kb upstream of the transcription start site (see, *e.g.,* Gogel et al. (1996) Chromosoma 104:511-518). The sequences of these specialized elements tend to have an altered chromatin structure, which may be detected, for example, by nuclease hypersensitivity or the presence of AT-rich regions that can give rise to bent DNA structures.

Sequences of intergenic spacer regions of plant rDNA include, but are not limited to, sequences contained in GenBank Accession numbers S70723 (from the 5S rDNA of barley *(Hordeum vulgare)),* AF013103 and X03989 (from maize *(Zea mays*))*,* X65489 (from potato *(Solanum tuberosum)),* X52265 (from tomato *(Lycopersicon esculentum)),* AF177418 (from *Arabidopsis neglecta),* AF177421 and AF17422 (from *Arabidopsis halleri*)*,* A71562, X15550, and X52631 (from *Arabidopsis thaliana;* see Gruendler et a/. (1991) J. Mol. Bio/. 221:1209-1222 and Gruendler et al. (1989) Nucleic Acids Res. 17:6395-6396), X54194 (from rice *(Oryza sativa))* and Y08422 and D76443 (from tobacco *(Nicotiana tabacum).* Sequences of intergenic spacer regions of plant rDNA further include sequences from rye (see Appels et al. (1986) Can. J. Genet. Cytol. 28:673-685), wheat (see Barker et al. (1988) J. Mol. Bio/. 201:1-17 and Sardana and Flavell (1996) Genome 39:288-292), radish (see Delcasso-Tremousaygue et al. (1988) Eur. J. Biochem. 172:767-776), *Vicia faba* and *Pisum sativum* (see Kato et al. (1990) Plant Mol. Biol. 14:983-993), mung bean (see Gerstner et al. (1988) Genome 30:723-733; and Schiebel et al. (1989) Mol. Gen. Genet. 218:302-307), tomato (see Schmidt-Puchta et al. (1989) Plant Mol. Biol. 73:251-253), *Hordeum bulbosum* (see Procunier et al. (1990) Plant Mol. Biol. 15:661-663) and *Lens culinaris Medik.,* and other legume species (see Fernandez et al. (2000) Genome 43:597-603). Nucleic acids containing intergenic spacer sequences from plants can be obtained by nucleic acid amplification of DNA from plant cells using oligonucleotide primers corresponding to the 3' end of the conserved 25S mature rRNA encoding region and the 5' end of the conserved 18S mature rRNA encoding region (see *e.g.,* PCT Application Publication No. W098/13505).

An exemplary sequence encompassing a mammalian origin of replication is provided in GENBANK accession no. X82564 at about positions 2430-5435. Exemplary sequences encompassing mammalian amplification-promoting sequences include nucleotides 690-1060 and 1105-1530 of GENBANK accession no. X82564 and are also provided in PCT Application Publication No. WO 97/40183. Exemplary sequences encompassing plant amplification-promoting sequences (APS) include those provided in U.S. Patent No. 6,100,092.

In human rDNA, a primary replication initiation site may be found a few kilobase pairs upstream of the transcribed region and secondary initiation sites may be found throughout the nontranscribed intergenic spacer region (see, e.g., Yoon et al. (1995) Mol. Cell. Bio/. 15:2482-2489). A complete human rDNA repeat unit is presented in GENBANK as accession no. U13369. Another exemplary sequence encompassing a replication initiation site may be found within the sequence of nucleotides 35355-42486 in GENBANK accession no. U 13369 particularly within the sequence of nucleotides 37912-42486 and more particularly within the sequence of nucleotides 37912-39288 of GENBANK accession no. U13369 (see Coffman et al. (1993) Exp. Cell. Res. 209:123-132*).*

### B. Preparation of Plant Artificial Chromosomes

Cell lines containing artificial chromosomes can be prepared by transforming cells, preferably a stable cell line, with heterologous nucleic acid and identifying cells that contain an artificial chromosome as described herein. The artificial chromosome is a chromosomal structure that is distinct from any chromosome that existed in the cell prior to introduction of the heterologous nucleic acid. A cell containing an artificial chromosome may be identified using a variety of procedures, alone or in combination, as described in detail herein. In particular embodiments of the methods described herein, the heterologous nucleic acid contains a sequence that targets the nucleic acid to an amplifiable region of a chromosome in the cell, such as, for example, the pericentric heterochromatin and/or rDNA. A variety of targeting sequences are provided herein.

Prior to analyzing transformed cells for the presence of an artificial chromosome, the cells to be analyzed may be enriched with artificial chromosome-containing cells using a variety of techniques depending on the heterologous nucleic acid that was introduced into the host cell to initiate generation of the artificial chromosomes. For example, if nucleic acid encoding a selectable marker was included in the heterologous nucleic acid, cells containing the marker may be selected for analysis. If the selectable marker is one that confers resistance to a cytotoxic agent, e.g., bialaphos, hygromycin or kanamycin, the transformed cells may be cultured under selective conditions which include the agent. Cells surviving growth under selective conditions are then analyzed for the presence of artificial chromosomes. If the selectable marker is a readily detectable reporter molecule, such as, for example, a fluorescent protein, the transformed cells may be selected on the basis of fluorescent properties. For example, cells containing the fluorescent protein may be isolated from nontransformed cells using a fluorescence-activated cell sorter (FACS).

In analyzing transformed cells for the presence of artificial chromosomes, it is also possible to identify cells that have a multicentric, typically dicentric, chromosome, formerly multicentric (typically dicentric) chromosome, minichromosome and/or heterochromatic structures, such as a megachromosome and a sausage chromosome. If cells containing multicentric chromosomes or formerly mulitcentric (typically formerly dicentric) chromosomes are initially selected, these cells can then be manipulated, if need be, as described herein to produce the minichromosomes and other artificial chromosomes, particularly the heterochromatic artificial chromosomes and other segmented, repeat region-containing artificial chromosomes, as described herein.

### 1. Cells used in the generation of plant artificial chromosomes

Any cells harboring plant centromere-containing chromosomes may be used in the generation of plant artificial chromosomes (PACs). Such cells include, but are not limited to, plant cells, protoplasts, and cells that are hybrid cells of one or more plant species. Preferred cells are those that harbor plant centromere-containing chromosomes and are readily susceptible to the introduction of heterologous nucleic acids therein.

Cells for use in the generation of plant artificial chromosomes include cells that harbor acrocentric plant chromosomes. Examples of acrocentric plant chromosomes include chromosomes 2 and 4 of the plant *Arabidopsis thaliana* (see, e.g., Mayer et al. (1999) Nature 402:769-777; Murata et al. (1997) The Plant Journal 12:31-37*; The* Arabidopsis Genome lnitiative (2000) Nature 408:796-815), four acrocentric chromosome pairs in *Helianthus annuus* (sunflower; see Schrader et al. (1997) Chromosome Res. 5:451-456), two pairs of acrocentric chromosomes in domesticated pepper plant *(Capsicum annuum)* and a nearly acrocentric chromosome in lentil plant. In particular embodiments of the methods described herein, cells harboring acrocentric plant chromosomes containing rDNA are used in generating plant artificial chromosomes.

Plant species from which cells may be obtained include, but are not limited to, vegetable crops, fruit and vine crops, field plants, bedding plants, trees, shrubs, and other nursery stock. Examples of vegetable crops include artichokes, kohlrabi, arugula, leeks, asparagus, lettuce, bok choy, malanga, broccoli, melons (e.g., muskmelon, watermelon, crenshaw, honeydew, cantaloupe), brussel sprouts, cabbage, cardoni, carots, napa, cauliflower, okra, onions, celery, parsley, chick peas, parsnips, chicory, chinese cabbage, peppers, collards, potatoes, cucumber plants, pumpkins, cucurbits, radishes, dry bulb onions, rutabaga, eggplant, salsify, escarole, shallots, endive, garlic, spinach, green onions, squash, greens, beet, sweet potatoes, swiss chard, horseradish, tomatoes, kale, turnips and spices. Fruit and vine crops include apples, apricots, cherries, nectarines, peaches, pears, plums, prunes, quince, almonds, chestnuts, filberts, pecans, pistachios, walnuts, citrus, blueberries, boysenberries, cranberries, currants, loganberries, raspberries, strawberries, blackberries, grapes, avocados, bananas, kiwi, persimmons, pomegrante, pineapple, tropical fruits, pomes, melon, mango, papaya and lychee.

Field crop plants include evening primrose, meadow foam, corn, maize, hops, jojoba, peanuts, rice, safflower, small grains (barley, oats, rye, wheat, and others) sorghum, tobacco, kapok, leguminous plants (beans, lentils, peas, soybeans), oil plants (canola, rape, mustard, poppy, olives, sunflowers, coconut, castor oil plants, cocoa beans, groundnuts), fibre plants (cotton, flax, hemp, jute), lauraceae (cinnamon, camphor) and plants such as coffee, sugarcane, tea and natural rubber plants. Other examples of plants include bedding plants such as flowers, cactus, succulents and ornamental plants, as well as trees such as forest (broad-leaved trees and evergreens, such as conifers), fruit, ornamental and nut-bearing trees, shrubs, algae, moss, and duckweed.

### 2. Heterologous nucleic acids for use in generating plant artificial chromosomes

### a. Selectable markers

The heterologous nucleic acid that is introduced into a cell in the generation of artificial chromosomes as described herein may include nucleic acid encoding a selectable marker. Any nucleic acid that includes a selectable marker sequence may be introduced into cells harboring plant centromere-containing chromosomes for the generation of plant artificial chromosomes. Examples of selectable markers include, but are not limited to, DNA encoding a product that confers resistance to a cytotoxic or cytostatic agent and DNA encoding a readily detectable product, such as a reporter protein.

### (1) Nucleic acids encoding products that confer resistance to a selection agent

Examples of selectable markers include the dihydrylfolate reductase (dhfr) gene, hygromycin phosphotransferase genes, the phosphinothricin acetyl transferase gene (bar gene) and neomycin phosphotransferase genes. Selectable markers that can be used in animal, e.g., mammalian cells include, but are not limited to the thymidine kinase gene and the cellular adenine-phosphribosyltransferase gene.

Of particular interest for purposes herein are nucleic acid selectable markers that, upon expression in the host cell, confer antibiotic or herbicide resistance to the cell, sufficient to provide for the maintenance of heterologous nucleic acids in the cell, and which facilitate the transfer of artificial chromosomes containing the marker DNA into new host cells. Examples of such markers include DNA encoding products that confer cellular resistance to hygromycin, kanamycin, G418, bialaphos, Basta, methotrexate, glyphosate, and puromycin. For example, *neo* (or *nptll)* provides kanamycin resistance and can be selected for using kanamycin, G418, paromomycin and other agents [see, e.g., Messing and Vierra (1982) Gene 19:259-268; and Bevan et al. (1983) Nature 304:184-187]; *bar* from *Steptomyces hygroscopicus,* which encodes the enzyme phosphinothricin acetyl transferase (PAT) confers bialaphos, glufosinate, Basta or phosphinothricin resistance [see *e.g.,* White et al. (1990) Nuc. Acids Res. 18:1062; Spencer et al. (1990) Theor. App/. Genet. 79:625-631; Vickers et a/. (1996) Plant Mol. Biol. Reporter 14:363-368; and Thompson et al. (1987) EMBO J. 6:2519-2523]; the *hph* gene which confers resistance to the antibiotic hygromycin (see, *e.g.,* Blochinger and Diggelmann, Mol. Cell. Bio/. 4:2929-2931); a mutant EPSP synthase protein [see Hinchee et al. (1988) Bio/techno/ 6:915-922] confers glyphosate resistance (see also U.S. Patent Nos. 4,940,935 and 5,188,642); and a nitrilase such as *bxn* from *Klebsiella ozaenae* confers resistance to bromoxynil [see Stalker et al. (1988) Science 242:419-42]. DNA encoding cystathionine gamma-synthase (CGS) can be used as a marker that confers resistance to ethionine (see PCT Application Publication No. WO 00/55303). Examples of markers that can be used in animal, *e.g.,* mammalian cells, include but are not limited to DNA encoding products that confer cellular resistance to streptomycin, zeocin, chloramphenicol and tetracycline.

### (2) Reporter Molecules

Nucleic acids encoding reporter molecules may also be included in the nucleic acid that is introduced into a recipient cell in the generation of artificial chromosomes. Reporter genes provide a means for identifying cells and chromosomes into which heterologous nucleic acids have been transferred and further provide a means for assessing whether or not, and to what extent, transferred DNA is expressed.

Nucleic acids encoding reporter molecules that may be used in monitoring transfer and expression of heterologous nucleic acids into cells, particularly plant cells include, but are not limited to, nucleic acid encoding *β-*glucuronidase (GUS) or the *uidA* gene product, which is an enzyme for which various chromogenic substrates are known [see Novel and Novel (1973) Mo/. Gen. Genet. 120:319-335; Jefferson et al. (1986) Proc. Natl. Acad. Sci. USA 83:8447-8451; US Patent No. 5,268,463; commercially available from Clontech Laboratories, Palo Alto, CA], DNA from an R-locus gene, which encodes a product that regulates the production of anthocyanin pigments (red color) in plant tissues [see, *e.g.,* Dellaporta et al. (1988) In "Chromosome Structure and Function: /mpact of New Concepts, 18th Stadler Genetics Symposium" 11:263-282], nucleic acid encoding *β*-lactamase [Sutcliffe (1978) Proc. Natl. Acad Sci. U.S.A. 75:3737-3741] which is an enzyme for which various chromogenic substrates are known *(e.g.,* PADAC, a chromogenic cephalosporin), DNA from a xy/E gene [see, *e.g.,* Zukowsky et al. (1983) Proc. Natl. Acad. Sci. U.S.A. 80:1101-1105]*,* which encodes a catechol dioxygenase that can convert chromogenic catechols; nucleic acid encoding α-amylase [see, *e.g.,* lkuta et al. (1990) Bio/technol. 8:241-242], nucleic acid encoding tyrosinase [see, *e.g.,* Katz et al. (1983) J. Gen. Microbiol. 129:2703-2714], an enzyme capable of oxidizing tyrosine to DOPA and dopaquinone which in turn condenses to form the readily detectable compound melanin, nucleic acid encoding *β*-galactosidase, an enzyme for which there are chromogenic substrates, nucleic acid encoding luciferase (*lux*) gene [see, *e.g.,* Ow et al. (1986) Science 234:856-859] which allows for bioluminesence detection, nucleic acid encoding aequorin [see, *e.g.,* Prasher et al. (1985) Biochem. Biophy. Res. Commun. 126:1259-1268] which may be employed in calcium-sensitive bioluminescence detection, nucleic acid encoding a green fluorescent protein (GFP) [see, *e.g.,* Sheen et al. (1995) Plant J. 8:777-784; Haselhoff et al. (1997) Proc. Natl. Acad. Sci. U.S.A. 94:2122-2127; Hasseloff and Amos (1995) Trends Genet 11:328-329; Reichel et al. (1996) Proc. Natl. Acad. Sci. U.S.A. 93:5888-5893; Tian et al. (1997) Plant Cell Rep. 16:267-271; Prasher et al. (1992) Gene 111:229-233*;* Chalfie et al. (1994) Science 263:802; PCT Application Publication Nos. W097/41228 and WO 95/07463; and commercially available from Clontech Laboratoreis, Palo Alto, C), nucleic acid encoding a red or blue fluorescent protein (RFP or BFP, respectively), or nucleic acid encoding chloramphenicol acetyltransferase (CAT).

Enhanced GFP (EGFP) is a mutant of GFP with a 35-fold increase in fluorescence. This variant has mutations of Ser to Thr at amino acid 65 and Phe to Leu at position 64 and is encoded by a gene with optimized human codons (see, e.g., U.S. Patent No. 6,054,312). EGFP is a red-shifted variant of wild-type GFP (Yang et al. (1996) Nucl. Acids Res. 24:4592-4593; Haas et al. (1996) Curr. Biol. 6:315-324; Jackson et al. (1990) Trends Biochem. 15:477-483) that has been optimized for brighter fluorescence and higher expression in mammalian cells (excitation maximum = 488 nm; emission maximum = 507 nm). EGFP encodes the GFPmut1 variant (Jackson (1990) Trends Biochem. 15:477-483) which contains the double-amino-acid substitution of Phe-64 to Leu and Ser-65 to Thr. Sequences flanking EGFP have been converted to a Kozak consensus translation initiation site (Huang et al. (1990) Nucleic Acids Res. 18: 937-947) to further increase the translation efficiency in eukaryotic cells.

Nucleic acid from the maize R gene complex can also be used as nucleic acid encoding a reporter molecule. The R gene complex in maize encodes a protein that acts to regulate the production of anthocyanin pigments in most seed and plant tissue. Maize strains can have one, or as many as four, R alleles which combine to regulate pigmentation in a developmental and tissue-specific manner. Thus, an R gene introduced into such cells will cause the expression of a red pigment and, if stably incorporated, can be visually scored as a red sector. If a maize line carries dominant alleles for genes encoding for the enzymatic intermediates in the anthocyanin biosynthetic pathway (C2, A1, A2, Bz1 and Bz2), but carries a recessive allele at the R locus, the transformation of any cell from that line with R will result in red pigment formation. Exemplary lines include Wisconsin 22 which contains the rg-Stadler allele and TR112, a K55 derivative which is r-g, b, Pl. Alternatively, any genotype of maize can be utilized if the C1 and R alleles are introduced together.

### b. Promoters and other sequences that influence gene expression

Expression of nucleic acid encoding a selectable marker (or any heterologous nucleic acid) in a recipient cell can be regulated by a variety of promoters. Promoters for use in regulating transcription of DNA in cells, particularly plant cells, include, but are not limited to, the nopaline synthase (NOS) and octopine synthase (OCS) promoters; cauliflower mosaic virus (CaMV) 19S and 35S promoters, the light-inducible promoter from the small subunit of ribulose bis-phosphate carboxylase (ssRUBlSCO, an abundant plant polypeptide), the mannopine synthase (MAS) promoter [see, *e.g.,* Velten et al. (1984) EMBO J. 3:2723-2730; and Velten and Schell (1985) Nuc. Acids Res. 13:6981-6998], the rice actin promoter, the ubiquitin promoter, for example, from Z. *mays* (see e.g., PCT Application Publication No. W000/60061)*, Arabidopsis thaliana* UBI 3 promoter [see e.g., Norris et a/. (1993) Plant Mol. Bio/. 22:895-906] and the chemically inducible PR-1 promoter from tobacco or *Arabidopsis* (see *e.g.,* U.S. Patent No. 5,689,044).

Selection of a suitable promoter may include several considerations, for example, recipient cell type (such as, for example, leaf epidermal cells, mesophyll cells, root cortex cells), tissue- or organ-specific (e.g., roots, leaves or flowers) expression of genes linked to the promoter, and timing and level of expression (as may be influenced by constitutive vs. regulatable promoters and promoter strength).

Additional sequences that may also be included in the nucleic acid containing a selectable marker include, but are not restricted to, transcription terminators and extraneous sequences to enhance expression such as introns. A variety of transcription terminators may be used which are responsible for termination of transcription beyond a coding region and correct polyadenylation. Appropriate transcription terminators include those that are known to function in plants such as, for example, the CaMV 35S terminator, the *tml* terminator, the nopaline synthase terminator and the pea *rbcS* E9 terminator, all of which may be used in both monocotyledonous and dicotyledonous plants.

Numerous sequences have been found to enhance gene expression from within the transcriptional unit and these sequences can be used in conjunction with selectable marker and other genes to increase expression of the genes in plant cells. For example, various intron sequences such as introns of the maize *Adhl* gene have been shown to enhance expression, particularly in monocotyledonous cells. In addition, a number of non-translated leader sequences derived from viruses are also known to enhance exprssion, and these are particularly effective in dicotyledonous cells.

### c. Nucleic acids containing targeting sequences

Development of a multicentric, particularly dicentric, chromosome typically is effected through integration of heterologous nucleic acid into heterochromatin, such as the pericentric heterochromatin, near or within the centromeric regions of chromosomes and/or into rDNA sequences. Thus, the development of artificial chromosomes may be facilitated by targeting the heterologous nucleic acid for integration into these regions, such as by introducing DNA, including, but not limited to, rDNA (e.g., rDNA intergenic spacer sequence), satellite DNA, pericentric DNA and lambda phage DNA, into the recipient host cell. The targeting sequence may be introduced alone or with other nucleic acids, including but not limited to selectable markers. For example, a targeting sequence can be linked to a selectable marker.

Examples of plant pericentric DNA and satellite DNA include, but are not limited to, pericentromeric sequences on tomato chromosome 6 [see, *e.g.,* Weide et al. (1998) Mol. Gen. Genet. 259:190-197], satellite DNA of soybean [see, *e.g.,* Morgante et al. (1997) Chromosome Res. 5:363-373; and Vahedian et al. (1995) Plant Mol. Biol. 29:857-862], pericentromeric DNA of *Arabidopsis thaliana* [see, e.g., Tutois et al. (1999) Chromosome Res. 7:143-156], satellite DNA of arabidopsis thaliana (GenBank accession nos. AB033593 and X58104), pericentric DNA of the chickpea *[Cicer arietinum* L.; see *e.g.,* Staginnus et al. (1999) Plant Mol. Biol. 39:1037-1050], satellite DNA on the rye B chromosome [see, *e,g*., Langdon et al. (2000) Genetics 154:869-884], subtelomeric satellite DNA from *Silene latifolia* [see, *e.g.,* Garrido-Ramos et al. (1999) Genome 42:442-446] and satellite DNA in the Saccharum complex [see, *e.g.,* Alix et al. (1998) Genome 41:854-864]*.*

Examples of rDNA targeting sequences include nucleic acids from plant and animal rDNA. Plant rDNA sequences include, but are not limited to, sequences contained in GENBANK Accession numbers D16103 [from rDNA of carrot *(Daucus carota)],* M23642 and M 11585 [from rDNA encoding 24S rRNA of rice (*Oryza sativa*)]*,* M26461 [from from rDNA encoding 18S rRNA of rice (*Oryza sativa)],* M16845 [from rDNA encoding 17S, 5.8S and 25S rRNA of rice (*Oryza sativa*)]*,* X82780 and X82781 [from rDNA encoding 5S rRNA of potato *(Solanum tuberosum)],* AJ 131161, AJ 131162, AJ131163, AJ131164, AJ131165, AJ131166 and AJ131167 [from rDNA encoding 5S rRNA of tobacco *(Nicotiana tabacum],* L36494 and U31016 through U31030 [from rDNA encoding 5S rRNA of barley *(Hordeum spontaneum*)]*,* U31004 through U31015 and U31031 [from rDNA encoding 5S rRNA of barley *(Hordeum bulbosum)],* Z11759 [from rDNA encoding 5.8S rRNA of barley *(Hordeum vulgare)],* X16077 (from rDNA encoding 18S rRNA of *Arabidopsis thaliana),* M65137 (rDNA encoding 5S rRNA of *Arabidopsis thaliana),* AJ232900 (from rDNA encoding 5.8S rRNA of *Arabidopsis thaliana)* and X52320 (from *Arabidopsis thaliana* genes for 5.8S and 25S rRNA with an 18S rRNA fragment).

Intergenic spacer regions of plant rDNA include, but are not limited to sequences contained in GENBANK Accession numbers S70723 (from the 5S rDNA of barley (*Hordeum vulgare*))*,* AF013103 and X03989 (from maize (*Zea mays*))*,* X65489 (from potato (*Solanum tuberosum*))*,* X52265 (from tomato (*Lycopersicon esculentum*))*,* AF177418 (from *Arabidopsis neglecta*)*,* AF177421 and AF17422 (from *Arabidopsis halleri*)*,* A71562, X15550, X52631, U43224, X52320, X52636 and X52637 (from *Arabidopsis thaliana;* see Gruendler et al. (1991) J. Mol. Biol. 221: 1209-1222 and Gruendler et al. (1989) Nucleic Acids Res. 17:6395-6396), X54194 [from rice *(Oryza sativa)]* Y08422 and D76443 [from tobacco (*Nicotiana tabacum*)]*,* AJ243073 [from wheat (*Triticum boeoticum*)] and X07841 [from wheat (*Triticum aestivum*)*].* Sequences of intergenic spacer regions of plant rDNA further include sequences from rye [see Appels et al. (1986) Can. J. Genet. Cytol. 28:673-685], wheat [see Barker et al. (1988) J. Mol. Bio/. 201:1-17 and Sardana and Flavell (1996) Genome 39:288-292], radish [see Delcasso-Tremousaygue et al. (1988) Eur. J. Biochem. 172:767-776]*, Vicia faba* and *Pisum sativum* [see Kato et al. (1990) Plant Mol. Biol. 14:983-993], mung bean [see Gerstner et al. (1988) Genome 30:723-733; and Schiebel et a/. (1989) Mol. Gen. Genet. 218:302-307], tomato [see Schmidt-Puchta et a/. (1989) Plant Mol. Bio/. 13:251-253], *Hordeum bulbosum* [see Procunier et al. (1990) Plant Mol. Biol, 15:661-663], *Lens culinaris Medik.,* and other legume species [see Fernandez et al. (2000) Genome 43:597-603] and tobacco [see U.S. Patent Nos. 6,100,092 and 6,096,546 and PCT Application Publication No. W099/66058; Borysyuk et al. (1997) Plant Mol. Biol. 35:655-660); Borysyuk et al. (2000) Nature Biotechnology 18:1303-1306].

Mammalian rDNA sequences include, but are not limited to, DNA of GENBANK accession no. X82564 and portions thereof, the DNA of GENBANK accession no. U13369 and portions thereof and DNA sequences provided in PCT Application Publication No. W097/40183 (particularly SEQ. lD. NOS. 18-24 of W097/40183). A particular vector for use in directing integration of heterologous nucleic acid into chromosomal rDNA is pTERPUD (see PCT Application Publication No. W097/40183). Satellite DNA sequences can also be used to direct the heterologous DNA to integrate into the pericentric heterochromatin. For example, vectors pTEMPUD and pHASPUD, which contain mouse and human satellite DNA, respectively (see PCT Application Publication No. W097/40183), are examples of vectors that may be used for introduction of heterologous nucleic acid into cells for *de novo* chromosome formation leading to artificial chromosomes.

### 3. Methods for introduction of heterologous nucleic acids into host cells

Any methods known in the art for introducing heterologous nucleic acids into host cells may be used in the methods of preparing artificial chromosomes. The particular method used may depend on the type of cell into which the heterologous nucleic acid is being transferred. For example, methods for the physical introduction of nucleic acids into plant cells, for example, protoplasts and plant cells in culture, include, but are not limited to polyethylene glycol (PEG)-mediated DNA uptake, electroporation, lipid-mediated delivery, including liposomes, calcium phosphate-mediated DNA uptake, microinjection, particle bombardment, silicon carbide whisker-mediated transformation and combinations of these methods, for example methods utilizing combinations of calcium phosphate and PEG for DNA uptake or methods utilizing a combination of electroporation, PEG and heat shock (see, *e.g.,* U.S. Patent Nos. 5,231,019 and 5,453,367). Physical methods such as these are known in the art and are effective in introducing DNA into a variety of dicotyledonous and monocotyledonous plants [see, *e.g.,* Paszkowski et al. (1984) EMBO J. 3:2717-2722; Potrykus et al. (1985) Mol. Gen. Genet. 199:169-177*;* Reich et al. (1986) Biotechnology 4:1001-1004; Klein et al. (1987) Nature 327:70-73; U.S. Patent No. 6,143,949; Paszkowski et al. (1989) in Cell Culture and Somatic Cell Genetics of Plants, Vo/. 6, Molecular Biology of Plant Nuclear Genes, eds. Schell, J and Vasil, L.K. Academic Publishers, San Diego, California, p. 52-68; and Frame et al. (1994) Plant J. 6:941-948].

In addition to these methods for the introduction of nucleic acids into plant cells based on physically, mechanically or chemically meidated processes, it is possible to introduce nucleic acids into plant cells by biological methods, such as those utilizing *Agrobacterium.* In this method, nucleic acid sequences located adjacent to T-DNA border repeats can be inserted into the genome of a plant cell, typically dicotyledonous plant cells, by utilizing the encoded function for DNA transfer found in the genus *Agrobacterium.* This method has also been shown to work for some monocotyledonous plant cells, such as rice cells.

Any method for introducing nucleic acids into plant cells can be used in the generation of artificial chromosomes, provided the method is capable of introducing the nucleic acid into an amplifiable region of a chromosome, for example, heterochromatin, and particularly in close proximity to a megareplicator region of a plant chromosome.

### a. Agrobacterium-mediated introduction of nucleic acids into plant cells

*Agrobacterium-mediated* transformation is particularly well-suited for transformation of dicotyledons because of its high efficiency of transformation and its broad utility with many different species, including tobacco, tomato (see, *e.g.,* European Patent Application no. 0 249 432), sunflower, cotton (see, e.g., European Patent Application no. 0 317 511), oilseed rape, potato, soybean, alfalfa and poplar (see, e.g., U.S. Patent No. 4,795,855) (see also PCT Application Publication no. W087/07299 with respect to transformation of *Brassica). Agrobacterium-mediated* transformation has also been used to transfer nucleic acids into monocotyledonous plants. *Agrobacterium-mediated* transformation of *Chlorophytum capense* and *Narcissus* cv "Paperwhite" [see, e.g., Hooykaas-Van Slogteren et al. (1984) Nature 311:763-764], corn and wheat [see, e.g., U.S. Patent Nos. 5,164,310, 5,187,073 and 5,177,010 and Mooney et al. (1991) Plant Cell, Tissue, Organ Culture 25:209-218], rice [see, e.g., Raineri et al. (1990) Bio/Technology 8:33-38 and Chan et al. (1993) Plant Mol. Biol. 22:491-506] and barley [see, e.g., Tingay et al. (1997) The Plant J. 11:1369-1376 and Qureshi et al. (1998) Proc. 42nd Conference of Australian Society for Biochemistry and Molecular Biology, September 28-October 1, 1998, Adelaide Australia] has been reported.

*Agrobacterium-mediated* delivery of nucleic acids is based on the capacity of certain *Agrobacterium* strains to introduce a part of their Ti (tumor-inducing) plasmid, i.e., the transforming DNA or T-DNA, into plant cells and to integrate this T-DNA into the genome of the cells. The part of the Ti plasmid that is transferred and integrated is delineated by specific DNA sequences, the left and right T-DNA border sequences. The natural T-DNA sequences between these border sequences can be replaced by foreign DNA [see, e.g., European Patent Publication 116 718 and Deblaere et al. (1987) Meth. Enzymol. 153:277-293]*.*

When *Agrobacterium* is used for transformation, the heterologous nucleic acid being transferred typically is cloned into a plasmid that contains T-DNA border regions and is replicated independently of the Ti plasmid (referred to as the binary vector system) or the heterologous nucleic acid is inserted between the T-DNA borders of the Ti plasmid (referred to as the co-integrate method). ln co-integrate methods, these vectors are be integrated into the Ti or Ri plasmid by homologous recombination owing to sequences that are homologus to sequences within the T-DNA region of the Ti or Ri plasmid. The Ti or Ri plasmid also contains the vir region necessary for transfer of the T-DNA.

Intermediate vectors cannot replicate in *Agrobacteria.* The intermediate vector can be transferred into *Agrobacterium* by means of a helper plasmid (conjugation, see Fraley et al. (1983) Proc. Natl. Acad. Sci. USA 80:4803). This method, typically referred to as triparental mating, introduces the heterologous nucleic acid sequence into the bacterium and allows for selection of a homologous recombination event that produces the desired *Agrobacterium* genotype. The triparental mating procedure typically employs *Escherichia coli* carrying the recombinant intermediate vector and a helper *E. coli* strain which carries a plasmid that is able to mobilize the recombinant intermediate vector to the target *Agrobacterium* strain. A modified Ti or Ri plasmid is obtained from the transfer and selection process, which contains a heterologous nucleic acid sequence located within the T-DNA region. The resultant *Agrobacterium* strain is capable of transferring the heterologous nucleic acid to plant cells.

Binary vectors can replicate both in *E. coli* and *Agrobacterium.* They typically contain a selection marker gene and a linker or polylinker which are flanked by the right and left T-DNA border regions and can be transformed directly into *Agrobacterium* [see, *e.g.,* Hofgen and Wilmitzer (1988) Nuc. Acids. Res. 16:9877 and Holsters et al. (1978) Mol. Gen. Genet. 163:181-187] or introduced through triparental mating. The *Agrobacterium* host cell contains a plasmid carrying a vir region needed for transfer of the T-DNA into a plant cell [see, *e.g.,* White in Plant Biotechnology, eds. Kung, S. and Arntzen, C.J., Butterworth Publishers, Boston, Mass., (1989) p. 3-34 and Fraley in Plant Biotechnology, eds. Kung, S. and Arntzen, C.J., Butterworth Publishers, Boston, Mass., (1989) p. 395-407].

*Agrobacterium-mediated* transformation typically involves the transfer of a binary vector carrying the heterologous nucleic acid of interest to an appropriate *Agrobacterium* strain, which may depend on the complement of vir genes carried by the host *Agrobacterium* strain either on a co-resident Ti plasmid or chromosomally (see, *e.g.,* Uknes et al. (1993) Plant Cell 5:159-169). The transfer of a recombinant binary vector to *Agrobacterium* is acomplished by a triparental mating procedure using *Eschreichia coli* carrying the recombinant binary vector, a helper *E. coli* strain which carries a plasmid which is able to mobilize the recombinant binary vector to the target *Agrobacterium* strain. Alternatively, the recombinant binary vector can be transferred to *Agrobacterium* by DNA transformation (see, *e.g.,* Hofgen & Wilimitzer (1988) Nuc. Acids. Res. 16:9877).

Many vectors are available for transfer of nucleic acids into *Agrobacterium tumefaciens* [see, *e.g.,* Rogers et al. (1987) Methods in Enzymol. 153:253-277]. These typically carry at least one T-DNA border sequence and include vectors such as pBlN1 9 [see, *e.g.,* Bevan (1984) Nuc. Acids. Res. 12:8711-8721]. Typical vectors suitable for *Agrobacterium* transformation include the binary vectors pClB200 and pClB2001, as well as the binary vector pClB10 and hygromycin selection derivatives thereof (see, *e.g.,* U.S. Patent No. 5,639,949). Other vectors that can be employed are the pCambia vectors (see www.cambia.org), including, for example, pCambia 3300 and pCambia 1302 (GenBank Accession No. AF234298).

A particularly useful Ti plasmid cassette vector for the transformation of dicotyledonous plants contains the enhanced CaMV35S promoter (EN35S) and the 3' end, including polyadenylation signals, of a soybean gene encoding the *α* subunit of *β*-conglycinin. Between these two elements is a multilinker containing multiple restriction sites for the insertion of genes of interest (see, e.g., U.S. Patent No. 6,023,013). The vector can contain a segment of pBR322 which provides an origin of replication in *E. coli* and a region for homologous recombination with the disarmed T-DNA in Agrobacterium strain ACO; the oriV region from the broad host range plasmid RK1; the streptomycin/spectinomycin resistance gene from Tn7; and a chimeric NPTII gene, containing the CaMV35S promoter and the nopaline synthase (NOS) 3' end, which provides kanamycin resistance in transformed plant cells. Optionally, the enhanced CaMV35S promoter may be replaced with the 1.5 kb mannopine synthase (MAS) promoter (see, e.g., Velton et al. (1984) EMBO J. 3:2723-2730). After incorporation of a DNA construct into the vector, it is introduced into *A. tumefaciens* strain ACO which contains a disarmed Ti plasmid. Cointegrate Ti plasmid vectors are selected and subsequentally may be used to transform a dicotyledenous plant.

Transformation of the target plant species by recombinant *Agrobacterium* usually involves co-cultivation of the *Agrobacterium* with explants from the plant and follows published protocols. Methods of inoculation of the plant tissue vary depending upon the plant species and the *Agrobacterium* delivery system. The plant tissue can be either protoplast, callus or organ tissue, depending on the plant species. A widely used approach is the leaf disc procedure which can be performed with any tissue explant that provides a good source for initiation of whole plant differentiation (see, e.g., Horsch et al. in Plant Molecular Biology Manual A5, Kluwer Academic Publishers, Dordrecht (1988) p. 1-9 and U.S. Patent No. 6,136,320). The addition of nurse tissue may be desirable under certain conditions. There are multiple choices of *Agrobacterium* strains (including, but not limited to, *A. tumefaciens* and *A. rhizogenes*) and plasmid construction strategies that can be used to optimize genetic transformation of plants. Transformed tissue carrying an antibiotic or herbicide resistance marker present between the binary plasmid and T-DNA borders can be regenerated on selectable medium.

*A. tumefaciens* ACO is a disarmed strain similar to pTiB6SE (see Fraley et al. (1985) Bio/Technology 3:629-635). For construction of ACO, the starting *Agrobacterium* strain was A208 which contains a nopaline-type Ti plasmid. The Ti plasmid was disarmed in a manner similar to that described by Fraley et al. (1985) Bio/Technology 3:629-635) so that essentially all of the native T-DNA was removed except for the left border and a few hundred base pairs of T-DNA inside the left border. The remainder of the T-DNA extending to a point just beyond the right border was replaced with a piece of DNA including (from left to right) a segment of pBR322, the oriV region from plasmid RK2, and the kanamycin resistance gene from Tn601. The pBR322 and oriV segments are similar to these segments and provide a region of homology for cointegrate formation (see U.S. Patent No. 6,023,013). Another useful strain of *Agrobacterium* is *A. tumefaciens* strain GV3101/pMP90 [see, *e.g.,* Koncz and Schell (1986) Mol. Gen. Genet. 204:383-396].

Advances in *Agrobacterium-mediated* transfer allow introduction of larger segments of nucleic acids [see, e.g., Hamilton (1997) Gene 4:200(1-2):107-116; Hamilton et al. (1996) Proc. Natl. Acad. Sci. U.S.A. 93:9975-9979; Liu et al. (1999) Proc. Natl. Acad Sci. U.S.A. 96:6535-6540]. The vectors used in these methods are designed to have the characteristics of both bacterial artificial chromosomes (BACs) and binary vectors for *Agrobacterium-mediated* transformation. Therefore, somewhat larger DNA fragments cloned in the T-DNA region can be transferred into a plant genome by *Agrobacterium.* Binary bacterial artificial chromosome (BIBAC) vector BIBAC2 (see U.S. Patent No. 5,733,744; available from the Plant Science Center, Cornell University) and the transformation-competent bacterial artificial chromosome (TAC) vector pYLTAC7 (available from the Plant Cell Bank of the RIKEN Gene Bank, Tsukuba, Japan) are examples of the types of vectors that may be used in transferring larger segments of nucleic acids, particularly heterologous nucleic acids containing targeting and/or selectable marker sequences as described herein, into plants via *Agrobacterium-*mediated DNA transfer processes.

Introduction of heterologous nucleic acids into plant cells without the use of *Agrobacterium* circumvents the requirements for T-DNA sequences in the transformation vector and consequently vectors lacking these sequences can be utilized in addition to vectors containing T-DNA sequences. Techniques for nucleic acid transfer that do not rely on *Agrobacterium* include transformation via particle bombardment, direct DNA uptake (e.g., PEG, lipids, electroporation) and mechanical methods such as microinjection or silicon "whiskers". The choice of vector that may be used in introduction of heterologous nucleic acids into plant cells can involve largely on the preferred selection for the species being transformed. Typical vectors suitable for transformation without *Agrobacterium* include pCIB3064, pSOG19 and pSOG35 (see, *e.g.,* U.S. Patent No. 5,639,949), or common plasmid, phage or cosmid vectors.

### b. Direct DNA Uptake

Introduction of heterologous nucleic acids into plant cells may be achieved using a variety of methods that facilitate direct DNA uptake, including calcium phosphate precipitation, polyethylene glycol (PEG) treatment, electroporation, and combinations thereof [see, *e.g.,* Potrykus et a/. (1985) Mol. Gen. Genet. 199:183; Lorz et al. (1985) Mol. Gen. Genet. 199:178; Fromm et al. (1985) Proc. Natl. Acad. Sci. U.S.A. 82:5824-5828; Uchimiya et al. (1986) Mol. Gen. Genet. 204:204*;* Callis et al. (1987) Genes Dev. 1: 1183-2000; Callis et al. (1987) Nuc. Acids Res. 15:5823-5831; Marcotte et al. (1988) Nature 355:454, Toriyama et al. (1988) Bio/Technology 6:1072-1074; Haim et al. (1985) Mol. Gen. Genet. 199:161-168; Deshayes et al. (1985) EMBO J. 4:2731-2737; Krens et al. (1982) Nature 296:72-74*;* Crossway et al. (1986) Mol. Gen. Genet. 20:179].

Typically, plant protoplasts are used for direct DNA uptake, or in some instances plant tissue that has been treated to remove a portion or the majority of the cell wall (see, *e.g.,* PCT Publication No. W093/21335 and U.S. Patent No. 5,472,869). Removal of the cell wall is believed to facilitate entry of DNA into plant cells, although in some instances electroporation may be used to introduce DNA into specialized plant cells, e.g., electroporation of pollen, without first removing the cell wall.

Techniques for the preparation of callus and protoplasts from maize, transformation of protoplasts using PEG or electroporation, and the regeneration of maize plants from transformed protoplasts are found, for example, in European Patent Application nos. 0 292 435 and 0 392 225 and PCT Application Publication no. W093/07278. Transformation of rice can also be undertaken by direct gene transfer techniques utilizing protoplasts [see, *e.g.,* Zhang et al. (1988) Plant Cell Rep. 7:379-384; Shimamoto et al. (1989) Nature 338:274-277; Datta et al. (1990) Biotechnology 8:736-740]. The regeneration of fertile transgenic barley by direct DNA transfer to protoplasts is described, for example, by Funatsuki et al. [(1995) Theor. Appl. Genet. 91:707-712]. Other plant species, including tobacco and *Arabidopsis,* may also serve as sources of protoplasts for use in introduction of heterologous nucleic acids into plant cells.

### c. Particle bombardment-mediated introduction of nucleic acids into plant cells

Microprojectile bombardment of plant cells can be an effective method for the introduction of nucleic acids into plant cells. In these methods, nucleic acids are carried through the cell wall and into the cytoplasm on the surface of small, typically metal, particles [see, *e.g.,* Klein et al. (1987) Nature 327:70; Klein et al. (1988) Proc. Natl. Acad. Sci. U.S.A. 85:8502-8505, Klein et al. in Progress in Plant Cellular and Molecular Biology, eds. Nijkamp, H.J.J., Van der Plas, J.H.W., and Van Aartrijk, J., Kluwer Academic Publishers, Dordrecht, (1988), p. 56-66; Seki et al. (1999) Mol. Biotechnol. 11:251-255; and McCabe et al. (1988) Bio/Technology 6:923-926]. Particles may be coated with nucleic acids and delivered into cells by a propelling force. Exemplary particles include those containing tungsten, gold or plantinum, as well as magnesium sulfate crystals. The metal particles can penetrate through several layers of cells and thus allow the transformation of cells within tissue explants.

In an illustrative embodiment [see, *e.g.,* U.S. Patent No. 6,023,013] of a method for delivering nucleic acids into plant cells, e.g., maize cells, by acceleration, a Biolistics Particle Delivery System may be used to propel particles coated with DNA or cells through a screen, such as a stainless steel or Nytex screen, onto a filter surface covered with plant (e.g., corn) cells cultured in suspension. The screen disperses the particles so that they are not delivered to the recipient cells in large aggregates. The intervening screen between the projectile apparatus and the cells to be bombarded may reduce the size of projectile aggregates and may contribute to a higher frequency of transformation by reducing damage inflicted on the recipient cells by projectiles that are too large.

For the bombardment, cells in suspension may be concentrated on filters or solid culture medium. Alternatively, immature embryos or other target cells may be arranged on solid culture medium. The cells to be bombarded are typically positioned at an appropriate distance below the macroprojectile stopping plate. lf desired, one or more screens may also be positioned between the acceleration device and the cells to be bombarded.

The prebombardment culturing conditions and bombardment parameters may be optimized to yield the maximum numbers of stable transformants. Both the physical and biological parameters for bombardment can be important in this technology. Physical factors include those that involve manipulating the DNA/microprojectile precipitate or those that affect the flight and velocity of either the macro- or microprojectiles. Biological factors include all steps involved in manipulation of cells before and immediately after bombardment, the osmotic adjustment of target cells to help alleviate the trauma associated with bombardment, and also the nature of the transforming nucleic acid, such as linearized DNA or intact supercoiled plasmids.

Physical parameters that may be adjusted include gap distance, flight distance, tissue distance and helium pressure. In addition, transformation may be optimized by adjusting the osmotic state, tissue hydration and subculture stage or cell cycle of the recipient cells.

Techniques for transformation of A188-derived maize line using particle bombardment are desribed in Gordon-Kamm et al. [(1990) Plant Cell 2:603-618] and Fromm et al. [(1990) Biotechnology 8:833-839]. Transformation of rice may also be accomplished via particle bombardment [see, *e.g.,* Christou et al. (1991) Biotechnology 9:957-962]. Particle bombardment may also be used to transform wheat [see, *e.g.,* Vasil et al. (1992) Biotechnology 10: 667-674 for transformation of cells of type C long-term regenerable callus; and Weeks et al. (1993) Plant Physio/. 102:1077-1084 for transformation of wheat using particle bombardment of immature embryos and immature embryo-derived callus]. The production of transgenic barley using bombardment methods is described, for example, by Koprek et al. ([1996) Plant Sci. 119:79-91].

### d. Electroporation-mediated introduction of nucleic acids into plant cells

The application of brief, high-voltage electric pulses to a variety of animal. and plant cells leads to the formation of nanometer-sized pores in the plasma membrane. Nucleic acids are taken directly into the cell cytoplasm either through these pores or as a consequence of the redistribution of membrane components that accompanies closure of the pores. Electroporation can be extremely efficient and can be used both for transient expression of cloned genes and for the establishment of cell lines that carry integrated copies of the gene of interest.

Certain cell wall-degrading enzymes, such as pectin-degrading enzymes, may be employed to render the target recipient cells more susceptible to transformation by electroporation than untreated cells. Alternatively, recipient cells may be more susceptible to transformation by mechanical wounding. To effect transformation by electroporation, friable tissues such as a suspension culture of cells or embryonic callus may be used or immature embryos or other organized tissues may be directly transformed [see, e.g., Fromm et al. (1986) Nature 319:791-793; and Neuman et al. (1982) EMBO J. 1:841-845].

### e. Microinjection-mediated introduction of nucleic acids into plant cells

In microinjection techniques, nucleic acids are mechanically injected directly into cells using very small micropipettes. For example, microinjection of protoplast cells with foreign DNA for transformation of plant cells has been reported for barley and tobacco [see, e.g., Holm et al. (2000) Transgenic Res. 9:21-32 and Schnorf et al. Transgenic Res. 1:23-30]*.*

### f. Lipid-mediated introduction of nucleic acids into plant cells

In lipid-mediated transfer, nucleic acids are contacted with lipids and/or encapsulated in lipid-containing structures, including but not limited to liposomes, and the liposome-containing nucleic acids are fused with plant protoplasts. The fusion can occur in the presence or absence of a fusogen, such as PEG. Lipid-mediated transformation of plant protoplasts has been reported [see e.g., Fraley and Papahadjopoulos (1982) Curr. Top. Microbiol. lmmunol. 96:171-191; Deshayes et al. (1985) EMBO J. 4:2731-2737 and Spoerlein and Koop (1991) Theor. Appl. Genetics 83:1-5].

### g. Other methods of introduction of nucleic acids into plant cells

Other methods to physically introduce nucleic acid into plant cells may be used, including silicon carbide fibers ("whiskers") that are used to pierce plant cell walls thereby facilitating nucleic acid uptake, the use of sound waves to introduce holes in plant cell membranes to facilitate nucleic acid uptake (e.g., sonoporation) and the use of laser beams to open holes in cell membranes facilitating the entry of nucleic acids (e.g., laser poration).

Nucleic acids may also be imbibed by hydrating plant tissue, providing another method for nucleic acid uptake into plant cells [see, e.g., Simon (1974) New Phytologist 37:377-420]. For example, nucleic acids may be taken into cereal and legume seed embryos by inbibition [see, *e.g.,* Toepfer et al. (1989) The Plant Cell 1:133-139].

### 4. Treatment of cells into which heterologous nucleic acids have been introduced

Cells into which heterologous nucleic acids have been introduced may be analyzed for *de novo* formation of artificial chromosomes described herein such as may result from amplification of chromosomal segments occurring in connection with integration of heterologous nucleic acids into chromosomes. Typically, amplification occurs over multiple generations of cell division leading to the formation of detectable changes in chromosome structure. Therefore, transfected cells are typically cultured through multiple cell divisions, from about 5 to about 60, or about 5 to about 55, or about 10 to about 55, or about 25 to about 55, or about 35 to about 55 cell divisions following introduction of nucleic acid into a cell. Artificial chromosomes may, however, appear after only about 5 to about 15 or about 10 to about 15 cell divisions. Cells into which heterologous nucleic have been introduced may be treated in a variety of ways prior to or during analysis thereof for the presence of artificial chromosomes.

For example, cells into which nucleic acid encoding a selectable marker required for growth in the presence of a selection agent has been transferred can be treated as the exemplified cells herein to facilitate generation of multicentric chromosomes, and fragmentation thereof, and/or the generation of artificial chromosomes. The cells may be grown in the presence of an appropriate concentration of selection agent, which may be determined empirically by growing untransfected cells in varying concentrations of the agent and identifying concentrations sufficient to prevent cell growth and/or facilitate amplification of chromosomal segments. Transfected cells may be grown in selective media for numerous generations and cell lines can be established that contain the introduced nucleic acid. The concentration of selection agent may also be increased over several generations to promote amplification of a region of a chromosome into which heterologous nucleic acid integrated. Transfected cells may also be treated to destabilize the chromosomes to facilitate generation and fragmentation of a multicentric, typically dicentric, chromosome.

Additional heterologous nucleic acid, e.g., nucleic acid encoding a selectable marker, may also be introduced into the transfected cells to facilitate amplification of chromosomal segments, such as the pericentric heterochromatin, contained in, for example, a fragment released from a multicentric chromosome (e.g., a formerly dicentric chromosome), and generation of a heterochromatic artificial chromosome. The resulting transformed cells can then be grown in the presence of a selection agent, which may be a second agent (if the heterologous nucleic acid introduced into the transfected cells encodes a selectable marker different from any selectable marker encoded by heterologous nucleic acid initially transferred into the original host cells), with or without the first selection agent.

Cells into which nucleic acids have been introduced may also be subjected to cell sorting. For example, protoplasts may be prepared from transfected plant cells or calli and subjected to sorting. If the sorting is conducted prior to chromosomal analysis of the cells for the presence of artificial chromosomes, it provides a population of transfected cells that may be enriched for artificial chromosomes and thus facilitates the subsequent chromosomal analysis of the cells.

The sorting is based on the presence of a detectable marker in the cells, as provided for by the introduced nucleic acid, which can provide the basis for isolating such cells from cells that do not contain the heterologous nucleic acid. For example, the nucleic acid introduced into the plant cells may contain nucleic acid encoding a fluorescent protein, such as a green, red or blue fluorescent protein, which may be used for selection, by flow cytometry and other methods, of recipient cells that have taken up and express the nucleic acid at readily detected levels.

In an exemplary protocol, GFP fluorescence of transfected cell cultures may be monitored visually during culture using an inverted microscope equipped with epifluorescence illumination (Axiovert 25; Zeiss, (North York ON) and #41017 Endow GFP filter set (Chroma Technologies, Brattleboro, VT). Enrichment of GFP expressing populations can be carried out as follows. Cell sorting may be carried out, for example, using a FACS Vantage flow cytometer (Becton Dickinson Immunocytometry Systems, San Jose, CA) equipped with turbo-sort option and 2 lnnova 306 lasers (Coherent, Palo Alto CA). For cell sorting a 70 *µ*m nozzle can be used. The buffer can be changed to PBS (maintained at 20 p.s.i.). GFP may be excited with a 488 nm laser beam and excitation detected in FL1 using a 500 EFLP filter. Forward and side scattering can be adjusted to select for viable cells. Gating parameters may be adjusted using untransfected cells as negative control and GFP CHO cells as positive control.

For the first round of sorting, transfected cells may be harvested post-transfection (e.g., about 7-14 days post-transfection), converted to protoplasts, resuspended in about 10 ml of growth medium and sorted for GFP-expressing populations using parameters described above. GFP-positive cells may be dispensed into a volume of about 5-10 ml of protoplast medium while non-expressing cells are directed to waste. The expressing cells may be cultured. Plant cells or calli can then be analyzed, for fluorescence in-situ hybridization screening.

### 5. Analysis of transformed cells and identification and manipulation of artificial chromosomes

Cells into which nucleic acids have been introduced, and which may or may not have been further treated as described herein, may be analyzed for indications of amplification of chromosomal segments, the presence of structures that may arise in connection with amplification and *de novo* artificial chromosome formation and/or the presence of desired artificial chromosomes as described herein. Analysis of the cells typically involves methods of visualizing chromosome structure, including, but not limited to, G-and C-banding, PCR, Southern blotting and FISH analyses, using techniques described herein and/or known to those of skill in the art. Such analyses can employ specific labelling of particular nucleic acids, such as satellite DNA sequences, heterochromatin, rDNA sequences and heterologous nucleic acid sequences, that may be subject to amplification. During analysis of transfected cells, a change in chromosome number and/or the appearance of distinctive, for example, by increased segmentation arising from amplification of repeat units, chromosomal structures will also assist in identification of cells containing artificial chromosomes. The following description of events and structures that may be observed in analyzing cells for evidence of chromosomal amplification and/or the presence of artificial chromosomes is intended to be illustrative of the observations and considerations that may occur in the analysis of cells of any type, including mammalian and plant cells. lt should be recognized that numerous types of structures may be formed during amplification of chromosomal segments and treatment of the cells. Additional, yet related, structures and variations of these structures are contemplated herein and are recognizable based on the descriptions and teachings of the generation and identification of artificial chromosomes presented herein. Each structure can be further manipulated, for example using procedures described herein, to derive additional chromosomal structures and compositions.

Typically, *de novo* centromere formation occurs in cells upon integration of heterologous nucleic acids into the cell chromosomes and amplification of chromosomal and heterologous nucleic acids. The integration and amplification that gives rise to *de novo* centromere formation typically occurs at the centromeric region of the short arm of a chromosome, typically an acrocentric chromosome. By employing methods such as chromosome-staining methods, including FISH and G-and C-banding, it may be possible to identify a chromosome at which the process occurs.

The amplification can lead to the formation of multicentric, typically dicentric, chromosomes. Because of the presence of two or more functionally active centromeres on the same chromosome, regular breakages occur between the centromeres. Such specific chromosome breakages can give rise to the appearance of a chromosome fragment carrying a neo-centromere. The neo-centromere may be found on a minichromosome (neo-minichromosome), while a formerly dicentric chromosome may carry traces of the heterologous nucleic acid.

### a. The neo-minichromosome

Breakage of a dicentric chromosome between the two functional centromeres can form at least two chromosomes, for example, a so-called minichromosome, and a formerly dicentric chromosome. Treatment of cells containing a dicentric chromosome, such as, for example, recloning, treatment with agents that destabilize the chromosomes, e.g., BrdU, and/or culturing under selective conditions, may facilitate breakage of the dicentric chromosome. Selection of transformed cells can yield cell lines containing a stable neo-minichromosome. The breakage of a multicentric, typically dicentric, chromosome in transformed cells, which separates the neo-centromere from the remainder of the endogenous chromosome, may occur, for example, in the G-band positive heterologous nucleic acid region as is suggested if traces of the heterologous nucleic acid sequences at the broken end of the formerly dicentric chromosome are observed.

Multiple E-type amplification (amplification of euchromatin) may form a neo-chromosome, which separates from the remainder of the dicentric chromosome through a specific breakage between the centromeres of the dicentric chromosome. Inverted duplication of the fragment bearing the neo-centromere can result in the formation of a stable neo-minichromosome. The minichromosome is generally about at least 20-30 Mb in size.

The presence of inverted chromosome segments can be associated with the chromosomes formed *de novo* at the centromeric region of a chromosome. During the formation of the neo-minichromosome, the event leading to the stabilization of the distal segment of the chromosome that bears the duplicated neo-centromere may be the formation of its inverted duplicate.

Although the neo-minichromosome typically carries only one functional centromere, both ends of the minichromosome can be heterochromatic, carrying, for example, satellite DNA sequences as discernable by *in situ* hybridization. Comparison of the G-band pattern of a chromosome fragment carrying the neo-centromere with that of a stable neo-minichromosome, can indicate that the neo-minichromosome is an inverted duplicate of the chromosome fragment that bears the neo-centromere.

Cells containing a de novo-formed minichromosome, which contains multiple repeats of the heterologous nucleic acids, can be used as recipient cells in cell transfection. Donor nucleic acids, such as heterologous nucleic acids containing DNA encoding a desired protein and DNA encoding a second selectable marker, can be introduced into the cells and integrated into the *de novo*-formed minichromosomes. To facilitate integration into the de *novo*-formed minichromosomes, the heterologous DNA may also contain sequences that are homologous to nucleic acids already present in the minichromosomes, which can, through homologous recombination, provide targeted integration into the minichromosome. Nucleic acids can also be integrated into the minichromosome through the use of site-specific recombinases by producing minichromosomes containing site-specific recombination sites as described herein. Integration can be verified by *in situ* hybridization and Southern blot analyses. Transcription and translation of heterologous DNA can be confirmed by primer extension, immunoblot analyses and reporter gene assays, if a reporter gene has been included in the heterologous DNA, using, for example, appropriate nucleic acid probes and/or product-specific antibodies.

The resulting engineered minichromosome that contains the heterologous DNA can also be transferred, for example by cell fusion, into a recipient cell line to further verify correct expression of the heterologous DNA. Following production of the cells, metaphase chromosomes can be obtained, such as by addition of colchicine, and the minichromosomes purified using methods as described herein. The resulting minichromosomes can be used for delivery to specific cells of interest using any known method or methods for transferring heterologous nucleic acids into cells, particularly plant cells, and/or methods described herein.

Thus, the neo-minichromosome is stably maintained in cells, replicates autonomously, and permits the persistent, long-term expression of genes under non-selective culture conditions, and in a whole, intact, regenerated plant. lt also can contain megabases of heterologous known DNA that can serve as target sites for homologous recombination and integration of DNA of interest. The neo-minichromosome is, thus, a vector for the delivery and expression of nucleic acids to cells.

Cell lines that contain artificial chromosomes, such as the minichromosome, the neo-chromosome, and the heterochromatic artificial chromosomes, are a convenient source of these chromosomes and can be manipulated, such as by cell fusion or production of microcells for fusion with selected cell lines, to deliver the chromosome of interest into a multiplicity of cell lines, including cells from a variety of different plant species.

### b. Heterochromatin-containing and predominantly heterochromatic artificial chromosomes

Manipulation of cells containing a fragment released upon breakage of the dicentric chromosome (e.g., a formerly dicentric chromosome), for example, by introducing additional heterologous nucleic acids, including, for example, DNA encoding a second selectable marker and growth under selective conditions, can yield heterochromatic structures. Included among such structures are compositions referred to as sausage chromosomes and megachromosomes. For example, a formerly dicentric chromosome may translocate to the end of another chromosome, such as an acrocentric chromosome. Additional heterologous nucleic acids added to cells containing a formerly dicentric chromosome can integrate into the pericentric heterochromatin of the formerly dicentric chromosome and be amplified several times with megabases of pericentric heterochromatic satellite DNA sequences forming a "sausage" chromosome carrying a newly formed heterochromatic chromosome arm. The size of this heterochromatic arm can vary, for example, between ∼150 and ∼800 Mb in individual metaphases. The chromosome arm can contain four to five satellite segments rich in satellite DNA, and evenly spaced integrated heterologous "foreign" DNA sequences. At the end of the compact heterochromatic arm of the sausage chromosome, a less condensed euchromatic terminal segment may be observed. By capturing a euchromatic terminal segment, this new chromosome arm is stabilized in the form of the "sausage" chromosome. In subclones of sausage chromosome-containing cell lines, the heterochromatic arm of the sausage chromosome may become unstable and show continuous intrachromosomal growth, particularly after treatment with BrdU and/or drug selection to induce further H-type amplification. ln extreme cases, the amplified chromosome arm can exceed 500 Mb or even 1000 Mb in size (gigachromosome). Thus, the gigachromsome is a structure in which a heterochromatic arm has amplified but not broken off from a euchromatic arm.

*ln situ* hybridization with, for example, biotin-labeled subfragments of the added heterologous nucleic acids may show a hybridization signal only in the heterochromatic arm of the sausage chromosome, indicating that the heterologous nucleic acid sequences are localized in the pericentric heterochromatin.

Gene expression, however, may be possible in the heterochromatic environment of a sausage chromosome. The level of heterologous gene expression may be determined by Northern hybridization with a subfragment of the selectable marker gene. Reporter genes included in heterologous nucleic acids also provide a readily detectable product for use in evaluating gene expression in a sausage or other heterochromatic or predominantly heterochromomatic chromosome. Southern hybridization of DNA isolated from subclones of sausage chromosome-containing cells with subfragments of reporter (and selectable marker) genes can show a close correlation between the intensity of hybridization and the length of the sausage chromosome.

Cell lines containing sausage chromosomes can be manipulated to yield additional heterochromatic structures and artificial chromosomes, including, for example, an artificial chromosome referred to as a megachromosome. Such manipulation includes fusion of the cell line with other cells and growth in the presence of one or more selection agents and/or BrdU.

Cells with a structure, such as the sausage chromosome, can be selected and fused with a second cell line, including other plant and non-plant species [see, e.g., Dudits et al. (1976) Heriditas 82:121-123 for the fusion of human cells with carrot protoplasts and Wiegand et al. (1987) J. Cell. Sci. (Pt. 2):145-149 for laser-induced fusion of plant protoplasts with mammalian cells] to eliminate other chromosomes that are not of interest. Structures such as sausage chromosomes formed during this process may be further manipulated, for example, by treating the cells with agents that destabilize chromosomes, *e.g.,* BrdU, so that the heterochromatic arm forms a chromosome that is substantially heterochromatic (*e.g.,* a megachromosome). Structures such as the gigachromosome in which the heterochromatic arm has amplified but not broken off from the euchromatic arm, may also be observed. Further manipulation, such as fusions and growth in selective conditions and/or BrdU treatment or other such treatment, can lead to fragmentation of the megachromosome to form smaller chromosomes that have the amplicon as the basic repeating unit.

lf a cell with a sausage chromosome is selected, it can be treated with an agent, such as BrdU, that destabilizes the chromosome so that the heterochromatic arm forms a chromosome that is substantially heterochromatic (e.g., a megachromosome). Prior to treating the cell with BrdU, it can be fused with another cell line carrying chromosomes of another species, in order to eliminate chromosomes of the original host cell and obtain a cell in which the only chromosome from the host cell is the sausage chromosome. The resulting hybrid cells can be grown in the presence of multiple selection agents to select for those that carry the sausage chromosome. *ln situ* hybridization with chromosome painting probes that detect chromosomes of both the host cell species and the species of cell to which the host cell was fused can provide an indication of the chromosomal make up of the hybrid cells.

Cell lines containing a sausage chromosome can be treated with a destabilizing agent, such as BrdU, followed by growth in selective medium and retreatment with BrdU. The BrdU treatments appear to destabilize the genome, resulting in a change in the sausage chromosome as well. A cell population in which a further amplification has occurred will arise. In addition to the heterochromatic arm (which may, for example, be ∼100-150 Mb) of the sausage chromosome, an extra centromere and another (for example, ∼150-250 Mb) heterochromatic chromosome arm may be formed. By the acquisition of another euchromatic terminal segment, a new submetacentric chromosome (e.g., megachromosome) can form.

Megachromosomes may also be produced through regrowth and establishment of sausage chromosome-containing cells in selective medium. Repeated BrdU treatment can produce cell lines that have a dwarf megachromosome (for example, about 150-200 Mb), a truncated megachromosome (for example, about 90-120 Mb), or a micro-megachromosome (for example, about 50-90 Mb). Cell lines containing smaller truncated megachromosomes can be used to generate even smaller megachromosomes, *e.g., ∼*10-30 Mb in size. This may be accomplished, for example, by breakage and fragmentation of a micro-megachromosome through exposing the cells to X-ray irradiation, BrdU or telomere-directed in vivo chromosome fragmentation.

Apart from the euchromatic terminal segments and the integrated foreign nucleic acid, the whole megachromosome, as well as other related types of predominantly heterochromatic artificial chromosomes, is constitutive heterochromatin. This can be demonstrated by C-banding of the megachromosome, which results in positive staining characteristic of constitutive heterochromatin. It can contain tandem arrays of satellite DNA. In a particular example, satellite DNA blocks are organized into a giant palindrome (amplicon) carrying integrated exogenous nucleic acid sequences at each end. It is of course understood that the specific organization and size of each component can vary among species, and also the chromosome in which the amplification event initiates.

In general, a clear segmentation may be observed in one or more arms of an amplification-based chromosome. For example, a megachromosome may contain building units that are amplicons of, for example, ∼30 Mb containing satellite DNA with the integrated "foreign" DNA sequences at both ends. The ∼30 Mb amplicons may be composed of two ∼15 Mb inverted doublets of ∼7.5 Mb satellite DNA blocks, which are separated from each other by a narrow band of non-satellite sequences. The wider non-satellite regions at the amplicon borders may contain integrated, exogenous (heterologous) nucleic acid, while any narrow bands of non-satellite DNA sequences within the amplicons may be integral parts of the pericentric heterochromatin of the host chromosomes. The sizes of the building units of a megachromosome or other amplification-based chromosome may vary depending on the species of the host chromosome from which the artificial chromosome was generated.

Further BrdU treatment can produce cell and/or calli that include cells with a truncated megachromosome. The megachromosome can be further fragmented *in vivo* using a chromosome fragmentation vector to ultimately produce a chromosome that comprises a smaller stable replicable unit, for example, about 15 Mb-60 Mb, containing one to four megareplicons.

Apart from the euchromatic terminal segments, the whole megachromosome is heterochromatic, and has structural homogeneity. Therefore, artificial chromosomes such as the megachromosome offer a unique possibility for obtaining information about the amplification process, and for analyzing some basic characteristics of the pericentric constitutive heterochromatin, as a vector for heterologous DNA, and as a target for further fragmentation.

### C. Isolation of Artificial Chromosomes

The artificial chomosomes provided herein can be isolated by any suitable method known to those of skill in the art. Also, methods are provided herein for effecting substantial purification, particularly of the artificial chromosomes.

Artificial chromosomes, may be sorted from endogenous chromosomes using any suitable procedures, and typically involve isolating metaphase chromosomes, distinguishing the artificial chromosomes from the endogenous chromosomes, and separating the artificial chromosomes from endogenous chromosomes. Such procedures will generally include the following basic steps for animal cells and protoplasts: (1) culture of a sufficient number of cells (typically about 2 x 10⁷ mitotic cells) to yield, preferably on the order of 1 x 10⁶ artificial chromosomes, (2) arrest of the cell cycle of the cells in a stage of mitosis, preferrably metaphase, using a mitotic arrest agent such as colchicine, (3) treatment of the cells, particularly by cell wall dissolution for plant cells and/or swelling of the cells in hypotonic buffer, to increase susceptibility of the cells to disruption, (4) by application of physical force to disrupt the cells in the presence of isolation buffers for stabilization of the released chromosomes, (5) dispersal of chromosomes in the presence of isolation buffers for stabilization of free chromosomes, (6) separation of artificial chromosomes from endogenous chromosomes and (7) storage (and shipping if desired) of the isolated artificial chromosomes in appropriate buffers. Modifications and variations of the general procedure for isolation of artificial chromosomes, for example to accommodate different cell types with differing growth characteristics and requirements and to optimize the duration of mitotic block with arresting agents to obtain the desired balance of chromosome yield and level of debris, may be empirically determined (see Examples).

Steps 1-5 relate to isolation of metaphase chromosomes. The separation of artificial from endogenous chromosomes (step 6) may be accomplished in a variety of ways. For example, the chromosomes may be stained with DNA-specific dyes such as Hoeschst 33258 and chromomycin A₃ and sorted into artificial chromosomes and endogenous chromosomes on the basis of dye content by employing fluorescence-activated cell sorting (FACS).

Artificial chromosomes have been isolated by fluorescence-activated cell sorting (FACS). This method takes advantage of the nucleotide base content of the artificial chromosomes. In the case of predominantly heterochromatic artificial chromosomes, by virtue of their high heterochromatic DNA content, they will differ from any other chromosomes in a cell. In a particular embodiment, metaphase chromosomes are isolated and stained with base-specific dyes, such as Hoechst 33258 and chromomycin A3. Fluorescence-activated cell sorting will separate artificial chromosomes from the endogenous chromosomes. A dual-laser cell sorter (such as, for example, a FACS Vantage Becton Dickinson Immunocytometry Systems) in which two lasers were set to excite the dyes separately, allowed a bivariate analysis of the chromosomes by base-pair composition and size. Cells containing such artificial chromosomes can be similarly sorted.

Preparative amounts of artificial chromosomes (for example, 5 x 10⁴ - 5 x 10⁷ chromosomes/ml) at a purity of 95% or higher can be obtained. The resulting artificial chromosomes are used for delivery to cells by methods such as, for example, microinjection, liposome-mediated transfer, and electroporation.

Additional methods provided herein for isolation of artificial chromosomes from endogenous chromosomes include procedures that are particularly well suited for large-scale isolation of artificial chromosomes. ln these methods, the size and density differences between artificial chromosomes and endogenous chromosomes are exploited to effect separation of these two types of chromosomes. To facilitate larger scale isolation of the artificial chromosomes, different separation techiniques may be employed such as swinging bucket centrifugation (to effect separation based on chromosome size and density) [see, e.g., Mendelsohn et al. (1968) J. Mol. Biol. 32:101-108],zonal rotor centrifugation (to effect separation on the basis of chromosome size and density) [see, e.g., Burki et al. (1973) Prep. Biochem. 3:157-182; Stubblefield et al. (1978) Biochem. Biophys. Res. Commun. 83:1404-1414, velocity sedimentation (to effect separation on the basis of chromosome size and shape) [see e.g., Collard et al. (1984) Cytometry 5:9-19].

Affinity-, particularly immunoaffinity-, based methods for separation of ACs from endogenous chromosomes are also provided herein. For example, artificial chromosomes which are predominantly heterochromatin may be separated from endogenous chromosomes through immunoaffinity procedures involving antibodies that specifically recognize heterochromatin, and/or the proteins associated therewith, when the endogenous chromosomes contain relatively little heterochromatin.

lmmuno-affinity purification may also be employed in larger scale artificial chromosomes isolation procedures. In this process, large populations of artificial chromosome-containing cells (asynchronous or mitotically enriched) are harvested en masse and the mitotic chromosomes (which can be released from the cells using standard procedures such as by incubation of the cells, such as freshly isolated protoplasts, in hypotonic buffer and/or detergent treatment of the cells in conjunction with physical disruption of the treated cells) are enriched by binding to antibodies that are bound to solid state matrices (e.g. column resins or magnetic beads). Antibodies suitable for use in this procedure bind to condensed centromeric proteins or condensed and DNA-bound histone proteins. For example, autoantibody LU851 (see Hadfaczky et al. (1989) Chromosoma 97:282-288), which recognizes mammalian centromeres, may be used for large-scale isolation of chromosomes prior to subsequent separation of artificial chromosomes from endogenous chromosomes using methods such as FACS. The bound chromosomes would be washed and eventually eluted for sorting.

lmmunoaffinity purification may also be used directly to separate artificial chromosomes from endogenous chromosomes. For example, in the case of artificial chromosomes that are predominantly heterochromatic, the artificial chromsomes may be generated in or transferred to (e.g., by microinjection or microcell fusion as described herein) a cell line that has chromosomes that contain relatively small amounts of heterochromatin, such as hamster cells (e.g., V79 cells or CHO-K1 cells). The predominantly heterochromatic artificial chromosomes are then separated from the endogenous chromosomes by utilizing anti-heterochromatin binding protein (Drosophila HP-1) antibody conjugated to a solid matrix. Such matrix preferentially binds artificial chromosomes relative to hamster chromosomes. Unbound hamster chromosomes are washed away from the matrix and the artificial chromosomes are eluted by standard techniques. Similarly, artificial chromosomes of one species, e.g., a plant-derived artificial chromosome, may be separated from a background of endogenous chromosomes of another species, e.g., animal, such as mammalian, chromosomes, based on immunological differences of the two species, provided that antibodies that specifically recognize one species and not the other are available or can be generated.

### D. Generation of Artificial Chromosomes Through Assembly of Component Elements

Artificial chromosomes can be constructed *in vitro* by assembling the structural and functional elements that contribute to a complete chromosome capable of stable replication and segregation alongside endogenous chromosomes in cells. The identification of the discrete elements that in combination yield a functional chromosome has made possible the *in vitro* assembly of artificial chromosomes. The process of *in vitro* assembly of artificial chromosomes, which can be rigidly controlled, provides advantages that may be desired in the generation of chromosomes that, for example, are required in large amounts or that are intended for specific use in transgenic organism systems.

For example, *in vitro* assembly may be advantageous when efficiency of time and scale are important considerations in the preparation of artificial chromosomes. Because *in vitro* assembly methods do not involve extensive cell culture procedures, they may be utilized when the time and labor required to transform, feed, cultivate, and harvest cells used in *de novo* cell-based production systems is unavailable.

Provided herein are *in vitro* assembly methods that include the joining of essential components, such as a centromere, telomere and an origin of replication, to yield an artificial chromosome, in particular, an artificial chromosome that functions in plants and that may contain components derived from plant chromosomes. Also provided are artificial chromosomes produced by the methods. Particular embodiments of the methods and chromosomes include a megreplicator. The megareplicator may contain rDNA, for example, mammalian or plant rDNA. *In vitro* assembled artificial chromosomes may contain any amount of heterochromatic and/or euchromatic nucleic acid. For example, an *in vitro* assembled artificial chromosome may be substantially all heterochromatin, while still containing protein-encoding DNA, or may contain increasing amounts of euchromatic DNA, such that, for example, it contains about 10%, 20%, 30%, 40%, 50%, 60%, 70%, 80%, 90% or greater than about 90% euchromatic DNA.

*ln vitro* assembly may also be rigorously controlled with respect to the exact manner in which the several elements of the desired artificial chromosome are combined and in what sequence and proportions they are assembled to yield a chromosome of precise specifications. This feature is of particular significance in the generation of plant artificial chromosomes containing one or more regions of segmentation as described herein with reference to amplification-based artificial chromosomes. For example, certain plant chromosome structures (such as acrocentric chromosomes and/or chromosomes containing adjacent regions of heterochromatin and rDNA) that may be desirable for use in the generation of particular types of plant artificial chromosomes via amplification-based methods as described herein may be limited in number or may not exist. These particular types of plant artificial chromosomes, e.g., certain predominantly heterochromatic plant artificial chromosomes, may also be generated via *in vitro* assembly of artificial chromosomes as described herein.

For example, plant artificial chromosomes containing regions of repeated nucleic acid units that are predominantly heterochromatic may be assembled by joining essential chromosomal components and repeat regions, or may be generated from an *in vitro* assembled artificial chromosome via amplification of heterochromatic DNA contained within an *in vitro* assembled artificial chromosome. For generation of such chromosomes via amplification of heterochromatic DNA contained within an *in vitro* assembled artificial chromosome, nucleic acids are introduced into a cell containing an *in vitro* assembled artificial chromosome and a resulting cell is selected that contains an artificial chromosome containing one or more regions of repeated nucleic acid units that are predominantly heterochromatic. The *in vitro* assembled artificial chromosome either contains a megareplicator to faciliate amplification of chromosomal DNA in connection with integration of nucleic acid into the chromosome or megareplicator-containing DNA is included in the nucleic acid that is integrated into thee *in vitro* assembled artificial chromosome.

The following describes the processes involved in the assembly of artificial chromosomes *in vitro,* utilizing a megachromosome as exemplary starting material.

### 1. Identification and isolation of the components of the artificial chromosome

The chromosomes provided herein are elegantly simple chromosomes for use in the identification and isolation of components to be used in the *in vitro* assembly of expression systems or artificial chromosomes. The ability to purify artificial chromosomes to a very high level of purity, as described herein, facilitates their use for these purposes. For example, the megachromosome, particularly truncated forms thereof, serve as starting materials. With respect to the construction of an artificial chromosome containing at least some mammalian cell derived components, possible starting materials can be obtained from, for example, cell lines such as 1 B3 and mM2C1, which are derived from H1 D3 (deposited at the European Collection of Animal Cell Culture (ECACC) under Accession No. 96040929). With respect to the construction of an artificial chromosome containing at least some plant cell derived components, possible starting materials include cells containing PACs, e.g., megachromosomes, generated as described herein.

For example, the mM2C1 cell line contains a micro-megachromosome (∼50-60 kB), which advantageously contains only one centromere, two regions of integrated heterologous DNA with adjacent rDNA sequences, with the remainder of the chromosomal DNA being mouse major satellite DNA. Other truncated megachromosomes can serve as a source of telomeres, or telomeres can be provided. The centromere of the mM2C1 cell line contains mouse minor satellite DNA, which provides a useful tag for isolation of the centromeric DNA.

Additional features of particular ACs provided herein, such as the micro-megachromosome of the mM2C1 cell line, that make them uniquely suited to serve as starting materials in the isolation and identification of chromosomal components include the fact that the centromeres of each megachromosome within a single specific cell line are identical. The ability to begin with a homogeneous centromere source (as opposed to a mixture of different chromosomes having differing centromeric sequences) greatly facilitates the cloning of the centromere DNA. By digesting purified megachromosomes, particularly truncated megachromosomes, such as the micro-megachromosome, with appropriate restriction endonucleases and cloning the fragments into commercially available and well known YAC vectors (see, e.g, Burke et al. (1987) Science 236:806-812), BAC vectors (see, e.g.,Shizuya et al. (1992) Proc. Natl. Acad. Sci. U.S.A. 89: 8794-8797 bacterial artificial chromosomes which have a capacity of incorporating 0.9 - 1 Mb of DNA) or PAC vectors (the P1 artificial chromosome vector which is a P1 plasmid derivative that has a capacity of incorporating 300 kb of DNA and that is delivered to E. coli host cells by electroporation rather than by bacteriophage packaging; see, e_{.}g., loannou et al. (1994) Nature Genetics 6:84-89; Pierce et al. (1992) Meth. Enzymol. 216:549-574; Pierce et al. (1992) Proc. Natl. Acad. Sci. U.S.A. 89:2056-2060; U.S. Patent No. 5,300,431 and International PCT application No. WO 92/14819) vectors, it is possible for as few as 50 clones to represent the entire micro-megachromosome.

### a. Centromeres

An exemplary centromere for use in the construction of an artificial chromosome is that contained within a megachromosome, such as those described herein. One example of a particular megachromosome-containing cell line provided is, for example, H 1 D3 and derivatives thereof, such as mM2C1 cells. Megachromosomes are isolated from such cell lines utilizing, for example, the procedures described herein, and the centromeric sequence is extracted from the isolated megachromosomes. For example, the megachromosomes may be separated into fragments utilizing selected restriction endonucleases that recognize and cut at sites that, for instance, are primarily located in the replication and/or heterologous DNA integration sites and/or in the satellite DNA. Based on the sizes of the resulting fragments, certain undesired elements may be separated from the centromere-containing sequences. The centromere-containing DNA could be as large as 1 Mb.

Probes that specifically recognize centromeric sequences, such as mouse minor satellite DNA-based probes [see, e.g., Wong et al. (1988) Nucl. Acids Res. 16:11645-11661], pCT4.2 probe, a 3.5 kb fragment of *Arabidopsis* 5S rDNA (Campbell et al. (1992) *Gene112*:225-228)*, Arabidopsis* cosmids E4.11 (30kb) adn E4.6 (33 kb, Bent et al. (1994) *Science*265:1856-1860; and 180 bp pAL1 repeat sequence (Maluszynska et al. (1991) *Plant J.1*: 159-166; and Martinez-Zapater et al. (1986) *Mol. Gen. Genet.* 204:417-423) may be used to isolate a centromere-containing YAC, BAC or PAC clone derived from the megachromosome. Alternatively, or in conjunction with the direct identification of centromere-containing megachromosomal DNA, probes that specifically recognize the non-centromeric elements, such as probes specific for mouse major satellite DNA, plant satellite DNA, the heterologous DNA and/or rDNA, may be used to identify and eliminate the non-centromeric DNA-containing clones.

Additionally, centromere cloning methods described herein may be utilized to isolate the centromere-containing sequence of the megachromosome.

Once the centromere fragment has been isolated, it may be sequenced and the sequence information may in turn be used in PCR amplification of centromere sequences from megachromosomes or other sources of centromeres. Isolated centromeres may also be tested for function *in vivo* by transferring the DNA into a host cell. Functional analysis may include, for example, examining the ability of the centromere sequence to bind centromere-binding proteins. The cloned centromere will be transferred to cells with a selectable marker gene and the binding of a centromere-specific protein, such as anti-centromere antibodies (e.g., LU851, see, Hadlaczky et al. (1986) Exp. Cell Res. 167:1-15) can be used to assess function of the centromeres.

### b. Telomeres

Telomeres that may be used in assembly of an artificial chromosome include a 1 kB synthetic telomere (see, e.g., PCT Application Publication No. WO 97/40183). A double synthetic telomere construct, which contains a 1 kB synthetic telomere linked to a dominant selectable marker gene that continues in an inverted orientation may be used for ease of manipulation. Such a double construct contains a series of TTAGGG repeats 3' of the marker gene and a series of repeats of the inverted sequence, i.e., GGGATT, 5' of the marker gene as follows:
(GGGATTT)ₙ---dominant marker gene---(TTAGGG)ₙ. Using an inverted marker provides an easy means for insertion, such as by blunt end ligation, since only properly oriented fragments will be selected.

Telomere sequences also include sequences described in plants, for example, an *Arabidopsis* sequence containing head-to-tail arrays of the monomer repeat CCCTAAA totaling a few, for example 3-4, kb in length. Telomere sequences vary in length and do not appear to have a strict length requirement. An example of a cloned telomere is found in GenBank accession no. M20158 (Richards and Ausubel (1988) Cell 53:127-136) and in U.S. Patent No. 5,270,201. Yeast telomere sequences include those provided in GenBank accession no. S70807 (Louis et al. (1994) Yeast 10:271-274). Additionally, a method for isolating a higher eukaryotic telomere *from A. thaliana* has been reported (Richards and Ausubel (1988) Cell53:127-136*;* and U.S. Patent No. 5,270,201).

### c. Megareplicator

The megareplicator sequences, such as those containing rDNA, provided herein are preferred for use in artificial chromosomes generated by assembly of component elements *in vitro.* The rDNA provides an origin of replication and also provides sequences that facilitate amplification of the artificial chromosome *in vivo* to increase the size of the chromosome to, for example, accommodate increasing copies of a heterologous gene of interest as well as continuous high levels of expression of the heterologous genes.

### d. Filler heterochromatin

Filler heterochromatin, particularly satellite DNA, is included to maintain structural integrity and stability of the artificial chromosome and provide a structural base for carrying genes within the chromosome. The satellite DNA is typically A/T-rich DNA sequence, such as mouse major satellite DNA, or G/C-rich DNA sequence, such as hamster natural satellite DNA. Sources of such DNA include any eukaryotic organisms that carry non-coding satellite DNA with sufficient A/T or G/C composition to promote ready separation by sequence, such as by FACS, or by density gradients. Examples of plant satellite DNA include, but are not limited to, satellite DNA of soybean (see, e.g., Morgante et al. (1997) Chromosome Res. 5:363-373; and Vahedian et al. (1995) Plant Mol. Biol. 29:857-862), satellite DNA on the rye B chromosome (see, e.g., Langdon et al. (2000) Genetics 154:869-884) and satellite DNA in the Saccharum complex (see, e.g., Alix et al. (1998) Genome 41:854-864). The satellite DNA may also be synthesized by generating sequence containing monotone, tandem repeats of highly A/T- or G/C-rich DNA units.

The most suitable amount of filler heterochromatin for use in construction of the artificial chromosome may be empirically determined by, for example, including segments of various lengths, increasing in size, in the construction process. Fragments that are too small to be suitable for use will not provide for a functional chromosome, which may be evaluated in cell-based expression studies, or will result in a chromosome of limited functional lifetime or mitotic and structural stability.

### e. Selectable marker

Any convenient selectable marker, including specific examples described herein, may be used and at any convenient locus in the expression system.

### 2. Combination of the isolated chromosomal elements

Once the isolated elements are obtained, they may be combined to generate the complete, functional artificial chromosome expression system. This assembly can be accomplished for example, by *in vitro* ligation either in solution, LMP agarose or on microbeads. The ligation is conducted so that one end of the centromere is directly joined to a telomere. The other end of the centromere, which serves as the gene-carrying chromosome arm, is built up from a combination of satellite DNA and megareplicator sequences, e.g., rDNA sequence, and may also contain a selectable marker gene. Another telomere is joined to the end of the gene-carrying chromosome arm. The gene-carrying arm is the site at which any heterologous genes of interest, for example, in expression of desired proteins encoded thereby, are incorporated either during *in vitro* assembly of the chromosome or sometime thereafter.

### 3. Analysis and testing of the artificial chromosome expression systems

Artificial chromosomes assembled *in vitro* may be tested for functionality in cell systems, such as plant and animal cells, using any of the methods described herein for the artificial chromosomes, minichromosomes, or known to those of skill in the art.

### 4. Introduction of desired heterologous DNA into the in vitro assembled chromosome

Heterologous DNA may be introduced into the *in vitro* synthesized chromosome using routine methods of molecular biology, may be introduced using the methods described herein for the artificial chromosomes, or may be incorporated into the *in vitro* assembled chromosome as part of one of the synthetic elements, such as the heterochromatin. The heterologous DNA may be linked to a selected repeated fragment, and then the resulting construct may be amplified *in vitro* using the methods for such *in vitro* amplification provided herein.

In a particular embodiment of these *in vitro* assembly methods, a site-specific recombination site is included in the assembly DNA or is added into the assembled chromosome, such as a plant *in vitro* assemble artificial chromosome, after initial assembly. The presence of a recombination site in the *in vitro* assembled artificial chromosome facilitates recombinase-catalyzed introduction of heterologous nucleic acid into the chromosome if the heterologous nucleic acid also contains a complementary recombination site. Such recombination systems include, but are not limited to, Cre//ox [see, e.g., Dale and Ow (1995) Gene 91:79-851, FLP/FRT [see, e.g., Nigel et al. (1995) The Plant Journal 8:637-652], R/RS [see, e.g., Onouchi et al. (1991) Nuc. Acids Res. 19:6373-6378], Gin/*gix* [see, *e.g.,* Maeser and Kahman (1991) Mol. Gen. Genet. 230:170-176] and int/*att*. The introduction *of att* recombination sites into a chromosome and the use of lambda phage integrase recombinase in conjunction therewith to permit engineering of natural and artificial chromosomes is desribed in copending U.S. provisional application Serial No. 60/294,758, by Perkins *et a*/*.* entitled "CHROMOSOME-BASED PLATFORMS" filed on May 30, 2001, U.S. provisional application Serial No. 60/366,891, by Perkins et al. entitled "CHROMOSOME-BASED PLATFORMS" filed on March 21, 2002, U.S. patent application Serial No. ___, by Perkins *et al.* entitled "CHROMOSOME-BASED PLATFORMS" filed on May 30, 2002, under attorney docket no. 24601-420, and PCT International Application No. ___, by Perkins *et al.* entitled "CHROMOSOME-BASED PLATFORMS" filed on May 30, 2002, under attorney docket no. 24601-420PC, each of which is incorporated herein in its entirety by reference thereto. Thus, also contemplated herein are *in vitro* assembled artificial chromosomes, in particular such chromosomes containing plant chromosome-derived components, that contain one or more recombination sites, such as an *att* site.

### E. Methods for the Production of Plant Acrocentric Chromosomes and Plant Chromosomes Containing Adjacent Regions of rDNA and Heterochromatin

Acrocentric human and mouse chromosomes in which the short arm contains only pericentric heterochromatin, an rDNA array, and telomeres can be used in the *de novo* formation of a satellite DNA based artificial chromosome (SATAC, also referred to as ACes). ln some embodiments of the methods of producing a plant artificial chromosome provided herein, it may be desirable to introduce heterologous nucleic acids into a plant chromosome with arms of unequal length (e.g., into the short arm of an acrocentric chromosome) and/or containing adjacent regions of rDNA and heterochromatin, such as pericentric heterochromatin or satellite DNA. Of particular interest in such methods are plant acrocentric chromosomes that contain rDNA located adjacent to the pericentric heterochromatin or satellite DNA, and, in particular, on the short arm of the chromosome with little to no euchromatic DNA between the rDNA and the pericentric heterochromatin. Utilizing such structures as the initial composition in the generation of plant artificial chromosomes may facilitate generation of plant artificial chromosomes that are predominantly heterochromatic. For example, introduction of heterologous nucleic acid into a cell containing such an acrocentric plant chromosome such that the nucleic acid integrates into the pericentric heterochromatin and/or rDNA of the short arm of the chromosome may be associated with amplification (possibly through "megareplicator" DNA sequences such as may reside in plant rDNA arrays, also known as the nucleolar organizing regions (NOR)) of heterochromatin that leads to the formation of a predominantly heterochromatic plant artificial chromosome.

Naturally occurring acrocentric plant chromosomes are limited in number, and plant chromosomes with a structure that includes adjacent regions of heterochromatin and rDNA may not exist or may not exist for a variety of plant species. Provided herein are methods for generating acrocentric plant chromosomes and plant chromosomes containing adjacent regions of rDNA and heterochromatin, in particular, pericentric and/or satellite heterochromatin. Further provided herein are methods for generating acrocentric plant chromosomes containing adjacent regions of heterochromatin, such as pericentric heterochromatin and/or satellite DNA, and rDNA on the short arm of the chromosome.

Also provided herein are plant acrocentric chromosomes in which the nucleic acid of one or both arms of the chromosome contains less than about 50%, or less than about 40%, or less than about 30%, or less than about 20%, or less than about 10%, or less than about 5%, or less than about 2%, or less than about 1 %, or less than about 0.5% or less than about 0.1 % euchromatin. In some embodiments of these chromosomes, the nucleic acid of only one arm, either the short arm or the long arm, contains less than these specified amounts of euchromatin. ln a particular embodiment of these chromosomes, the nucleic acid of the short arm contains less these specified amounts of euchromatin.

Further provided herein are plant chromosomes containing adjacent regions of heterochromatin, in particular pericentric heterochromatin or satellite DNA, and rDNA with little to no euchromatin between the two regions. With reference to such plant chromosomes, "litte to no" means that the amount of euchromatic DNA, if any, located between the rDNA and heterochromatin (such as pericentric heterochromatin and/or satellite DNA), generally does not stain diffusely and recognizably as euchromatin and/or does not contain protein-encoding genes. Thus, in these chromosomes, between the heterochromatin (such as pericentric heterochromatin and/or satellite DNA) and the rDNA, there is substantially no chromatin that is less condensed than the heterochromatin (e.g., pericentric heterochromatin). The plant chromosomes containing adjacent regions of rDNA and heterochromatin (such as pericentric heterochromatin) provided herein may be acrocentric chromosomes. In a particular embodiment of these plant chromosomes, the adjacent regions of rDNA and heterochromatin, in particular pericentric heterochromatin, are contained on the short arm of the chromosome.

Further provided are methods of utilizing such plant chromosomes in the generation of plant artificial chromosomes, and, in particular, predominantly heterochromatic plant artificial chromosomes, such as ACes (also referred to as SATACs). In particular methods of producing plant artificial chromosomes provided herein, nucleic acids are introduced into a cell containing a plant chromosome that is acrocentric and/or contains adjacent regions of rDNA and heterochromatin, such as pericentric heterochromatin, the cells are cultured through at least one cell division and a cell comprising an artificial chromosome, such as a predominantly heterochromatic artificial chromosome, is selected. In these methods, the plant chromosome into which nucleic acid is introduced may be an acrocentric chromosome containing adjacent regions of rDNA and heterochromatin on the short or long arm, and, in particular, on the short arm.

The plant chromosomes provided herein can be generated using site-specific recombination between plant chromosome regions. The regions may be on the same chromosome or separate chromosomes. Through site-specific recombination, sections of plant chromosomes may be altered to remove, invert and/or insert sequences such that a desired plant chromosome results. The resulting plant chromosome is acrocentric and/or contains adjacent regions of heterochromatic DNA and rDNA, which may or may not be on the short arm of an acrocentric chromosome. Thus, the starting chromosome in these methods may be a plant chromosome or may be a plant acrocentric chromosome that does not contain adjacent regions of rDNA and heterochromatin, such as pericentric heterochromatin or satellite DNA. If the starting chromosome is acrocentric, then it may be used in the generation of a plant acrocentric chromosome that contains adjacent regions of heterochromatic DNA (e.g., pericentric heterochromatin and/or satellite DNA) and rDNA, particularly on the short arm of the chromosome, or to generate a plant acrocentric chromosome in which the nucleic acid of one or both arms contains less than about 50%, or less than about 40%, or less than about 30%, or less than about 20%, or less than about 10%, or less than about 5%, or less than about 2%, or less than about 1 %, or less than about 0.5% or less than about 0.1 % euchromatin.

ln one of the methods provided herein for producing a plant chromosome that is acrocentric and/or contains adjacent regions of rDNA and heterochromatin, nucleic acid containing a site-specific recombination site and nucleic acid containing a complementary site-specific recombination site are introduced into a cell containing one or more plant chromosomes. The nucleic acids may be introduced into the cell sequentially or simultaneously. The nucleic acids may also be targeted to particular chromosomes and/or particular sequences of a chromosome. Such targeting may be accomplished by including in the nucleic acids sequences homologous to particular sequences in the chromosome(s).

The cell is then exposed to a recombinase activity. The recombinase activity can be provided by introduction of nucleic acid encoding the activity into the cell for expression of the activity therein, or may be added to the cell from an exogenous source. The recombinase activity is one that catalyzes recombination between sequences at the two recombination sites. An appropriate recombination event produces a plant chromosome that is acrocentric and/or contains adjacent regions of rDNA and heterochromatin (such as pericentric heterochromatin and/or satellite DNA) which may be readily identified therein based on its particular structure (e.g., arms of unequal length if the chromosome is acrocentric) and/or other features, e.g., the presence of particular added sequences, such as recombination sites and DNA encoding a selectable marker, the absence of particular sequences, such as excised euchromatic DNA, and the arrangement of sequences, such as the placement of rDNA segments adjacent to pericentric heterochromatin and/or satellite DNA. Such attributes may be detected using techniques known in the art for the analysis of nucleic acids and chromosomes, such as, for example, *in situ* hybridization.

A number of site-specific recombination systems may be used in the production of plant chromosomes that are acrocentric and/or contain rDNA adjacent to heterochromatin, such as pericentric heterochromatin, as described herein. Such systems include, but are not limited to, Cre//ox [see, e.g., Dale and Ow (1995) Gene 91:79-85], FLP/*FRT* [see, e.g., Nigel et a/. (1995) The Plant Journal 8:637-652], R/RS [see, e.g., Onouchi et al. (1991) Nuc. Acids Res. 19:6373-6378], Gin/*gix* [see, e.g., Maeser and Kahman (1991) Mol. Gen. Genet. 230:170-176] and int/att. The introduction *of att* recombination sites into a chromosome and the use of lambda phage integrase recombinase in conjunction therewith to permit engineering of natural chromosomes is desribed in copending U.S. provisional application Serial No. 60/294,758 by Perkins et al*,* entitled "CHROMOSOME-BASED PLATFORMS" filed on May 30, 2001, U.S. provisional application Serial No. 60/366,891, by Perkins et al. entitled "CHROMOSOME-BASED PLATFORMS" filed on March 21, 2002, U.S. patent application Serial No. ___, by Perkins *et al.*entitled "CHROMOSOME-BASED PLATFORMS" filed on May 30, 2002, under attorney docket no. 24601-420, and PCT International Application No. ___,by Perkins *et al.*entitled "CHROMOSOME-BASED PLATFORMS" filed on May 30, 2002, under attorney docket no. 24601-420PC, each of which is incorporated herein in its entirety by reference thereto. These systems, as well as others known in the art, can be used to specifically excise or invert DNA (for example, in an intrachromosomal recombination), exchange regions of DNA (for example, in an inter-chromosomal recombination) or insert DNA (for example, through recombination between homologous sequences at a recombination site and the DNA to be inserted). The precise event is controlled by the orientation of the recombination site DNA sequences.

In particular embodiments of the methods for producing an acrocentric plant chromosome provided herein, nucleic acid containing complementary recombinase recognition sites for site-specific recombination is introduced into a cell containing one or more plant chromosomes wherein one of the sites integrates into, or close to, the pericentric heterochromatin and/or satellite DNA (in particular, proximal satellite DNA) of one plant chromosome in the cell. In a further embodiment, nucleic acid containing complementary recombinase recognition sites for site-specific recombination is introduced into a cell containing one or more plant chromosomes wherein one of the sites integrates into the distal end of an arm of a plant chromosome in the cell. In these embodiments, recombination between the sites in the presence of a recombinase that recognizes the sites can result in deletion of a portion of an arm of a chromosome, reciprocal translocation between a distal portion of a chromosome arm and a more proximal portion of another chromosome arm or reciprocal translocation between pericentric heterochromatin and/or satellite DNA of one chromosomal arm and a more distal portion of another chromosome arm. Each of these recombination events can serve to reduce the length of a chromosome arm and give rise to an acrocentric chromosome.

ln another embodiment, a nucleic acid containing a site-specific recombination site is introduced into a cell containing plant chromosomes wherein it integrates into the pericentric heterochromatin and/or satellite DNA of one plant chromosome in the cell and nucleic acid containing a complementary site-specific recombination site is introduced into the cell wherein it integrates into the distal end of an arm of another plant chromosome in the cell. ln this embodiment, recombination between the sites in the presence of a recombinase that recognizes the sites can result in reciprocal translocation between the pericentric heterochromatin and/or satellite DNA of one chromosome and the distal portion of another chromosome arm thereby bringing these two regions into close proximity on one chromosomal arm and reducing the amount of DNA between the pericentric region of the arm and the end of the arm to generate an acrocentric plant chromosome.

These methods for producing an acrocentric plant chromosome may also be conducted such that nucleic acid containing a site-specific recombination site is introduced into a cell containing a plant chromosome wherein it integrates into, or close to, the pericentric heterochromatin and/or satellite DNA of a plant chromosome in the cell and nucleic acid containing a complementary site-specific recombination site is introduced into the cell wherein it integrates into the distal end of the same arm of the same chromosome. In this embodiment, recombination between the sites in direct (i.e., the same, or head-to-tail) orientation in the presence of a recombinase that recognizes the sites can result in intrachromosomal recombination between the pericentric heterochromatin (and/or satellite DNA) and the distal portion of the chromosomal arm thereby excising DNA between these two regions and reducing the amount of DNA between them to generate an acrocentric plant chromosome.

In particular embodiments of the methods provided herein for producing a plant chromosome containing adjacent regions of rDNA and heterochromatin, such as pericentric heterochromatin and/or satellite DNA, nucleic acid containing complementary recombinase recognition sites for site-specific recombination is introduced into a cell containing one or more plant chromosomes wherein one of the sites integrates into heterochromatin of one plant chromosome in the cell. ln a further embodiment, nucleic acid containing complementary recombinase recognitions sites for site-specific recombination is introduced into a cell containing one or more plant chromosomes wherein one of the sites integrates into rDNA or a nucleolar organizing region (NOR) of a plant chromosome in the cell. In these embodiments, recombination between the sites in the presence of a recombinase that recognizes the sites can result in deletion of DNA between a heterochromatic region, such as the pericentric heterochromatin (and/or satellite DNA), and rDNA, inversion of DNA that includes heterochromatin or rDNA of a plant chromosome or reciprocal translocation between heterochromatin of one chromosomal arm and rDNA of another chromosomal arm. Each of these recombination events can serve to arrange chromosomal DNA such that a region of heterochromatic DNA, such as pericentric heterochromatin and/or satellite DNA, is adjacent to a region of rDNA on a plant chromosome.

In another embodiment, nucleic acid containing a site-specific recombination site is introduced into a cell containing plant chromosomes wherein it integrates into heterochromatin, such as, for example, pericentric heterochromatin and/or satellite DNA, of one plant chromosome in the cell and nucleic acid containing containing a complementary site-specific recombination site is introduced into the cell wherein it integrates into rDNA of another plant chromosome in the cell. In this embodiment, recombination between the sites can result in reciprocal translocation between the heterochromatin of one chromosome and the rDNA of another chromosome thereby bringing these two regions into close proximity on one plant chromosome with little to no euchromatin between them.

These methods for producing a plant chromosome containing adjacent regions of heterochromatic DNA and rDNA may also be conducted such that nucleic acid containing site-specific recombination sites is introduced into a cell containing a plant chromosome wherein it integrates into heterochromatin, for example, pericentric heterochromatin and/or satellite DNA, of a plant chromosome and nucleic acid containing a complementary site-specific recombination site is introduced into the cell wherein it integrates into rDNA of the same chromosome. ln this embodiment, recombination between the sites in direct orientation in the presence of a recombinase that recognizes the sites can result in intrachromosomal recombination between heterochromatin, such as pericentric heterochromatin (and/or satellite DNA), and rDNA thereby excising DNA, including euchromatic DNA, between these two regions. Recombination of the sites in indirect (i.e., head-to-head) orientation in the presence of a recombinase can result in inversion of DNA between the sites thereby replacing DNA, such as euchromatin, located between pericentric heterochromatin (and/or satellite DNA) and rDNA on the chromosome with rDNA. Thus, in the resulting plant chromosome, rDNA is located adjacent to pericentric heterochromatin (and/or satellite DNA), and DNA that was present between the pericentric heterochromatin (and/or satellite DNA) and the rDNA is located distal to the rDNA in a position previously occupied by the rDNA.

In particular embodiments for producing an acrocentric plant chromosome containing adjacent regions of heterochromatin, such as pericentric heterochromatin (and/or satellite DNA), and rDNA, the short arm of the acrocentric chromosome may be generated in the same recombination event that places the heterochromatin and rDNA regions adjacent to each other or in a separate recombination event. For example, nucleic acid containing a site-specific recombination site may be introduced into a cell containing one or more plant chromosomes wherein it integrates into the pericentric heterochromatin of one plant chromosome and nucleic acid containing a complementary site-specific recombination site may be introduced into the cell wherein it integrates into rDNA that is located at a distal portion of another plant chromosome or the same arm of the same of the same chromosome. Recombination of the sites in the presence of a recombinase can result in intra- or inter-chromosomal recombination that not only brings the pericentric heterchromatin (and/or satellite DNA) and rDNA into close proximity on one chromosomal arm, but also sufficiently reduces the length of that arm such that the resulting chromosome is acrocentric.

If a single recombination event such as this does not generate an acrocentric plant chromosome, multiple recombination events may be used to produce an acrocentric plant chromosome containing adjacent regions of heterochromatic DNA and rDNA. For example, nucleic acid containing a site-specific recombination site may be introduced into a cell containing one or more plant chromosomes wherein it integrates into the pericentric heterochromatin (and/or satellite DNA) of one plant chromosome and nucleic acid containing a complementary site-specific recombination site may be introduced into the cell wherein it integrates into rDNA of the same or a different plant chromosome. As described abouve, recombination between the sites in the presence of a recombinase can result in deletion, inversion or reciprocal translocation of DNA to arrange chromosomal DNA such that pericentric heterochromatin (and/or satellite DNA) is adjacent to a region of rDNA on a plant chromosome. ln order to reduce the length of the arm of the chromosome on which the adjacent regions of heterochromatin and rDNA are located, an additional recombination event can be induced by introducing nucleic acid containing a site-specific recombination site into a cell containing this plant chromosome wherein it integrates into a region of the chromosome distal to the rDNA and nucleic acid containing a complementary site-specific recombination site into the cell wherein it integrates into the distal end of the same chromosome arm or of another plant chromosome arm. Recombination between the recognition sites can result in deletion or reciprocal translocation of DNA to reduce the length of the chromosome arm distal to the rDNA and give rise to an acrocentric plant chromosome containing adjacent regions of heterochromatin and rDNA on the short arm of the chromosome.

In each of the aforementioned methods for producing a plant chromosome that is acrocentric and/or contains adjacent regions of heterochromatin and rDNA, the nucleic acid containing the two or more recombination sites may be introduced simultaneously or sequentially into a cell or cells using nucleic acid transfer methods described herein or known in the art. The nucleic acids may randomly integrate into plant chromosomes or may be targeted for integration into a particular region or site on a plant chromosome through homologous recombination between sequences in the nucleic acid and sequences within the chromosome. The recombinase activity may be provided by introduction of nucleic acid encoding an appropriate recombinase into the cell for expression therein. The recombinase-encoding nucleic acid may be introduced into the cell prior to, during or after introduction of nucleic acids encoding recombination sites.

To facilitate identification of cells containing the transferred nucleic acids and/or in which a recombination event has occurred, nucleic acid encoding a selectable marker may be introduced into the cell. For example, one or both of the nucleic acids containing a recombination site may also contain DNA encoding a selectable marker (e.g., a resistance-encoding marker or a reporter molecule) operatively linked to a promoter which is oriented such that integration of the nucleic acid into a chromosome places the marker DNA between two directly oriented recombination sites on an arm of a chromosome. A cell containing the nucleic acid will thus be resistant to a selection agent or will detectably express a reporter molecule. Exposure of the cell to the appropriate recombinase can result in a recombination event that excises the DNA between the two recombination sites, which includes DNA encoding the selectable marker. Thus, recombination could be detected as loss of reporter molecule expression or decreased resistance to a selection agent. After exposure to a recombinase, the cells into which nucleic acids containing recombination sites have been transferred may be analyzed for the presence of acrocentric plant chromosomes using, for example, FISH analysis and other chromosome visualization techniques.

In another method provided herein for producing a plant chromosome that is acrocentric and/or contains adjacent regions of heterchromatin and rDNA, the recombination event or events that lead to formation of the chromosome occur through crossing of transgenic plants that contain chromosomes which contain complementary site-specific recombination sites. Thus, in one embodiment of these methods, nucleic acid containing a recombination site adjacent to nucleic acid encoding a selectable marker is introduced into a first plant cell and a first transgenic plant is generated from the first plant cell. Nucleic acid containing a promoter functional in a plant cell, a recombination site and a recombinase coding region in operative linkage is introduced into a second plant cell from which a second transgenic plant is generated. The first and second transgenic plants are crossed to obtain one or more plants resistant to an agent that selects for cells containing the nucleic acid encoding the selectable marker, and a resistant plant that contains cells comprising a plant chromosome that is acrocentric and/or contains adjacent regions of heterochromatin and rDNA is selected.

In an example of this method, nucleic acids containing site-specific recombination sites are introduced into cells of *Nicotiana tabacum.* The nucleic acids are introduced separately by infecting leaf explants with *Agrobacterium tumefaciens* which carries the kanamycin-resistance gene (Kan^{R}). Kanamycin-resistant transgenic plants are generated from the infected leaf explants. One transgenic plant contains nucleic acid encoding a promoterless hygromycin-resistance gene preceded by a *lox-*site specific recombination sequence *(lox-hpt),* the other plant contains a cauliflower mosaic virus 35S promoter linked to a *lox* sequence and the *cre* DNA recombinase coding region *(*35S*-lox-cre).* The resultant Kan^{R} transgenic plants are crossed (see, e.g., protocols of Qin et al. (1994) Proc. Natl. Acad. Sci. U.S.A. 91:1706-1710, 1994). Plants in which the appropriate DNA recombination event has occurred are identified by hygromycin-resistance.

The Kan^{R} cultivars initially may be screened, such as by FISH, to identify two sets of candidate transgenic plants. One set has one construct integrated in regions adjacent to the pericentric heterochromatin (and/or satellite DNA) on the short arm of any chromosome. The second set of candidate plants has the other construct integrated in rDNA, such as the NOR region, of appropriate chromosomes. To obtain reciprocal translocation both sites must be in the same orientation. Therefore a series of crosses may be required, marker-resistant plants generated, and FISH analyses performed to identify an "acrocentric" plant chromosome or chromosomes that contain adjacent regions of heterochromatin. As described above, such an acrocentric chromosome may be used for *de novo* plant artificial chromosome formation, particularly predominantly heterochromatic plant artificial chromosomes. The selection of appropriate plant lines can be done, for example, using marker-assisted selection.

### F. Incorporation of Heterologous Nucleic Acids into Artificial Chromosomes

Heterologous nucleic acids can be introduced into artificial chromosomes during or after formation. Incorporation of particular desired nucleic acids into an artificial chromosome during generation thereof may be accomplished by including the desired nucleic acids along with the nucleic acid encoding a selectable marker and any other nucleic acids used in artificial chromosome generation (e.g., targeting sequences that direct the heterologous nucleic acid to the pericentric region of a chromosome) in the transformation of a cell to initiate amplification and formation of a artificial chromosomes.

Alternatively, heterologous nucleic acids may be incorporated into an artificial chromosome following formation thereof through transfection of a cell containing the artificial chromosome with the heterologous nucleic acids. In general, incorporation of such nucleic acids into the artificial chromosome is assured through site-directed integration, such as may be accomplished by including nucleic acids homologous or identical to DNA contained within the artificial chromosome in with the heterologous nucleic acid when transferring it to the artificial chromosome. An additional selective marker gene may also be included.

Additionally, introduction of nucleic acids, particularly DNA molecules to an artificial chromosome can be accomplished by the use of site-specific recombinases as described herein (see, also, copending U.S. provisional application Serial No. 60/294,758 by Perkins *et a*/*.* entitled "CHROMOSOME-BASED PLATFORMS" filed on May 30, 2001, U.S. provisional application Serial No. 60/366,891, by Perkins et al. entitled "CHROMOSOME-BASED PLATFORMS" filed on March 21, 2002, U.S. patent application Serial No. ___, by Perkins *et al.* entitled "CHROMOSOME-BASED PLATFORMS" filed on May 30, 2002, under attorney docket no. 24601-420, and PCT International Application No. ___, by Perkins *et a*/*.* entitled "CHROMOSOME-BASED PLATFORMS" filed on May 30, 2002, under attorney docket no. 24601-420PC; each of which is incorporated in its entirety by reference thereto). Artificial chromosomes can be produced containing recombinase recognition sequences, to allow the site-specific introduction of DNA molecules into the same. Another use for an introduced recombinase site is to provide a region for site-specific integration of a new trait by the use of recombinase mediated gene insertion.

### G. Introduction of Artificial Chromosomes into Plant Cells and Recovery of Plants Containing Artificial Chromosomes

Artificial chromosomes can be introduced into plant cells by a variety of methods familiar to those skilled in the art. These methods include chemical and physical methods for introduction of foreign DNA, as well as cell culture methods to transfer chromosomes from one cell to another cell.

Any type of artificial chromosome can be used. Plant artificial chromosomes (PACs) can be prepared by the *in vivo* and *in vitro* methods described herein. PACs can be prepared inside plant protoplasts and then transferred to other plant species and tissues, in particular to other plant protoplasts, via fusion in the presence or absence of PEG as described herein (Draper et al. (1982) Plant Cell Physiol. 23:451-458; Krens et al. (1982) Nature 72-74). PACs can be isolated from the protoplasts in which they were prepared, encapsulated into liposomes, and delivered to other plant protoplasts (Deshayes et al. (1985) EMBO J. 4:2731-2737). Alternatively, the PACs can be isolated and delivered directly to plant protoplasts, plant cells, or other plant targets via a PEG-mediated process, calcium phosphate-mediated process, electroporation, microinjection, (particle bombardment), lipid-mediated method with or without sonoporation, sonoporation alone, or any method known in the art as described herein (Haim et al. (1985) Mol. Gen. Genet. 199:161-168; Fromm et al. (1986) Nature 319:791-793; Fromm et al. (1985) Proc. Nat. Acad. Sci. USA 82:5824-5828; Klein et al. (1987) Nature 327:70; Klein et al. (1988) Proc. Nat. Acad. Sci. USA 85:8502-8505; and International PCT application publication no. WO 91/00358), Plant artificial chromosomes can also be transferred to other plant species by preparation of protoplast-derived plant microcells, and fusion of the microcells containing the plant artificial chromosome with plant cells of other plant species.

Mammalian artificial chromosomes (MACs) can be transferred to plant cells. Mammalian artificial chromosomes are prepared by the *in vivo* and *in vitro* methods described in US Patent Nos. 6,025,155 and 6,077,697, and International PCT application No. WO 97/40183. MACs can be prepared as microcells, and the microcells can be fused with plant protoplasts in the presence or absence of PEG (Dudits et al. (1976) Hereditas 82:121-123; Wiegland et al. (1987) J. Cell. Sci. Pt. 2 145-149). Alternatively, the MACs can be isolated and delivered directly to plant cells, protoplasts, and other plant targets using a PEG-mediated process, calcium phosphate-mediated process, electroporation, microinjection, lipid-mediated method with or without sonoporation, sonoporation alone, or any method known in the art as described herein and in US Patent Nos. 6,025,155 and 6,077,697, and International PCT application publication No. WO 97/40183.

After PACs or MACs are introduced into plant targets and the plant targets are grown and analyzed for transfection, the plant transformed plant targets can be developed using standard conditions into roots, shoots, plantlets, or any structure capable of growing into a plant.

Accordingly, methods for the introduction of artificial chromosomes represent the first step in the production of plant cells and whole plants containing artificial chromosomes from a variety of sources.

The ability to introduce genes into plants, such that they are stably expressed and transmissible from generation to generation, has revolutionized plant biology and opens up new possibilities for using plants as green factories for the production of commercially useful products as well as for other applications described herein. There are several approaches to the generation of stably transformed plants, and the adopted approach varies according to the aims of the project. For introduction of artificial chromosomes into plants, a variety of methods may be employed. transgenic plants, the transformation process involves the methods of foreign DNA delivery to plant host cells, the growth and analysis of transformed plant host cells, and the generation and regeneration of transgenic plants from transformed plant host cells.

### 1. Introduction of artificial chromosomes into plant host cells

Numerous methods for producing or developing transgenic plants are available to those of skill in the art. The method used is primarily a function of the species of plant. Artificial chromosomes containing heterologous DNA, such as artificial chromosomes prepared by the methods described herein, can be introduced into plant host cells, including, but not limited to, plant cells and protoplasts, by, for example, non-vector mediated DNA transfer processes (see, also copending U.S. application Serial No. 09/815,979, which describes methods for delivery that can be adapted for use with plant cells and used with plant protoplasts).

Non-vector mediated, or direct, gene transfer systems involve the introduction of heterologous DNA, in particular artificial chromosomes, into host cells, including but not limited to plant cells and protoplasts, without the use of a biological vector. The artificial chromosome that is introduced into these plant host cells can lead to the development of transformed, regenerable transgenic plants. The direct gene transfer systems for transgenic plants are designed to overcome the barrier to DNA uptake caused by the cell wall and the plasma membrane of plant cells. The approaches for direct gene transfer include, but are not limited to, chemical, electrical, and physical methods, which can also be adapted to optimize transfer of artificial chromosomes (see, e.g.,Uchimiya et al.(1989) J. of Biotech. 12: 1-20 for a review of such procedures, see also, e.g.,U.S. Patent Nos. 5,436,392; 5,489,520; Potrykus et al. (1985) Mol. Gen. Genet. 199:183*;* Lorz et al. (1985) Mol. Gen. Genet. 199:178*;* Fromm et al. (1985) Proc. Natl. Acad. Sci. U.S.A. 82:5824-5828; Uchimiya et al. (1986) Mol. Gen. Genet. 204:204; Callis et al. (1987) Genes Dev. 1: 1183-2000; Callis et a/. (1987) Nuc. Acids Res. 15:5823-5831 ; Marcotte et al. (1988) Nature 355:454 and Toriyama et al. (1988) Bio/Technology 6:1072-1074).

### a. Chemical methods

Uptake of artificial chromosomes into plant cells, such as protoplasts, can be accomplished in the absence or presence of polyethylene glycol (PEG), which is a fusogen, or by any variations of such methods known to those of skill in the art [see, e.g.,U.S. Patent No. 4,684,611 to Schilperoot et al.; Paskowski et al. (1984) EMBO J. 3:2717-2722; U.S. Patent Nos. 5,231,019 and 5,453,367]. ln one approach, plant protoplasts are incubated with a solution of foreign DNA, in particular artificial chromosomes, and PEG at a concentration that allows for high cell survival and high efficiency chromosome uptake. The protoplasts are then washed and cultured [Datta and Datta (1999) Meth. in Molecular Biol. 111:335-348]. In an alternative approach, plant protoplasts are incubated with artificial chromosomes in the presence of calcium phosphate for direct artificial chromosome uptake (Haim et al. (1985) Mol. Gen. Genet.199:161-168). Alternatively, the artificial chromosome, in particular plant artificial chromosome (PAC), is formed in a plant protoplast which is, in turn, fused with another plant protoplast in the presence or absence of PEG to transfer the PAC to the plant host protoplast. Such methods for treating protoplasts with PEG and foreign DNA are well known in the art (Draper et al. (1982) Plant Cell Physiol. 23:451-458; Krens et al. (1982) Nature 72-74).

Another chemical direct gene transfer method involves lipid-mediated delivery of artificial chromosomes to plant protoplasts. In this process, liposomes with encapsulated artificial chromosomes are allowed to fuse with protoplasts alone or in the presence of PEG as the fusogen to transfer the foreign DNA, in particular artificial chromosome, to the plant host protoplast (Deshayes et al. (1985) EMBO J. 4:2731-2737; Fraley and Paphadjopoulos (1982) Curr Top Microbiol lmmunol 96:171-191).

Another direct gene transfer method involves the use of microcells. The chromosomes can be transferred by preparing microcells containing artificial chromosomes and then fusing the microcells with plant protoplasts. Methods for the preparation and fusion of microcells with other cells are well known in the art (see Example No. 4 and see also, e.g., U.S. Patent Nos. 5,240,840; 4,806,476;5,298,429; 5,396,767; Fournier (1981) Proc. Natl. Acad. Sci. U.S.A. 78:6349-6353; and Lambert et al.(1991) Proc. Natl. Acad. Sci. U.S.A. 88:5907-59; Dudits et al. (1976) Hereditas 82:121-123; Wiegland et al. (1987) J. Cell. Sci. Pt. 2 145-149).

### b. Electrical methods

Electroporation, which involves high-voltage electrical pulses to a solution containing a mixture of protoplasts or plant cells and foreign DNA, in particular artificial chromosomes, to create nanometer-sized, reversible pores, is a common method to introduce DNA into plant cells or protoplasts. The exogenous DNA may be added to the protoplasts in any form such as, for example, naked linear, circular or supercoiled DNA, artificial chromosomes encapsulated in liposomes, DNA in spheroplasts, artificial chromosomes in other plant protoplasts, artificial chromosomes complexed with salts, and other methods. The foreign DNA, in particular artificial chromosome, can also include a phenotypic marker to identify plant cells that are successfully transformed.

When plant cells or protoplasts are subjected to short electrical DC (direct current) pulses, they may experience an increase in the permeability of the plasma membrane and/or cell wall to hydrophilic molecules such as nucleic acids, which are normally unable to enter the plant cell directly. Nucleic acids are taken directly into the cell cytoplasm either through these pores or as a consequence of the redistribution of membrane components that accompanies closure of the pores. Certain cell wall-degrading enzymes, such as pectin-degrading enzymes, may be employed to render the plant target recipient cells more susceptible to DNA or artificial chromosome uptake by electroporation than untreated cells. Plant recipient cells may also be susceptible to transformation by mechanical wounding. To effect transformation by electroporation, friable tissues such as a suspension culture of cells or embryonic callus may be used or immature embryos or other organized tissues may be directly transformed (see, e.g., Fromm et al. (1986) Nature 319:791-793). Methods for effecting electroporation are well known in the art (see, e.g.,U.S. Patent Nos. 4,784,737; 4,970,154; 5,304,486; 5,501,967; 5,501,662; 5,019,034; 5,503,999; see, also Fromm et al.(1985) Proc. Natl. Acad. Sci. U.S.A. 82:5824-5828; Zimmerman et al. (1981) Biophys Biochem Acta 641:160-165; Neuman et al. (1982) EMBO J. 1:841-845; Riggs et al. (1986) Proc. Nat. Acad. Sci. USA 83:5602-5606; Lurquin (1997) Mol. Biotechnol. 7:5-35; Bates (1999) Methods in Molecular Biology 111:359-366). Electroporation can be used to introduce nucleic acids into tobacco mesophyll cells (Morikawa et al. (1986) Gene 41:121-124; leaf bases of rice (Dekeyser et al. (1990) Plant Cell 2:591-602; immature maize embryos (Songstad et al. (1993) Plant Cell Tiss. Orgn. Cult. 40:1-15; macerated immature maize embryos (D'Halluin et al. (1992) Plant Cell 4:1495-1505; suspension cultured maize cells (Laursen et al. (1994) Plant Mol. Biol. 24: 51-61; and sugar cane (Arencibia et al. (1995) Plant Cell Rep. 14:305-309).

Artificial chromosomes may be delivered to plant cells, in particular plant seeds, by the use of electroporation and pollen to derive pollen comprising an artificial chromosome. Methods that may be used for delivery of artificial chromosomes into pollen include, for example, techniques described in U.S. Patent No. 5,049,500 and by Negrutiu et al. [in Biotechnology and Ecology of Pollen, Mulcahy et al. eds., (1986) Springer Verlag, N.Y., pp. 65-69] and Fromm et al. [(1986) Nature 319:791; including methods for introducing DNA into mature pollen using various procedures such as heat shock, PEG and electroporation]. The pollen is capable of germinating and fertilizing an egg cell, leading to the formation of a plant seed comprising an artificial chromosome.

### c. Physical methods

The physical methods approach for introducing foreign DNA, in particular artificial chromosomes , into plant cells overcomes the cell wall barrier to DNA movement. Physical, or mechanical means, are used to introduce transgenes directly into protoplasts or plant cells and include, but are not limited to, microinjection, particle bombardment, and sonoporation.

### (1) Microinjection

Microinjection involves the mechanical injection of heterologous DNA, in particular artificial chromosomes, into plant cells, including cultured cells and cells in intact plant organs and embryoids in tissue culture via very small micropipettes, needles, or syringes (Neuhaus et al. (1987) Theor. Appl Genet. 75:30-36; Reich et al. (1986) Can. J. Bot. 64:1255-1258; Crossway et al. (1986) BioTechniques 4:320-334; Crossway et al. (1986) Mol. Gen. Genet. 20:179; U.S. Patent No. 4,743,548; silicon carbide whiskers (Kaeppler et al. (1990) Plant Cell Rep. 9:415-418; Frame *et al.* (1994). For example, microinjection of protoplast cells with foreign DNA for transformation of plant cells has been reported for barley and tobacco (see, *e.g.,* Holm et al. (2000) Transgenic Res. 9:21-32 and Schnorf et al. Transgenic Res. 1:23-30). Single artificial chromosomes may be front-loaded into microinjection needles and then injected into cells ("pick-and-inject") following procedures as described by Co et al. [(2000) Chromosome Res. 8:183-191].

### (2) Particle bombardment

Microprojectile bombardment (acceleration of small high density particles, which contain the DNA, to high velocity with a particle gun apparatus, which forces the particles to penetrate plant cell walls and membranes)have also been used to introduce heterologous DNA into plant cells. Microprojectile bombardment techniques for the introduction of nucleic acids into plant cells, in addition to being an effective means of reproducibly stably transforming plant cells, particularly monocots, do not require isolation of protoplasts or susceptibility of the host cell to *Agrobacterium* infection. In these methods, nucleic acids are carried through the cell wall and into the cytoplasm on the surface of small, typically metal, particles (see, *e.g.,* Klein et al. (1987) Nature 327:70; Klein et al. (1988) Proc. Natl. Acad. Sci. U.S.A. 85:8502-8505, Klein et a/. in Progress in Plant Cellular and Molecular Biology, eds. Nijkamp, H.J.J., Van der Plas, J.H.W., and Van Aartrijk, J., Kluwer Academic Publishers, Dordrecht, (1988), p. 56-66 and McCabe et al. (1988) Bio/Technology 6:923-926; Sautter et al. (1991) Biol. Technol. 9:1080-1085; Gordon-Kamm et al. (1990) Plant Cell 2:603-618; Finer et al. (1999) Curr. Top. Microbiol. lmmunol. 240:59-80; Vasil and Vasil (1999) Methods in Molecular Biology 111:349-358; Seki et al. (1999) Mo. Biotechnol. 11:251-255). Particles may be coated with nucleic acids and delivered into cells by a propelling force. Exemplary particles include those containing tungsten, gold or platinum, as well as magnesium sulfate crystals. The metal particles can penetrate through several layers of cells and thus allow the transformation of cells within tissue explants.

ln an illustrative embodiment (see, *e.g.,* U.S. Patent No. 6,023,013) of a method for delivering foreign nucleic acids into plant cells, *e.g.,* maize cells, by acceleration, a Biolistics Particle Delivery System may be used to propel particles coated with DNA or cells through a screen, such as a stainless steel or Nytex screen, onto a filter surface covered with plant *(e.g.,* corn) cells cultured in suspension. The screen disperses the particles so that they are not delivered to the recipient cells in large aggregates. The intervening screen between the projectile apparatus and the cells to be bombarded may reduce the size of projectile aggregates and may contribute to a higher frequency of transformation by reducing damage inflicted on the recipient cells by projectiles that are too large.

For the bombardment, cells in suspension may be concentrated on filters or solid culture medium. Alternatively, immature embryos or other plant target cells may be arranged on solid culture medium. The cells to be bombarded are typically positioned at an appropriate distance below the microprojectile stopping plate. If desired, one or more screens may also be positioned between the acceleration device and the cells to be bombarded.

The prebombardment culturing conditions and bombardment parameters may be optimized to yield the maximum numbers of stable transformants. Both the physical and biological parameters for bombardment are important in this technology. Physical factors include those that involve manipulating the DNA/microprojectile precipitate or those that affect the flight and velocity of either the macro- or microprojectiles. Biological factors include all steps involved in manipulation of cells before and immediately after bombardment, the osmotic adjustment of target cells to help alleviate the trauma associated with bombardment, and also the nature of the transforming nucleic acid, such as linearized DNA, intact supercoiled plasmids, or artificial chromosomes.

Physical parameters that may be adjusted include gap distance, flight distance, tissue distance and helium pressure. ln addition, transformation may be optimized by adjusting the osmotic state, tissue hydration and subculture stage or cell cycle of the recipient cells. Ballistic particle acceleration devices are available from Agracetus, Inc. (Madison, Wl) and BioRad (Hercules, CA).

Techniques for transformation of A188-derived maize line using particle bombardment are described in Gordon-Kamm et al. (1990) Plant Cell 2:603-618 and Fromm et at (1990) Biotechnology 8:833-839. Transformation of rice may also be accomplished via particle bombardment (see, e.g., Christou et al. (1991) Biotechnology 9:957-962). Particle bombardment may also be used to transform wheat (see, *e.g.,* Vasil et al. (1992) Biotechnology 10:667-674 for transformation of cells of type C long-term regenerable callus; and Weeks et al. (1993) Plant Physiol. 102:1077-1084 for transformation of wheat using particle bombardment of immature embryos and immature embryo-derived callus). The production of transgenic barley using bombardment methods is described, for example, by Koprek et al. (1996) Plant Sci. 119:79-91.

### (3) Sonoporation

Foreign DNA, in paticular artificial chromosomes, may be introduced into plant protoplasts using ultrasound treatment, in particular mild ultrasound treatment (10-100kHz), to create pores for DNA uptake (see e.g. lnternational PCT application publication no. WO 91/00358) or may be introduced into plant protoplasts via a sonoporation machine (lmaRx Pharmaceutical Corp., Tucson, AZ).

Alternatively, the delivery of artificial chromosomes into plant host cells is performed by any method described herein or well known in the art. For example, needle-like whiskers (US 5,302,523, 1994, US 5,464,765) have been used to delivery foreign DNA.

Suitable plant targets into which foreign DNA, in particular artificial chromosomes, is transferred include, but are not limited to, protoplasts, cell culture cells, cells in plant tissue, meristem cells, microspores, callus, pollen, pollen tubes, microspores, egg-cells, embryo-sacs, zygotes or embryos in different stages of development, seeds, seedlings, roots, stems, leaves, whole plants, algae, or any plant part capable of proliferation and regeneration of plants. (see, e.g., U.S. Patent Nos. 5,990,390; 6,037,526 and 5,990,390). The growth of the transformed plant targets described herein can done with tissue-culture or non-tissue culture methods, with the preferred methods being tissue culture methods.

All plant cells into which foreign DNA, in particular artificial chromosomes, are introduced and that is regenerated from the transformed cells are used directly for expressed purposes (e.g. herbicide resistance, insect/pest resistance, disease resistance, environmental/stress resistance, nutrient utilization, male sterility, improved nutritional content, production of chemicals or biologicals, non-protein expressing sequences, and preparation and screening of libraries) as described herein or are used to produce transformed whole plants for the applications and uses described herein. The particular protocol and means for the introduction of the artificial chromosome into the plant host is adapted or refined to suit the particular plant species or cultivar.

Chromosomes may be transferred to cells by microcell mediated chromosome transfer (MMCT) (Telenius et al., Chromosome Research 7:3-7, 1999; Ramulu et al., Methods in Molecular Biology 111: 227-242, 1999). ln general, donor plant cultures or donor mammalian cell cultures are incubated in media supplemented with reagents that inhibit DNA synthesis (e.g., hydroxy urea, aphidicolin) and/or reagents that inhibit attachment of chromosomes to the mitotic spindle (e.g.,colcemid, coichicines, amiprophos-methyl, cremart). The cell walls of plant cells are digested with enzymes (e.g., cellulase, maceroenzyme) producing protoplasts. Donor plant protoplasts or donor mammalian cells are loaded on a Percoll gradient in the presence of cytochalasin-B (which causes the cell cytoskeleton to depolymerize into monomer protein subunits) and centrifuged at 10⁵ × g. During centrifugation the metaphase chromosomes are extruded through the plasma membrane forming plant 'microprotoplasts' or mammalian 'microcells.' The microprotoplasts/microcells are filtered through nylon sieves of decreasing pore size (8-3 *µ*m) to isolate smaller ones that contain predominately 1 metaphase chromosome. The microprotoplasts/microcells are fused to recipient plant protoplasts or mammalian cells by polyethelene glycol (peg) treatment. The fusion mixture is cultured in appropriate media. If the chromosome of interest is expressing a selection marker gene the fusion mixtures may be cultured in appropriate media supplemented with the appropriate selection drug (e.g. hygromycin, kanamycin).

### 2. The growth of transformed plant host cells

In tissue culture methods, plant cells or protoplasts transformed by the chemical, physical, electrical methods described herein are grown, or cultured, under selective conditions. The selective markers are integrated into the heterologous DNA, in particular artificial chromosome, before its introduction to plant hosts or are integrated into the plant host after transfection. An additional marker can be used for double selection. Generally, the plant cells or protoplasts are grown for numerous generations, after which the transformed cells are identified.

The transformed cells are subjected to conditions known in the art for callus initiation. Tissue that develops during the initiation period is placed in a regeneration or selection medium where shoot and root development occur. The plantlets are analyzed for the determination of transformation (International PCT application publication no. WO 00/60061). In the case of maize, embryonic callus cultures are initiated from immature maize embryos, bombarded with genes, and transformed into plantlets by the methods described in International PCT application publication no. WO 00/60061. In tissue culture methods, Rice calli are transformed with DNA encoding insecticidal proteins CrylA(b) and CrylA(c) for insect resistance. Common tissue culture methods can also be used to transform tobacco and tomato (see, e.g., US Patent No. 6,136,320), embryogenic maize calli (US Pat. Nos. 5,508,468; 5,538,877; 5,538,880; 5,780,708; 6,013,863; 5,554,798; 5,990,390; and 5,484,956;) and other crop species, e.g., potato and tobacco (Sijmons et al. (1990) Bio/Technol 8:217-221; tobacco (Vanderkerckhove et al. (1989) Bio/Technol 7:929-932 and Owen and Pen eds. Transgenic Plants: A Production System for Industrial and Pharmaceutical Proteins, John Wiley & Sons, Chichester, 1996) and rice (Zhu et al. (1994) Plant Cell Tiss Org Cult 36:197-204).

### 3. Analysis of transformed plant host cells

Once foreign DNA, in particular artificial chromosomes, is introduced into plant hosts and the cells or protoplasts are grown and developed under the conditions described herein, the plant cells or protoplasts which were transformed with artificial chromosomes are identified. The plant cell, protoplast, callus, leaf disc, or other plant target are screened for the presence of artificial chromosomes by various methods well known in the art including, but not limited to, assays for the expression of reporter genes, PCR of the isolated plant chromosomes or DNA, electron microscopy, visualization methods, and in situ hybridization of chromosome painting probe as described herein. Moreover, cells treated with artificial chromosomes are isolated during metaphase using a mitotic arrest agent, such as colchicine, and the artificial chromosome are distinguished from endogenous chromosomes by fluorescence-activated cell sorting, size and density differences, or by any method well known in the art. Alternatively, when a selectable marker gene is transmitted with or as part of the artificial chromosome, selective agents are used to detect the expression of the selectable marker (International PCT application publication no. WO 00/60061; US Patent No. 6,136,320; Owen and Pen Eds. Transgenic Plants: A Production System for Industrial and Pharmaceutical Proteins). Enzymatic assays, immunological assays, bioassays, germination assays, or chemical assays are used to assess the phenotypic effects of artificial chromosomes such as insect or fungal resistance or any other expression of genes in artificial chromosomes (Cheng et al. (1998) 95:2767-2772; US Patent No. 6,126,320; International PCT application publication no. WO 00/60061; Owen and Pen eds. Transgenic Plants: A Production System for Industrial and Pharmaceutical Proteins, John Wiley & Sons, Chichester, 1996). The plant cells, protoplasts, or other plant hosts that are successfully transformed with artificial chromosomes are used directly to express the gene of interest or are used to generate transgenic plants.

Fluorescent in situ hybridization (FISH) may be used to screen for the transfer of artificial chromosomes into plant cells. Using DNA probes specfic for the artificial chromosome (e.g., mouse major satellite DNA probe for murine satellite DNA based artificial chromosomes; or a kanamycin, hygromycin or GUS gene DNA probe for a plant artificial chromosome carrying such a gene) standard FISH techniques for plant cells have been described (de Jong et al., Trends in Plant Science 4: 258-263, 1999).

ldU labeling can be used to determine the optimum conditions for chromosome transfer (microcells) or isolated artificial chromosomes. The incorporated ldU increases the fragility of the chromosome and will increase the probability of cellular mutation. Hence, the cells are fixed within 48-hours after transfection/fusion and analyzed for chromosome uptake using various procedures. Once the optimum transfer conditions have been determined, long-term expression experiments are performed with unlabeled artificial chromosomes or microcells.

### H. Re-generation of transgenic plants

Plants containing artificial chromosomes are generated from plant cells, protoplasts, calli, or other plant tissue targets into which foreign DNA, in particular artificial chromosomes, have been introduced. Regeneration techniques for many commercially important plant species are well-known in the art. The artificial chromosome that is inserted into plant hosts to produce transgenic plants are PACs or MACs.

Plants are re-generated by the planting of transformed roots, plantlets, seeds, seedlings and structures capable of growing into a whole plant capable of reproduction (see, *e.g.,* US Patent Nos. 6,136,320 and International PCT application No. WO 00/60061). The re-generation of maize plants from transformed protoplasts is found, for example, in European Patent Application nos. 0 292 435 and 0 392 225 and International PCT Application Publication no. WO 93/07278; the regeneration of rice following gene transfer is found in Zhang et al. (1988) Plant Cell Rep. 7:379-384; Shimamoto et al. (1989) Nature 338:274-277; Datta et al. (1990) Biotechnology 8:736-740; and the re-generation of fertile transgenic barley by direct DNA transfer to protoplasts is described by Funatsuki et al. (1995) Theor. Appl. Genet. 91:707-712. Alternatively, plants containing artificial chromosomes are obtained by crossing a plant containing an artificial chromosome with another plant to produce plants having an artificial chromosome in their genomes (see e.g. US Patent No. 6,150,585).

Plants containing an artificial chromosome are propagated through seed, cuttings, or vegetatively. The seed from plants containing an artificial chromosome are grown in the field, in pots, indoors, outdoors, in greenhouses, on glass, or in or on any suitable medium, and the resulting sexually mature transgenic plants are self-pollinated to generate true breeding plants. The progeny from these transgenic plants become true breeding lines (International PCT application publication Nos. WO 00/60061 and EP 1017268; US Patent Nos. 5,631,152; 5,955,362; 6,015,940; 6,013,523; 6,096,546; 6,037,527; 6,153,812; Weissbach and Weissbach (1988) Methods for Plant Molecular Biology, Academic Press, Inc.; Fromm et al. (1990) Bio/Technology 8:833-839; Gordon-Kamm et al. (1990) Plant Cell 2:603-608; Koziel et al. (1993) Bio/Technology 11:194-200; and Golovkin et al. (1993) Plant Sci. 90:41-52).

### 1. PACs

Plant artificial chromosomes (PACs) are prepared by the *in vivo* and *in vitro* methods described herein. PACs may be prepared inside plant protoplasts and then transferred to plant targets, in particular to other plant protoplasts, via fusion in the presence or absence of PEG as described herein (Draper et al. (1982) Plant Cell Physiol. 23:451-458; Krens et al. (1982) Nature 72-74). PACs are isolated from the protoplasts in which they were prepared, encapsulated into liposomes, and delivered to other plant protoplasts (Deshayes et al. (1985) EMBO J. 4:2731-2737). Alternatively, the PACs are isolated and delivered directly to plant protoplasts, plant cells, or other plant targets via a PEG-mediated process, calcium phosphate-mediated process, electroporation, microinjection, sonoporation, or any method known in the art as described herein (Haim et al. (1985) Mol. Gen. Genet. 199:161-168; Fromm et al. (1986) Nature 319:791-793; Fromm et al. (1985) Proc. Nat. Acad. Sci. USA 82:5824-5828; Klein et al. (1987) Nature 327:70; Klein et al. (1988) Proc. Nat. Acad. Sci. USA 85:8502-8505; and lnternational PCT application publication no. WO 91/00358).

### 2. MACs

Mammalian artificial chromosomes (MACs) are prepared by the *in vivo* and *in vitro* methods described in US Patent Nos. 6,025,155 and 6,077,697, and lnternational PCT application No. WO 97/40183. MACs are prepared as microcells, and the microcells are fused with plant protoplasts in the presence or absence of PEG (Dudits et al. (1976) Hereditas 82:121-123; Wiegland et al. (1987) J. Cell. Sci. Pt. 2 145-149). Alternatively, the MACs are isolated and delivered directly to plant cells, protoplasts, and other plant targets a PEG-mediated process, calcium phosphate-mediated process, electroporation, microinjection, sonoporation , or any method known in the art as described herein and in US Patent Nos. 6,025,155 and 6,077,697, and International PCT application publication No. WO 97/40183.

After PACs or MACs are introduced into plant targets and the plant targets are grown and analyzed for transfection, the transformed plant targets are developed using standard conditions into roots, shoots, plantlets, or any structure capable of growing into a plant. Transgenic plants can, in turn, be generated by the planting of transformed roots, plantlets, seeds, seedlings and structures capable of growing into a plant. Transgenic plants can be propagated, for example, through seed, cuttings, or vegetative propagation.

### l. Applications and Uses of Artificial Chromosomes

Artificial chromosomes provide convenient and useful vectors, and in some instances (e.g., in the case of very large heterologous genes) the only vectors, for introduction of heterologous genes into hosts. Virtually any gene of interest is amenable to introduction into a host via artificial chromosomes.

As described herein, there are numerous methods for using artificial chromosomes to introduce coding sequences into plant cells. These include methods for using artificial chromosomes to express genes encoding commerically valuable enzymes and therapeutic compounds in plant cells, introduction of agronomically important traits or applications related to the manipulation of large regions of DNA.

The artificial chromosomes provided herein may be used in methods of protein and gene product production, particularly using plant cells as host cells for production of such products, and in cellular production systems in which the artificial chromosomes provide a reliable, stable and efficient means for optimizing the biomanufacturing of important compounds for medicine and industry. They are also intended for use in methods of gene therapy and for production of transgenic organisms, particularly plants (discussed above, below and in the EXAMPLES).

### 1. Production of products in plants

Methods for expression of heterologous proteins in plant cells ("molecular farming") are provided. At present, many foreign proteins have been expressed in whole plants or selected plant organs. Plants can offer a highly effective and economical means to produce recombinant proteins as they can be grown on a large scale at modest cost. The production of heterologous proteins in plants has included genes that are fused to strong constitutive plant promoters (e.g., 35S from cauliflower mosaic virus (Sijmons et al., 1990, Bio/Technology, 8:217-221, Benfey and Chua, US 5,110, 732, Fraley et al., US 5,858,742, McPherson and Kay, US 5,359,142); seed specific promoters (Hall et al., US 5,504,200, Knauf et al., US 5,530,194, Thomas et al., US 5,905,186, Moloney, US 5,792,922, US 5,948,682) or promoters active in other plant organs such as fruit (Radke et al., 1988, Theoret. Appl. Genet., 75:685-694, Bestwick et al., US 5,783,394, Houck and Pear, US 4,943,674) or storage organs such as tubers (Rocha-Sosa et al., US 5,436,393, US 5,723,757). The genes under the control of these promoters can be any protein and include, for example, genes that encode receptors, cytokines, enzymes, proteases, hormones, growth factors, antibodies, tumor suppressor genes, vaccines, therapeutic products and multigene pathways.

For example, industrial enzymes that can be produced include, for example, α-amylase, glucanase, phytase and xylanase (see, Goddijn and Pen (1995) Trends Biotechnol. 13:379-387; Pen et al. (1992) Bio/Technology 10:292-296; Horvath et al. (2000) Proc. Natl. Acad. Sci. U.S.A. 97:1914-1919; and *e.g.,* Herbers and Sonnewald (1996) in Transgenic Plants: A Production System for lndustrial and Pharmaceutical Proteins" Owen and Pen Eds., John Wiley & Sons, West Sussex, England), proteases such as subtilisin and other industrially important enzymes. Additional proteins that can be produced in crops by molecular farming include other industrial enzymes, for example, proteases, carbohydrate modifying enzymes such as glucose oxidase, cellulases, hemicellulases, xylanases, mannanases or pectinases, (e.g. Baszczynski et all., US 5,824,870, US 5,767,379, Bruce et al., US 5,804,694). Additionally, the production of enzymes particularly valuable in the pulp and paper industry such as ligninases or xylanases also can be expressed, (Austin-Philips et al., US 5,981,835). Other examples of enzymes include phosphatases, oxidoreductases and phytases, (van Ooijen et al., US 5,714,474).

Additionally, expression and delivery of vaccines in plants has been proposed(Arntzen and Lam, US 6,136,320, US, 5,914,123, Curtiss and Cardineau, US 5,679,880, US 5,679,880, US 5,654,184, Lam and Arntzen, US 5,612,487, US 6,034,298, Rymerson et al., WO9937784A1, as well as antibodies (Conrad et al., WO 972900A1, Hein et al., US 5,959,177, Hiatt and Hein, US 5,202,422, US 5,639,947, Hiatt et al., US 6,046,037), peptide hormones (Vandekerckhove, J.S., US 5,487;991, Brandle et al., WO9967401A2), blood factors and similar therapeutic molecules. Expression of vaccines in edible plants can provide a means for drug delivery which is cost effective and particularly suited for the administration of therapeutic agents in rural or under developed countries. The plant material containing the therapeutic agents could be cultivated and incorporated into the diet (Lam, D.M., and Arntzen, C.J., US 5,484,719). Similarly, plants used for animal feed can be engineered to express veterinary biologics that can provide protection against animal disease, (Rymerson et al., WO9937784A1). Antibodies also can be produced in plants, including, for example, a gene fusion encoding an antigen-binding single chain Fv protein (scFv) that recognizes the hapten oxazolone (Fiedler and Conrad (1995) Bio/Technology 13:1090-1093) and lgG (Ma et al. (1995) Science 268:716-719). Monoclonal antibodies for therapeutic and diagnostic applications are of particular interest.

Examples of human biopharmaceuticals that can be expressed in plants include, but are not limited to, albumin (Sijmons *et al.* (1990)), enkephalins (Vandekerckhove *et al.* (1989) ), interferon-á (Zhu *et al.* (1994) and GM-CSF (Ganz et al. (1996) in Transgenic Plants: A Production System for lndustrial and Pharmaceutical Proteins, Owen and Pen Eds., John Wiley & Sons, West Sussex, England, pp. 281-297; and Sardana et al. (1998) in Methods in Biotechnology, Vol. 3: Recombinant Proteins from Plants: Production and lsolation of Clinically Useful Compounds, Cunningham and Porter, Eds., Humana Press, New Jersey; pp. 77-87).

Cells containing the artificial chromosomes provided herein can advantageously be used in *in vitro* plant cell-based systems for production of proteins, particularly several proteins from one cell line, such as multiple proteins involved in a biochemical pathway or multivalent vaccines. The genes encoding the proteins are introduced into the artificial chromosomes which are then introduced into plant cells. Plant cells useful for this purpose are those that grow well in culture, or most preferably, plant cells capable of being regenerated to whole plants. Plants can then be cultivated by common methods to produce plant material comprising said heterologous proteins. The heterologous proteins can be subject to purification or the plant tissue or extracts thereof can be used directly for vaccination, amelioration of disease, or processing of material, such as bleaching during pulp and paper processing or enzymatic conversion of industrial materials or feedstocks. Alternatively, the heterologous gene(s) of interest are transferred into a production cell line or plant line that already contains artificial chromosomes in a manner that targets the gene(s) to the artificial chromosomes. The cells or plants are grown under conditions whereby the heterologous proteins are expressed. Because the proteins are expressed at high levels in a stable permanent extra-genomic chromosomal system, selective conditions are not required.

Selection of host lines for use in artificial chromosome-based protein production systems is within the skill of the art, but often will depend on a variety of factors, including the properties of the heterologous protein to be produced, potential toxicity of the protein in the host cell, any requirements for post-translational modification (e.g., glycosylation, amination, phosphorylation) of the protein, transcription factors available in the cells, the type of promoter element(s) being used to drive expression of the heterologous gene, whether production is completely intracellular or the heterologous protein will preferably be secreted from the cell, or be sequestered or localized, and the types of processing enzymes in the cell.

Artificial chromosomes can be engineered as platforms for the production of specific molecules in plant cells. For example, production of complex mammalian molecules, such as multichain antibodies, requires a number of protein activities not normally found in plant species. It is possible to produce an artificial chromosome that comprises all of the mamalian activities needed to produce human antibodies, correctly modified and processed, by introducing into an artificial chromosome the genes needed to carry out these activities. Said genes would be modified, for example, by placing each gene under the control of a plant promoter, or by placing the master control gene, i.e., a gene that controls expression of the various genes, under the control of a plant promoter. Alternatively, mammalian transcriptional control factors could be introduced, under the control of plant active promoters, to be expressed in a plant cell and cause the expression of said target proteins, for example multichain antibodies.

ln this fashion, plant artificial chromosomes are developed, each capable of supporting the efficient production of a specific class of valuable products, for example, antibodies, blood clotting factors, etc. Thus, production of products within a class, for example, human antibodies would simply involve the introduction of a specific antibody coding sequence, without modification into the artificial chromosome engineered specifically for the production of human antibodies. The artificial chromosome would comprise all of the required genetic activities for the proper expression, translation and post-translational modification of human antibodies. Such artificial chromosomes can be used in a variety of applications, such as, but are not limited to, large scale production of numerous specific human antibodies.

Advantages of plant cells as host cell lines in the production of recombinant proteins include, but are not limited to, the following: (1) proteins are post-translationally modified similar to mammalian systems, (2) plants can be directed to secrete proteins into stable, dry, intracellular compartments of seeds called endosperm protein bodies, which can easily be collected, (3) the amount of recombinant product that can be produced approaches industrial scale levels and (4) health risks due to contamination with potential pathogens/toxins are minimized.

The artificial chromosome-based system for heterologous protein production has many advantageous features. For example, as described above, because the heterologous DNA is located in an independent, extra-genomic artificial chromosome (as opposed to randomly inserted in an unknown area of the host cell genome or located as extrachromosomal element(s) providing only transient expression), it is stably maintained in an active transcription unit and is not subject to ejection via recombination or elimination during cell division. Accordingly, it is unnecessary to include a selection gene in the host cells and thus growth under selective conditions is also unnecessary. Furthermore, because the artificial chromosomes are capable of incorporating large segments of DNA, multiple copies of the heterologous gene and linked promoter element(s) can be retained in these chromosomes, thereby providing for high-level expression of the foreign protein(s). Alternatively, multiple copies of the gene can be linked to a single promoter element and several different genes can be linked in a fused polygene complex to a single promoter for expression of, for example, all the key proteins constituting a complete metabolic pathway (see, e.g., Beck von Bodman et al. (1995) Biotechnology 13:587-591). Alternatively, multiple copies of a single gene can be operatively linked to a single promoter, or each or one or several copies can be linked to different promoters or multiple copies of the same promoter. Additionally, because artificial chromosomes have an almost unlimited capacity for integration and expression of foreign genes, they can be used not only for the expression of genes encoding end-products of interest, but also for the expression of genes associated with optimal maintenance and metabolic management of the host cell, e.g., genes encoding growth factors, as well as genes that facilitate rapid synthesis of correct form of the desired heterologous protein product, *e.g,,* genes encoding processing enzymes and transcription factors as described above.

The artificial chromosomes are suitable for expression of any proteins or peptides, including proteins and peptides that require *in vivo* posttranslational modification for their biological activity. Such proteins include, but are not limited to antibody fragments, full-length antibodies, and multimeric antibodies, tumor suppressor proteins, naturally occurring or artificial antibodies and enzymes, heat shock proteins, and others.

Thus, such cell-based "protein factories" employing artificial chromosomes can be generated using artificial chromosomes constructed with multiple copies (theoretically an unlimited number or at least up to a number such that the resulting artificial chromosome is about up to the size of a genomic chromosome (i.e., endogenous)) of protein-encoding genes with appropriate promoters, or multiple genes driven by a single promoter, i.e., a fused gene complex (such as a complete metabolic pathway in plant expression system; see, e.g.,eck von Bodman (1995) Biotechnology 13:587-591). Once such an artificial chromosome is constructed, it can be transferred to a suitable plant species capable of being propagated under field conditions, or under conditions that permit the recovery of the intended product. Plant cell cultures such as algae can be used in a system analogous to mammalian cell culture systems. The advantage of plant based systems such as this include low input costs for growth, rapid growth rates and ability to produce a large biomass economically.

The ability of artificial chromosomes to provide for high-level expression of heterologous proteins in host cells is demonstrated, for example, by analysis of mammalian cells containing a mammalian artificial chromosome, H1 D3 and G3D5 cell lines described herein. Northern blot analysis of mRNA obtained from these cells reveals that expression of the hygromycin-resistance and *β-*galactosidase genes in the cells correlates with the amplicon number of the megachromosome(s) contained therein.

Transgenic plants producing these compounds are made by the introduction and expression of one or potentially many genes using the artificial chromosomes provided herein. The vast array of possibilities include, but are not limited to, any biological compound which is presently produced by any organism such as proteins, nucleic acids, primary and intermediary metabolites, carbohydrate polymers, enzymes for uses in bioremediation, enzymes for modifying pathways that produce secondary plant metabolites such as flavonoids or vitamins, enzymes that could produce pharmaceuticals and for introducing enzymes that could produce compounds of interest to the manufacturing industry such as specialty chemicals and plastics. The compounds are roduced by the plant, extracted upon harvest and/or processing, and used for any presently recognized useful purpose such as pharmaceuticals, fragrances, and industrial enzymes. Alternatively, plants produced in accordance with the methods and compositions provided herein can be made to metabolize certain compounds, such as hazardous wastes, thereby allowing bioremediation of these compounds.

The artificial chromosomes provided herein can be used in methods of protein and gene product production, particularly using plant cells as host cells for production of such products, and in cellular production systems in which the artificial chromosomes provide a reliable, stable and efficient means for optimizing the biomanufacturing of important compounds for medicine and industry.

### 2. Genetic alteration of organisms to possess desired traits

Artificial chromosomes are ideally suited for preparing organisms, such as plants, that possess certain desired traits, such as, for example, disease resistance, resistance to harsh environmental conditions, altered growth patterns and enhanced physical characteristics. With respect to plants, the choice of the particular nucleic acid that will be delivered to recipient cells via artificial chromosomes often will depend on the purpose of the transformation. One of the major purposes of transformation of crop and tree species is to add some commercially desirable, agronomically important traits to the plant. Such traits include, but are not limited to, input and output traits such as herbicide resistance or tolerance, insect resistance or tolerance, disease resistance or tolerance (viral, bacterial, fungal or nematode), stress tolerance and/or resistance, as exemplified by resistance or tolerance to drought, heat, chilling, freezing, excessive moisture, salt stress and oxidative stress, increased yields, food content and makeup, physical appearance, male sterility, drydown, standability, prolificacy, starch quantity and quality, oil quantity and quality, protein quantity and quality and amino acid composition. It may be desirable to incorporate one or more genes conferring such desirable traits into host plants.

### a. Herbicide resistance

The genes encoding phosphinothricin acetyltransferase *(bar* and *pat),* glyphosate tolerant EPSP synthase genes, the glyphosate degradative enzyme gene gox encoding glyphosate oxidoreductase, *deh* (encoding a dehalogenase enzyme that inactivates dalapon), herbicide resistant (*e.g.*, sulfonylurea and imidazolinone) acetolactate synthase, and *b×n* genes (encoding a nitrilase enzyme that degrades bromoxynil) are all examples of herbicide resistant genes for use in plant transformation. The *bar* and *pat* genes code for an enzyme, phosphinothricin acetyltransferase (PAT), which inactivates the herbicide phosphinothricin and prevents this compound from inhibiting gluatamine synthetase enzymes. The enzyme 5-enolpyruvylshikimate 3-phosphate synthase (EPSP synthase) is normally inhibited by the herbicide N-(phosphonomethyl)glycine (glyphosate). However, genes are known that encode glyphosate-resistant EPSP synthase enzymes. The *deh* gene encodes the enzyme dalapon dehalogenase and confers resistance to the herbicide dalapon. The *b×n* gene codes for a specific nitrilase enzyme that converts bromoxynil to a non-herbicidal degradation product.

### b. Insect and other pest resistance

lnsect-resistant organisms may be prepared in which resistance or decreased susceptibility to insect-induced disease is conferred by introduction into the host organism or embryo of artificial chromosomes containing DNA encoding gene products (*e.g.,* ribozymes and proteins that are toxic to certain pathogens) that destroy or attenuate pathogens or limit access of pathogens to the host. Potential insect resistance genes that can be introduced into plants via artificial chromosomes include *Bacillus thuringiensis* crystal toxin genes or Bt genes (see, *e.g.,,* Watrud *et al.* (1985) *in Engineered Organisms and the Environment).* Bt genes may provide resistance to lepidopteran or coleopteran pests such as the European Corn Borer (ECB). Such Bt toxin genes include the *Cry*/*A (b)* and *Cry*/*A (c)* genes. Endotoxin genes from other species of *B. thuringiensis* which affect insect growth or development also may be employed in this regard. Bt gene sequences can be modified to effect increased expression in plants, and particularly monocot plants. Means for preparing synthetic genes are well known in the art and are disclosed in, for example, U.S. Patent Nos. 5,500,365 and 5,689,052. Examples of such modified Bt toxin genes include a synthetic Bt *Cry*/*A (b)* gene (see, *e.g.,* Perlak et al. (1991) Proc. Natl. Acad. Sci. U.S.A. 88:3324-3328) and the synthetic *CrylA(c)* gene termed 1800b (see PCT Application publication no. WO95/06128).

Examples of the types of genes that may be transferred into plants via artificial chromosomes to generate disease- and/or insect-resistant transgenic plants include, but are not limited to, the *crylA(b)* and *crylA(c)* genes which yield products that are highly toxic to two major rice insect pests (the striped stem borer and the yellow stem borer) (see, e.g., Cheng et al. (1998) Proc. Natl. Acad. Sci. U.S.A. 95:2767-2772), *cry3* genes which encode products that are toxic to Coleopteran insects that attack a variety of plants, including grains and legumes (see, *e.g.,* U.S. Patent No. 6,023,013), genes *(e.g.,* DNA encoding tricothecene 3-O-acetyltransferase) that confer resistance to tricothecenes such as those produced by plant fungi *(e.g., Fusarium)* in plants particularly susceptible to fungi *(e.g.,* wheat, rye, barley, oats, and maize) (see, *e.g.,* PCT Application publication no. WO 00/60061), and genes involved in multi-gene biosynthetic pathways that yield antipathogenic substances that have a deleterious effect on the growth of plant pathogens (see, *e.g.,* U.S. Patent No. 5,639,949).

Protease inhibitors may also provide insect resistance (see, *e.g.,* Johnson *et al.* (1989) and will thus have utility in plant transformation. The use of a protease inhibitor ll gene, *pinll,* from tomato or potato may be particularly useful. The combined effect of the use of a *pinll* gene with a Bt toxin gene can produce synergistic insecticidal activity. Other genes that encode inhibitors of the insect's digestive system, or those that encode enzymes or co-factors that facilitate the production of inhibitors, also may be useful. This group may be exemplified by oryzacystatin and amylase inhibitors such as those from wheat and barley.

Genes encoding lectins may confer additional or alternative insecticide properties. Lectins (originally termed phytohemagglutinins) are multivalent carbohydrate-binding proteins which have the ability to agglutinate red blood cells from a range of species. Lectins have been identified as insecticidal agents with activity against weevils, ECB and rootworm (see, *e.g.,* Murdock et al. (1990) Phytochemistry 29:85-89; Czapla & Lang (1990) J. Econ. Entomol, 83:2480-2485). Lectin genes that may be useful include, for example, barley and wheat germ agglutinin (WGA) and rice lectins (Gatehouse et al. (1984) J. Sci. Food. Agric. 35:373-380).

Genes controlling the production of large and small polypeptides active against insects when introduced into the insect pests, such as, for example, lytic peptides, peptide hormones and toxins and venoms, may also be useful in generating pest-resistant plants. For example, expression of juvenile hormone esterase, directed toward specific insect pests, also may result in insecticidal activity, or cause cessation of metamorphosis (see, *e.g.,* Hammock et al. (1990) Nature 344:468-461).

Transgenic plants expressing genes which encode enzymes that affect the integrity of the insect cuticle are additional examples of genes that may be transferred to plants via artificial chromosomes to confer resistance to insects. Such genes include those encoding, for example, chitinase, proteases, lipases and also genes for the production of nikkomycin, a compound that inhibits chitin synthesis, the introduction of any of which may be used to produce insect-resistant plants. Genes that affect insect molting, such as those affecting the production of ecdysteroid UDP-glucosyl transferase, also can be useful transgenes.

Genes that code for enzymes that facilitate the production of compounds that reduce the nutritional quality of the host plant to insect pests may also be used to confer insect resistance on plants. lt may be possible, for instance, to confer insecticidal activity on a plant by altering its sterol composition. Sterols are obtained by insects from their diet and are used for hormone synthesis and membrane stability. Therefore, alterations in plant sterol composition by expression of genes that directly promote the production of undesirable sterols or those that convert desirable sterols into undesirable forms, could have a negative effect on insect growth and/or development and hence endow the plant with insecticidal activity. Lipoxygenases are naturally occurring plant enzymes that have been shown to exhibit anti-nutritional effects on insects and to reduce the nutritional quality of their diet. Therefore, transgenic plants with enhanced lipoxygenase activity may be resistant to insect feeding.

*Tripsacum dactyloides* is a species of grass that is resistant to certain insects, including corn root worm. *Tripsacum* may thus include genes encoding proteins that are toxic to insects or are involved in the biosynthesis of compounds toxic to insects. Such genes may be useful in conferring resistance to insects. It is known that the basis of insect resistance in *Tripsacum* is genetic, because said resistance has been transferred to Zea *mays* via sexual crosses (Branson and Guss, 1972). It is further anticipated that other cereal, monocot or dicot plant species may have genes encoding proteins that are toxic to insects which would be useful for producing insect resistant plants.

Further genes encoding proteins characterized as having potential insecticidal activity also may be used as transgenes in accordance herewith. Such genes include, for example, the cowpea trypsin inhibitor (CpT1: Hilder *et al.,* 1987) which may be used as a rootworm deterrent, genes encoding avermectin *(*Avermectin and Abamectin., Campbell, W.C., Ed., 1989: Ikeda et al., 1987) which may prove particularly useful as a corn rootworm deterent, ribosome inactivating protein genes and even genes that regulate plant structures. Transgenic plants including anti-insect antibody genes and genes that code for enzymes that can convert a non-toxic insecticide (proinsecticide) applied to the outside of the plant into an insecticide inside the plant also are contemplated.

### c. Disease resistance

Transgenic organisms, such as plants, that express genes that confer resistance or reduce susceptibility to disease are of particular interest. For example, the transgene may encode a protein that is toxic to a pathogen, such as a virus, fungus, mycotoxin-producing organism, nematode or bacterium, but that is not toxic to the transgenic host.

Because multiple genes can be introduced on an artificial chromosome, a series of genes encoding a genetic pathway involved in disease resistance or tolerance can be introduced into crop plants. For example, it is known that often numerous genes are expressed upon pathogen invasion, typically one or more "PR", or pathogen related, proteins are expressed in response to invasion of a plant bacterial or fungal pathogen. One or more of the proteins involved in conferring resistance to pathogens can be contained within an artificial chromosome and therefore be expressed in a plant cell, in particular a whole transgenic plant as described herein. In addition, production of single-chain Fv recombinant antibodies in plants may extend the range of possibilities for the introduction of pathogen protection in crop plants (see, e.g., Tavladoraki et al. (1993) Nature 366:469-472).

lt has been demonstrated that expression of a viral coat protein in a transgenic plant can impart resistance to infection of the plant by that virus and perhaps other closely related viruses (Cuozzo *et al.,* 1988. Hemenway *et a*/*.,* 1988, Abel *et al.,* 1986). Expression of antisense genes targeted at essential viral functions may also impart resistance to viruses. For example, an antisense gene targeted at the gene responsible for replication of viral nucleic acid may inhibit replication and lead to resistance to the virus. Interference with other viral functions through the use of antisense genes also may increase resistance to viruses. Further, it may be possible to achieve resistance to viruses through other approaches, including, but not limited to the use of satellite viruses. Artificial chromosomes are ideally suited for carrying a multiplicity of these genes and DNA sequences which are useful for conferring a broad range of resistance to many pathogens.

Genes encoding so-called "peptide antibiotics," pathogenesis related (PR) proteins, toxin resistance, and proteins affecting host-pathogen interactions such as morphological may also be useful, particularly in conferring increased resistance to diseases caused by bacteria and fungi. Peptide antibiotics are polypeptide sequences which are inhibitory to growth of bacteria and other microorganisms. For example, the classes of peptides referred to as cepropins and magainins inhibit growth of may species of bacteria and fungi. Expression of PR proteins in monocotyledonous plants such as maize may be useful in conferring resistance to bacterial disease. These genes are induced following pathogen attack on a host plant and have been divided into at lease five classes of proteins (Bio. Linthorst, and Cornelissen, 1990). lncluded among the PR proteins are *β*-1, 3-glucanases, chitinases, and osmotin and other proteins that are believed to function in plant resistance to disease organisms. Other genes have been identified that have antifungal properties, e.g., UDA (stinging nettle lectin) and hevein (Broakaert *et al.,* 1989; Barkai-Golan *et al.,* 1978). It is known that certain plant diseases are caused by the production of phytotoxins. Resistance to these diseases may be achieved through expression of a gene that encodes an enzyme capable of degrading or otherwise inactivating the phytotoxin. lt also is contemplated that expression of genes that alter the interactions between the host plant and pathogen may be useful in reducing the ability of the disease organism to invade the tissues of the host plant, *e.g.,* an increase in the waxiness of the leaf cuticle or other morphological characteristics.

### d. Environment or stress resistance

Improvement of a plant's ability to tolerate various environmental stresses such as, but not limited to, drought, excess moisture, chilling, freezing, high temperature, salt, and oxidative stress, also can be effected through expression of genes therein. It is proposed that benefits may be realized in terms of increased resistance to freezing temperatures through the introduction of an "antifreeze" protein such as that of the Winter Flounder (Cutler *et al.,* 1989) or synthetic gene derivatives thereof. Improved chilling tolerance also may be conferred through increased expression of glycerol-3-phosphate acetyltransferase in chloroplasts (Wolter *et al.,* 1992). Resistance to oxidative stress in some crop species (often exacerbated by conditions such as chilling temperatures in combination with high light intensities) can be conferred by expression of superoxide dismutase (Gupta *et al.,* 1993), and may be improved by glutathione reductase (Bowler et al., 1992). Such strategies may allow for tolerance to freezing in newly emerged fields as well as extending later maturity higher yielding varieties to earlier relative maturity zones.

It is contemplated that the expression of genes that favorably effect plant water content, total water potential, osmotic potential, and turgor will enhance the ability of the plant to tolerate drought. As used herein, the terms "drought resistance" and drought tolerance" are used to refer to a plant's increased resistance or tolerance to stress induced by a reduction in water availability, as compared to normal circumstances, and the ability of the plant to function and survive in lower-water environments. The expression of genes encoding for the biosynthesis of osmotically-active solutes, such as polyol compounds, may impart protection against drought. Within this class are genes encoding for mannitol-L-phosphate dehydrogenase (Lee and Saier, 1982) and trehalose-6-phosphate synthase (Kaasen et al., 1992). Through the subsequent action of native phosphatases in the cell or by the introduction and coexpression of a specific phosphatase, these introduced genes will result in the accumulation of either mannitol or trehalose, respectively, both of which have been well documented as protective compounds able to mitigate the effects of stress. Mannitol accumulation in transgenic tobacco has been verified and preliminary results indicate that plants expressing high levels of this metabolite are able to tolerate an applied osmotic stress (Tarczynski *et al.,* 1992, 1993).

Similarly, the efficacy of other metabolites in protecting either enzyme function (e.g., alanopine or propionic acid) or membrane integrity (e.g., alanopine) has been documented (Loomis *et al.,* 1989), and therefore expression of genes encoding for the biosynthesis of these compounds might confer drought resistance in a manner similar to or complimentary to mannitol. Other examples of naturally occurring matabolites that are osmotically active and/or provide some direct protective effect during drought and/or desiccation include fructose, erythritol (Coxson *et al.,* 1992), sorbitol, dulcitol (Karsten *et al.,* 1992), glucosylglycerol (Reed *et al.,* 1984; ErdMann *et al.,* 1992), sucrose, stachyose (Koster and Leopold, 1988: Blackman *et al.,* 1992), raffinose (Bernal-Lugo and Leopold, 1992), proline (Rensburg *et al.,* 1993), glycine betaine, ononitol and pinitol (Vernon and Bohnert, 1992). Continued canopy growth and increased reproductive fitness during times of stress will be augmented by introduction and expression of genes such as those controlling the osmotically active compounds discussed above and other such compounds. Genes which promote the synthesis of an osmotically active polyol compound include genes which encode the enzymes mannitol-1-phosphate dehydrogenase, trehalose-6-phosphate synthase and myoinositol O-methyltransferase. Artificial chromosomes can carry a multiplicity of genes to provide durable stress tolerance, for example, concominant expression of proline and ketane and/or poly-ols.

lt is contemplated that the expression of specific proteins also may increase drought tolerance under certain conditions or in certain crop species. These may include proteins such as Late Embryogenic Proteins (see Dure *et al.,* 1989). All three classes of LEAs have been demonstrated in maturing *(i.e.* desiccating) seeds. Within LEA proteins, the Type-ll (dehydrin-type) have generally been implicated in drought and/or desiccation tolerance in vegetative plant parts *(i.e.* Mundy and Chua, 1988: Piatkowski *et al.,* 1990: Yamaguchi-Shinozaki *et al.,* 1992). Recently, expression of a Type-lll LEA (HVA-1) in tobacco was found to influence plant height, maturity and drought tolerance (Fitzpatrick, 1993). In rice, expression of the HVA-1 gene influenced tolerance to water deficit and salinity (Xu *et al .,* 1996). Expression of structural genes from all three LEA groups may therefore confer drought tolerance. Other types of proteins induced during water stress include thiol proteases, aldolases and transmembrane transporters (Guerrero *et al.,* 1999), which may confer various protective and/or repair-type functions during drought stress. lt is also is contemplated that genes that effect lipid biosynthesis and hence membrane composition might also be useful in conferring drought resistance on the plant.

Many of these genes for improving drought resistance have complementary modes of action. Thus, combinations of these genes might have additive and/or synergistic effects in improving drought resistance in plants. Many of these genes also improve freezing tolerance (or resistance): the physical stresses incurred during freezing and drought are similar in nature and may be mitigated in similar fashion. Benefit may be conferred via constitutive expression of these genes, but the preferred means of expressing these genes may be through the use of a turgor-induced promoter (such as the promoters for the turgor-induced genes described in Guerrero *et* a/., 1990 and Shagan *et al.,* 1993 which are incorporated herein by reference). Spatial and temporal expression patterns of these genes may enable plants to better withstand stress.

lt is proposed that expression of genes that are involved with specific morphological traits that allow for increased water extractions from drying soil would be of benefit. For example, introduction and expression of genes that alter root characteristics may enhance water uptake. lt also is contemplated that expression of genes that enhance reproductive fitness during times of stress would be of significant value. For example, expression of genes that improve the synchrony of pollen shed and receptiveness of the female flower parts, *i.e.,* silks, would be of benefit. ln addition it is proposed that expression of genes that minimize kernel abortion during times of stress would increase the amount of grain to be harvested and hence be of value.

Given the overall role of water in determining yield, it is contemplated that enabling plants to utilize water more efficiently, through the introduction and expression of genes, will improve overall performance even when soil water availability is not limiting. By introducing genes that improve the ability of plants to maximize water usage across a full range of stresses relating to water availability, yield stability or consistency of yield performance may be realized.

### e. Plant agronomic characteristics

Plants possessing desired traits that might, for example, enhance utility, processibility and commercial value of the organisms in areas such as the agricultural and ornamental plant industries may also be generated using artificial chromosomes in the same manner as described above for production of disease-resistant organisms. In such instances, the artificial chromosomes that are introduced into the organism or embryo contain DNA encoding gene products that serve to confer the desired trait in the organism.

For example, transgenic plants having improved flavor properties, stability and/or quality are of commercial interest. One possible method for generating such plants may include the expression of transgenes, e.g., genes encoding cystathionine gamma synthase (CGS), that result in increased free methionine levels (see, e.g., PCT Application publication no. WO 00/55303).

Two of the factors determining where crop plants can be grown are the average daily temperature during the growing season and the length of time between frosts. Within the areas where it is possible to grow a particular crop, there are varying limitations on the maximal time it is allowed to grow to maturity and be harvested. For example, a variety to be grown in a particular area is selected for its ability to mature and dry down to harvestable moisture content within the required period of time with maximum possible yield. Therefore, crops of varying maturities are developed for different growing locations. Apart from the need to dry down sufficiently to permit harvest, it is desirable to have maximal drying take place in the field to minimize the amount of energy required for additional drying post-harvest. Also, the more readily a product such as grain can dry down, the more time there is available for growth and kernel fill. Genes that influence maturity and/or dry down can be identified and introduced into plant lines using transformation techniques to create new varieties adapted to different growing locations or the same growing location, but having improved yield to moisture ratio at harvest. Expression of genes that are involved in regulation of plant development may be especially useful.

Genes that would improve standability and other plant growth characteristics may also be introduced into plants. Expression of new genes in plants which confer stronger stalks, improved root systems, or prevent or reduce ear droppage would be of great value to the farmer. Introduction and expression of genes that increase the total amount of photoassimilate available by, for example, increasing light distribution and/or interception would be advantageous. In addition, the expression of genes that increase the efficiency of photosynthesis and/or the leaf canopy would further increase gains in productivity. Expression of a phytochrome gene in crop plants may be advantageous. Expression of such a gene may be reduce apical dominance, confer semidwarfism on a plant, and increase shade tolerance (U.S. Patent No. 5,268,526). Such approaches would allow for increased plant populations in the field.

### f. Nutrient utilization

The ability to utilize available nutrients may be a limiting factor in growth of crop plants. lt may be possible to alter nutrient uptake, tolerate pH extremes, mobilization through the plant, storage pools, and availability for metabolic activities by the introduction of new agents. These modifications would allow a plant such as maize to more efficiently utilize available nutrients. An increase in the activity of, for example, an enzyme that is normally present in the plant and involved in nutrient utilization may increase the availability of a nutrient. An example of such an enzyme would be phytase. It is further contemplated that enhanced nitrogen utilization by a plant is desirable. Expression of a glutamate dehydrogenase gene in plants, *e.g., E. coli gdhA* genes, may lead to enhanced resistance to the herbicide glufosinate by incorporation of excess ammonia into glutamate, thereby detoxifying the ammonia. Gene expression may make a nutrient source available that was previously not accessible, e.g., an enzyme that releases a component of nutrient value from a more complex molecule, perhaps a macromolecule. Alternatively, artificial chromosomes can carry the multiplicity of genes governing nodulation and nitrogen fixation in legumes. The artificial chromosomes could be used to promote nodulation in non-legume species.

### g. Male sterility

Male sterility is useful in the production of hybrid seed. Male sterility may be produced through gene expression. For example, it has been shown that expression of genes that encode proteins that interfere with development of the male inflorescence and/or gametophyte result in male sterility. Chimeric ribonuclease genes that express in the anthers of transgenic tobacco and oilseed rape have been demonstrated to lead to male sterility (Mariani *et al.,* 1990). Other methods of conferring male sterility have been described, including gene encoding antisense RNA capable of causing male sterility (U.S. Patent Nos. 6,184,439, 6,191,343 and 5,728,926) and methods utilizing two genes to confer sterility, see, *e.g.,* U.S. Patent No. 5,426,041.

A number of mutations were discovered in maize that confer cytoplasmic male sterility. One mutation in particular, referred to as T cytoplasm, also correlates with sensitivity to Southern corn leaf blight. A DNA sequence, designated TURF-13 (Levings, 1990), was identified that correlates with T cytoplasm. It is proposed that it would be possible through the introduction of TURF-13 via transformation, to separate male sterility from disease sensitivity. As it is necessary to be able to restore male fertility for breeding purposes and for grain production, it is proposed that genes encoding restoration of male fertility also may be introduced.

### h. Improved nutritional content

Genes may be introduced into plants to improve the nutrient quality or content of a particular crop. Introduction of genes that alter the nutrient composition of a crop may greatly enhance the feed or food value. For example, the protein of many grains is suboptimal for feed and food purposes especially when fed to pigs, poultry, and humans. The protein is deficient in several amino acids that are essential in the diet of these species, requiring the addition of supplements to the grain. Limiting essential amino acids may include lysine, methionine, tryptophan, threonine, valine, arginine, and histidine. Some amino acids become limiting only after corn is supplemented with other inputs for feed formulations. The levels of these essential amino acids in seeds and grain may be elevated by mechanisms which include, but are not limited to, the introduction of genes to increase the biosynthesis of the amino acids, increase the storage of the amino acids in proteins, or increase transport of the amino acids to the seeds or grain.

The protein composition of a crop may be altered to improve the balance of amino acids in a variety of ways including elevating expression of native proteins, decreasing expression of those with poor composition changing the composition of native proteins, or introducing genes encoding entirely new proteins possessing superior composition.

The introduction of genes that alter the oil content of a crop plant may also be of value. lncreases in oil content may result in increases in metabolizable-energy-content and density of seeds for use in feed and food. The introduced genes may encode enzymes that remove or reduce rate-limitations or regulated steps in fatty acid or lipid biosynthesis. Such genes may include, but are not limited to, those that encode acetyl-CoA carboxylase, ACP-acyltransferase, *β*-ketoacyl-ACP synthase, plus other well known fatty acid biosynthetic activities. Other possibilities are genes that encode proteins that do not possess enzymatic activity such as acyl-carrier proteins. Genes may be introduced that alter the balance of fatty acids present in the oil providing a more healthful or nutritive feedstuff. The introduced DNA also may encode sequences that block expression of enzymes involved in fatty acid biosynthesis, altering the proportions of fatty acids present in crops.

Genes may be introduced that enhance the nutritive value of the starch component of crops, for example by increasing, or in some cases decreasing, the degree of branching, resulting in improved utilization of the starch in livestock by delaying its metabolism. Additionally, other major constituents of a crop may be altered, including genes that affect a variety of other nutritive, processing, or other quality aspects. For example, pigmentation may be increased or decreased.

Feed or food crops may also possesses insufficient quantities of vitamins, requiring supplementation to provide adequate nutritive value. Introduction of genes that enhance vitamins biosynthesis may be envisioned including, for example, vitamins A (e.g. rice with Vitamin A or golden rice), E, B12 choline, and the like. Mineral content may also be sub-optimal. Thus genes that affect the accumulation or availability of compounds containing phosphorus, sulfur, calcium, manganese, zinc, and iron among others would be valuable.

Numerous other examples of improvements of crops may be effected using the artificial chromosomes, with appropriate heterologous genes contained therein, in accordance with the methods and compositions provided herein. The improvements may not necessarily involve grain, but may, for example, improve the value of a crop for silage. Introduction of DNA to accomplish this might include sequences that alter lignin production such as those that result in the "brown midrib" phenotype associated with superior feed value for cattle.

In addition to direct improvements in feed or food value, genes also may be introduced which improve the processing of crops and improve the value of the products resulting from the processing. One use of crops is via wetmilling. Thus, genes that increase the efficiency and reduce the cost of such processing, for example, by decreasing steeping time may also find use. Improving the value of wetmilling products may include altering the quantity or quality of starch, oil, corn gluten meal, or the components of gluten feed. Elevation of starch may be achieved through the identification and elimination of rate limiting steps in starch biosynthesis or by decreasing levels of the other components of crops resulting in proportional increases in starch.

Oil is another product of wetmilling, the value of which may be improved by introduction and expression of genes. Oil properties maybe be altered to improve its performance in the production and use of cooking oil, shortenings, lubricants or other oil-derived products or improvements of its health attributes when used in the food-related applications. Fatty acids also may be synthesized which upon extraction can serve as starting materials for chemical syntheses. The changes in oil properties may be achieved by altering the type, level, or lipid arrangement of the fatty acids present in the oil. This in turn may be accomplished by the addition of genes that encode enzymes that catalyze the synthesis of new fatty acids and the lipids possessing them or by increasing levels of native fatty acids while possibly reducing levels of precursors. Alternatively, DNA sequences may be introduced which slow or block steps in fatty acid biosynthesis resulting in the increase in precursor fatty acid intermediates. Genes that might be added include desaturases, epoxidases, hydratases, dehydratases and other enzymes that catalyze reactions involving fatty acid intermediates. Representative examples of catalytic steps that might be blocked include the desaturations from stearic to oleic acid and oleic to linolenic acid resulting in the respective accumulations of stearic and oleic acids. Another example is the blockage of elongation steps resulting in the accumulation of C8 to C12 saturated fatty acids.

### i. Production of chemicals or biologicals

Transgenic plants can be used as protein production systems to generate recombinant products ranging from industrial enzymes, viral antigens, vaccines, antibodies, human blood proteins, cytokines, growth factors, enkephalins, serum albumin and other proteins of clinical relevance and pharmaceuticals. For example, enzymes including *α*-amylase, glucanase, phytase and xylanase (see, Goddijn and Pen (1995) Trends Biotechnol. 13:379-387; Pen et al. (1992) Bio/Technology 10:292-296; Horvath et al. (2000) Proc. Natl. Acad. Sci. U.S.A. 97:1914-1919; and e.g., Herbers and Sonnewald (1996) in Transgenic Plants: A Production System for lndustrial and Pharmaceutical Proteins" Owen and Pen Eds., John Wiley & Sons, West Sussex, England).

Examples of medically relevant proteins that may be produced in plants include surface antigens of viral pathogens, such as hepatitis B virus and transmissible gastroenteritis virus spike protein, for use in vaccines. The proteins thus produced may be isolated and administered through standard vaccine introduction methods or through the consumption of the edible transgenic plant as food which can be taken orally (see, e.g., U.S. Patent No. 6,136,320 and Mason et al. (1992) Proc. Natl. Acad. Sci. U.S.A. 89:11745-11749). HIV, rhinovirus, malarial and rabies virus antigens are additional examples of that may be expressed in plants as candidate vaccines (see, e.g., Porta et al. (1994) Virol. 202:949-955; Turpen et al. (1995) Bio/Technology 13:53-57; and McGarvey et al. (1995) Bio/Technology 13:1484-1487). Antibodies may also be produced in plants, including, for example, a gene fusion encoding an antigen-binding single chain Fv protein (scFv) that recognizes the hapten oxazolone (Fiedler and Conrad (1995) Bio/Technology 13:1090-1093) and IgG (Ma et al. (1995) Science 268:716-719).

Examples of human biopharmaceuticals that may be expressed in plants include, but are not limited to, albumin (Sijmons *et al.* (1990)), enkephalins (Vandekerckhove *et al.* (1989) ), interferon-*α* (Zhu *et al.* (1994) and GM-CSF (Ganz et al. (1996) in Transgenic Plants: A Production System for lndustrial and Pharmaceutical Proteins, Owen and Pen Eds., John Wiley & Sons, West Sussex, England, pp. 281-297; and Sardana et al. (1998) in Methods in Biotechnology, Vol. 3: Recombinant Proteins from Plants: Production and lsolation of Clinically Useful Compounds, Cunningham and Porter, Eds., Humana Press, New Jersey; pp. 77-87).

Transgenic plants producing these compounds are made possible by the introduction and expression of one or potentially many genes using the artificial chromosomes provided herein. The vast array of possibilities include, but are not limited to, any biological compound which is presently produced by any organism such as proteins, nucleic acids, primary and intermediary metabolites, carbohydrate polymers, enzymes for uses in bioremediation, enzymes for modifying pathways that produce secondary plant metabolites such as flavonoids or vitamins, enzymes that could produce pharmaceuticals and for introducing enzymes that could produce compounds of interest to the manufacturing industry such as specialty chemicals and plastics. The compounds may be produced by the plant, extracted upon harvest and/or processing, and used for any presently recognized useful purpose such as pharmaceuticals, fragrances, and industrial enzymes to name a few. Alternatively, plants produced in accordance with the methods and compositions provided herein may be made to metabolize certain compounds, such as hazardous wastes, thereby allowing bioremediation of these compounds.

### j. Non-protein-expressing sequences

Nucleic acids may be introduced into plants that are designed to down-regulate or supress a plant-encoded gene. A number of different means to achieve down regulation have been demonstrated in the art, including antisense RNA, ribozymes and co-suppression. The use of antisense RNA to suppress plant genes is described, for example, in U.S. Patent Nos. 4,801,540, 5,107,065 and 5,453,566. ln such methods, an "antisense" gene is constructed that encodes an RNA that is complementary to the mRNA of a resident plant gene, such that expression of the antisense gene inhibits the translation of the mRNA of the resident plant gene. Thus, the activity of the resident gene is down-regulated.

An additional method of down regulating gene activities involves ribozymes, or catalytic hammerhead hairpin RNA structures. The use of ribozymes is described, for example, in U.S. Patent Nos. 4,987,071, 5,037,746, 5,116,742 and 5,354,855. These methods rely on the expression of small catalytic "hammerhead" RNA molecules that are capable of binding to and cleaving specific RNA sequences. Ribozymes designed to specifically recognize a resident plant mRNA can be used to cleave the mRNA and prevent its proper expression.

Essentially a more or less equivalent down-regulation control of gene activities by ribozymes and antisense can be achieved by adding additional copies of the gene to be regulated. The process is referred to as co-suppression and is described in, for example, U.S. Patent Nos. 5,034,323, 5,283,184 and 5,231,020.

Numerous plant genes may be targeted for down regulation. For example, a gene may be down-regulated that encodes an enzyme that catalyzes a reaction in a plant. Reduction of the enzyme activity may reduce or eliminate products of the reaction which include any enzymatically synthesized compound in the plnat such as fatty acids, amino acids, carbohydrates, nucleic acids and the like. Alternatively, the protein may be a storage protein, such as zein, or a structural protein, the decreased expression of which may lead to changes in seed amino acid composition or plant morphological changes, respectively. The possibilities cited above are provided only by way of example and do not represent the full range of applications.

### (1). Antisense RNA

Genes may be constructed, which when transcribed, produce antisense RNA that is complementary to all or part(s) of a targeted messenger RNA(s). The antisense RNA reduces production of the polypeptide product of the messenger RNA. The polypeptide product may be any protein encoded by the plant genome. The aforementioned genes will be referred to as antisense genes. An antisense gene may thus be introduced into a plant by transformation methods to produce a transgenic plant with reduced expression of a selected protein of interest. For example, the protein may be an enzyme that catalyzes a reaction in the plant. Reduction of the enzyme activity may reduce or eliminate products of the reaction which include any enzymatically synthesized compound in the plant such as fatty acids, amino acids, carbohydrates, nucleic acids and the like. Alternatively, the protein may be a storage protein, such as a zein, or a structural protein, the decreased expression of which may lead to changes in seed amino acid composition or plant morphological changes respectively. The possibilities cited above are provided only by way of example and do not represent the full range of applications.

### (2.) Ribozymes

Genes also may be constructed or isolated, which when transcribed, produce RNA enzymes (ribozymes) which can act as endoribonucleases and catalyze the cleavage of RNA molecules with selected sequences. The cleavage of selected messenger RNAs can result in the reduced production of their encoded polypeptide products. These genes may be used to prepare transgenic plants which possess them. The transgenic plants may possess reduced levels of polypeptides including, but not limited to, the polypeptides cited above.

Ribozymes are RNA-protein complexes that cleave nucleic acids in a site-specific fashion. Ribozymes have specific catalytic domains that possess endonuclease activity (Kim and Cech, 1987; Gerlach *et al.,* 1987; Forster and Symons, 1987). For example, a large number of ribozymes accelerate phosphoester transfer reactions with a high degree of specificity, often cleaving only one of several phophoesters in an oligonucleotide substrate (Cech *et al.,* 1981; Michel and Westhof, 1990); Reinhold-Hurek and Shub, 1992). This specificity has been attributed to the requirement that the substrate bind *via* specific base-pairing interactions to the internal guide sequence ("lGS") of the ribozyme prior to chemical reaction.

Ribozyme catalysis has primarily been observed as part of sequence-specific cleavage/ligation reactions involving nucleic acids (Joyce, 1989; Cech *et al.,* 1981). For example, U.S. Patent 5,354,855 reports that certain ribozymes can act as endonucleases with a sequence specificity greater than that of known ribonucleases and approaching that of the DNA restriction enzymes.

Several different ribozyme motifs have been described with RNA cleavage activity (Symons, 1992). Examples include sequences from the Group I self splicing introns including Tobacco Ringspot Virus (Prody *et al.,* 1986), Avacado Sunblotch Viroid (Palukaitis *et al.,* 1979; Symons, 1981) and Lucerne Transient Streak Virus (Forster and Symons, 1987). Sequences from these and related viruses are referred to as hammerhead ribozyme based on a predicted folded secondary structure.

Other suitable ribozymes include sequences from RNase P with RNA cleavage activity (Yuan *et al.,* 1992; Yuan and Altman, 1994; U.S. Patents 5,168,053 and 5,624,824), hairpin ribozyme structures (Berzal-Herranz *et al.,* 1992; Chowrira *et al.,* 1993) and Hepatitis Delta virus based ribozymes (U.S. Patent 5,625,047). The general design and optimization of ribozyme directed RNA cleavage activity has been discussed in detail (Haselhoff and Gerlach, 1988; Symons, 1992; Chowrira *et al.,* 1994; Thompson *et al*., 1995).

The other variable on ribozyme design is the selection of a cleavage site on a given target RNA. Ribozymes are targeted to a given sequence by virtue of annealing to a site by complementary base pair interactions. Two stretches of homology are required for this targeting. These stretches of homologous sequences flank the catalytic ribozyme structure defined above. Each stretch of homologous sequence can vary in length from 7 to 15 nucleotides. The only requirement for defining the homologous sequences is that, on the target RNA, they are separated by a specific sequence which is the cleavage site. For hammerhead ribozyme, the cleavage site is a dinucleotide sequence on the target RNA is a uracil (U) followed by either an adenine, cytosine or uracil (A, C or U) (Perriman *et al.,* 1992; Thompson *et al.,* 1995). The frequency of this dinucleotide occurring in any given RNA is statistically 3 out of 16. Therefore, for a given target messenger RNA of 1,000 bases, 187 dinucleotide cleavage sites are statistically possible.

Designing and testing ribozymes for efficient cleavage of a target RNA is a process well known to those skilled in the art. Examples of scientific methods for designing and testing ribozymes are described by Chowrira *et al.* (1994) and Lieber and Strauss (1995), each incorporated by reference. The identification of operative and preferred sequences for use in down regulating a given gene is simply a matter of preparing and testing a given sequence, and is a routinely practiced "screening" method known to those of skill in the art.

### (3.) Induction of gene silencing

It also is possible that genes may be introduced to produce transgenic plants which have reduced expression of a native gene product by the mechanism of co-suppression. It has been demonstrated in tobacco, tomato, and petunia (Goring *et al.,* 1991; Smith *et a*/*.,* 1990; Napoli *et a*/*.,* 1990; van der Krol *et al.,* 1990) that expression of the sense transcript of a native gene will reduce or eliminate expression of the native gene in a manner similar to that observed for antisense genes. The introduced gene may encode all or part of the targeting native protein but its translation may not be required for reduction of levels of that native protein.

### (4.) Non-RNA-expressing sequences

DNA elements including those of transposable elements such as Ds, Ac, or MU, may be inserted into a gene to cause mutations. These DNA elements may be inserted in order to inactivate (or activate) a gene and thereby "tag" a particular trait. In this instance the transposable element does not cause instability of the tagged mutation, because the utility of the element does not depend on its ability to move in the genome. Once a desired trait is tagged, the introduced DNA sequence may be used to clone the corresponding gene, e.g., using the introduced DNA sequence as a PCR primer together with PCR gene cloning techniques (Shapiro, 1983; Dellaporta *et al.,* 1988). Once identified, the entire gene(s) for the particular trait, including control or regulatory regions where desired, may be isolated, cloned and manipulated as desired. The utility of DNA elements introduced into an organism for purposes of gene tagging is independent of the DNA sequence and does not depend on any biological activity of the DNA sequence, *i.e.,* transcription into RNA or translation into protein. The sole function of the DNA element is to disrupt the DNA sequence of a gene.

It is contemplated that unexpressed DNA sequences, including synthetic sequences, could be introduced into cells as proprietary "labels" of those cells and plants and seeds thereof. It would not be necessary for a label DNA element to disrupt the function of a gene endogenous to the host organism, as the sole function of this DNA would be to identify the origin of the organism. For example, one could introduce a unique DNA sequence into a plant and this DNA element would identify all cells, plants, and progeny of these cells as having arisen from that labeled source. It is proposed that inclusion of label DNAs would enable one to distinguish proprietary germplasm or germplasm derived from such, from unlabelled germplasm.

Another possible element which may be introduced is a matrix attachment region element (MAR), such as the chicken lysozyme A element (Stief, 1989), which can be positioned around an expressible gene of interest to effect an increase in overall expression of the gene and diminish position dependent effects upon incorporation into the plant genome (Stief *et al.,* 1989; Phi-Van *et al.,* 1990). Sequences such as MARs can be included on the artificial chromosome to enhance gene expression.

### 3. Transgenic models for evaluation of genes and discovery of new traits

Of significant interest is the use of plants and plant cells containing artificial chromosomes for the evaluation of new genetic combinations and discovery of new traits. Artificial chromosomes, by virtue of the fact that they can contain significant amounts of DNA can also therefore encode numerous genes and accordingly a multiplicity of traits. It is contemplated here that artificial chromosomes, when formed from one plant species, can be evaluated in a second plant species. The resultant phenotypic changes observed, for example, can indicate the nature of the genes contained within the DNA containing the artificial chromosome, and hence permit the identification of new genetic activities. Artificial chromsomes containing euchromatic DNA or partially containing euchromatic DNA can serve as a valuable source of new traits when transferred to an alien plant cell environment. For example, it is contemplated that artificial chromosomes derived from dicot plant species can be introduced into monocot plant species by transfering a dicot artificial chromosome. The dicot artificial chromosome containing a region of euchromatic DNA containing expressed genes.

The artificial chromosomes can be generated or manipulated in such a fashion that a large region of naturally occurring plant DNA becomes incorporated into the artificial chromosome. This allows the artificial chromosome to contain new genetic activities and hence carry new traits. For example, an artificial chromosome can be introduced into a wild relative of a crop plant under conditions whereby a portion of the DNA present in the chromosomes of the wild relative is transferred to the artificial chromosome. After isolation of the artificial chromosome, this naturally occurring region of DNA from the wild relative, now located on the artificial chromosome can be introduced into the domesticated crop species and the genes encoded within the transferred DNA expressed and evaluated for utility. New traits and gene systems can be discovered in this fashion.

Artificial chromosomes modified to recombine with plant DNA offer many advantages for the discovery and evaluation of traits in different plant species. When the artificial chromosome containing DNA from one plant species is introduced into a new plant species, new traits and genes can be introduced. This use of an artificial chromosome allows for the ability to overcome the sexual barrier that prevents transfer of genes from one plant species to another species. Using artificial chromosomes in this fashion allows for many potentially valuable traits to be identified including traits that are typically found in wild species. Other valuable applications for artificial chromosomes include the ability to transfer large regions of DNA from one plant species to another, DNA encoding potentially valuable traits such as altered oil, carbohydrate or protein composition, multiple genes encoding enzymes capable of producing valuable plant secondary metabolites, genetic systems encoding valuable agronomic traits such as disease and insect resistance, genes encoding functions that allow association with soil bacterium such as growth promoting bacteria or nitrogen fixing bacteria, or genes encoding traits that confer freezing, drought or other stress tolerances. In this fashion, artificial chromosomes can be used to discover regions of plant DNA that encode valuable traits.

The artificial chromosome can also be designed to allow the transfer and subsequent incorporation of these valuable traits now located on the artificial chromosome into the natural chromosomes of a plant species. In this fashion the artificial chromosomes can be used to transfer large regions of DNA encoding traits normally found in one plant species into another plant species. In this fashion, it is possible to derive a plant cell that no longer needs to carry an artificial chromosome to posses the new trait. Thus the artificial chromosome would serve as the transfer mechanism to permit the formation of plants with greater degree of genetic diversity.

An artificial chromosome can be designed in a variety of ways to accomplish the afore-mentioned purposes. An artificial chromosome can be modified to contain sequences that promote homologous recombination within plant cells, or be modified to contain a genetic system that functions as a site-specific recombination system. For example, the DNA sequence of *Arabidopsis* is now known. To construct an artificial chromosome capable of recombining with a specific region of *Arabidopsis* DNA, a sequence of *Arabidopsis* DNA, normally located near a chromosomal location encoding genes of potential interest can be introduced into an artificial chromosome by methods provided herein. It may be desireable to include a second region of DNA within the artificial chromosome that provides a second flanking sequence to the region encoding genes of potential interest, to promote a double recombination event which would ensure transfer of the entire chromosomal region encoding genes of potential interest to the artificial chromosome. The modified artificial chromosome, containing the DNA sequences capable of homologous recombination region can then be introduced into *Arabidopsis* cells and the homologous recombination event is selected.

It is convenient to include a marker gene to allow for the selection of a homologous recombination event. The marker gene is preferably inactive unless activated by an appropriate homologous recombination event. For example, US 5,272,071, describes a method where an inactive plant gene is activated by a recombination event such that desired homologous recombination events can be easily scored. Similarly, US 5,501,967 describes a method for the selection of homologous recombination events by activation of a silent selection gene first introduced into the plant DNA, the gene being activated by an appropriate homologous recombination event. Both of these methods can be applied to enable a selective process to be included in to select for recombination between an artificial chromosome and a plant chromosome. Once the homologous recombination event is detected, the artificial chromosome, once selected, is isolated and introduced into a recipient cell, for example, tobacco, corn, wheat or rice, and the expression of the newly introduced DNA sequences evaluated. Selection of recombinant events can take place in cell culture, or following seed formation and screening of seedling plants or seed itself.

Phenotypic changes in the recipient plant cells containing the artificial chromosome, or in regenerated plants containing the artificial chromosome, allows for the evaluation of the nature of the traits encoded by the genes of interest, for example, *Arabidopsis* DNA, under conditions naturally found in plant cells, including the naturally occurring arrangement of DNA sequences responsible for the developmental control of the traits in the normal chromosomal environment.

Traits such as durable fungal or bacterial disease resistance, new oil and carbohydrate compositions, valuable secondary metabolites such as phytosterols, flavonoids, efficient nitrogen fixation or mineral utilization, resistance to extremes of drought, heat or cold are all found within different populations of plant species and are often governed by multiple genes. The use of single gene transformation technologies does not permit the evaluation of the multiplicity of genes controlling many valuable traits. Thus, incorporation of these genes into artificial chromosomes allows the rapid evaluation of the utility of these genetic combinations in heterologous plant species.

The large scale order and structure of the artificial chromosome provides a number of unique advantages in screening for new utilities or new phenotypes within heterologous plant species. The size of new DNA that can be carried by an artificial chromosome can be millions of base pairs of DNA, representing potentially numerous genes that may have different or new utility in a heterologous plant cell. The artificial chromosome is a "natural" environment for gene expression, the problems of variable gene expression and silencing seen for genes transferred by random insertion into a genome should not be observed. Similarly, there is no need to engineer the genes for expression, and the genes inserted would not need to be recombinant genes. Thus, transferred genes are fully expected to be expressed in the typical temporal and spatial fashion as observed in the species from where the genes were initially isolated. A valuable feature for these utilities is the ability to isolate the artificial chromosomes and to further isolate, manipulate and introduce into other cells artificial chromosomes carrying unique genetic compositions.

Thus, the use of artificial chromosomes and homologous recombination in plant cells can be used to isolate and identify many valuable crop traits. In addition to the use of artificial chromosomes for the isolation and testing of large regions of naturally occurring DNA, methods for the use of artificial chromosomes and cloned DNA are also contemplated. Similar to that described above, artificial chromsomes can be used to carry large regions of cloned DNA, including that derived from other plant species.

The ability to incorporate DNA elements into artificial chromosomes as they are being formed allows for the development of artificial chromosomes specifically engineered as a platform for testing of new genetic combinations, or "genomic" discoveries for model species such as *Arabidopsis.* Specific "recombinase" systems can be used in plant cells to excise or re-arrange genes; these same systems can be used to derive new gene combinations contained on an artificial chromosome. In this regard, it is contemplated that the use of site specific recombination sequences can have considerable utility in developing artificial chromosomes containing DNA sequences recognized by recombinase enzymes and capable of accepting DNA sequences containing same. The use of site-specific recombination as a means to target an introduced DNA to a specific locus has been demonstrated in the art and such methods can be employed. The recombinase systems can also be used to transfer the cloned DNA regions contained within the artificial chromosome to the naturally occurring plant chromosomes.

Many site specific recombinases have been described in the literature (Kilby et al., Trends in Genetics, 9(12): 413-418, 1993). Among these are: an activity identified as R encoded by the pSR1 plasmid of *Zygosaccharomyes rouxii,* FLP encoded for the 2um circular plasmid from *Saccharomyces cerevisiae* and *Cre-lox* from the phage P1.

The integration function of site specific recombinases is contemplated as a means to assist in the derivation of genetic combinations on artificial chromosomes. In order to accomplish this, it is contemplated that a first step of introducing site-specific recombinase sites into the genome of a plant cell in an essentially random manner is conducted, such that the plant cell has one or more site-specific recombinase recognition sequences on one or more of the plant chromosomes. An artificial chromosome is then introduced into the pant cell, the artificial chromosome engineered to contain a recombinase recognition site capable of being recognized by a site specific recombinase. Optionally a gene encoding a recombinase enzyme is also included, preferably under the control of an inducible promoter. Expression of the site specific recombinase enzyme in the plant cell, either by induction of a inducible recombinase gene, or transient expression of a recombinase sequence causes a site-specific recombination event to take place, leading to the insertion of a region of the plant chromosomal DNA containing the recombinase recognition site into the recombinase recognition site of the artificial chromosome, forming an artificial chromosome containing plant chromosomal DNA. The artificial chromosome can be isolated and introduced into a heterologous host, preferably a plant host, and expression of the newly introduced plant chromosomal DNA can be monitored and evaluated for desirable phenotypic changes. Accordingly, carrying out this recombination with a population of plant cells wherein the chromosomally located recombinase recognition site is randomly scattered throughout the chromosomes of the plant can lead to the formation of a population of artificial chromosomes, each with a different region of plant chromosomal DNA, each representing a new genetic combination.

This particular method involves the precise site-specific insertion of chromosomal DNA into the artificial chromosome. This precision has been demonstrated in the art. For example, Fukushige and Sauer (Proc. Natl. Acad. Sci. USA, 89:7905-7909, 1992) demonstrated that the *Cre-lox* homologous recombination system could be successfully employed to introduce DNA into a predefined locus in a chromosome of mammalian cells. In this demonstration a promoter-less antibiotic resistance gene modified to include a *lox* sequence at the 5' end of the coding region was introduced into CHO cells. Cells were retransformed by electroporation with a plasmid that contained a promoter with a *lox* sequence and a transiently expressed Cre recombinase gene. Under the conditions employed, the expression of the Cre enzyme catalyzed the homologous recombination between the *lox* site in the chromosomally located promoter-less antibiotic resistance gene and the *lox* site in the introduced promoter sequence leading to the formation of a functional antibiotic resistance gene. The authors demonstrated efficient and correct targeting of the introduced sequence, 54 of 56 lines analyzed corresponded to the predicted single copy insertion of the DNA due to *Cre* catalyzed site specific homologous recombination between the *lox* sequences.

The use of the same *Cre-lox* system has been demonstrated in plants (Dale and Ow, Gene 91:79-85, 1995) to specifically excise, delete or insert DNA. The precise event is controlled by the orientation of *lox* DNA sequences, in *cis* the *lox* sequences direct the *Cre* recombinase to either delete *(lox* sequences in direct orientation) or invert *(lox* sequences in inverted orientation) DNA flanked by the sequences, while *in trans* the *lox* sequences can direct a homologous recombination event resulting in the insertion of a recombinant DNA. Accordingly a *lox* sequence may be first added to a genome of a plant species capable of being transformed and regenerated to a whole plant to serve as a recombinase target DNA sequence for recombination with an artificial chromosome. The *lox* sequence may be optimally modified to further contain a selectable marker which is inactive but can be activated by insertion of the *lox* recombinase recognition sequence into the artificial chromosome.

A promoterless marker gene or selectable marker gene linked to the recombinase recognition sequence, which is first inserted into the chromosomes of a plant cell can be used to engineer a platform chromosome. A promoter is linked to a recombinase recognition site, in an orientation that allows the promoter to control the expression of the marker or selectable marker gene upon recombination within the artificial chromosome. Upon a site-specific recombination event between a recombinase recognition site in a plant chromosome and the recombinase recognition site within the the introduced artificial chromosome, a cell is derived with a recombined artificial chromosome, the artificial chromosome containing an active marker or selectable marker acitivity that permits the identification and or selection of the cell.

The artificial chromosomes can be transferred to other plant species and the functionality of the new combinations tested. The ability to conduct such an inter-chromosomal transfer of sequences has been demonstrated in the art. For example, the use of the Cre-lox recombinase system to cause a chromosome recombination event between two chromatids of different chromosomes has been shown

Any number of recombination systems may be employed (see, U.S. provisional application Serial No. filed the same day herewith under attorney docket no. 24601-P420). Such systems include, but are not limited to, bacterially derived systems such as the lnt/*att* system of phage lambda and the Gin/*gix* system.

More than one recombination system may be employed, including, for example, one recombinase system for the introduction of DNA into an artificial chromosome, and a second recombinase system for the subsequent transfer of the newly introduced DNA contained within an artificial chromosome into the naturally occurring chromosome of a second plant species. The choice of the specific recombination system used will be dependent on the nature of the modification contemplated.

By having the ability to isolate an artificial chromosome and in particular artificial chromosomes containing plant chromosomal DNA introduced via site-specific recombination and re-introduce the chromosome into other cells, particularly plant cells, these new combinations can be evaluated in different crop species without the need to first isolate and modify the genes, or carry out multiple transformations or gene transfers to achieve the same combination isolation and testing combinations of the genes in plants. The use of a site specific recombinase and artificial chromosomes also allows the convenient recovery of the plant chromosomal region into other recombinant DNA vectors and systems for manipulation and study.

The artificial chromosomes can be engineered as platforms to accept large regions of cloned DNA, such as that contained in Bacterial Artificial Chromosomes (BACs) or Yeast Artificial Chromosomes (YACs). It is further contemplated, that as a result of the typical structure of amplification-based artificial chromosomes, such as, for example, SATACS (or ACes), containing tandemly repreated DNA blocks, that more than cloned DNA sequence can be introduced by recombination processes. ln particular recombination within a predefined region of the tandemly repreated DNA within the artifical chromosome provides a mechanism to "stack" numerous regions of cloned DNA, including large regions of DNA contained within BACs or YACs clones. Thus, multiple combinations of genes can be introduced onto artificial chromosomes and these combinations tested for functionality. In particular, it is contemplated that multiple YACs or BACs can be stacked onto an artificial chromsomes, the BACs or YACs containing multiple genes of complex pathways or mutlipe genetic pathways. The BACs or YACs are typically selected based on genetic information available within the public domain, for example from the Arabidopsis Information Management System **(http://aims.cps.msu.edu/aims/index.html)** or the information related to the plant DNA sequences available from the Institute for Genomic Research **(http://www.tigr.org)** and other sites known to those skilled in the art. Alternatively, clones can be chosen at random and evaluated for functionality. It is contemplated that combinations providing a desired phenotype can be identified by isolation of the artificial chromosome containing the combination and analyzing the nature of the inserted cloned DNA.

In another embodiment of the methods provided herein for discovering genes associated with plant traits, the artificial chromosome used to transfer plant DNA to a host cell for evaluation therein will contain large regions of plant DNA, in particular plant euchromatin, as a result of the process by which the artificial chromosome is produced. In particular, the artificial chromosome may be an amplification-based artificial chromosome, including, but not limited to: (1) a minichromosome arising from breakage of a dicentric chromosome, (2) an artificial chromosome containing one or more regions of repeating nucleic acid units wherein the repeat region(s) contain substantially equivalent amounts of euchromatic and heterochromatic nucleic acid, (3) an artificial chromosome containing one or more regions of repeating nucleic acid units wherein the repeat region(s) is made up predominantly of euchromatic DNA or contains about 10%, 20%, 30%, 40%, 50%, 60%, 70%, 80%, 90% or greater than 90% euchromatic DNA, (4) an artificial chromosome containing one or more regions of repeating nucleic acid units wherein the artificial chromosome is made up of substantially equivalent amounts of heterochromatin and euchromatin, (5) an artificial chromosome that containing one or more regions of repeating nucleic acid units having common nucleic acid sequences that represent euchromatic and heterochromatic nucleic acid and (6) a sausage-like structure that contains a portion or all of a euchromatin-containing arm of a plant chromosome.

ln these methods for discovering genes associated with plant traits, because the artificial chromosome used to transfer plant DNA to a host cell for evaluation therein is generated to already contain large amounts of plant DNA, in particular plant euchromatin, there is no need to introduce plant euchromatin into the artificial chromosomes, by homologous or site-specific recombination.

### 4. Use of artificial chromosomes for preparation and screening of libraries

Since large fragments of DNA can be incorporated into artificial chromosomes (ACs), they are well-suited for use as cloning vehicles that can accommodate entire genomes in the preparation of genomic DNA libraries, which then can be readily screened for functionality as described above or for specific gene sequences for further modification and study. For example, it is possible to use artificial chromosomes to prepare artificial chromosome libraries containing plant genomic DNA library useful in the identification and isolation of functional DNA components such as genes, centromeric DNA and telomeric DNA from a variety of different species of plants.

The following examples are included for illustrative purposes only and are not intended to limit the scope of the invention.

### Example 1

### Generation of Arabidopsis protoplasts

Plant protoplasts are typically generated from plant cells following standard techniques (for example, Maheshwari et al., Crit. Rev. Plant Sci. 14:149-178, 1995; Ramulu et al., Methods in Molecular Biology 111 227-242, 1999). Typically plant protoplasts are prepared from fresh plant tissue, e.g., leaf, or can be prepared by converting cell suspension cultures to protoplasts by removal of the cell walls enzymatically. For production of *Arabidopsis* protoplasts, the methods of Karesh et al. (Plant Cell Reports 9: 575-578, 1991) and Mathur et al. (Plant Cell Reports 14:21-226, 1995) were used to generate *Arabidopsis* suspension cultures by modifications thereof as described below. These cells were maintained in liquid culture and subcultured as required, usually between 7 and 10 days in culture.

### Establishment of suspension cultures

Cell suspension cultures derived from root callus of *Arabidopsis* thaliana cv. Columbia, RLD and Landsburg l erecta*'* were used. Calli were induced from roots of 3 week-old seedlings on callus induction medium containing MS basic media (Murashige and Skoog (1962) Physiol. Plant 15:473-497) with 3% sucrose, 0.5mg/l napthalene acetic acid (NAA), 0.05 mg/l Kinetin (Sigman Aldrich Canada). The cell suspension cultures were grown from the calli in liquid callus induction medium at 22°C with shaking at 120 rpm. They were subcultured every 7 days.

### Generation of protoplasts

One gram of 4-5 day-old suspension culture was incubated in 6 ml enzyme solution containing 1% Cellulase 'Onozuka' R-10 and 0.25% Macerozyme R-10 in 35 g/l CaCl₂-2H₂O (Hartmann et al. (1998) Plant Mol. Biol. 36:741 -754) and incubated at 22°C in the dark with shaking at 70 rpm for 15 h. The protoplast mixture was poured through a 100 *µ*m nylon mesh sieve and centrifuged at 250xg for 5 min. The protoplasts were washed with 35 g/l CaCl₂.2H₂O and resuspended in 10 ml floating medium containing B5 medium (Gamborg et al. (1968) Exp. Cell Res. 50:151-158) with 144 g/l sucrose and 1 mg/l 2,4-dichlorophenoxyacetic acid (2,4-D). The protoplasts were centrifuged at 80xg for 10 min, collected at the interface and used immediately for transfection.

### Example 2

### Generation of Tobacco Mesophyll Protoplasts

Mesophyll protoplasts were generated from leaves of sterile plantlets of N. *tabacum* cv. Xanthi. The plantlets were grown aseptically on MSO medium (MS basal media, 3% sucrose, 0.05% morpholinoethanesulfonic acid (MES), 1.0 mg/l benzyl adenine (BA), 0.1 mg/l NAA and 0.8% agar, pH 5.8) at 22°C under a 16/8 h photoperiod (see also Bilang et al. (1994) Plant Molecular Biology, Manual A 1:1-6*).* Fully expanded leaves (2 x 4 cm) were cut in half, the main vein removed and the upper epidermis scored with parallel cuts. Leaf pieces were immersed in 6 ml enzyme solution containing 1.2% Cellulase 'Onozuka' R-10 and 0.4% Macerozyme R-10 in K4 medium (Nagy and Maliga (1976) Z. Pflanzenpysiol. 78:453-455) and incubated at 22°C for 15 h without shaking. The protoplasts were purified by pouring through a 100 *µ*m nylon mesh sieve. Suspension of protoplasts was carefully overlayed with 1 ml W5 solution (Bilang et al. (1994) Plant Molecular Biology Manual A 1: 1-6) and centrifuged at 80xg for 10 min. Protoplasts were then resuspended in W5 solution at a density of 1 × 10⁶ protoplasts/ml and stored at 4°C for 1 to 2 hours prior to treatment, for example, DNA uptake or chromosome transfer.

### Example 3

### Production of Tobacco Protoplasts from Suspension Cultures

Tobacco BY-2 protoplasts are prepared from suspension cultures according to the method of Nagata et al. [(1981) Molecular and General Genetics, 184:161-165].

### Example 4

### Generation of Brassica Hypocotyl Protoplasts

Genotypes of *Brassica napus, B. oleracea, B. juncea and B. carinata* may be used to generate protoplasts. Seeds of *Brassica napus* were surface-sterilized (for 2 min with 70% ethanol, then for 20 min with 2.4% sodium hypochlorite containing one drop of Tween 20 per 100 ml). Seeds were rinsed thoroughly with sterile distilled water and grown aseptically on autoclaved germination medium (half-strength basal Murashige and Skoog's medium (MS), 1% sucrose, 0.8% agar, pH 5.8). Unless otherwise indicated, the protoplast generation procedures were performed aseptically and solutions and media were filter-sterilized. Alternatively, protoplasts can be generated and cultured successfully from different explants using various protocol modifications (for example, Kao et al. (1991) Plant Science 75:63-72; Kao et al. (1990) Plant Cell Rep. 9:311-315; Kao and Seguin-Swartz (1987) Plant Cell Tiss. Org. Cult. 10:79-90; Kao (1977) Mol. Gen. Genet. 150:225-230).

### Generation of Hypocotyl Protoplasts

Hypocotyls were excised from 4 or 5 day-old seedlings grown aseptically in the dark with or without light exposure for a few hours prior to use. The explants were cut transversely into 2-5 mm pieces and incubated in enzyme solution (salts, vitamins and organic acids of Kao's medium (Kao (1977) Mol. Gen. Genet. 150:225-230), 0.4 g/l CaCl₂.2H₂0, 13% sucrose, 1% Cellulase*'* Onozuka R10', 0.1% Pectolyase Y23, pH 5.6) in petri dishes, in darkness, without agitation for 14-18 hours, then with agitation on a rotary shaker (ca. 50 rpm) for 15-30 min.

The mixture was filtered through a 63 *µ*m nylon screen into centrifuge tubes, and an equal volume of 17.5% sucrose was added to each tube. Following centrifugation (ca. 100×g, 8 min), the protoplast band that formed at the top of each tube was collected. Protoplasts were washed 3 times by resuspension in wash solution [solution W5 of Menczel and Wolfe (1984, Plant Cell Rep 3:196-198) at a reduced strength (0.8×)] followed by centrifugation at 100×g for 3-5 min and discarding the supernatant.

Protoplasts were cultured in Kao's medium containing the salts, vitamins and organic acids with 30 g/I sucrose, 68.4 g/I glucose, 0.5 mg/l NAA, 0.5 mg/l BA, 0.5 mg/l 2,4-D, pH 5.7, at a density of 1 × 10⁵ per ml and incubated at 25°C, 16 h photoperiod, in dim fluorescent light (25 *µ*Em⁻² S⁻¹).

After 5-8 days in culture, 1-1.5 ml of feeder medium containing the above medium except with 55.8 g/I glucose instead of 68.4 g/l, were added to each dish, and the dishes were placed under brighter fluorescent light (50 *µ*Em⁻² s⁻¹). At about 14 days, 1-2 ml of medium were removed from each dish, and 2-3 ml of feeder medium containing basal B5 medium (Gamborg et al. (1968) Exp. Cell Res. 50:151-158), 3% sucrose, 3.8% glucose, 0.5 mg/l BA, 0.5 mg/l NAA, and 0.5 mg/l 2,4-D, pH 5.7, were added. At about 21 days, if microcolonies have not yet formed, the cultures can be fed with the last feeder medium except with 2.2% glucose instead of 3.8%. Protoplast cultures can be washed when necessary by adding new feeder medium, gently swirling petri dishes, allowing cells to settle, removing most of the supernatant and adding fresh medium to the dishes.

At 3-5 weeks, microcolonies were embedded with medium containing a 1:1 mixture of the last feeder medium and proliferation medium which contains the components of the feeder medium with 0.9% glucose and 1.6% agarose to make a concentration of 0.8% in the final mixture. Cultures were incubated as described above in bright fluorescent light (80-100 *µ*Em⁻² s⁻¹). After 10 days-2 weeks, green colonies were plated onto the regeneration medium.

### Example 5

### Preparation of a Transformation Vector Useful for the Induction of Plant Artificial Chromosome Formation

Plant artificial chromosomes (PACs) can be generated by introducing nucleic acid, such as DNA, which can include an amplification-inducing DNA and/or a targeting DNA, for example rDNA or lambda DNA, into a plant cell, allowing the cell to grow, and then identifying from among the resulting cells those that include a chromosome with a structure that is distinct from that of any chromosome that existed in the cell prior to introduction of the nucleic acid. The structure of a PAC reflects amplification of chromosomal DNA, for example, segmented, repeat region-containing and heterochromatic structures. It is also possible to select cells that contain structures that are precursors to PACs, for example, chromosomes containing more than one centromere and/or fragments thereof, and culture and/or manipulate them to ultimately generate a PAC within the cell.

In the method of generating PACs, the nucleic acid can be introduced into a variety of plant cells. The nucleic acid can include targeting DNA and/or a plant expressable DNA encoding one or multiple selectable markers (e.g., DNA encoding bialophos (bar) resistance) or scorable markers (e.g., DNA encoding GFP). Examples of targeting DNA include, but are not limited to, *N. tabacum* rDNA intergenic spacer sequence (lGS) and *Arabidopsis* rDNA such as the 18S, 5.8S, 26S rDNA and/or the intergenic spacer sequence. The DNA can be introduced using a variety of methods, including, but not limited to *Agrobacterium-mediated* methods, PEG-mediated DNA uptake and electroporation using, for example, standard procedures according to Hartmann et al [(1998) Plant Molecular Biology 36:741]. The cell into which such DNA is introduced can be grown under selective conditions and can initially be grown under non-selective conditions and then transferred to selective media. The cells or protoplasts can be placed on plates containing a selection agent to grow, for example, individual calli. Resistant calli can be scored for scorable marker expression. Metaphase spreads of resistance cultures can be prepared, and the metaphase chromosomes examined by FISH analysis using specific probes in order to detect amplification of regions of the chromosomes. Cells that have artificial chromosomes with functioning centromeres or artificial chromosomal intermediate structures, including, but not limited to, dicentric chromosomes, formerly dicentric chromosomes, minichromosomes, heterochromatin structures (e.g. sausage chromosomes), and stable self-replicating artificial chromosomal intermediates as described herein, are identified and cultured. In particular, the cells containing self-replicating artificial chromosomes are identified.

The DNA introduced into a plant cell for the generation of PACs can be in any form, including in the form of a vector. An exemplary vector for use in methods of generating PACs can be prepared as follows.

For the production of artificial chromosomes, plant transformation vectors, as exemplified by pAglla and pAgllb, containing a selectable marker, a targeting sequence, and a scorable marker were constructed using procedures well known in the art to combine the various fragments. The vectors can be prepared using vector pAg1 as a base vector and inserting the following DNA fragments into pAg1: DNA encoding *β*-glucoronidase under the control of the nopaline synthase (NOS) promoter fragment and flanked at the 3' end by the NOS terminator fragment, a fragment of mouse satellite DNA and an N. *tabacum* rDNA intergenic spacer sequence (IGS). In constructing plant transformation vectors, vector pAg2 can also be used as the base vector.

### 1. Construction of pAG 1

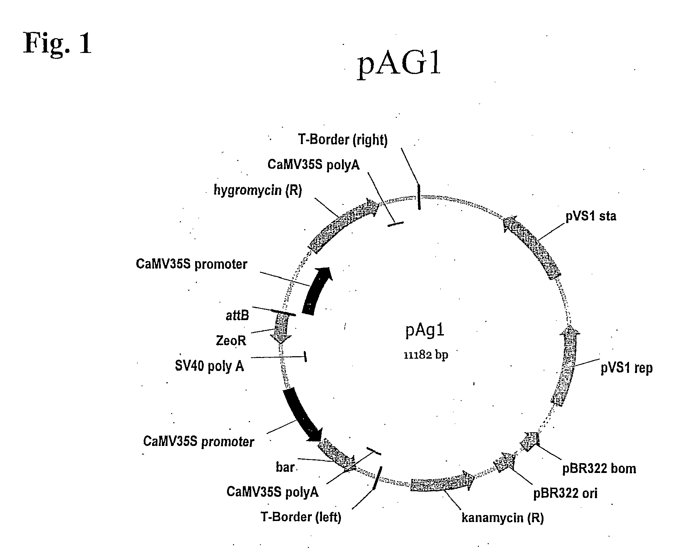

Vector pAg1 (SEQ. lD. NO: 1; see Figure 1) is a derivative of the CAMBlA vector named pCambia 3300 (Center for the Application of Molecular Biology to International Agriculture, i.e., CAMBIA, Canberra, Australia; www.cambia.org), which is a modified version of vector pCambia 1300 to which has been added DNA from the bar gene confering resistance to phosphinothricin. The nucleotide sequence of pCambia 3300 is provided in SEQ. lD. NO: 2. pCambia 3300 also contains a lacZ alpha sequence containing a polylinker region.

pAg1 was constructed by inserting two new functional DNA fragments into the polylinker of pCambia 3300: one sequence containing an attB site and a promoterless zeomycin resistance-encoding DNA flanked at the 3' end by a SV40 polyA signal sequence, and a second sequence containing DNA from the hygromycin resistance gene (hygromycin phosphotransferase) confering resistance to hygromycin for selection in plants. Although the zeomycin-SV40 polyA signal fusion is not expected to provide the basis for zeomycin selection in plant cells, it can be activated in mammalian cells by insertion of a functional promoter element into the attB site by site-specific recombination catalyzed by the Lambda att integrase. Thus, the inclusion of the attB-zeomycin sequences allows for evaluation of functionality of plant artificial chromosomes in mammalian cells by activation of the zeomycin resistance-encoding DNA, and provides an att site for further insertion of new DNA sequences into plant artificial chromosomes formed as a result of using pAg1 for plant transformation. The second functional DNA fragment allows for selection of plant cells with hygromycin. Thus, pAg1 contains DNA from the bar gene confering resistance to phosphinothricin, DNA from the hygromycin resistance gene, both resistance-encoding DNAs under the control of a separate cauliflower mosaic virus (CaMV) 35S promoter, and the attB-promoterless zeomycin resistance-encoding DNA.

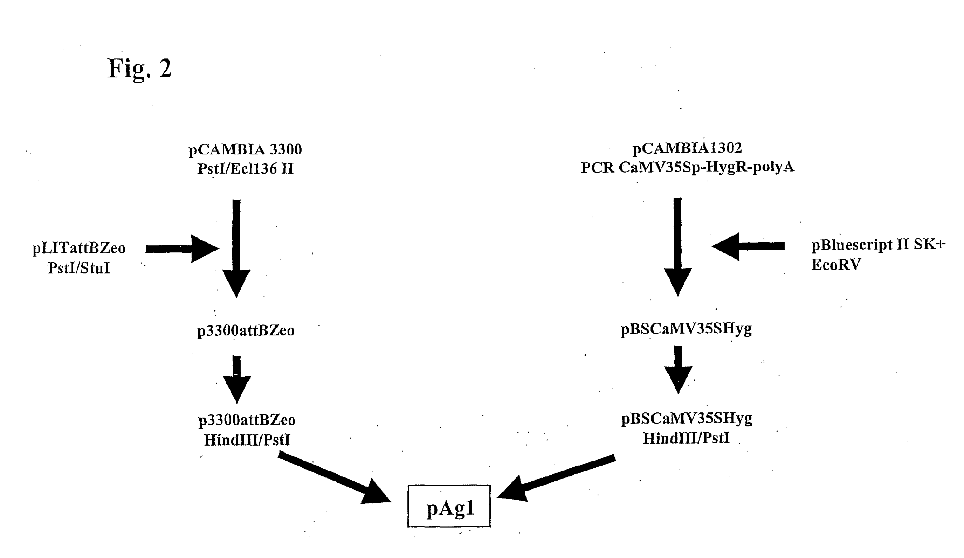

pAg1 is a binary vector containing *Agrobacterium* right and left T-DNA border sequences for use in *Agrobacterium-*mediated transformation of plant cells or protoplasts with the DNA located between the border sequences. pAg1 also contains the pBR322 Ori for replication in *E.coli.* pAg1 was constructed by ligating *Hind*lll/*Pst*l-digested p3300attBZeo with *Hind*lll/*Pst*l-digested pBSCaMV35SHyg as follows (see Figure 2).

### a. Generation of p3300attBZeo

Plasmid pCambia 3300 was digested with *Pst*l/*Ecl*136 ll and ligated with *Pst*l/S*tu*l-digested pLITattBZeo (the nucleotide sequence of pLITattBZeo is provided in SEQ. lD. NO: 19 to generate p3300attBZeo which contains an attB site, a promoterless zeomycin resistance-encoding DNA flanked at the 3' end by a SV40 polyA signal, and a reconstructed Pstl site.

### b. Generation of pBSCaMV35SHyg

A DNA fragment containing DNA encoding hygromycin phosphotransferase flanked by the CaMV 35S promoter and the CaMV 35S polyA signal sequence was obtained by PCR amplification of plasmid pCambia 1302 (GenBank Accession No. AF234298 and SEQ. lD. NO: 3). The primers used in the amplification reaction were as follows:
CaMV35SpolyA:
   5*'*-CTGAATTAACGCCGAATTAATTCGGGGGATCTG-3*'* SEQ. lD. NO: 4
CaMV35Spr:
   5'-CTAGAGCAGCTTGCCAACATGGTGGAGCA-3' SEQ. lD. NO: 5
The 2100-bp PCR fragment was ligated with *Eco*RV-digested pBluescript ll SK + (Stratagene, La Jolla, CA, U.S.A.) to generate pBSCaMV35SHyg.

### c. Generation of pAg1

To generate pAg1, pBSCaMV35SHyg was digested with *Hind*lll/*Pst*l and ligated with *Hind*lll/*Pst*l-digested p3300attBZeo. Thus, pAg1 contains the pCambia 3300 backbone with DNA conferring resistance to phophinothricin and hygromycin under the control of separate CaMV 35S promoters, an attB-promoterless zeomycin resistance-encoding DNA recombination cassette and unique sites for adding additional markers, e.g., DNA encoding GFP. The attB site facilitates the addition of new DNA sequences to plant or animal, e.g., mammalian, artificial chromosomes, including PACs formed as a result of using the pAg1 vector, or derivatives thereof, in the production of PACs. The attB site provides a convenient site for recombinase-mediated insertion of DNAs containing a homologous att site.

### 2. pAG2

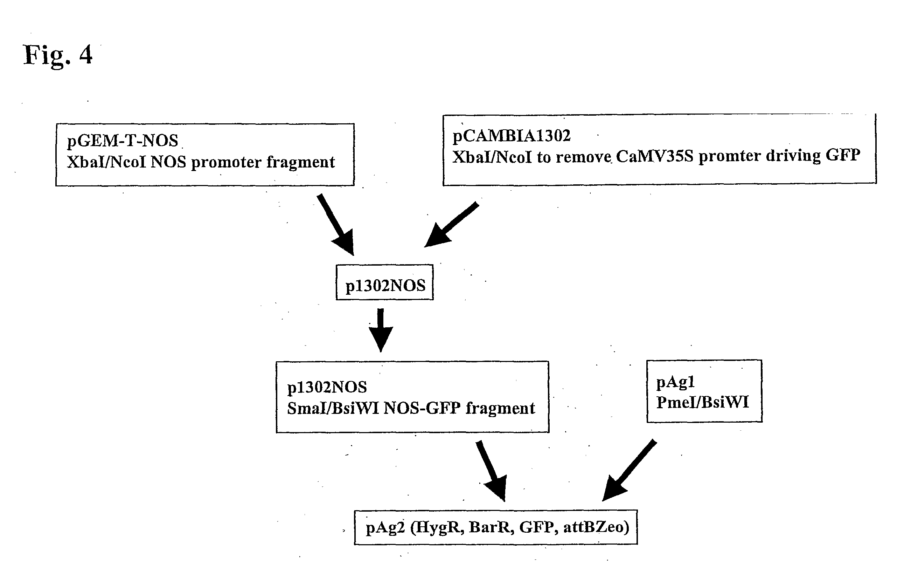

The vector pAg2 (SEQ. lD. NO: 6; see Figure 3) is a derivative of vector pAg1 formed by adding DNA encoding a green fluorescent protein (GFP), under the control of a NOS promoter and flanked at the 3' end by a NOS polyA signal, to pAg1. pAg2 was constructed as follows (see Figure 4). A DNA fragment containing the NOS promoter was obtained by digestion of pGEM-T-NOS, or pGEMEasyNOS (SEQ. lD. NO: 7), containing the NOS promoter in the cloning vector pGEM-T-Easy (Promega Biotech, Madison, WI, U.S.A.), with *Xba*l/*Nco*l and was ligated to an *Xba*l/*Nco*l fragment of pCambia 1302 containing DNA encoding GFP (without the CaMV 35S promoter) to generate p1302NOS (SEQ. lD. NO: 8) containing GFP-encoding DNA in operable association with the NOS promoter. Plasmid p1302NOS was digested with *Sma*l/*Bsi*Wl to yield a fragment containing the NOS promoter and GFP-encoding DNA. The fragment was ligated with *Pme*l/*BsiW*l-digested pAg1 to generate pAg2. Thus, pAg2 contains DNA from the bar gene confering resistance to phosphinothricin, DNA conferring resistance to hygromycin, both resistance-encoding DNAs under the control of a cauliflower mosaic virus 35S promoter, DNA encoding kanamycin resistance, a GFP gene under the control of a NOS promoter and the attB-zeomycin resistance-encoding DNA. One of skill in the art will appreciate that other fragments can be used to generate the pAg1 and pAg2 derivatives and that other heterlogous DNA can be incorporated into pAg 1 and pAg2 derivatives using methods well known in the art.

### 3. pAglla and pAgllb transformation vectors

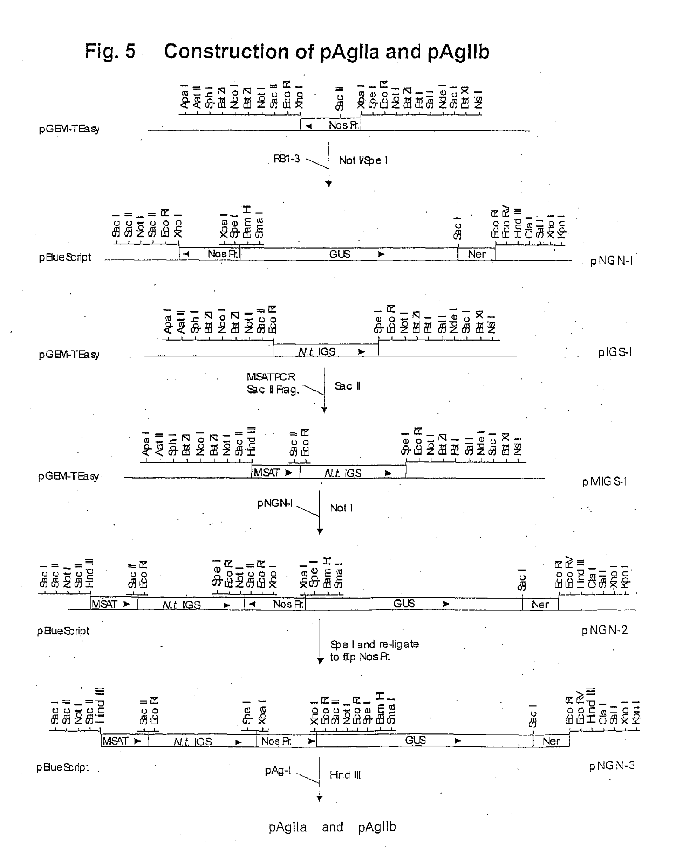

Vectors pAglla and pAgllb were constructed by inserting the following DNA fragments into pAg1: DNA encoding *β*-glucoronidase, the nopaline synthase terminator fragment, the nopaline synthase (NOS) promoter fragment, a fragment of mouse satellite DNA and an N. *tabacum* rDNA intergenic spacer sequence (IGS). The construction of pAglla and pAgllb was as follows (see Figure 5).

An N. *tabacum* rDNA intergenic spacer (IGS) sequence (SEQ. lD. NO: 9); see also GenBank Accession No. Y*O*8422; see also Borysyuk et al. (2000) Nature Biotechnology 18:1303-1306; Borysyuk et a/. (1997) Plant Mol. Bio/.35:655-660; U.S. Patent Nos. 6,100,092 and 6,355,860) was obtained by PCR amplification of tobacco genomic DNA. The lGS can be used as a targeting sequence by virtue of its homology to tobacco rDNA genes; the sequence is also an amplification promoter sequence in plants. This fragment was amplified using standard PCR conditions (e.g., as described by Promega Biotech, Madison, Wl, U.S.A.) from tobacco genomic DNA using the primers shown below:
NTlGS-Fl
5'- GTG CTA GCC AAT GTT TAA CAA GAT G- 3' (SEQ lD No. 10) and NTlGS-Rl
5'-ATG TCT TAA AAA AAA AAA CCC AAG TGA C- 3' (SEQ lD No. 11) Following amplification, the fragment was cloned into pGEM-T Easy to give plGS-l.

A fragment of mouse satellite DNA (Msat1 fragment; GenBank Accession No. V00846; and SEQ lD No. 12) was amplified via PCR from pSAT-1 using the following primers:
MSAT-F1
5'- AAT ACC GCG GAA GCT TGA CCT GGA ATA TCG C -3*'*(SEQ lD No. 13)
and
MSAT-Ri
5'-ATA ACC GCG GAG TCC TTC AGT GTG CA T- 3' (SEQ lD No. 14)
This amplification added a *Sac*ll and a *Hind*lll site at the 5'end and a *Sac*ll site at the 3' end of the PCR fragment. This fragment was then cloned into the *Sac*ll site in plGS-l to give pMlGS-1, providing a eukaryotic centromere-specific DNA and a convenient DNA sequence for detection via FlSH.

A functional marker gene containing a NOS-promoter:GUS:NOS terminator fusion was then constructed containing the NOS promoter (GenBank Accession No. U09365; SEQ lD No. 15), *E. coli β*-glucuronidase coding sequence (from the GUS gene; GenBank Accession No. S69414; and SEQ lD No. 16), and the nopaline synthase terminator sequence (GenBank Accession No. U09365; SEQ lD No. 18). The NOS promoter in pGEM-T-NOS was added to a promoterless GUS gene in pBlueScript (Stratagene, La Jolla, CA, U.S.A.) using *Not*l/*Spe*l to form pNGN-1, which has the NOS promoter in the opposite orientation relative to the GUS gene.

pMlGS-1 was digested with *Not*l/*Spe*l to yield a fragment containing the mouse major satellite DNA and the tobacco IGS which was then added to *Notl-*digested pNGN-1 to yield pNGN-2. The NOS promoter was then re-oriented to provide a functional GUS gene, yielding pNGN-3, by digestion and religation with Spel. Plasmid pNGN-3 was then digested with *Hind*lll*,* and the *Hind*lll fragment containing the *β*-glucuronidase coding sequence and the rDNA intergenic spacer, along with the Msat sequence, was added to pAG-1 to form pAglla, using the unique *Hind*lll site in pAg1 located near the right T-DNA border of pAg1, within the T-DNA region.

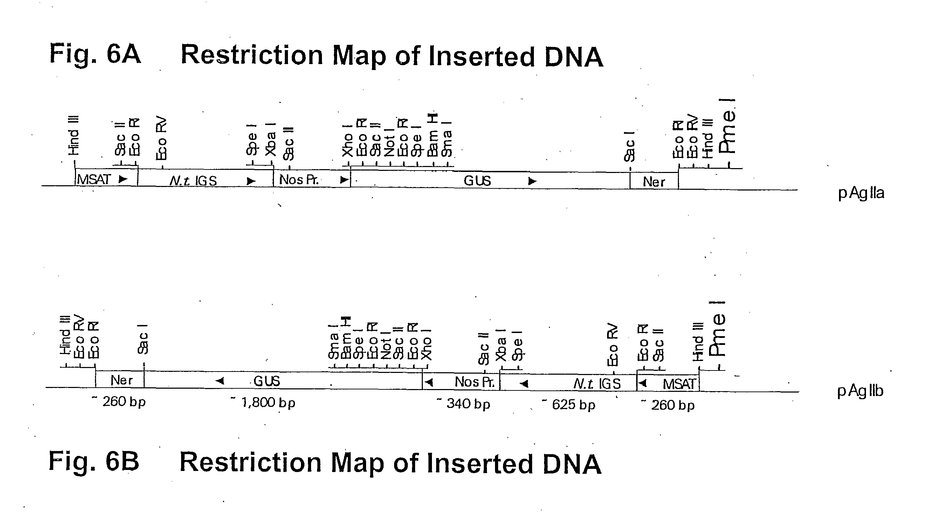

Another plasmid vector, referred to as pAgllb, was also recovered, which contained the inserted *Hind*lll fragment in the opposite orientation relative to that observed in pAglla. Thus, pAglla and pAgllb differ only in the orientation of the *Hind*lll fragment containing the mouse major satellite sequence, the GUS DNA sequence and the IGS sequence (see Figure 6). The nucleotide sequence of pAglla is provided in SEQ lD. NO: 21.

Vectors pAg1, pAg2, pAglla and pAgllb, as well as similarly designed vectors containing a recombination site and a promoter (e.g., plant or animal promoter), and possibly other regulatory sequences, in operable association with DNA encoding a protein or other product for the expression in a host cell, such as a plant or animal cell, can be used in the transfer of any protein (or other product)-encoding nucleic acid of interest into a cell for expression thereof. For example, any protein (or other product)-encoding nucleic acid of interest (in operable association with transcriptional regulatory suitable for use in a particular host cell) can be inserted into any of the vectors pAg1, pAg2, pAglla and pAgllb and thereby incorporated into a plant, animal or other artificial chromosome, particularly a platform artificial chromosome ACes, as desribed herein.

### Example 6

### Agrobacterium-Mediated Transformation of Plant Cells

Plant cells were transformed via *Agrobacterium-mediated* transformation according to standard procedures (see, for example, Horsch *et al.* (1988) *Plant Molecular Biology Manual, A5:1-9,* Kluwer Academic Publisher, Dordrecht, Belgium). *Briefly, Agrobacterium* strain GV 3101/pMP90 (see Koncz and Schell (1986) *Molecular and General Genetics 204*:383-396) was transformed with pAglla and pAgllb (see Example 5) by heat shock, and the plasmid integrity of pAglla and pAgllb after transformation was verified by *Hind*lll digest pattern. pAglla/pMP90 or pAgllb/pMP90 were cultured in 5 ml AB minimum medium (Horsch et al. (1988) Plant Molecular Biology Manual, A5:1-9, Kluwer Academic Publisher, Dordrecht, Belgium) containing 25 *µ*g/ml kanamycin and 25 *µ*g/ml gentamycin at 28°C for two days.

Leaf disks of tobacco and *Arabidopsis* and root segments of *Arabidopsis* were prepared as follows: tobacco leaves from 3 to 4 week-old explants were cut into 1 cm in diameter, and *Arabidopsis* leaves were taken from 3 week-old seedlings and transversely cut in two halves. Roots of 3 week-old *Arabidopsis* were excised into segments of 1 cm in length. Cocultivation was carried out by immersing leaf disks or root segments in bacterial culture for 2 minutes and then transferring the infected tissues to culture medium without antibiotics for 2 days at 22°C for 16-hours/day under cool white fluorescent light. The leaf disks of tobacco and *Arabidopsis* were cultured on MS104 medium (MS, 3% sucrose, 0.05% MES, 1.0 mg/l BA, 0.1 mg/l NAA and 0.8% agar, pH 5.8) and root segments on callus-inducing medium, CIM 0.5/0.05 (B5, 2% glucose, 0.05% MES, 0.5 mg/l 2,4-D, 0.05 mg/l kinetin and 0.8% agar, pH 5.8).

The transformed leaf disks and root segments were then transferred to selection medium of MS104 or ClM 0.5/0.05, respectively, containing 20 mg/l hygromycin and 300 mg/l Timentin for the elimination of Agrobacterium. The selection medium was refreshed every two weeks and green shoots regenerated. Plants were analyzed for the expression of the DNA encoding GUS by standard histochemical and fluorescent assays and evidence of amplification of the inserted DNA by quantitative PCR. Numerous plants were obtained that expressed high levels of GUS, and multiple copies of the GUS gene were observed by Fluorescent ln Situ Hybridization (FISH) and PCR analysis. Thus, amplification the chromosomal regions containing the inserted DNA was observed. One of skill in the art will appreciate that GUS expression, or the expression of any other gene, can be assessed using methods well known in the art.

### Example 7

### Transfection and culture of Arabidopsis protoplasts

*E. coli* strain Stb14 (Gibco Life Sciences) was transformed with pAglla, pAgllb, and one of two targeting plasmids containing the rDNA repeat sequence from *Arabidopsis* (plasmid pJHD-14A or the 26S rDNA from *Arabidopsis* plasmid pJHD2-19A, as described by Doelling et al. [(1993) Proc. Natl. Acad. Sci. U.S.A. 90:7528-7532]) via electroporation according to standard procedures. A single colony was grown up in 250 ml LB medium containing 50 *µ*g/ml kanamycin (for selection based on the kanamycin resistance-encoding DNA in pAglla and pAgllb) or 50 *µ*g/ml ampicillin (for selection based on the ampicillin resistance-encoding DNA in pJHD-14A & pJHD2-19A) and cultured at 30°C with shaking at 225 rpm for 16 hours. The plasmids were isolated according to standard procedures well known in the art. The structural integrity of the plasmids was checked by restriction digestion pattern, and the plasmids were linearized with restriction enzymes. Plasmids were sterilized with chloroform and 70% ethanol before use for transfection.

*Arabidopsis* protoplasts were resuspended in the culture medium (see Example 1) at a density of 2 ×10⁶ protoplasts/ml. A 300 µ*l* protoplast suspension was pipetted into a 15 ml tube, and 30 *µ*l of plasmid (pAglla or pAgllb) and targeting DNA (pJHD-14A or pJHD2-19A) was added containing 10 *µ*g plasmid and 100 *µ*g targeting sequence followed immediately by slowly adding 300 *µ*l of 10% PEG. The targeting plasmids were included in the transfection procedure in order ensure that the amount of rDNA targeting DNA (i.e., tobacco rDNA from pAglla or b and Arabidopsis DNA from the targeting vectors) was sufficient to effect recombination of the introduced DNA at a homologous site in an Arabidopsis chromosome. DNA was typically used in a ratio of 10:1, targeting DNA (pJHD-14A or pJDH2-19A, or Lambda DNA) to plasmid DNA (pAglla or pAgllb, or a selectable marker plasmid), or in a ratio of 5:1. Generally, the number of base pairs of targeting DNA to be sufficient for insertion into a plant chromosome is at least about 50 bp, or about 60 bp, or about 70 bp, or about 80 bp, or about 90 bp, or about 100 bp, or about 150 bp, or about 200 bp, or about 300 bp, or about 400 bp, or about 500 bp, or about 600 bp, or about 700 bp, or about 800 bp, or about 900 bp, or about 1 kb, or about 2 kb or about 3 kb, or about 4 kb, or about 5 kb, or about 6 kb, or about 7 kb, or about 8 kb, or about 9 kb, or about 10 kb or more. The amount and length of targeting DNA sufficient to effect introduction into a chromosome can be determined empirically and can vary for different plant species.

The mixture was shaken gently, and immediately 300 *µ*l of 10% PEG solution was added slowly with gentle shaking. The protoplast mixture was incubated at 22°C for 10-15 min with several cycles of gentle shaking. DNA uptake was quenched by the addition of 5 ml 72.4 g/l Ca(N0₃)₂. The protoplasts were then centrifuged at 80xg for 7 min and resuspended in culture medium. For selection, 10 to 40 mg/l hygromycin was added to protoplast cultures 14 days aftertransfection, and the culture medium was refreshed every 7 days. The protoplast cultures could also be selected after embedding in 0.6% agarose by transferring to a culture medium containing 20 mg/l hygromycin. The cultures were incubated for 14 days or longer at 22°C.

The *Arabidopsis* protoplasts were analyzed for the presence and expression of the DNA encoding GUS. Recovered microcalli strongly expressed GUS and were resistant to selective agents, indicating amplification of the inserted DNA. Alternatively, the transfection of *Arabidopsis* protoplasts can be conducted without using targeting DNA sequences since pAglla and pAgllb include a region of rDNA (i.e. the tobacco rDNA IGS) that can act as a targeting sequence as long as a sufficient amount of pAglla/b plasmid is used in the transfection procedure. **Example 8**

### Transfection and Culture of Tobacco Protoplasts

As described in Example 7, E. *coli* strain Stbl4 was transformed with pAglla, pAgllb, pJHD-14A (targeting DNA) and pJHD2-19A (targeting DNA) via electroporation, and plasmid DNA was recovered and linearized with restriction enzymes. Plasmids were sterilized with chloroform and 70% ethanol before use for transfection.

The tobacco protoplasts (see Examples 2 and 3) were resuspended in the culture medium (see Example 2) at a density of 2 ×10⁶ protoplasts/ml. A 300 *µ*l protoplast suspension was pipetted into a 15 ml tube, and 30 *µ*l of plasmid and targeting DNA was added as described in Example 7. The mixture was shaken gently, and immediately 300 *µ*l of 10% PEG solution was added slowly with gentle shaking. The tobacco protoplast mixture was incubated at 22°C for 10-15 min with several cycles of gentle shaking. DNA uptake was quenched by the addition of 5 ml 72.4 g/L Ca(NO₃)₂. The protoplasts were then centrifuged at 80xg for 7 min and resuspended in culture medium.

The recovery of viable tobacco protoplasts following DNA uptake ranged from 65-75% following treatment. Typically greater than 35% of the protoplasts initiated cell division within 7 days of treatment. Protoplast cells were analyzed for gene expression (in this case for the expression of the reporter DNA GUS, but alternatively, the expression of other genes can be monitored). Between 4% and 6% of the recovered cells exhibited GUS expression.

The protoplasts were subject to selection procedures to recover transformed cells. For selection of tobacco cells, 10 to 40 mg/l hygromycin was added to protoplast cultures 10-14 days after transfection, and the culture medium was refreshed every 7 days. Leaf disc selection was performed in the presence of 40 mg/l hygromycin. Transformed microcalli were recovered and analyzed for the expression of the GUS reporter gene. GUS positive calli were isolated and subjected to FISH analysis (see Example 13). Plant cells that exhibited amplification of the inserted DNA were identified.

### Example 9

### Transfection and Culture of Brassica Protoplasts

Brassica protoplasts (see Example 4), following the final washing step after filtering through a 63 *µ*m nylon screen and centrifugation, are collected and used for DNA transfection as described in Example 8. *Brassica* protoplast cultures following DNA uptake or transformation by *Agrobacterium* can be selected with either hygromycin or glufosinate ammonium in liquid culture or in embedded semi-solid cultures. The effective concentration of hygromycin is 10 to 40 mg/l for 2 to 4 weeks or continuously, whereas that for glufosinate ammonium is 2 to 60 mg/l for 5 days to 2 weeks. Selection can impede growth, and additional transfers to similar media may be required.

### Example 10

### Plant Regeneration from Brassica Protoplasts

Colonies of *Brassica* protoplasts (1 mm or larger in diameter) are plated onto regeneration medium (basal Murashige and Skoog's medium, 1 % sucrose, 2 mg/l BA, 0.01 mg/l NAA, 0.8% agarose, pH 5.6). Cultures are incubated under the conditions described in Example 4. Cultures are transferred onto fresh regeneration medium every 2 weeks. Regenerated shoots are transferred onto autoclaved rooting medium (basal Murashige and Skoog's medium, 1 % sucrose, 0.1 mg/l NAA, 0.8% agar, pH 5.8) and incubated under dim fluorescent light (25 *µ*Em⁻² s⁻¹). Plantlets are potted in a soil-less mix (for example, Terra-lite Redi-Earth, W.R. Grace & Co., Canada Ltd., Ajax, Ontario) containing fertilizer (Nutricote 1414-14 type 100, Plant Products Co. Ltd, Brampton, Ontario) and grown in a growth room (20°C/15°C, 16 h photoperiod, 100-140 *µ*Em⁻² s⁻¹) with fluorescent and incandescent light at soil level. Plantlets are covered with transparent plastic cups for one week to allow for acclimatization.

### Example 11

### lsolation of Nuclei from Protoplasts

To facilitate analysis, plant cells can be subjected to nuclei isolation, and the isolated nuclei can be analyzed by FISH or PCR. To isolate the nuclei, protoplast calli were reprotoplasted according to the procedure of Mathur *et al.* with modifications (see Mathur et al. Plant Cell Report (1995) 14: 221-226). The protoplast calli were digested with 1.2% Cellulase 'Onozuka' R-10 and 0.4% w/v Macerozyme R-10 in nuclei isolation buffer (10 mM MES-pH 5.5, 0.2M sucrose, 2.5 mM EDTA, 2.5 mM DTT, 0.1 mM spermine, 10 mM NaCl, 10 mM KCI and 0.15% Triton X-100) for 3 hours. After centrifugation at 80 x g for 10 minutes, the pellets of protoplasts were resuspended in hypertonic buffer of 12.5% W5 solution (Hinnisdaels et al. (1994) Plant Molecular Biology, Manual G2:1-13, Kluwer Academic Publisher, Belgium) for 10 minutes. To promote disruption of protoplasts, the protoplast suspension was forced through a syringe needle four times. The disrupted protoplasts were filtered through 5 *µ*m meshes to remove debris and centrifuged at 200 x g for 10 min. By repeated washing of the pellet in a nuclei isolation buffer containing phenylmethylsulfonylfluoride (PMSF) and centrifugation at 200 x g for 10 minutes, nuclei were collected as a white pellet freed from cytoplasm contamination and cellular debris. Samples were fixed in 3:1 methanol:glacial acetic acid and were analyzed by FISH.

### Example 12

### Mitotic Arrest of Plant Cells for Detection of Amplification and Artificial Chromosome Formation

ln general, plant cells or protoplasts are typically cultured for two or more generations prior to mitotic arrest. Typically, 5 *µ*g/ml colchicine is added to the cultures for 12 hours to accumulate mitotic plant cells. The mitotic cells are harvested by gentle centrifugation. Alternatively, plant cells (grown on plastic or in suspension) can be arrested in different stages of the cell cycle with chemical agents other than colchicine, such as, but not limited to, hydroxyurea, vinblastine, colcemid or aphidicolin or through the deprivation of nutrients, hormones, or growth factors. Chemical agents that arrest the cells in stages other than mitosis, such as, but not limited to, hydroxyurea and aphidicolin, are used to synchronize the cycles of all cells in the population and are then removed from the cell medium to allow the cells to proceed, more or less simultaneously, to mitosis at which time they can be harvested to disperse the chromosomes.

### Example 13

### Detection of Amplification and Artificial Chromosome Formation by Fluorescence in situ hybridization (FISH)

A variety of plant cells can analyzed by fluorescence in situ hybridization (FISH) methods (Fransz et al. (1996) Plant J. 9:421-430; Fransz et al. (1998) Plant J. 13:867-876; Wilkes et al. (1995) Chromosome Research 3:466-472; Busch et al. (1994) Chromosome Research 2:15-20; Nkongolo (1993) Genome 36:701-705; Leitch et a/. (1994) Methods in Molecular Biology 28:177-185; Murata et aL. (1997) Plant J. 12:31-37) to identify amplification events and artificial chromosome formation.

FISH is used to detect specific DNA sequences on chromosomes, in particular to detect regions of plant chromosomes that have undergone amplification as a result of the introduction of heterologous DNA as described herein, or to detect artificial chromosome formation in plant cells. FISH chromosome spreads of *Arabidopsis* and tobacco plant cells into which heterologous DNA has been introduced are generated using colchicine or similar cell cycle arresting agents and various DNA probes (e.g. rDNA probe, Lambda DNA probe, selectable marker probe). The cells are analyzed for the presence of amplified regions of chromosomes, in particular amplification of the rDNA regions, and those cells exhibiting amplification are further cultured and analyzed for the formation of artificial chromosomes.

The chromosomes of plant cells subjected to introduction of heterologous DNA and growth to generate artificial chromosomes can also be analyzed by scanning electron microscopy. Preparation of mitotic chromosomes for scanning electron microscopy can be performed using methods known in the art (see, *e.g.,* Sumner (1991) Chromosome 100:410-418). The chromosomes can be observed, for example, with a Hitachi S-800 field emission scanning electron microscope operated with an accelerating voltage of 25kV.

### Example 14

### Detection of Amplification and Artificial Chromosome Formation by Idu Labeling of Chromosomes

The structure of the chromosomes in plant cells can be analyzed by labeling the chromosomes with iododeoxyuridine (ldU), or other nucleotide analog, and using an ldU-specific antibody to visualize the chromosome structure. Plant cell cultures selected following introduction of heterologous DNA are labeled with IdU following standard protocols (Fujishige and Taniguchi (1998) Chromosome Research 6:611-619; Yanpaisan et al. (1998) Biotechnology and Bioengineering, 58:515-528; Trick and Bates (1996) Plant Cell Reports, 15:986-990; Binarova et al. (1993) Theoretical and Applied Genetics, 87:9-16; Wang et al. (1991) Journal of Plant Physiology, 138:200-203). Plant cells in culture, typically suspension culture, are used. A series of sub-cultures are initiated, and IdU labeling is performed as described above. Cells are allowed to incorporate IdU for up to a week, depending on the doubling time of the culture. Labeled chromosomes can be detected in plant cells (Fujishige and Taniguchi (1998) Chromosome Research 6:611-619; Binarova et al. (1993) Theoretical and Applied Genetics 87:9-16) and in mammalian cells (Gratzner and Leif (1981) Cytometry 1:385-393) using procedures well known in the art. IdU-labeled chromosomes are detected by immunocytochemical techniques. An anti-ldU fluorescein isothiocyanate (FITC)-conjugated B44 clone antibody (Becton Dickinson) is used to bind the IDU-DNA adduct in the DNA and is detected by fluorescence microscopy (490 nm excitation, 519 nm emission). Analysis of labeled chromosomes reveals the presence of amplified DNA regions and the formation of artificial chromosomes.

### Example 15

### Isolation of Metaphase Chromosomes from Protoplasts

Artificial chromosomes, once detected in plant cells, may be isolated for transfer to other organisms and in particular other plant species. Several procedures may be used to isolate metaphase chromosomes from mitoticarrested plant cells, including, but not limited to, a polyamine-based buffer system (Cram et al. (1990) Methods in Cell Biology 33:377-3821), a modified hexylene glycol buffer system (Hadlaczky et al. (1982) Chromosoma 86:643-65), a magnesium sulfate buffer system (Van den Engh et al. (1988) Cytometry 9:266-270 and Van den Engh et al. (1984) Cytometry 5:108), an acetic acid fixation buffer system (Stoehr et al. (1982) Histochemistry 74:57-61), and a technique utilizing hypotonic KCl and propidium iodide (Cram et al. (1994) XVll meeting of the International Society for Analytical Cytology, October 16-21, Tutorial lV Chromosome Analysis and Sorting with Commerical Flow Cytometers; Cram et al. (1990) Methods in Cell Biology 33:376; de Jong et al. (1999) Cytometry 35:129-133).

In an exemplary procedure, a hexylene glycol buffer is used to isolate plant chromosomes from mitotic-arrested plant cells that have been converted to protoplasts (Hadlaczky et al. (1982) Chromosoma 86:643-659). Chromosomes are isolated from about 10⁶ mitotic cells re-suspended in a glycine-hexylene glycol buffer (100 mM glycine, 1 % hexylene glycol, pH 8.4-8.6, adjusted with a solution of saturated Ca(OH)₂) supplemented with 0.1 % Triton X-1 00 (GHT buffer). The cells are incubated for 10 minutes at 37°C, and the chromosomes are purified by differential centrifugation to pellet the nuclei (200xg for 20 min) and sucrose gradient centrifugation (5-30% sucrose, 5600xg for 60 min, 0-4°C). To avoid proteolytic degradation of chromosomal proteins, 1 mM PMSF (phenylmethylsulfonylfluoride) is used in the presence of 1 % isopropyl alcohol. The proteins can be extracted from the isolated chromosomes using dextran sulfate-heparin (DSH) extraction, and the chromosomes can be visualized via electron microscopy using techniques known in the art (Hadlaczky et al. (1982) Chromosoma (Berl.) 86:643-659; Hadlaczky et al. (1981) Chromosoma (Berl.) 81:537-555). Additionally, modifications of these procedures, including, but not limited to, modification of the buffer composition (Carrano et al. (1979) Proc. Natl. Acad. Sci. U.S.A. 76:1382-1384) and variation of the centrifugation time or speed, to accommodate different plant species can be implemented by any skilled artisan.

### Example 16

### Transfer of Artificial Chromosomes into Plant Cells: Transfer of Mammalian Artificial Chromosomes into a Dicot Plant: Arabidopsis

One method of delivery of mammalian artificial chromosomes (MACs) into plant cells is the formation of microcells containing murine MACs and the CaPO₄-mediated uptake or the PEG-mediated fusion of these microcells with plant protoplasts. In this example, microcells and plant protoplasts, such as but not limited to tobacco and *Arabidopsis* protoplasts, were mixed (in a series of 25:1, 10:1, 5:1, or 2:1 microcells:protoplasts ratio) and fusion was observed. Protocols for the formation of microcells are known in the art and are described, for example, in US Patent Nos. 5,240,840, 4,806,476 and 5,298,429 and in Fournier Proc. Natl. Acad. Sci. U.S.A. (1981) 78:6349-6353 and Lambert et al. Proc. Natl. Acad. Sci. U.S.A. (1991) 88: 5907-5912. The murine microcells can be labeled with ldu or the lVlACs stained with a specific dye such as, but not limited to, e.g., propidium iodide or DAPI, prior to fusion with plant protoplasts including, but not limited to, *Arabidopsis* and tobacco protoplasts, to facilitate detection of the presence of lVlACs in the protoplasts.

In this example, MACs were introduced into *Arabidopsis* cells using microcell-PEG mediated fusion. Microcells were formed from murine cells containing an artificial chromosome (see U.S. Patent No. 6,077,697) and were fused with freshly prepared *Arabidopsis* protoplasts in a ratio of 10:1, microcells to protoplasts. Fusion occurred in the presence of 25% PEG 6000, 204 mM CaCl₂, pH 6.9 within the first 5 minutes of mixing. Typically less than about one minute of mixing is required to observe fusion between microcells and protoplasts. Fused cells were washed with 240 mM CaCl₂, then floated on top of a solution of 204mM sucrose in B5 salts. Cells were then transferred to cell suspension culture media (MS, 87mM sucrose, 2.7 *µ*M napthalene acetic acid, 0.23 *µ*M kinetin, pH 5.8). Empirical observations can be used to determine the optimal concentration and composition of PEG and the concentration of calcium that provides the highest degree of fusion with the least toxicity.

Fused protoplasts were allowed to grow for one or more generations. The presence of a mouse chromosomal sequence, including MACs, was demonstrated by southern hybridization with MAC probes, by FISH analysis and by PCR analysis using, for example, satellite sequences known to exist on the MAC chromosome. Thus, the mouse sequences were detected in the *Arabidopsis* protoplasts.

To further demonstrate the transfer of mouse chromosomal sequence to *Arabidopsis* protoplasts, *Arabidopsis* plant cell nuclei were isolated according to Example 11 and were subjected to FISH analysis according to Example 13, using the mouse major satellite DNA (SEQ IDNo 12). portion of the nuclei contained a significant signal using the mouse major satellite DNA, indicating successful transfer of at least a mouse chromosome and/or MAC to the *Arabidopsis* nuclei.

Similarly, PACs may be introduced into Arabidopsis protoplasts using PEG- and/or calcium-mediated fusion procedures. Generation of microprotoplasts and protoplasts can be conducted as described, for example, in Example 1. Microprotoplasts formed from plant cells containing a plant artificial chromosome are fused with freshly prepared Arabidopsis protoplasts, for example, in a ratio of 10:1, microprotoplasts to protoplasts. Protoplasts from other plants, including but not limited to, tobacco, wheat, maize and rice, can also be used as the recipient of MACs and/or PACs. Fused protoplasts are recovered and allowed to grow for one or more generations. The presence of the transferred PACs can be analyzed using methods such as, for example, those described herein (including Southern hybridization with PAC probes, FlSH analysis and PCR analysis using DNA sequences specific to the PAC).

### Example 17

### Transfer of Artificial Chromosomes into Plant Cells: Transfer of Mammalian Artificial Chromosomes into a Second Dicot Plant: Tobacco

MACs were introduced into tobacco cells using microcell-PEG mediated fusion using the same microcells, MAC, and protocol as described in Example 16. Microcells were formed from murine cells containing an artificial chromosome and were fused with freshly prepared tobacco BY-2 protoplasts in a ratio of 10:1, microcells to protoplasts. Fusion occurred in the presence of 20% PEG 4000 and 100-200 mM calcium chloride. Empirical observations are used to determine the optimal concentration and composition of PEG and the concentration of calcium that provides the highest degree of fusion with the least toxicity.

DAPI staining of the microcells (e.g. by preincubation of the microcells with DAPl by adding DAPI to the microcells to a final concentration of 1 *µ*g/ml) allowed visualization of the fusion and transfer of the chromosomes to the tobacco protoplasts. Fused protoplasts were recovered and allowed to grow for one or more generations. The fused protoplasts can be analyzed for the presence of a MAC in a number of ways, including those described herein. Fused tobacco cell nuclei were isolated from tobacco protoplasts that had been fused with microcells according to Example 11 and were subjected to FlSH analysis according to Example 13, using the mouse major satellite DNA (SEQ lD No. 12). Numerous nuclei were found to have incorporated a mouse chromosome.

### Example 18

### Transfer of isolated Artificial Chromosomes by Lipid-Mediated Transfer into a Monocot Plant: Rice

Isolated murine artificial chromosomes (MACs) prepared by sorting through a FACS apparatus (de Jong et al. Cytometry (1999) 35:129-133) were transferred into rice plant protoplasts by cationic lipid-mediated transfection of the purified MAC. Purified MACs (see Example 15 and U.S. Patent No. 6,077,697) were mixed with LipofectAMlNE 2000 (Gibco, Md, USA) as follows. Typically, 15 *µ*l of LipofectAMlNE 2000 were added to 1 × 10⁶ artificial chromosomes in liquid buffer, the solution allowed to complex for up to three hours, and then the solution was added to freshly prepared 1 × 10⁵ rice protoplasts prepared using standard protoplast methods well known in the art. The uptake of the lipid-complexed artificial chromosome was monitored by adding to the mixture of protoplasts and purified artificial chromosomes a fluorescent dye that stains DNA. Microscopic examination of the protoplast/artificial chromosome mixture over the next several hours allowed the visualization of the artificial chromosome being transported across the protoplast cellular membrane and the presence of the readily identifiable MAC in the cytoplasm of the rice plant cell.

The same procedure as described in this Example for cationic lipid-mediated transfer of an isolated MAC into rice protoplasts can be used to transfer isolated MACs, as well as PACs, into rice and other plant protoplasts, including but not limited to, tobacco, wheat, maize and *Arabidopsis.* Fused protoplasts are recovered and allowed to grow for one or more generations. The presence of the transferred MACs and PACs can be analyzed using methods such as, for example, those described herein (including, but not limited to, Southern hybridization with PAC probes, FISH analysis and PCR analysis using DNA sequences specific to the PAC).

### Example 19

### Delivery of Plant Regulatory and Coding Sequences via a Promoterless attBZeo Marker Gene in pAg2 onto a MAC Platform

As described in Examples 6-15, the plasmid pAg2, comprising plant regulatory and selectable marker genes (SEQ lD NO: 6; prepared as set forth in Example 5) can be used for the production of a MAC containing said plant expressible genes. In this example, pAg2, by virtue of the attBZeo DNA sequences contained on the plasmid, is used for the loading of plant regulatory and selectable marker genes onto MACs in mammalian cells using the attB sequences to recombine with attP sequences present on a platform MAC. In this example, platform MACs are produced with attP sequences and the plasmid pAg2 is then loaded onto the platform MAC. New MACs so produced are useful for introduction into plan cells by virtue of the plant expressible markers contained therein.

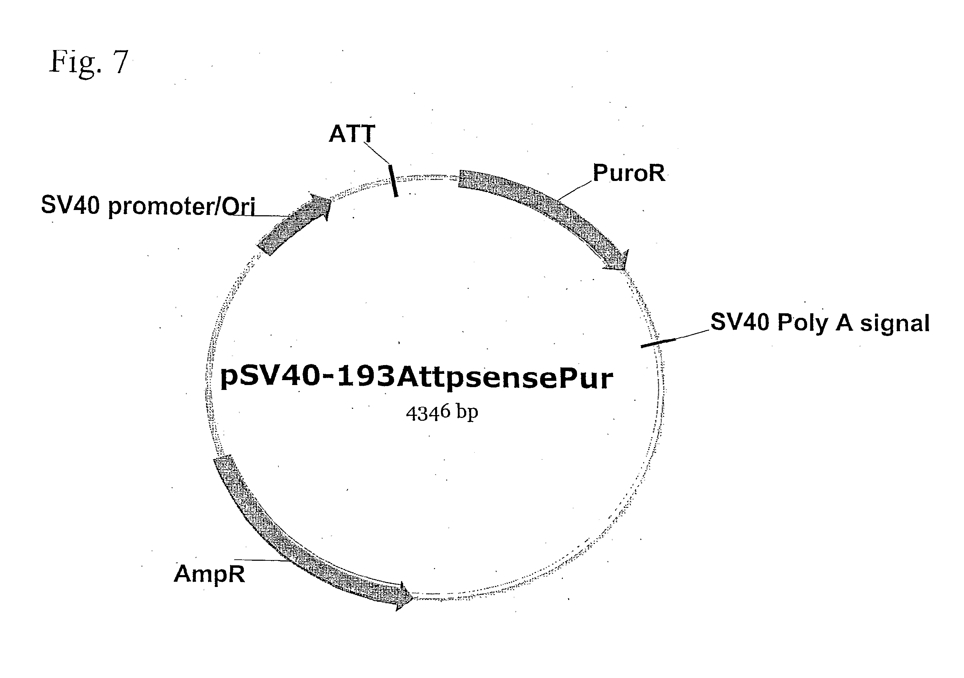

### A. Construction of Platform MAC containing pSV40attPsensePUR (Figure 7; SEQ lD NO: 26).

An example of a selectable marker system for the creation of a MAC-based platform into which the plasmid pAg2 can target plant regulatory and coding sequences is shown in Figure 7. This system includes a vector containing the SV40 early promoter immediately followed by (1) a 282 base pair (bp) sequence containing the bacteriophage lambda attP site and (2) the puromycin resistance marker. Initially a *Pvu*ll/*Stu*l fragment containing the SV40 early promoter from plasmid pPUR (Clontech Laboratories, Inc., Palo Alto, CA; SEQ lD No. 22) was subcloned into the *EcoR*l/*CR*l site of pNEB193 (a PUC19 derivative obtained from New England Biolabs, Beverly, MA; SEQ lD No. 23) generating the plasmid pSV40193.

The attP site was PCR amplified from lambda genome (GenBank Accession # NC 001416) using the following primers:
attPUP: CCTTGCGCTAATGCTCTGTTACAGG SEQ lD No. 24
attPDWN: CAGAGGCAGGGAGTGGGACAAAATTG SEQ lD No. 25

After amplification and purification of the resulting fragment, the attP site was cloned into the *Sma*l site of pSV40193 and the orientation of the attP site was determined by DNA sequence analysis (plasmid pSV40193attP). The gene encoding puromycin resistance (Puro) was isolated by digesting the plasmid pPUR (Clontech Laboratories, lnc. Palo Alto, CA) with *Age*l/*BamH*l followed by filling in the overhangs with Klenow and subsequently cloned into *the Asc*l site downstream of the attP site of pSV40193attP generating the plasmid pSV40193attPsensePUR (Figure 7; SEQ lD NO:26)).

The plasmid pSV40193attPsensePUR was digested with *Sca*l and cotransfected with the plasmid pFK161 into mouse LMtk- cells and platform artificial chromosomes were identified and isolated as described herein. Briefly, Puromycin resistant colonies were isolated and subsequently tested for artificial chromosome formation via fluorescent *in situ* hybridization (FISH) (using mouse major and minor DNA repeat sequences, the puromycin gene and telomeres sequences as probes), and their fluorescent activating cell sorted (FACS). From this sort, a subclone was isolated containing an artificial chromosome, designated B19-38. FISH analysis of the B19-38 subclone demonstrated the presence of telomeres and mouse minor on the MAC. DOT PCR has been done revealing the absence of uncharacterized euchromatic regions on the MAC. The process for generating this exemplary MAC platform containing multiple site-specific recombination sites is summarized in Figure 5. This MAC chromosome may subsequently be engineered to contain target gene expression nucleic acids using the lambda integrase mediated site-specific recombination system as described below.

### B. Construction of Targeting Vector.

The construction of the targeting vector pAg2 is set forth in Example 5 herein.

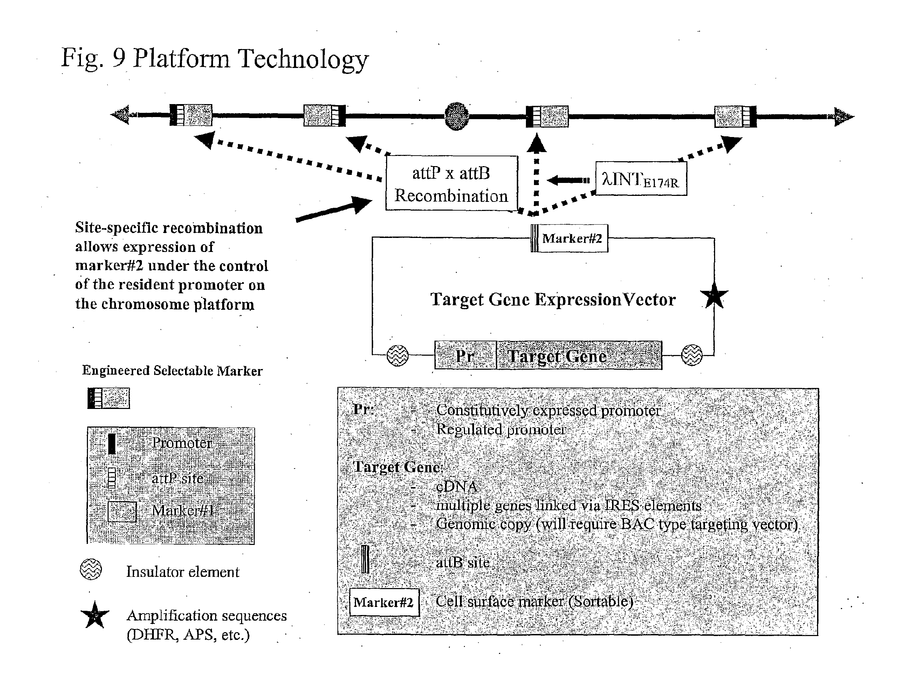

### C. Transfection of Promotorless Marker and Selection With Drug (See Figure 9).

The mouse LMtk- cell line containing the MAC B19-38 (constructed as set forth above and also referred to as a 2^{nd} generation platform ACE), is plated onto four 10cm dishes at approximately 5 million cells per dish. The cells are incubated overnight in DMEM with 10% fetal calf serum at 37°C and 5% C0_{2.} The following day the cells are transfected with 5*µ*g of the vector pAg2 (prepared as described in Example 5 above) and 5*µ*g of pCXLamlntR (encoding a lambda integrase having an E to R amino acid substitution at position 174), for a total of 10*µ*g per 10cm dish. Lipofectamine Plus reagent is used to transfect the cells according to the manufacturers protocol. Two days post-transfection zeocin is added to the medium at 500ug/ml. The cells are maintained in selective medium until colonies are formed. The colonies are then ring-cloned and genomic DNA is analyzed.

### D. Analysis Of Clones (PCR, SEQUENCING).

Genomic DNA (including MACs) is isolated from each of the candidate clones with the Wizard kit (Promega) and following the manufacturers protocol. The following primer set is used to analyze the genomic DNA isolated from the zeocin resistant clones: 5PacSV40 - CTGTTAATTAACTGTGGAATGTGTG TCAGTTAGGGTG (SEQ lD NO: 28); Antisense Zeo - TGAACAGGGTCACGTCGTCC (SEQ lD NO: 29). PCR amplification using the above primers and genomic DNA, which included MACs, from the candidate clones results in a PCR product indicating the correct sequence for the desired site-specific integration event.

The MACs containing the pAg2 vector are identified and used for transfer into plant (such as described in Examples 16 and 17) or animal cells for the expression of the desired coding sequences contained therein. The MACs containing pAg2 carry two plan selectable markers (hygromycin resistance, resistance to phosphinothricin) and a visual selectable marker (green fluorescent protein).

### Example 20

### Construction of Plant-derived Shuttle Artificial Chromosome.

In another embodiment, the plant artificial chromosomes provided herein are useful as selectable shuttle vectors that are able to move one or more desired genes back and forth between plant and mammalian cells. In this particular embodiment, the plant artificial chromosome is bi-functional in that proper integration of donor nucleic acid can be selected for in both plant and mammalian cells.

For example, a plant artificial chromosome is prepared as described in Examples 6-15 above using ing the plasmid pAg2 (Example 5; SEQ lD NO: 6) that has been modified to include the SV40attPsensePur coding region from the plasmid pSV40193attPsensePur (described above in Example 19.A.). Thus, the resulting plant-derived shuttle artificial chromosome contains DNA from the bar gene confering resistance to phosphinothricin in plant cells, DNA from the hygromycin resistance gene conferring resistance to hygromycin in plant cells, both resistance-encoding DNAs under the control of a separate cauliflower mosaic virus (CaMV) 35S promoter, the attB-promoterless zeomycin resistance-encoding DNA, and DNA conferring resistance to puromycin under the control of a mammalian SV40 promoter. Accordingly, the presence of the shuttle PAC in either a plant or mammalian cell can be selected for by treatment with, for example, either hygromycin (plant) or puromycin (mammalian).

Because the resulting plant-derived shuttle artificial chromosome contains at least one SV40attP site therein similar to the platform MAC prepared in Example 19.A. above, a donor vector containing an attB-selectable marker sequence, such as a plasmid comprising an attBzeo (e.g. pAg2) can be used to selectively introduce desired heterologous nucleic acids from any species (such as plants, animals, insects and the like) into the shuttle artificial chromosome that is present in a mammalian cell.

Likewise, a plant promoter region, such as CaMV35S, can be used to replace the SV40 promoter in the SV40attPPur region of the modified pAg2 plasmid described above. In this embodiment, because the resulting plant-derived shuttle artificial chromosome contains at least one CaMV35SattP site therein analogous to the platform MAC prepared in Example 19.A. above, a donor vector containing an attB-selectable marker sequence, such as a plasmid having attBkanamycin, or other plant selectable or scorable marker can be used to selectively introduce desired heterologous nucleic acids from any species (such as plants, animals, insects and the like) into the shuttle artificial chromosome that is present in a plant cell.

Since modifications will be apparent to those of skill in this art, it is intended that this invention be limited by only the scope of the appended claims.

Also described are:
1. A method for producing an artificial chromosome, comprising:
   introducing nucleic acid into a cell comprising one or more plant chromosomes; and
   selecting a cell comprising an artificial chromosome thatcomprises one or more repeat regions
   wherein:
   one or more nucleic acid units is (are) repeated in a repeat region;
   repeats of a nucleic acid unit have common nucleic acid sequences; and
   the repeat region(s) contain substantially equivalent amounts of euchromatic and
   heterochromatic nucleic acid.
2. The method of 1, wherein the artificial chromosome is predominantly made up of one or more repeat regions.
3. The method of 1, wherein the nucleic acid introduced into the cell comprises a nucleic acid sequence that facilitates amplification of a region of a plant chromosome or targets it to an amplifiable region of a plant chromosome.
4. The method of 1, wherein the nucleic acid introduced into the cell comprises one or more nucleic acids selected from the group consisting of rDNA, lambda phage DNA and satellite DNA.
5. The method of 4, wherein the nucleic acid comprises plant rDNA.
6. The method of 5, wherein the rDNA is from a plant selected from the group consisting *of Arabidopsis, Nicotiana, Solanum, Lycopersicon, Daucus, Hordeum, Zea mays, Brassica, Triticum* and *Oryza.*
7. The method of 4, wherein the nucleic acid comprises animal rDNA.
8. The method of 7, wherein the rDNA is mammalian rDNA.
9. The method of 4, wherein the nucleic acid comprises rDNA comprising sequence of an intergenic spacer region.
10. The method of 9, wherein the intergenic spacer region is from DNA from a plant selected from the group consisting *of Arabidopsis, Solanum, Lycopersicon, Hordeum, Zea, Oryza,* rye, wheat, radish and mung bean.
11. The method of 1, wherein the nucleic acid introduced into the cell comprises a nucleic acid sequence that facilitates identification of cells containing the nucleic acid.
12. The method of 11, wherein the nucleic acid sequence encodes a fluorescent protein.
13. The method of 12, wherein the protein is a green fluorescent protein.
14. The method of 1, wherein the step of selecting a cell comprising an artificial chromosome comprises sorting of cells into which nucleic acid was introduced.
15. The method of 1, wherein the step of selecting a cell comprising an artificial chromosome comprises fluorescent in situ hybridization (FISH) analysis of cells into which nucleic acid was introduced.
16. The method of 1, wherein the one or more plant chromosomes contained in the cell is (are) selected from the group consisting of *Arabidopsis,* tobacco and *Helianthus* cells.
17. The method of 16, wherein the cell is a plant protoplast.
18. The method of 1, wherein the nucleic acid introduced into the cell comprises nucleic acid encoding a selectable marker.
19. The method of 18, wherein the selectable marker confers resistance to phosphinothricin, ammonium glufosinate, glyphosate, kanamycin, hygromycin, dihydrofolate or sulfonylurea.
20. A isolated plant artificial chromosome comprising one or more repeat regions, wherein:
   one or more nucleic acid units is (are) repeated in a repeat region;
   repeats of a nucleic acid unit have common nucleic acid sequences; and
   the repeat region (s) contain substantially equivalent amounts of euchromatic and
   heterochromatic nucleic acid.
21. The plant artificial chromosome of 20, wherein the artificial chromosome is predominantly made up of one or more repeat regions.
22. A plant cell comprising an artificial chromosome, wherein the artificial chromosome is produced by the method of 1 or 2.
23. A method of producing a transgenic plant, comprising introducing the artificial chromosome of 20 or 21 into a plant cell.
24. The method of 23, wherein the artificial chromosome comprises heterologous nucleic acid encoding a gene product.
25. The method of 24, wherein the heterologous nucleic acid encodes a product selected from the group consisting of enzymes, antisense RNA, tRNA, rDNA, structural proteins, marker proteins, ligands, receptors, ribozymes, therapeutic proteins and biopharmaceutical proteins.
26. The method of 24, wherein the heterologous nucleic acid encodes a product selected from the group consisting of vaccines, blood factors, antigens, hormones, cytokines, growth factors and antibodies.
27. The method of 24, wherein the heterologous nucleic acid encodes a product that provides for resistance to diseases, insects, herbicides or stress in the plant.
28. The method of 24, wherein the heterologous nucleic acid encodes a product that provides for an agronomically important trait in the plant.
29. The method of 24, wherein the heterologous nucleic acid encodes a product that alters the nutrient utilization and/or improves the nutrient quality of the plant.
30. The method of 24, wherein the heterologous nucleic acid is contained within a bacterial artificial chromosome (BAC) or a yeast artificial chromosome (YAC).
31. A method of identifying plant genes encoding particular traits, comprising:
   generating an artificial chromosome comprising euchromatic DNA from a first species of plant;
   introducing the artificial chromosome into a plant cell of a second species of plant;
   and
   detecting phenotypic changes in the plant cell comprising the artificial chromosome and/or a plant generated from the plant cell comprising the artificial chromosome.
32. The method of 31, wherein the artificial chromosome is a plant artificial chromosome or a mammalian artificial chromosome.
33. The method of 31, wherein the artificial chromosome is produced by a method comprising:
   introducing nucleic acid into a cell comprising one or more plant chromosomes; and
   selecting a plant cell comprising an artificial chromosome that comprises one or more repeat regions, wherein:
      repeats of a nucleic acid unit have common nucleic acid sequences; and
      the repeat region (s) contain substantially equivalent amounts of euchromatic and heterochromatic nucleic acid.
34. The method of 31, wherein the artificial chromosome is produced by a method comprising:
   introducing nucleic acid into a plant cell; and
   selecting a plant cell comprising a SATAC.
35. The method of 31, wherein the artificial chromosome is a minichromosome produced by a method comprising: introducing nucleic acid into a plant cell; and selecting a cell comprising a minichromosome comprising a neocentomere and euchromatin.
36. The method of any of 33-35, wherein the nucleic acid introduced into the plant cell comprises DNA encoding a selectable marker.
37. The method of 36, wherein the selectable marker confers resistance to phosphinothricin, ammonium gluf osinate, glyphosate, kanamycin, hygromycin, dihydrofolate or sulfonylurea.
38. The method of 31, wherein the artificial chromosome comprising euchromatic DNA from a first plant species is produced by a method comprising:
   introducing into a plant cell of a first plant species an artificial chromosome capable of undergoing homologous recombination with the DNA of the first plant species;
   selecting for a recombination event between the artificial chromosome and the DNA of the first plant species; and
   selecting an artificial chromosome comprising euchromatic DNA from the first plant species.
39. The method of 31, wherein the artificial chromosome comprising euchromatic DNA from a first plant species is produced by a method comprising:
   introducing into a plant cell of a first species an artificial chromosome capable of undergoing site-specific recombination with the DNA of the first plant species;
   selecting for a site-specific recombination event between the artificial chromosome and the DNA of the first plant species, and
   selecting an artificial chromosome comprising euchromatic DNA from the first plant species.
40. The method of 39, wherein the DNA of the plant cell of a first species is modified to comprise a site-specific recombination sequence.
41. The method of 39, wherein the artificial chromosome comprises a site-specific recombination sequence.
42. The method of 39, wherein the DNA of the plant cell of a first species is modified to comprise a site-specific recombination sequence and the artificial chromosome comprises a site-specific recombination sequence.
43. The method of 39, wherein the DNA of the plant cell of a first species is modified to comprise a site-specific recombination sequence and the artificial chromosome comprises a site-specific recombination sequence that is complementary to the site-specific recombination sequence of the plant cell of a first plant species.
44. The method of 39, wherein the site-specific recombination is catalyzed by a recombinase enzyme.
45. A method for producing an acrocentric plant chromosome, comprising:
   introducing a first nucleic acid comprising a site-specific recombination site into a first chromosome of a plant cell;
   introducing a second nucleic acid comprising a site-specific recombination site into a second chromosome of the plant cell;
   introducing a recombinase activity into the plant cell, wherein the activity catalyzes recombination between the first and second chromosomes and whereby an acrocentric plant chromosome is produced.
46. The method of 45, wherein the first nucleic acid is introduced into the pericentric heterochromatin of the first chromosome.
47. The method of 45, wherein the second nucleic acid is introduced into the distal end of the arm of the second chromosome.
48. The method of 45, wherein the first nucleic acid is introduced into the pericentric heterochromatin of the first chromosome and the second nucleic acid is introduced into the distal end of the arm of the second chromosome.
49. A method for producing an acrocentric plant chromosome, comprising:
   introducing a first nucleic acid comprising a site-specific recombination site into the pericentric heterochromatin of a chromosome in a plant cell;
   introducing a second nucleic acid comprising a site-specific recombination site into the distal end of the chromosome, wherein
   the first and second recombination sites are located on the same arm of the chromosome;
   introducing a recombinase activity into the cell, wherein the activity catalyzes recombination between the first and second recombination sites in the chromosome and whereby an acrocentric plant chromosome is produced.
50. A method for producing an acrocentric plant chromosome, comprising:
   introducing nucleic acid comprising a recombination site adjacent to nucleic acid encoding a selectable marker into a first plant cell;
   generating a first transgenic plant from the first plant cell;
   introducing nucleic acid comprising a promoter functional in a plant cell, a recombination site and a recombinase coding region in operative linkage into a second plant cell;
   generating a second transgenic plant from the second plant cell;
   crossing the first and second plants;
   obtaining plants resistant to an agent that selects for cells containing the nucleic acid encoding the selectable marker; and selecting a resistant plant that contains cells comprising an acrocentric plant chromosome.
51. The method of any of 45-50, wherein the DNA of the short arm of the acrocentric chromosome contains less than 5% euchromatic DNA.
52. The method of any of 45-50, wherein the DNA of the short arm of the acrocentric chromosome contains less than 1% euchromatic DNA.
53. The method of any of 45-50, wherein the short arm of the acrocentric chromosome does not contain euchromatic DNA.
54. The method of any of 45-49, wherein the nucleic acid introduced into a chromosome comprises nucleic acid encoding a selectable marker.
55. An acrocentric plant artificial chromosome, wherein the short arm of the acrocentric chromosome does not contain euchromatic DNA.
56. A method of producing a plant artificial chromosome, comprising:
   introducing nucleic acid into a plant acrocentric chromosome in a cell, wherein the short arm of the acrocentric chromosome does not contain euchromatic DNA;
   culturing the cell through at least one cell division; and
   selecting a cell comprising an artificial chromosome is predominantly heterochromatic.
57. The method of 56, wherein the acrocentric chromosome is produced by the method of any of 45-49.
58. A method for producing an artificial chromosome, comprising:
   introducing nucleic acid into a plant cell; and
   selecting a plant cell comprising an artificial chromosome that comprises one or more repeat regions wherein:
   one or more nucleic acid units is (are) repeated in a repeat region;
   repeats of a nucleic acid unit have common nucleic acid sequences; and
   the common nucleic acid sequences comprise sequences that represent euchromatic and heterochromatic nucleic acid.
59. The method of 4, wherein the nucleic acid comprises plant rDNA from a dicot plant species.
60. The method of 4, wherein the nucleic acid comprises plant rDNA from a monocot plant species.
61. The method of 9, wherein the intergenic spacer region is from DNA from a Nicotiana plant.
62. The method of 9, wherein the rDNA is plant rDNA.
63. The method of 62, wherein the plant is a dicot plant species.
64. The method of 62, wherein the plant is a monocot plant species.
65. The method of 1, wherein the cell is a dicot plant cell.
66. The method of 1, wherein the cell is a monocot plant cell.
67. An isolated plant artificial chromosome comprising one or more repeat regions, wherein:
   one or more nucleic acid units is (are) repeated in a repeat region;
   repeats of a nucleic acid unit have common nucleic acid sequences; and
   the common nucleic acid sequences comprise sequences that represent euchromatic and heterochromatic nucleic acid.
68. The method of 31, wherein the artificial chromosome is produced by a method comprising:
   introducing nucleic acid into a plant cell; and
   selecting a plant cell comprising an artificial chromosome that comprises one or more repeat regions, wherein:
      repeats of a nucleic acid unit have common nucleic acid sequences; and
      the common nucleic acid sequences comprise sequences that represent euchromatic and heterochromatic nucleic acid.
69. The method of 44, wherein the recombinase is selected from the group consisting of a bacteriophage P1 Cre recombinase, a yeast R recombinase and a yeast FLP recombinase.
70. The method of 50, further comprising selecting first and second transgenic plants wherein:
   one of the plants comprises a chromosome comprising a recombination site located on a short arm of the chromosome in a region adjacent to the pericentric heterochromatin; and
   the other plant comprises a chromosome comprising a recombination site located in rDNA of the chromosome.
71. The method of 70, wherein the recombination sites on the two chromosomes are in the same orientation.
72. A method for producing an acrocentric plant chromosome, comprising:
   introducing nucleic acid comprising two site-specific recombination sites into a cell comprising one or more plant chromosomes;
   introducing a recombinase activity into the cell, wherein the activity catalyzes recombination between the two recombination sites, whereby a plant acrocentric chromosome is produced.
73. The method of 72, wherein the two site-specific recombination sites are contained on separate nucleic acid fragments.
74. The method of 73, wherein the separate nucleic acid fragments are introduced into the cell simultaneously or sequentially.
75. The method of 56, wherein the artificial chromosome is predominantly heterochromatic.
76. A method of producing a plant artificial chromosome, comprising:
   introducing nucleic acid into a plant chromosome in a cell, wherein the chromosome contains adjacent regions of rDNA and heterochromatic DNA;
   culturing the cell through at least one cell division; and
   selecting a cell comprising an artificial chromosome.
77. The method of 76, wherein the artificial chromosome is predominantly heterochromatic.
78. The method of 76 or 77, wherein the plant chromosome into which the nucleic acid is introduced is an acrocentric chromosome.
79. The method of 78, wherein the short arm of the chromosome contains adjacent regions of rDNA and heterochromatic DNA.
80. The method of any of 76-79, wherein the heterochromatic DNA is pericentric heterochromatin.
81. A vector, comprising:
   nucleic acid encoding a selectable marker that is not operably associated with any promoter, wherein the selectable marker permits growth of animal cells in the presence of an agent normally toxic to the animal cells; and
   wherein the agent is not toxic to plant cells;
   a recognition site for recombination; and
   a sequence of nucleotides that facilitates amplification of a region of a plant chromosome or targets the vector to an amplifiable region of a plant chromosome.
82. The vector of 81, wherein the amplifiable region comprises heterochromatic nucleic acid.
83. The vector of 81, wherein the amplifiable region comprises rDNA.
84. The vector of 81, wherein the sequence of nucleotides that facilitates amplification of a region of a plant chromosome or targets the vector to an amplifiable region of a plant chromosome comprises a sufficient portion of an intergenic spacer region of rDNA to facilitate amplification or effect the targeting.
85. The vector of 84, wherein the sufficient portion contains at least 14, 20, 30, 50, 100, 150, 300 or 500 contiguous nucleotides from an intergenic spacer region.
86. The vector of 81, wherein the selectable marker encodes a product that confers resistance to zeomycin.
88. The vector of 81, wherein the recognition site comprises an att site.
89. The vector 81, that is pAgila or pAgilb.
90. A vector, comprising:
   nucleic acid encoding a selectable marker that is not operably associated with any promoter, wherein the selectable marker permits growth of animal cells in the presence of an agent normally toxic to the animal cells; and
   wherein the agent is not toxic to plant cells;
   a recognition site for recombination; and
   nucleic acid encoding a protein operably linked to a plant promoter.
91. The vector of 90, wherein the recognition site comprises an att site.
92. The vector of 90, further comprising a sequence of nucleotides that facilitates amplification of a region of a plant chromosome or targets the vector to an amplifiable region of a plant chromosome.
93. The vector of 90, wherein the promoter is opaline synthase (NOS) or CaMV35S.
94. The vector of 93 that is pAg1 or pAg 2.
95. The vector of 92, wherein the amplifiable region comprises heterochromatic nucleic acid.
96. The vector of 92, wherein the amplifiable region comprises rDNA.
97. The vector of 96, wherein the sequence of nucleotides that facilitates amplification of a region of a plant chromosome or targets the vector to an amplifiable region of a plant chromosome comprises a sufficient portion of an intergenic spacer region of rDNA to effect the amplification or the targeting.
98. The vector of 90, wherein the protein is a selectable marker that permits growth of plant cells in the presence of an agent normally toxic to the plant cells.
99. The vector of 98, wherein the selectable marker confers resistance to hygromycin or to phosphothricin.
100. The vector of 90, wherein the protein is a fluorescent protein.
101. The vector of 90, wherein the fluorescent protein is selected from the group consisting of green, blue and red fluorescent proteins.
102. A vector, comprising:
   nucleic acid encoding a selectable marker that is not operably associated with any promoter, wherein the selectable marker permits growth of plant cells in the presence of an agent normally toxic to the plant cells; and
   wherein the agent is not toxic to animal cells;
   a recognition site for recombination; and
   nucleic acid encoding a protein operably linked to a plant promoter.
103. A vector, comprising:
   a recognition site for recombination; and
   a sequence of nucleotides that facilitates amplification of a region of a plant chromosome or targets the vector to an amplifiable region of a plant chromosome, wherein the plant is selected from the group consisting *of Arabidopsis, Nicotiana, Solanum, Lycopersicon, Daucus, Hordeum, Zea mays, Brassica, Triticum, Helianthus, Glycine,* soybean, *Gossypium,* cotton, *Helianthus,* sunflower and *Oryza.*
104. The vector of 103, wherein the recognition site comprises an att site.
105. A cell, comprising a vector of any of.81-104.
106. The cell of 105 that is a plant cell.
107. A method, comprising:
   introducing a vector of 90 into a cell, wherein:
      the cell comprises an animal platform ACes that contains a recognition site that recombines with the recognition site in the vector in the presences of the recombinase therefor, thereby incorporating the selectable marker that is not operably associated with any promoter and the nucleic acid encoding a protein operably linked to a plant promoter into the platform ACes to produce a resulting platform ACes.
108. The method of 107, wherein the recombination sites are att sites.
109. The method of 107, wherein the animal is a mammal.
110. The method of 107, wherein the platform ACes comprises a promoter that upon recombination is operably linked to the selectable marker that in the vector is not operably associated with a promoter.
111. The method of any of 107-110, further comprising, transferring the resulting platform ACes into a plant cell to produce a plant cell the compriese the platform Aces.
112. The method of 111, wherein the resulting platform ACes is isolated prior to transfer.
113. The method of 111, wherein the isolated ACes is introduced into a plant cell by a method selected from the group consisting of protoplast transfection, lipid-mediated delivery, liposomes, electroporation, sonoporation, microinjection, particle bombardment, silicon carbide whisker-mediated transformation, polyethylene glycol (PEG)-mediated DNA uptake, lipofection and lipid-mediated carrier systems.
114. The method of 111, wherein the resulting platform ACes is transferred by fusion of the cells.
115. The method of 111, wherein the cells are plant protoplasts.
116. The method of any of 107, wherein the cell is an animal cell.
117. The method of 116, wherein the animal cell is a mammalian cell.
118. The method of 111, further comprising culturing the plant cell that comprises the platform Aces under conditions whereby the protein encoded by the nucleic acid that is operably linked to a plant promoter is expressed.
119. A method, comprising:
   introducing a vector of 81 into a plant cell;
   culturing the plant cells; and
   selecting a plant cell comprising an artificial chromosome that comprises one or more repeat regions.
120. The method of 119, wherein sufficient portion of the vector integrates into a chromosome in the plant cell to result in amplification of chromosomal DNA.
121. The method of 119 or 120, wherein:
   one or more nucleic acid units is (are) repeated in a repeat region;
   repeats of a nucleic acid unit have common nucleic acid sequences; and
   the repeat region (s) contain substantially equivalent amounts of euchromatic and
   heterochromatic nucleic acid.
122. The method of 119, further comprising isolating the artificial chromosome.
123. A method, comprising:
   introducing a vector into a cell, wherein:
      i) the vector comprises:
         a) nucleic acid encoding a selectable marker that is not operably associated with any promoter, wherein the selectable marker permits growth of animal cells in the presence of an agent normally toxic to the animal cells; and
            wherein the agent is not toxic to plant cells;
         b) a recognition site for recombination; and
         c) nucleic acid encoding a protein operably linked to an animal promoter;
      ii) the cell comprises: a platform plant artifical chromosome (PAC) that comprises a recombination site and an animal promoter that upon recombination is operably linked to the selectable marker that, in the vector, is not operably associated with a promoter;
      iii) introduction is effected under conditions whereby the vector recombines with the PAC to produce a plant platform PAC that contains the selectable marker operably linked to the promoter; and
   culturing the resulting cell under conditions, whereby the protein encoded by nucleic acid operably linked to an animal promoter is expressed.
124. The method of 119, wherein the artificial chromosome is an ACes.
125. The method of 123, wherein the plant platform PAC is an ACes.
126. The method of 1, wherein the nucleic acid introduced into the cell comprises nucleic acid encoding a selectable marker.
127. The vector of 81, further comprising one or more selectable markers that when expressed in the plant cell permit the selection of the cell.
128. A plant transformation vector, comprising:
   a recognition site for recombination;
   a sequence of nucleotides that facilitates amplification of a region of a plant chromosome or targets the vector to an amplifiable region of a plant chromosome;
   and one or more selectable markers that when expressed in a plant cell permit the selection of the cell;
   wherein the plant transformation vector is for Agrobacterium-mediated transformation of plants.
129. A method of producing a plant artificial chromosome, comprising:
   introducing the vector of any of 81,127 and 128 into a cell comprising one or more plant chromosomes; and
   selecting a cell comprising an artificial chromosome that comprises one or more repeat regions;
   wherein one or more nucleic acid units is (are) repeated in a repeat region;
   repeats of a nucleic acid unit have common nucleic acid sequences; and
   the common nucleic acid sequences comprise sequences that represent euchromatic and heterochromatic nucleic acid.
130. A method of producing a plant artificial chromosome, comprising:
   introducing the vector of any of 81,127 and 128 into a cell comprising one or more plant chromosomes; and selecting a cell comprising an artificial chromosome that
   comprises one or more repeat regions;
   wherein one or more nucleic acid units is (are) repeated in a repeat region; repeats of a nucleic acid unit have common nucleic acid sequences; and the repeat region (s) contain substantially equivalent amounts of euchromatic and heterochromatic nucleic acid.
131. The method of 123, wherein the cell into which the vector is introduced is an animal cell.
132. The method of 131, wherein the cell is a mammalian cell.

## Claims

1. A method for producing a plant artificial chromosome, comprising:
introducing nucleic acid into a plant cell containing one or more plant chromosomes;
culturing the cell through multiple cell divisions; and
selecting a cell comprising an artificial chromosome that comprises repeat regions, wherein:
the repeat regions contain euchromatic and hetcrochromatic nucleic acid and each repeat region contains substantially equivalent amounts of euchromatin and heterochromatin;
substantially equivalent means that the repeat regions contain about 40% to about 50% of euchromatic or heterochromatic nucleic acid and about 50% to about 60% of the other type of nucleic acid; and
the artificial chromosome contains substantially equivalent amounts of euchromatic and heterochromatic nucleic acid.

2. The method of claim 1, wherein the cell is a dicot plant cell or a monocot plant cell.

3. The method of claim 1 or claim 2, wherein:
the nucleic acid introduced into the cell comprises a nucleic acid sequence that facilitates amplification of a region of a plant chromosome or that targets the nucleic acid to an amplifiable region of a plant chromosome; and/or the nucleic acid introduced into the cell comprises one or more nucleic acids selected from among rDNA., lambda phage RNA and satellite DNA.

4. The method of claim 3, wherein the rDNA is from a plant selected from *among Arabidopsis, Nicotiana. Solarium, Lycopersicon, Daucus, Hordeum, Zea mays, Brassica, Triticum* and *Oryza.*

5. The method of claim 3, wherein the nucleic acid comprises plant rDNA from a dicot plant species or a monocots plant species.

6. The method of claim 3, wherein the nucleic acid introduced into the cell comprises rDNA that comprises an intergenic spacer region.

7. The method of claim 6, wherein the intergenic spacer region is from DNA from a plant selected from among *Arabidopsis, Solarium, Lycopersicon, Hordeum, Zea, Oryza,* rye, wheat, radish, mung bean and *Nicotiana.*

8. The method of any of claims 1-7, wherein the nucleic acid introduced into the cell comprises a nucleic acid sequence that facilitates identification of cells containing the nucleic acid.

9. The method of claim 8, wherein the nucleic acid sequence that facilitates identification encodes a fluorescent protein.

10. The method of any of claims 1-9, wherein the step of selecting a cell comprising an artificial chromosome comprises sorting of cells into which nucleic acid was introduced or fluroreseent in situ hybridization (FISH) analysis of cells into which the nucleic acid was introduced.

11. The method of any of claims 1-10, wherein the cell is a plant protoplast.

12. The method of any of claims 1-11, wherein the nucleic acid introduced into the cell comprises nucleic acid encoding a selectable marker.

13. The method of claim 12, wherein the selectable marker confers resistance to phosphinothricin, ammonium glufosinate, glyphosate, kanamycin, hygromycin, dihydrofolate or sulfonylurea.

14. An isolated plant artificial chromosome produced by the method of any of claims 1-13.

15. A plant cell, comprising an artificial chromosome of claim 14.

16. A method of producing a transgenic plant, comprising introducing the artificial chromosome of claim 15 into a plant cell.

17. The method of any of claims 1-13 and 16, wherein the nucleic acid introduced into the cell encodes a gene product or the artificial chromosome comprises heteroiogous nucleic acid encoding a gene product.

18. The method of claim 17, wherein the nucleic acid encodes a product selected from among enzymes, antisense RNA, tRNA, rDNA, structural proteins, marker proteins, ligands, receptors, ribozymes, therapeutic proteins and biopharmaceutica! proteins.

19. The method of claim 17 or claim 18, wherein the nucleic acid encodes a product selected from the group consisting of vaccines, blood factors, antigens, hormones, cytokines, growth factors, antibodies, a product that provides for resistance to diseases, insects, herbicides or stress in the plant and a product that alters the nutrient utilization and/or improves the nutrient quality of the plant.


SEQUENCE LISTING

<110> Agrisoma, Inc. and Calyx Bio-Ventures Inc.

<120> Plant Artificial Chromosomes, Uses thereof, and Methods of Preparing
      Plant Artificial Chromosomes

<130> P64751EP01

<150> US 60/294,687
<151> 2001-05-30

<150> US 60/296,329
<151> 2001-06-04

<160> 51

<170> FastSEQ for Windows Version 4.0

<210> 1
<211> 11182
<212> DNA
<213> Artificial Sequence

<220>
<223> pAg1 plasmid

<400> 1
catgccaacc acagggttcc cctcgggatc aaagtacttt gatccaaccc ctccgctgct 60
atagtgcagt cggcttctga cgttcagtgc agccgtcttc tgaaaacgac atgtcgcaca 120
agtcctaagt tacgcgacag gctgccgccc tgcccttttc ctggcgtttt cttgtcgcgt 180
gttttagtcg cataaagtag aatacttgcg actagaaccg gagacattac gccatgaaca 240
agagcgccgc cgctggcctg ctgggctatg cccgcgtcag caccgacgac caggacttga 300
ccaaccaacg ggccgaactg cacgcggccg gctgcaccaa gctgttttcc gagaagatca 360
ccggcaccag gcgcgaccgc ccggagctgg ccaggatgct tgaccaccta cgccctggcg 420
acgttgtgac agtgaccagg ctagaccgcc tggcccgcag cacccgcgac ctactggaca 480
ttgccgagcg catccaggag gccggcgcgg gcctgcgtag cctggcagag ccgtgggccg 540
acaccaccac gccggccggc cgcatggtgt tgaccgtgtt cgccggcatt gccgagttcg 600
agcgttccct aatcatcgac cgcacccgga gcgggcgcga ggccgccaag gcccgaggcg 660
tgaagtttgg cccccgccct accctcaccc cggcacagat cgcgcacgcc cgcgagctga 720
tcgaccagga aggccgcacc gtgaaagagg cggctgcact gcttggcgtg catcgctcga 780
ccctgtaccg cgcacttgag cgcagcgagg aagtgacgcc caccgaggcc aggcggcgcg 840
gtgccttccg tgaggacgca ttgaccgagg ccgacgccct ggcggccgcc gagaatgaac 900
gccaagagga acaagcatga aaccgcacca ggacggccag gacgaaccgt ttttcattac 960
cgaagagatc gaggcggaga tgatcgcggc cgggtacgtg ttcgagccgc ccgcgcacgt 1020
ctcaaccgtg cggctgcatg aaatcctggc cggtttgtct gatgccaagc tggcggcctg 1080
gccggccagc ttggccgctg aagaaaccga gcgccgccgt ctaaaaaggt gatgtgtatt 1140
tgagtaaaac agcttgcgtc atgcggtcgc tgcgtatatg atgcgatgag taaataaaca 1200
aatacgcaag gggaacgcat gaaggttatc gctgtactta accagaaagg cgggtcaggc 1260
aagacgacca tcgcaaccca tctagcccgc gccctgcaac tcgccggggc cgatgttctg 1320
ttagtcgatt ccgatcccca gggcagtgcc cgcgattggg cggccgtgcg ggaagatcaa 1380
ccgctaaccg ttgtcggcat cgaccgcccg acgattgacc gcgacgtgaa ggccatcggc 1440
cggcgcgact tcgtagtgat cgacggagcg ccccaggcgg cggacttggc tgtgtccgcg 1500
atcaaggcag ccgacttcgt gctgattccg gtgcagccaa gcccttacga catatgggcc 1560
accgccgacc tggtggagct ggttaagcag cgcattgagg tcacggatgg aaggctacaa 1620
gcggcctttg tcgtgtcgcg ggcgatcaaa ggcacgcgca tcggcggtga ggttgccgag 1680
gcgctggccg ggtacgagct gcccattctt gagtcccgta tcacgcagcg cgtgagctac 1740
ccaggcactg ccgccgccgg cacaaccgtt cttgaatcag aacccgaggg cgacgctgcc 1800
cgcgaggtcc aggcgctggc cgctgaaatt aaatcaaaac tcatttgagt taatgaggta 1860
aagagaaaat gagcaaaagc acaaacacgc taagtgccgg ccgtccgagc gcacgcagca 1920
gcaaggctgc aacgttggcc agcctggcag acacgccagc catgaagcgg gtcaactttc 1980

agttgccggc ggaggatcac accaagctga agatgtacgc ggtacgccaa ggcaagacca 2040
ttaccgagct gctatctgaa tacatcgcgc agctaccaga gtaaatgagc aaatgaataa 2100
atgagtagat gaattttagc ggctaaagga ggcggcatgg aaaatcaaga acaaccaggc 2160
accgacgccg tggaatgccc catgtgtgga ggaacgggcg gttggccagg cgtaagcggc 2220
tgggttgtct gccggccctg caatggcact ggaaccccca agcccgagga atcggcgtga 2280
cggtcgcaaa ccatccggcc cggtacaaat cggcgcggcg ctgggtgatg acctggtgga 2340
gaagttgaag gccgcgcagg ccgcccagcg gcaacgcatc gaggcagaag cacgccccgg 2400
tgaatcgtgg caagcggccg ctgatcgaat ccgcaaagaa tcccggcaac cgccggcagc 2460
cggtgcgccg tcgattagga agccgcccaa gggcgacgag caaccagatt ttttcgttcc 2520
gatgctctat gacgtgggca cccgcgatag tcgcagcatc atggacgtgg ccgttttccg 2580
tctgtcgaag cgtgaccgac gagctggcga ggtgatccgc tacgagcttc cagacgggca 2640
cgtagaggtt tccgcagggc cggccggcat ggccagtgtg tgggattacg acctggtact 2700
gatggcggtt tcccatctaa ccgaatccat gaaccgatac cgggaaggga agggagacaa 2760
gcccggccgc gtgttccgtc cacacgttgc ggacgtactc aagttctgcc ggcgagccga 2820
tggcggaaag cagaaagacg acctggtaga aacctgcatt cggttaaaca ccacgcacgt 2880
tgccatgcag cgtacgaaga aggccaagaa cggccgcctg gtgacggtat ccgagggtga 2940
agccttgatt agccgctaca agatcgtaaa gagcgaaacc gggcggccgg agtacatcga 3000
gatcgagcta gctgattgga tgtaccgcga gatcacagaa ggcaagaacc cggacgtgct 3060
gacggttcac cccgattact ttttgatcga tcccggcatc ggccgttttc tctaccgcct 3120
ggcacgccgc gccgcaggca aggcagaagc cagatggttg ttcaagacga tctacgaacg 3180
cagtggcagc gccggagagt tcaagaagtt ctgtttcacc gtgcgcaagc tgatcgggtc 3240
aaatgacctg ccggagtacg atttgaagga ggaggcgggg caggctggcc cgatcctagt 3300
catgcgctac cgcaacctga tcgagggcga agcatccgcc ggttcctaat gtacggagca 3360
gatgctaggg caaattgccc tagcagggga aaaaggtcga aaaggtctct ttcctgtgga 3420
tagcacgtac attgggaacc caaagccgta cattgggaac cggaacccgt acattgggaa 3480
cccaaagccg tacattggga accggtcaca catgtaagtg actgatataa aagagaaaaa 3540
aggcgatttt tccgcctaaa actctttaaa acttattaaa actcttaaaa cccgcctggc 3600
ctgtgcataa ctgtctggcc agcgcacagc cgaagagctg caaaaagcgc ctacccttcg 3660
gtcgctgcgc tccctacgcc ccgccgcttc gcgtcggcct atcgcggccg ctggccgctc 3720
aaaaatggct ggcctacggc caggcaatct accagggcgc ggacaagccg cgccgtcgcc 3780
actcgaccgc cggcgcccac atcaaggcac cctgcctcgc gcgtttcggt gatgacggtg 3840
aaaacctctg acacatgcag ctcccggaga cggtcacagc ttgtctgtaa gcggatgccg 3900
ggagcagaca agcccgtcag ggcgcgtcag cgggtgttgg cgggtgtcgg ggcgcagcca 3960
tgacccagtc acgtagcgat agcggagtgt atactggctt aactatgcgg catcagagca 4020
gattgtactg agagtgcacc atatgcggtg tgaaataccg cacagatgcg taaggagaaa 4080
ataccgcatc aggcgctctt ccgcttcctc gctcactgac tcgctgcgct cggtcgttcg 4140
gctgcggcga gcggtatcag ctcactcaaa ggcggtaata cggttatcca cagaatcagg 4200
ggataacgca ggaaagaaca tgtgagcaaa aggccagcaa aaggccagga accgtaaaaa 4260
ggccgcgttg ctggcgtttt tccataggct ccgcccccct gacgagcatc acaaaaatcg 4320
acgctcaagt cagaggtggc gaaacccgac aggactataa agataccagg cgtttccccc 4380
tggaagctcc ctcgtgcgct ctcctgttcc gaccctgccg cttaccggat acctgtccgc 4440
ctttctccct tcgggaagcg tggcgctttc tcatagctca cgctgtaggt atctcagttc 4500
ggtgtaggtc gttcgctcca agctgggctg tgtgcacgaa ccccccgttc agcccgaccg 4560
ctgcgcctta tccggtaact atcgtcttga gtccaacccg gtaagacacg acttatcgcc 4620
actggcagca gccactggta acaggattag cagagcgagg tatgtaggcg gtgctacaga 4680
gttcttgaag tggtggccta actacggcta cactagaagg acagtatttg gtatctgcgc 4740
tctgctgaag ccagttacct tcggaaaaag agttggtagc tcttgatccg gcaaacaaac 4800
caccgctggt agcggtggtt tttttgtttg caagcagcag attacgcgca gaaaaaaagg 4860
atctcaagaa gatcctttga tcttttctac ggggtctgac gctcagtgga acgaaaactc 4920
acgttaaggg attttggtca tgcattctag gtactaaaac aattcatcca gtaaaatata 4980
atattttatt ttctcccaat caggcttgat ccccagtaag tcaaaaaata gctcgacata 5040
ctgttcttcc ccgatatcct ccctgatcga ccggacgcag aaggcaatgt cataccactt 5100
gtccgccctg ccgcttctcc caagatcaat aaagccactt actttgccat ctttcacaaa 5160
gatgttgctg tctcccaggt cgccgtggga aaagacaagt tcctcttcgg gcttttccgt 5220
ctttaaaaaa tcatacagct cgcgcggatc tttaaatgga gtgtcttctt cccagttttc 5280
gcaatccaca tcggccagat cgttattcag taagtaatcc aattcggcta agcggctgtc 5340
taagctattc gtatagggac aatccgatat gtcgatggag tgaaagagcc tgatgcactc 5400
cgcatacagc tcgataatct tttcagggct ttgttcatct tcatactctt ccgagcaaag 5460
gacgccatcg gcctcactca tgagcagatt gctccagcca tcatgccgtt caaagtgcag 5520
gacctttgga acaggcagct ttccttccag ccatagcatc atgtcctttt cccgttccac 5580
atcataggtg gtccctttat accggctgtc cgtcattttt aaatataggt tttcattttc 5640

tcccaccagc ttatatacct tagcaggaga cattccttcc gtatctttta cgcagcggta 5700
tttttcgatc agttttttca attccggtga tattctcatt ttagccattt attatttcct 5760
tcctcttttc tacagtattt aaagataccc caagaagcta attataacaa gacgaactcc 5820
aattcactgt tccttgcatt ctaaaacctt aaataccaga aaacagcttt ttcaaagttg 5880
ttttcaaagt tggcgtataa catagtatcg acggagccga ttttgaaacc gcggtgatca 5940
caggcagcaa cgctctgtca tcgttacaat caacatgcta ccctccgcga gatcatccgt 6000
gtttcaaacc cggcagctta gttgccgttc ttccgaatag catcggtaac atgagcaaag 6060
tctgccgcct tacaacggct ctcccgctga cgccgtcccg gactgatggg ctgcctgtat 6120
cgagtggtga ttttgtgccg agctgccggt cggggagctg ttggctggct ggtggcagga 6180
tatattgtgg tgtaaacaaa ttgacgctta gacaacttaa taacacattg cggacgtttt 6240
taatgtactg aattaacgcc gaattaattc gggggatctg gattttagta ctggattttg 6300
gttttaggaa ttagaaattt tattgataga agtattttac aaatacaaat acatactaag 6360
ggtttcttat atgctcaaca catgagcgaa accctatagg aaccctaatt cccttatctg 6420
ggaactactc acacattatt atggagaaac tcgagtcaaa tctcggtgac gggcaggacc 6480
ggacggggcg gtaccggcag gctgaagtcc agctgccaga aacccacgtc atgccagttc 6540
ccgtgcttga agccggccgc ccgcagcatg ccgcgggggg catatccgag cgcctcgtgc 6600
atgcgcacgc tcgggtcgtt gggcagcccg atgacagcga ccacgctctt gaagccctgt 6660
gcctccaggg acttcagcag gtgggtgtag agcgtggagc ccagtcccgt ccgctggtgg 6720
cggggggaga cgtacacggt cgactcggcc gtccagtcgt aggcgttgcg tgccttccag 6780
gggcccgcgt aggcgatgcc ggcgacctcg ccgtccacct cggcgacgag ccagggatag 6840
cgctcccgca gacggacgag gtcgtccgtc cactcctgcg gttcctgcgg ctcggtacgg 6900
aagttgaccg tgcttgtctc gatgtagtgg ttgacgatgg tgcagaccgc cggcatgtcc 6960
gcctcggtgg cacggcggat gtcggccggg cgtcgttctg ggctcatggt agactcgaga 7020
gagatagatt tgtagagaga gactggtgat ttcagcgtgt cctctccaaa tgaaatgaac 7080
ttccttatat agaggaaggt cttgcgaagg atagtgggat tgtgcgtcat cccttacgtc 7140
agtggagata tcacatcaat ccacttgctt tgaagacgtg gttggaacgt cttctttttc 7200
cacgatgctc ctcgtgggtg ggggtccatc tttgggacca ctgtcggcag aggcatcttg 7260
aacgatagcc tttcctttat cgcaatgatg gcatttgtag gtgccacctt ccttttctac 7320
tgtccttttg atgaagtgac agatagctgg gcaatggaat ccgaggaggt ttcccgatat 7380
taccctttgt tgaaaagtct caatagccct ttggtcttct gagactgtat ctttgatatt 7440
cttggagtag acgagagtgt cgtgctccac catgttatca catcaatcca cttgctttga 7500
agacgtggtt ggaacgtctt ctttttccac gatgctcctc gtgggtgggg gtccatcttt 7560
gggaccactg tcggcagagg catcttgaac gatagccttt cctttatcgc aatgatggca 7620
tttgtaggtg ccaccttcct tttctactgt ccttttgatg aagtgacaga tagctgggca 7680
atggaatccg aggaggtttc ccgatattac cctttgttga aaagtctcaa tagccctttg 7740
gtcttctgag actgtatctt tgatattctt ggagtagacg agagtgtcgt gctccaccat 7800
gttggcaagc tgctctagcc aatacgcaaa ccgcctctcc ccgcgcgttg gccgattcat 7860
taatgcagct ggcacgacag gtttcccgac tggaaagcgg gcagtgagcg caacgcaatt 7920
aatgtgagtt agctcactca ttaggcaccc caggctttac actttatgct tccggctcgt 7980
atgttgtgtg gaattgtgag cggataacaa tttcacacag gaaacagcta tgaccatgat 8040
tacgaattcg agccttgact agagggtcga cggtatacag acatgataag atacattgat 8100
gagtttggac aaaccacaac tagaatgcag tgaaaaaaat gctttatttg tgaaatttgt 8160
gatgctattg ctttatttgt aaccattata agctgcaata aacaagttgg ggtgggcgaa 8220
gaactccagc atgagatccc cgcgctggag gatcatccag ccggcgtccc ggaaaacgat 8280
tccgaagccc aacctttcat agaaggcggc ggtggaatcg aaatctcgta gcacgtgtca 8340
gtcctgctcc tcggccacga agtgcacgca gttgccggcc gggtcgcgca gggcgaactc 8400
ccgcccccac ggctgctcgc cgatctcggt catggccggc ccggaggcgt cccggaagtt 8460
cgtggacacg acctccgacc actcggcgta cagctcgtcc aggccgcgca cccacaccca 8520
ggccagggtg ttgtccggca ccacctggtc ctggaccgcg ctgatgaaca gggtcacgtc 8580
gtcccggacc acaccggcga agtcgtcctc cacgaagtcc cgggagaacc cgagccggtc 8640
ggtccagaac tcgaccgctc cggcgacgtc gcgcgcggtg agcaccggaa cggcactggt 8700
caacttggcc atggatccag atttcgctca agttagtata aaaaagcagg cttcaatcct 8760
gcaggaattc gatcgacact ctcgtctact ccaagaatat caaagataca gtctcagaag 8820
accaaagggc tattgagact tttcaacaaa gggtaatatc gggaaacctc ctcggattcc 8880
attgcccagc tatctgtcac ttcatcaaaa ggacagtaga aaaggaaggt ggcacctaca 8940
aatgccatca ttgcgataaa ggaaaggcta tcgttcaaga tgcctctgcc gacagtggtc 9000
ccaaagatgg acccccaccc acgaggagca tcgtggaaaa agaagacgtt ccaaccacgt 9060
cttcaaagca agtggattga tgtgataaca tggtggagca cgacactctc gtctactcca 9120
agaatatcaa agatacagtc tcagaagacc aaagggctat tgagactttt caacaaaggg 9180
taatatcggg aaacctcctc ggattccatt gcccagctat ctgtcacttc atcaaaagga 9240
cagtagaaaa ggaaggtggc acctacaaat gccatcattg cgataaagga aaggctatcg 9300

ttcaagatgc ctctgccgac agtggtccca aagatggacc cccacccacg aggagcatcg 9360
tggaaaaaga agacgttcca accacgtctt caaagcaagt ggattgatgt gatatctcca 9420
ctgacgtaag ggatgacgca caatcccact atccttcgca agaccttcct ctatataagg 9480
aagttcattt catttggaga ggacacgctg aaatcaccag tctctctcta caaatctatc 9540
tctctcgagc tttcgcagat ccgggggggc aatgagatat gaaaaagcct gaactcaccg 9600
cgacgtctgt cgagaagttt ctgatcgaaa agttcgacag cgtctccgac ctgatgcagc 9660
tctcggaggg cgaagaatct cgtgctttca gcttcgatgt aggagggcgt ggatatgtcc 9720
tgcgggtaaa tagctgcgcc gatggtttct acaaagatcg ttatgtttat cggcactttg 9780
catcggccgc gctcccgatt ccggaagtgc ttgacattgg ggagtttagc gagagcctga 9840
cctattgcat ctcccgccgt gcacagggtg tcacgttgca agacctgcct gaaaccgaac 9900
tgcccgctgt tctacaaccg gtcgcggagg ctatggatgc gatcgctgcg gccgatctta 9960
gccagacgag cgggttcggc ccattcggac cgcaaggaat cggtcaatac actacatggc 10020
gtgatttcat atgcgcgatt gctgatcccc atgtgtatca ctggcaaact gtgatggacg 10080
acaccgtcag tgcgtccgtc gcgcaggctc tcgatgagct gatgctttgg gccgaggact 10140
gccccgaagt ccggcacctc gtgcacgcgg atttcggctc caacaatgtc ctgacggaca 10200
atggccgcat aacagcggtc attgactgga gcgaggcgat gttcggggat tcccaatacg 10260
aggtcgccaa catcttcttc tggaggccgt ggttggcttg tatggagcag cagacgcgct 10320
acttcgagcg gaggcatccg gagcttgcag gatcgccacg actccgggcg tatatgctcc 10380
gcattggtct tgaccaactc tatcagagct tggttgacgg caatttcgat gatgcagctt 10440
gggcgcaggg tcgatgcgac gcaatcgtcc gatccggagc cgggactgtc gggcgtacac 10500
aaatcgcccg cagaagcgcg gccgtctgga ccgatggctg tgtagaagta ctcgccgata 10560
gtggaaaccg acgccccagc actcgtccga gggcaaagaa atagagtaga tgccgaccgg 10620
atctgtcgat cgacaagctc gagtttctcc ataataatgt gtgagtagtt cccagataag 10680
ggaattaggg ttcctatagg gtttcgctca tgtgttgagc atataagaaa cccttagtat 10740
gtatttgtat ttgtaaaata cttctatcaa taaaatttct aattcctaaa accaaaatcc 10800
agtactaaaa tccagatccc ccgaattaat tcggcgttaa ttcagatcaa gcttggcact 10860
ggccgtcgtt ttacaacgtc gtgactggga aaaccctggc gttacccaac ttaatcgcct 10920
tgcagcacat ccccctttcg ccagctggcg taatagcgaa gaggcccgca ccgatcgccc 10980
ttcccaacag ttgcgcagcc tgaatggcga atgctagagc agcttgagct tggatcagat 11040
tgtcgtttcc cgccttcagt ttaaactatc agtgtttgac aggatatatt ggcgggtaaa 11100
cctaagagaa aagagcgttt attagaataa cggatattta aaagggcgtg aaaaggttta 11160
tccgttcgtc catttgtatg tg 11182

<210> 2
<211> 8428
<212> DNA
<213> Artificial Sequence

<220>
<223> pCambia3300 plasmid

<400> 2
catgccaacc acagggttcc cctcgggatc aaagtacttt gatccaaccc ctccgctgct 60
atagtgcagt cggcttctga cgttcagtgc agccgtcttc tgaaaacgac atgtcgcaca 120
agtcctaagt tacgcgacag gctgccgccc tgcccttttc ctggcgtttt cttgtcgcgt 180
gttttagtcg cataaagtag aatacttgcg actagaaccg gagacattac gccatgaaca 240
agagcgccgc cgctggcctg ctgggctatg cccgcgtcag caccgacgac caggacttga 300
ccaaccaacg ggccgaactg cacgcggccg gctgcaccaa gctgttttcc gagaagatca 360
ccggcaccag gcgcgaccgc ccggagctgg ccaggatgct tgaccaccta cgccctggcg 420
acgttgtgac agtgaccagg ctagaccgcc tggcccgcag cacccgcgac ctactggaca 480
ttgccgagcg catccaggag gccggcgcgg gcctgcgtag cctggcagag ccgtgggccg 540
acaccaccac gccggccggc cgcatggtgt tgaccgtgtt cgccggcatt gccgagttcg 600
agcgttccct aatcatcgac cgcacccgga gcgggcgcga ggccgccaag gcccgaggcg 660
tgaagtttgg cccccgccct accctcaccc cggcacagat cgcgcacgcc cgcgagctga 720
tcgaccagga aggccgcacc gtgaaagagg cggctgcact gcttggcgtg catcgctcga 780
ccctgtaccg cgcacttgag cgcagcgagg aagtgacgcc caccgaggcc aggcggcgcg 840
gtgccttccg tgaggacgca ttgaccgagg ccgacgccct ggcggccgcc gagaatgaac 900
gccaagagga acaagcatga aaccgcacca ggacggccag gacgaaccgt ttttcattac 960
cgaagagatc gaggcggaga tgatcgcggc cgggtacgtg ttcgagccgc ccgcgcacgt 1020
ctcaaccgtg cggctgcatg aaatcctggc cggtttgtct gatgccaagc tggcggcctg 1080
gccggccagc ttggccgctg aagaaaccga gcgccgccgt ctaaaaaggt gatgtgtatt 1140

tgagtaaaac agcttgcgtc atgcggtcgc tgcgtatatg atgcgatgag taaataaaca 1200
aatacgcaag gggaacgcat gaaggttatc gctgtactta accagaaagg cgggtcaggc 1260
aagacgacca tcgcaaccca tctagcccgc gccctgcaac tcgccggggc cgatgttctg 1320
ttagtcgatt ccgatcccca gggcagtgcc cgcgattggg cggccgtgcg ggaagatcaa 1380
ccgctaaccg ttgtcggcat cgaccgcccg acgattgacc gcgacgtgaa ggccatcggc 1440
cggcgcgact tcgtagtgat cgacggagcg ccccaggcgg cggacttggc tgtgtccgcg 1500
atcaaggcag ccgacttcgt gctgattccg gtgcagccaa gcccttacga catatgggcc 1560
accgccgacc tggtggagct ggttaagcag cgcattgagg tcacggatgg aaggctacaa 1620
gcggcctttg tcgtgtcgcg ggcgatcaaa ggcacgcgca tcggcggtga ggttgccgag 1680
gcgctggccg ggtacgagct gcccattctt gagtcccgta tcacgcagcg cgtgagctac 1740
ccaggcactg ccgccgccgg cacaaccgtt cttgaatcag aacccgaggg cgacgctgcc 1800
cgcgaggtcc aggcgctggc cgctgaaatt aaatcaaaac tcatttgagt taatgaggta 1860
aagagaaaat gagcaaaagc acaaacacgc taagtgccgg ccgtccgagc gcacgcagca 1920
gcaaggctgc aacgttggcc agcctggcag acacgccagc catgaagcgg gtcaactttc 1980
agttgccggc ggaggatcac accaagctga agatgtacgc ggtacgccaa ggcaagacca 2040
ttaccgagct gctatctgaa tacatcgcgc agctaccaga gtaaatgagc aaatgaataa 2100
atgagtagat gaattttagc ggctaaagga ggcggcatgg aaaatcaaga acaaccaggc 2160
accgacgccg tggaatgccc catgtgtgga ggaacgggcg gttggccagg cgtaagcggc 2220
tgggttgtct gccggccctg caatggcact ggaaccccca agcccgagga atcggcgtga 2280
cggtcgcaaa ccatccggcc cggtacaaat cggcgcggcg ctgggtgatg acctggtgga 2340
gaagttgaag gccgcgcagg ccgcccagcg gcaacgcatc gaggcagaag cacgccccgg 2400
tgaatcgtgg caagcggccg ctgatcgaat ccgcaaagaa tcccggcaac cgccggcagc 2460
cggtgcgccg tcgattagga agccgcccaa gggcgacgag caaccagatt ttttcgttcc 2520
gatgctctat gacgtgggca cccgcgatag tcgcagcatc atggacgtgg ccgttttccg 2580
tctgtcgaag cgtgaccgac gagctggcga ggtgatccgc tacgagcttc cagacgggca 2640
cgtagaggtt tccgcagggc cggccggcat ggccagtgtg tgggattacg acctggtact 2700
gatggcggtt tcccatctaa ccgaatccat gaaccgatac cgggaaggga agggagacaa 2760
gcccggccgc gtgttccgtc cacacgttgc ggacgtactc aagttctgcc ggcgagccga 2820
tggcggaaag cagaaagacg acctggtaga aacctgcatt cggttaaaca ccacgcacgt 2880
tgccatgcag cgtacgaaga aggccaagaa cggccgcctg gtgacggtat ccgagggtga 2940
agccttgatt agccgctaca agatcgtaaa gagcgaaacc gggcggccgg agtacatcga 3000
gatcgagcta gctgattgga tgtaccgcga gatcacagaa ggcaagaacc cggacgtgct 3060
gacggttcac cccgattact ttttgatcga tcccggcatc ggccgttttc tctaccgcct 3120
ggcacgccgc gccgcaggca aggcagaagc cagatggttg ttcaagacga tctacgaacg 3180
cagtggcagc gccggagagt tcaagaagtt ctgtttcacc gtgcgcaagc tgatcgggtc 3240
aaatgacctg ccggagtacg atttgaagga ggaggcgggg caggctggcc cgatcctagt 3300
catgcgctac cgcaacctga tcgagggcga agcatccgcc ggttcctaat gtacggagca 3360
gatgctaggg caaattgccc tagcagggga aaaaggtcga aaaggtctct ttcctgtgga 3420
tagcacgtac attgggaacc caaagccgta cattgggaac cggaacccgt acattgggaa 3480
cccaaagccg tacattggga accggtcaca catgtaagtg actgatataa aagagaaaaa 3540
aggcgatttt tccgcctaaa actctttaaa acttattaaa actcttaaaa cccgcctggc 3600
ctgtgcataa ctgtctggcc agcgcacagc cgaagagctg caaaaagcgc ctacccttcg 3660
gtcgctgcgc tccctacgcc ccgccgcttc gcgtcggcct atcgcggccg ctggccgctc 3720
aaaaatggct ggcctacggc caggcaatct accagggcgc ggacaagccg cgccgtcgcc 3780
actcgaccgc cggcgcccac atcaaggcac cctgcctcgc gcgtttcggt gatgacggtg 3840
aaaacctctg acacatgcag ctcccggaga cggtcacagc ttgtctgtaa gcggatgccg 3900
ggagcagaca agcccgtcag ggcgcgtcag cgggtgttgg cgggtgtcgg ggcgcagcca 3960
tgacccagtc acgtagcgat agcggagtgt atactggctt aactatgcgg catcagagca 4020
gattgtactg agagtgcacc atatgcggtg tgaaataccg cacagatgcg taaggagaaa 4080
ataccgcatc aggcgctctt ccgcttcctc gctcactgac tcgctgcgct cggtcgttcg 4140
gctgcggcga gcggtatcag ctcactcaaa ggcggtaata cggttatcca cagaatcagg 4200
ggataacgca ggaaagaaca tgtgagcaaa aggccagcaa aaggccagga accgtaaaaa 4260
ggccgcgttg ctggcgtttt tccataggct ccgcccccct gacgagcatc acaaaaatcg 4320
acgctcaagt cagaggtggc gaaacccgac aggactataa agataccagg cgtttccccc 4380
tggaagctcc ctcgtgcgct ctcctgttcc gaccctgccg cttaccggat acctgtccgc 4440
ctttctccct tcgggaagcg tggcgctttc tcatagctca cgctgtaggt atctcagttc 4500
ggtgtaggtc gttcgctcca agctgggctg tgtgcacgaa ccccccgttc agcccgaccg 4560
ctgcgcctta tccggtaact atcgtcttga gtccaacccg gtaagacacg acttatcgcc 4620
actggcagca gccactggta acaggattag cagagcgagg tatgtaggcg gtgctacaga 4680
gttcttgaag tggtggccta actacggcta cactagaagg acagtatttg gtatctgcgc 4740
tctgctgaag ccagttacct tcggaaaaag agttggtagc tcttgatccg gcaaacaaac 4800

caccgctggt agcggtggtt tttttgtttg caagcagcag attacgcgca gaaaaaaagg 4860
atctcaagaa gatcctttga tcttttctac ggggtctgac gctcagtgga acgaaaactc 4920
acgttaaggg attttggtca tgcattctag gtactaaaac aattcatcca gtaaaatata 4980
atattttatt ttctcccaat caggcttgat ccccagtaag tcaaaaaata gctcgacata 5040
ctgttcttcc ccgatatcct ccctgatcga ccggacgcag aaggcaatgt cataccactt 5100
gtccgccctg ccgcttctcc caagatcaat aaagccactt actttgccat ctttcacaaa 5160
gatgttgctg tctcccaggt cgccgtggga aaagacaagt tcctcttcgg gcttttccgt 5220
ctttaaaaaa tcatacagct cgcgcggatc tttaaatgga gtgtcttctt cccagttttc 5280
gcaatccaca tcggccagat cgttattcag taagtaatcc aattcggcta agcggctgtc 5340
taagctattc gtatagggac aatccgatat gtcgatggag tgaaagagcc tgatgcactc 5400
cgcatacagc tcgataatct tttcagggct ttgttcatct tcatactctt ccgagcaaag 5460
gacgccatcg gcctcactca tgagcagatt gctccagcca tcatgccgtt caaagtgcag 5520
gacctttgga acaggcagct ttccttccag ccatagcatc atgtcctttt cccgttccac 5580
atcataggtg gtccctttat accggctgtc cgtcattttt aaatataggt tttcattttc 5640
tcccaccagc ttatatacct tagcaggaga cattccttcc gtatctttta cgcagcggta 5700
tttttcgatc agttttttca attccggtga tattctcatt ttagccattt attatttcct 5760
tcctcttttc tacagtattt aaagataccc caagaagcta attataacaa gacgaactcc 5820
aattcactgt tccttgcatt ctaaaacctt aaataccaga aaacagcttt ttcaaagttg 5880
ttttcaaagt tggcgtataa catagtatcg acggagccga ttttgaaacc gcggtgatca 5940
caggcagcaa cgctctgtca tcgttacaat caacatgcta ccctccgcga gatcatccgt 6000
gtttcaaacc cggcagctta gttgccgttc ttccgaatag catcggtaac atgagcaaag 6060
tctgccgcct tacaacggct ctcccgctga cgccgtcccg gactgatggg ctgcctgtat 6120
cgagtggtga ttttgtgccg agctgccggt cggggagctg ttggctggct ggtggcagga 6180
tatattgtgg tgtaaacaaa ttgacgctta gacaacttaa taacacattg cggacgtttt 6240
taatgtactg aattaacgcc gaattaattc gggggatctg gattttagta ctggattttg 6300
gttttaggaa ttagaaattt tattgataga agtattttac aaatacaaat acatactaag 6360
ggtttcttat atgctcaaca catgagcgaa accctatagg aaccctaatt cccttatctg 6420
ggaactactc acacattatt atggagaaac tcgagtcaaa tctcggtgac gggcaggacc 6480
ggacggggcg gtaccggcag gctgaagtcc agctgccaga aacccacgtc atgccagttc 6540
ccgtgcttga agccggccgc ccgcagcatg ccgcgggggg catatccgag cgcctcgtgc 6600
atgcgcacgc tcgggtcgtt gggcagcccg atgacagcga ccacgctctt gaagccctgt 6660
gcctccaggg acttcagcag gtgggtgtag agcgtggagc ccagtcccgt ccgctggtgg 6720
cggggggaga cgtacacggt cgactcggcc gtccagtcgt aggcgttgcg tgccttccag 6780
gggcccgcgt aggcgatgcc ggcgacctcg ccgtccacct cggcgacgag ccagggatag 6840
cgctcccgca gacggacgag gtcgtccgtc cactcctgcg gttcctgcgg ctcggtacgg 6900
aagttgaccg tgcttgtctc gatgtagtgg ttgacgatgg tgcagaccgc cggcatgtcc 6960
gcctcggtgg cacggcggat gtcggccggg cgtcgttctg ggctcatggt agactcgaga 7020
gagatagatt tgtagagaga gactggtgat ttcagcgtgt cctctccaaa tgaaatgaac 7080
ttccttatat agaggaaggt cttgcgaagg atagtgggat tgtgcgtcat cccttacgtc 7140
agtggagata tcacatcaat ccacttgctt tgaagacgtg gttggaacgt cttctttttc 7200
cacgatgctc ctcgtgggtg ggggtccatc tttgggacca ctgtcggcag aggcatcttg 7260
aacgatagcc tttcctttat cgcaatgatg gcatttgtag gtgccacctt ccttttctac 7320
tgtccttttg atgaagtgac agatagctgg gcaatggaat ccgaggaggt ttcccgatat 7380
taccctttgt tgaaaagtct caatagccct ttggtcttct gagactgtat ctttgatatt 7440
cttggagtag acgagagtgt cgtgctccac catgttatca catcaatcca cttgctttga 7500
agacgtggtt ggaacgtctt ctttttccac gatgctcctc gtgggtgggg gtccatcttt 7560
gggaccactg tcggcagagg catcttgaac gatagccttt cctttatcgc aatgatggca 7620
tttgtaggtg ccaccttcct tttctactgt ccttttgatg aagtgacaga tagctgggca 7680
atggaatccg aggaggtttc ccgatattac cctttgttga aaagtctcaa tagccctttg 7740
gtcttctgag actgtatctt tgatattctt ggagtagacg agagtgtcgt gctccaccat 7800
gttggcaagc tgctctagcc aatacgcaaa ccgcctctcc ccgcgcgttg gccgattcat 7860
taatgcagct ggcacgacag gtttcccgac tggaaagcgg gcagtgagcg caacgcaatt 7920
aatgtgagtt agctcactca ttaggcaccc caggctttac actttatgct tccggctcgt 7980
atgttgtgtg gaattgtgag cggataacaa tttcacacag gaaacagcta tgaccatgat 8040
tacgaattcg agctcggtac ccggggatcc tctagagtcg acctgcaggc atgcaagctt 8100
ggcactggcc gtcgttttac aacgtcgtga ctgggaaaac cctggcgtta cccaacttaa 8160
tcgccttgca gcacatcccc ctttcgccag ctggcgtaat agcgaagagg cccgcaccga 8220
tcgcccttcc caacagttgc gcagcctgaa tggcgaatgc tagagcagct tgagcttgga 8280
tcagattgtc gtttcccgcc ttcagtttaa actatcagtg tttgacagga tatattggcg 8340
ggtaaaccta agagaaaaga gcgtttatta gaataacgga tatttaaaag ggcgtgaaaa 8400
ggtttatccg ttcgtccatt tgtatgtg 8428

<210> 3
<211> 10549
<212> DNA
<213> Artificial Sequence

<220>
<223> pCambia1302 plasmid

<300>
<308> Genbank #AF234298
<309> 2000-04-24

<400> 3
catggtagat ctgactagta aaggagaaga acttttcact ggagttgtcc caattcttgt 60
tgaattagat ggtgatgtta atgggcacaa attttctgtc agtggagagg gtgaaggtga 120
tgcaacatac ggaaaactta cccttaaatt tatttgcact actggaaaac tacctgttcc 180
gtggccaaca cttgtcacta ctttctctta tggtgttcaa tgcttttcaa gatacccaga 240
tcatatgaag cggcacgact tcttcaagag cgccatgcct gagggatacg tgcaggagag 300
gaccatcttc ttcaaggacg acgggaacta caagacacgt gctgaagtca agtttgaggg 360
agacaccctc gtcaacagga tcgagcttaa gggaatcgat ttcaaggagg acggaaacat 420
cctcggccac aagttggaat acaactacaa ctcccacaac gtatacatca tggccgacaa 480
gcaaaagaac ggcatcaaag ccaacttcaa gacccgccac aacatcgaag acggcggcgt 540
gcaactcgct gatcattatc aacaaaatac tccaattggc gatggccctg tccttttacc 600
agacaaccat tacctgtcca cacaatctgc cctttcgaaa gatcccaacg aaaagagaga 660
ccacatggtc cttcttgagt ttgtaacagc tgctgggatt acacatggca tggatgaact 720
atacaaagct agccaccacc accaccacca cgtgtgaatt ggtgaccagc tcgaatttcc 780
ccgatcgttc aaacatttgg caataaagtt tcttaagatt gaatcctgtt gccggtcttg 840
cgatgattat catataattt ctgttgaatt acgttaagca tgtaataatt aacatgtaat 900
gcatgacgtt atttatgaga tgggttttta tgattagagt cccgcaatta tacatttaat 960
acgcgataga aaacaaaata tagcgcgcaa actaggataa attatcgcgc gcggtgtcat 1020
ctatgttact agatcgggaa ttaaactatc agtgtttgac aggatatatt ggcgggtaaa 1080
cctaagagaa aagagcgttt attagaataa cggatattta aaagggcgtg aaaaggttta 1140
tccgttcgtc catttgtatg tgcatgccaa ccacagggtt cccctcggga tcaaagtact 1200
ttgatccaac ccctccgctg ctatagtgca gtcggcttct gacgttcagt gcagccgtct 1260
tctgaaaacg acatgtcgca caagtcctaa gttacgcgac aggctgccgc cctgcccttt 1320
tcctggcgtt ttcttgtcgc gtgttttagt cgcataaagt agaatacttg cgactagaac 1380
cggagacatt acgccatgaa caagagcgcc gccgctggcc tgctgggcta tgcccgcgtc 1440
agcaccgacg accaggactt gaccaaccaa cgggccgaac tgcacgcggc cggctgcacc 1500
aagctgtttt ccgagaagat caccggcacc aggcgcgacc gcccggagct ggccaggatg 1560
cttgaccacc tacgccctgg cgacgttgtg acagtgacca ggctagaccg cctggcccgc 1620
agcacccgcg acctactgga cattgccgag cgcatccagg aggccggcgc gggcctgcgt 1680
agcctggcag agccgtgggc cgacaccacc acgccggccg gccgcatggt gttgaccgtg 1740
ttcgccggca ttgccgagtt cgagcgttcc ctaatcatcg accgcacccg gagcgggcgc 1800
gaggccgcca aggcccgagg cgtgaagttt ggcccccgcc ctaccctcac cccggcacag 1860
atcgcgcacg cccgcgagct gatcgaccag gaaggccgca ccgtgaaaga ggcggctgca 1920
ctgcttggcg tgcatcgctc gaccctgtac cgcgcacttg agcgcagcga ggaagtgacg 1980
cccaccgagg ccaggcggcg cggtgccttc cgtgaggacg cattgaccga ggccgacgcc 2040
ctggcggccg ccgagaatga acgccaagag gaacaagcat gaaaccgcac caggacggcc 2100
aggacgaacc gtttttcatt accgaagaga tcgaggcgga gatgatcgcg gccgggtacg 2160
tgttcgagcc gcccgcgcac gtctcaaccg tgcggctgca tgaaatcctg gccggtttgt 2220
ctgatgccaa gctggcggcc tggccggcca gcttggccgc tgaagaaacc gagcgccgcc 2280
gtctaaaaag gtgatgtgta tttgagtaaa acagcttgcg tcatgcggtc gctgcgtata 2340
tgatgcgatg agtaaataaa caaatacgca aggggaacgc atgaaggtta tcgctgtact 2400
taaccagaaa ggcgggtcag gcaagacgac catcgcaacc catctagccc gcgccctgca 2460
actcgccggg gccgatgttc tgttagtcga ttccgatccc cagggcagtg cccgcgattg 2520
ggcggccgtg cgggaagatc aaccgctaac cgttgtcggc atcgaccgcc cgacgattga 2580
ccgcgacgtg aaggccatcg gccggcgcga cttcgtagtg atcgacggag cgccccaggc 2640
ggcggacttg gctgtgtccg cgatcaaggc agccgacttc gtgctgattc cggtgcagcc 2700
aagcccttac gacatatggg ccaccgccga cctggtggag ctggttaagc agcgcattga 2760
ggtcacggat ggaaggctac aagcggcctt tgtcgtgtcg cgggcgatca aaggcacgcg 2820

catcggcggt gaggttgccg aggcgctggc cgggtacgag ctgcccattc ttgagtcccg 2880
tatcacgcag cgcgtgagct acccaggcac tgccgccgcc ggcacaaccg ttcttgaatc 2940
agaacccgag ggcgacgctg cccgcgaggt ccaggcgctg gccgctgaaa ttaaatcaaa 3000
actcatttga gttaatgagg taaagagaaa atgagcaaaa gcacaaacac gctaagtgcc 3060
ggccgtccga gcgcacgcag cagcaaggct gcaacgttgg ccagcctggc agacacgcca 3120
gccatgaagc gggtcaactt tcagttgccg gcggaggatc acaccaagct gaagatgtac 3180
gcggtacgcc aaggcaagac cattaccgag ctgctatctg aatacatcgc gcagctacca 3240
gagtaaatga gcaaatgaat aaatgagtag atgaatttta gcggctaaag gaggcggcat 3300
ggaaaatcaa gaacaaccag gcaccgacgc cgtggaatgc cccatgtgtg gaggaacggg 3360
cggttggcca ggcgtaagcg gctgggttgt ctgccggccc tgcaatggca ctggaacccc 3420
caagcccgag gaatcggcgt gacggtcgca aaccatccgg cccggtacaa atcggcgcgg 3480
cgctgggtga tgacctggtg gagaagttga aggccgcgca ggccgcccag cggcaacgca 3540
tcgaggcaga agcacgcccc ggtgaatcgt ggcaagcggc cgctgatcga atccgcaaag 3600
aatcccggca accgccggca gccggtgcgc cgtcgattag gaagccgccc aagggcgacg 3660
agcaaccaga ttttttcgtt ccgatgctct atgacgtggg cacccgcgat agtcgcagca 3720
tcatggacgt ggccgttttc cgtctgtcga agcgtgaccg acgagctggc gaggtgatcc 3780
gctacgagct tccagacggg cacgtagagg tttccgcagg gccggccggc atggccagtg 3840
tgtgggatta cgacctggta ctgatggcgg tttcccatct aaccgaatcc atgaaccgat 3900
accgggaagg gaagggagac aagcccggcc gcgtgttccg tccacacgtt gcggacgtac 3960
tcaagttctg ccggcgagcc gatggcggaa agcagaaaga cgacctggta gaaacctgca 4020
ttcggttaaa caccacgcac gttgccatgc agcgtacgaa gaaggccaag aacggccgcc 4080
tggtgacggt atccgagggt gaagccttga ttagccgcta caagatcgta aagagcgaaa 4140
ccgggcggcc ggagtacatc gagatcgagc tagctgattg gatgtaccgc gagatcacag 4200
aaggcaagaa cccggacgtg ctgacggttc accccgatta ctttttgatc gatcccggca 4260
tcggccgttt tctctaccgc ctggcacgcc gcgccgcagg caaggcagaa gccagatggt 4320
tgttcaagac gatctacgaa cgcagtggca gcgccggaga gttcaagaag ttctgtttca 4380
ccgtgcgcaa gctgatcggg tcaaatgacc tgccggagta cgatttgaag gaggaggcgg 4440
ggcaggctgg cccgatccta gtcatgcgct accgcaacct gatcgagggc gaagcatccg 4500
ccggttccta atgtacggag cagatgctag ggcaaattgc cctagcaggg gaaaaaggtc 4560
gaaaaggtct ctttcctgtg gatagcacgt acattgggaa cccaaagccg tacattggga 4620
accggaaccc gtacattggg aacccaaagc cgtacattgg gaaccggtca cacatgtaag 4680
tgactgatat aaaagagaaa aaaggcgatt tttccgccta aaactcttta aaacttatta 4740
aaactcttaa aacccgcctg gcctgtgcat aactgtctgg ccagcgcaca gccgaagagc 4800
tgcaaaaagc gcctaccctt cggtcgctgc gctccctacg ccccgccgct tcgcgtcggc 4860
ctatcgcggc cgctggccgc tcaaaaatgg ctggcctacg gccaggcaat ctaccagggc 4920
gcggacaagc cgcgccgtcg ccactcgacc gccggcgccc acatcaaggc accctgcctc 4980
gcgcgtttcg gtgatgacgg tgaaaacctc tgacacatgc agctcccgga gacggtcaca 5040
gcttgtctgt aagcggatgc cgggagcaga caagcccgtc agggcgcgtc agcgggtgtt 5100
ggcgggtgtc ggggcgcagc catgacccag tcacgtagcg atagcggagt gtatactggc 5160
ttaactatgc ggcatcagag cagattgtac tgagagtgca ccatatgcgg tgtgaaatac 5220
cgcacagatg cgtaaggaga aaataccgca tcaggcgctc ttccgcttcc tcgctcactg 5280
actcgctgcg ctcggtcgtt cggctgcggc gagcggtatc agctcactca aaggcggtaa 5340
tacggttatc cacagaatca ggggataacg caggaaagaa catgtgagca aaaggccagc 5400
aaaaggccag gaaccgtaaa aaggccgcgt tgctggcgtt tttccatagg ctccgccccc 5460
ctgacgagca tcacaaaaat cgacgctcaa gtcagaggtg gcgaaacccg acaggactat 5520
aaagatacca ggcgtttccc cctggaagct ccctcgtgcg ctctcctgtt ccgaccctgc 5580
cgcttaccgg atacctgtcc gcctttctcc cttcgggaag cgtggcgctt tctcatagct 5640
cacgctgtag gtatctcagt tcggtgtagg tcgttcgctc caagctgggc tgtgtgcacg 5700
aaccccccgt tcagcccgac cgctgcgcct tatccggtaa ctatcgtctt gagtccaacc 5760
cggtaagaca cgacttatcg ccactggcag cagccactgg taacaggatt agcagagcga 5820
ggtatgtagg cggtgctaca gagttcttga agtggtggcc taactacggc tacactagaa 5880
ggacagtatt tggtatctgc gctctgctga agccagttac cttcggaaaa agagttggta 5940
gctcttgatc cggcaaacaa accaccgctg gtagcggtgg tttttttgtt tgcaagcagc 6000
agattacgcg cagaaaaaaa ggatctcaag aagatccttt gatcttttct acggggtctg 6060
acgctcagtg gaacgaaaac tcacgttaag ggattttggt catgcattct aggtactaaa 6120
acaattcatc cagtaaaata taatatttta ttttctccca atcaggcttg atccccagta 6180
agtcaaaaaa tagctcgaca tactgttctt ccccgatatc ctccctgatc gaccggacgc 6240
agaaggcaat gtcataccac ttgtccgccc tgccgcttct cccaagatca ataaagccac 6300
ttactttgcc atctttcaca aagatgttgc tgtctcccag gtcgccgtgg gaaaagacaa 6360
gttcctcttc gggcttttcc gtctttaaaa aatcatacag ctcgcgcgga tctttaaatg 6420
gagtgtcttc ttcccagttt tcgcaatcca catcggccag atcgttattc agtaagtaat 6480

ccaattcggc taagcggctg tctaagctat tcgtataggg acaatccgat atgtcgatgg 6540
agtgaaagag cctgatgcac tccgcataca gctcgataat cttttcaggg ctttgttcat 6600
cttcatactc ttccgagcaa aggacgccat cggcctcact catgagcaga ttgctccagc 6660
catcatgccg ttcaaagtgc aggacctttg gaacaggcag ctttccttcc agccatagca 6720
tcatgtcctt ttcccgttcc acatcatagg tggtcccttt ataccggctg tccgtcattt 6780
ttaaatatag gttttcattt tctcccacca gcttatatac cttagcagga gacattcctt 6840
ccgtatcttt tacgcagcgg tatttttcga tcagtttttt caattccggt gatattctca 6900
ttttagccat ttattatttc cttcctcttt tctacagtat ttaaagatac cccaagaagc 6960
taattataac aagacgaact ccaattcact gttccttgca ttctaaaacc ttaaatacca 7020
gaaaacagct ttttcaaagt tgttttcaaa gttggcgtat aacatagtat cgacggagcc 7080
gattttgaaa ccgcggtgat cacaggcagc aacgctctgt catcgttaca atcaacatgc 7140
taccctccgc gagatcatcc gtgtttcaaa cccggcagct tagttgccgt tcttccgaat 7200
agcatcggta acatgagcaa agtctgccgc cttacaacgg ctctcccgct gacgccgtcc 7260
cggactgatg ggctgcctgt atcgagtggt gattttgtgc cgagctgccg gtcggggagc 7320
tgttggctgg ctggtggcag gatatattgt ggtgtaaaca aattgacgct tagacaactt 7380
aataacacat tgcggacgtt tttaatgtac tgaattaacg ccgaattaat tcgggggatc 7440
tggattttag tactggattt tggttttagg aattagaaat tttattgata gaagtatttt 7500
acaaatacaa atacatacta agggtttctt atatgctcaa cacatgagcg aaaccctata 7560
ggaaccctaa ttcccttatc tgggaactac tcacacatta ttatggagaa actcgagctt 7620
gtcgatcgac agatccggtc ggcatctact ctatttcttt gccctcggac gagtgctggg 7680
gcgtcggttt ccactatcgg cgagtacttc tacacagcca tcggtccaga cggccgcgct 7740
tctgcgggcg atttgtgtac gcccgacagt cccggctccg gatcggacga ttgcgtcgca 7800
tcgaccctgc gcccaagctg catcatcgaa attgccgtca accaagctct gatagagttg 7860
gtcaagacca atgcggagca tatacgcccg gagtcgtggc gatcctgcaa gctccggatg 7920
cctccgctcg aagtagcgcg tctgctgctc catacaagcc aaccacggcc tccagaagaa 7980
gatgttggcg acctcgtatt gggaatcccc gaacatcgcc tcgctccagt caatgaccgc 8040
tgttatgcgg ccattgtccg tcaggacatt gttggagccg aaatccgcgt gcacgaggtg 8100
ccggacttcg gggcagtcct cggcccaaag catcagctca tcgagagcct gcgcgacgga 8160
cgcactgacg gtgtcgtcca tcacagtttg ccagtgatac acatggggat cagcaatcgc 8220
gcatatgaaa tcacgccatg tagtgtattg accgattcct tgcggtccga atgggccgaa 8280
cccgctcgtc tggctaagat cggccgcagc gatcgcatcc atagcctccg cgaccggttg 8340
tagaacagcg ggcagttcgg tttcaggcag gtcttgcaac gtgacaccct gtgcacggcg 8400
ggagatgcaa taggtcaggc tctcgctaaa ctccccaatg tcaagcactt ccggaatcgg 8460
gagcgcggcc gatgcaaagt gccgataaac ataacgatct ttgtagaaac catcggcgca 8520
gctatttacc cgcaggacat atccacgccc tcctacatcg aagctgaaag cacgagattc 8580
ttcgccctcc gagagctgca tcaggtcgga gacgctgtcg aacttttcga tcagaaactt 8640
ctcgacagac gtcgcggtga gttcaggctt tttcatatct cattgccccc cgggatctgc 8700
gaaagctcga gagagataga tttgtagaga gagactggtg atttcagcgt gtcctctcca 8760
aatgaaatga acttccttat atagaggaag gtcttgcgaa ggatagtggg attgtgcgtc 8820
atcccttacg tcagtggaga tatcacatca atccacttgc tttgaagacg tggttggaac 8880
gtcttctttt tccacgatgc tcctcgtggg tgggggtcca tctttgggac cactgtcggc 8940
agaggcatct tgaacgatag cctttccttt atcgcaatga tggcatttgt aggtgccacc 9000
ttccttttct actgtccttt tgatgaagtg acagatagct gggcaatgga atccgaggag 9060
gtttcccgat attacccttt gttgaaaagt ctcaatagcc ctttggtctt ctgagactgt 9120
atctttgata ttcttggagt agacgagagt gtcgtgctcc accatgttat cacatcaatc 9180
cacttgcttt gaagacgtgg ttggaacgtc ttctttttcc acgatgctcc tcgtgggtgg 9240
gggtccatct ttgggaccac tgtcggcaga ggcatcttga acgatagcct ttcctttatc 9300
gcaatgatgg catttgtagg tgccaccttc cttttctact gtccttttga tgaagtgaca 9360
gatagctggg caatggaatc cgaggaggtt tcccgatatt accctttgtt gaaaagtctc 9420
aatagccctt tggtcttctg agactgtatc tttgatattc ttggagtaga cgagagtgtc 9480
gtgctccacc atgttggcaa gctgctctag ccaatacgca aaccgcctct ccccgcgcgt 9540
tggccgattc attaatgcag ctggcacgac aggtttcccg actggaaagc gggcagtgag 9600
cgcaacgcaa ttaatgtgag ttagctcact cattaggcac cccaggcttt acactttatg 9660
cttccggctc gtatgttgtg tggaattgtg agcggataac aatttcacac aggaaacagc 9720
tatgaccatg attacgaatt cgagctcggt acccggggat cctctagagt cgacctgcag 9780
gcatgcaagc ttggcactgg ccgtcgtttt acaacgtcgt gactgggaaa accctggcgt 9840
tacccaactt aatcgccttg cagcacatcc ccctttcgcc agctggcgta atagcgaaga 9900
ggcccgcacc gatcgccctt cccaacagtt gcgcagcctg aatggcgaat gctagagcag 9960
cttgagcttg gatcagattg tcgtttcccg ccttcagttt agcttcatgg agtcaaagat 10020
tcaaatagag gacctaacag aactcgccgt aaagactggc gaacagttca tacagagtct 10080
cttacgactc aatgacaaga agaaaatctt cgtcaacatg gtggagcacg acacacttgt 10140

ctactccaaa aatatcaaag atacagtctc agaagaccaa agggcaattg agacttttca 10200
acaaagggta atatccggaa acctcctcgg attccattgc ccagctatct gtcactttat 10260
tgtgaagata gtggaaaagg aaggtggctc ctacaaatgc catcattgcg ataaaggaaa 10320
ggccatcgtt gaagatgcct ctgccgacag tggtcccaaa gatggacccc cacccacgag 10380
gagcatcgtg gaaaaagaag acgttccaac cacgtcttca aagcaagtgg attgatgtga 10440
tatctccact gacgtaaggg atgacgcaca atcccactat ccttcgcaag acccttcctc 10500
tatataagga agttcatttc atttggagag aacacggggg actcttgac 10549

<210> 4
<211> 33
<212> DNA
<213> Artificial Sequence

<220>
<223> CaMV35SpolyA Primer

<400> 4
ctgaattaac gccgaattaa ttcgggggat ctg 33

<210> 5
<211> 29
<212> DNA
<213> Artificial Sequence

<220>
<223> CaMV35Spr Primer

<400> 5
ctagagcagc ttgccaacat ggtggagca 29

<210> 6
<211> 12592
<212> DNA
<213> Artificial Sequence

<220>
<223> pAg2 Plasmid

<400> 6
gtacgaagaa ggccaagaac ggccgcctgg tgacggtatc cgagggtgaa gccttgatta 60
gccgctacaa gatcgtaaag agcgaaaccg ggcggccgga gtacatcgag atcgagctag 120
ctgattggat gtaccgcgag atcacagaag gcaagaaccc ggacgtgctg acggttcacc 180
ccgattactt tttgatcgat cccggcatcg gccgttttct ctaccgcctg gcacgccgcg 240
ccgcaggcaa ggcagaagcc agatggttgt tcaagacgat ctacgaacgc agtggcagcg 300
ccggagagtt caagaagttc tgtttcaccg tgcgcaagct gatcgggtca aatgacctgc 360
cggagtacga tttgaaggag gaggcggggc aggctggccc gatcctagtc atgcgctacc 420
gcaacctgat cgagggcgaa gcatccgccg gttcctaatg tacggagcag atgctagggc 480
aaattgccct agcaggggaa aaaggtcgaa aaggtctctt tcctgtggat agcacgtaca 540
ttgggaaccc aaagccgtac attgggaacc ggaacccgta cattgggaac ccaaagccgt 600
acattgggaa ccggtcacac atgtaagtga ctgatataaa agagaaaaaa ggcgattttt 660
ccgcctaaaa ctctttaaaa cttattaaaa ctcttaaaac ccgcctggcc tgtgcataac 720
tgtctggcca gcgcacagcc gaagagctgc aaaaagcgcc tacccttcgg tcgctgcgct 780
ccctacgccc cgccgcttcg cgtcggccta tcgcggccgc tggccgctca aaaatggctg 840
gcctacggcc aggcaatcta ccagggcgcg gacaagccgc gccgtcgcca ctcgaccgcc 900
ggcgcccaca tcaaggcacc ctgcctcgcg cgtttcggtg atgacggtga aaacctctga 960
cacatgcagc tcccggagac ggtcacagct tgtctgtaag cggatgccgg gagcagacaa 1020
gcccgtcagg gcgcgtcagc gggtgttggc gggtgtcggg gcgcagccat gacccagtca 1080
cgtagcgata gcggagtgta tactggctta actatgcggc atcagagcag attgtactga 1140
gagtgcacca tatgcggtgt gaaataccgc acagatgcgt aaggagaaaa taccgcatca 1200
ggcgctcttc cgcttcctcg ctcactgact cgctgcgctc ggtcgttcgg ctgcggcgag 1260
cggtatcagc tcactcaaag gcggtaatac ggttatccac agaatcaggg gataacgcag 1320

gaaagaacat gtgagcaaaa ggccagcaaa aggccaggaa ccgtaaaaag gccgcgttgc 1380
tggcgttttt ccataggctc cgcccccctg acgagcatca caaaaatcga cgctcaagtc 1440
agaggtggcg aaacccgaca ggactataaa gataccaggc gtttccccct ggaagctccc 1500
tcgtgcgctc tcctgttccg accctgccgc ttaccggata cctgtccgcc tttctccctt 1560
cgggaagcgt ggcgctttct catagctcac gctgtaggta tctcagttcg gtgtaggtcg 1620
ttcgctccaa gctgggctgt gtgcacgaac cccccgttca gcccgaccgc tgcgccttat 1680
ccggtaacta tcgtcttgag tccaacccgg taagacacga cttatcgcca ctggcagcag 1740
ccactggtaa caggattagc agagcgaggt atgtaggcgg tgctacagag ttcttgaagt 1800
ggtggcctaa ctacggctac actagaagga cagtatttgg tatctgcgct ctgctgaagc 1860
cagttacctt cggaaaaaga gttggtagct cttgatccgg caaacaaacc accgctggta 1920
gcggtggttt ttttgtttgc aagcagcaga ttacgcgcag aaaaaaagga tctcaagaag 1980
atcctttgat cttttctacg gggtctgacg ctcagtggaa cgaaaactca cgttaaggga 2040
ttttggtcat gcattctagg tactaaaaca attcatccag taaaatataa tattttattt 2100
tctcccaatc aggcttgatc cccagtaagt caaaaaatag ctcgacatac tgttcttccc 2160
cgatatcctc cctgatcgac cggacgcaga aggcaatgtc ataccacttg tccgccctgc 2220
cgcttctccc aagatcaata aagccactta ctttgccatc tttcacaaag atgttgctgt 2280
ctcccaggtc gccgtgggaa aagacaagtt cctcttcggg cttttccgtc tttaaaaaat 2340
catacagctc gcgcggatct ttaaatggag tgtcttcttc ccagttttcg caatccacat 2400
cggccagatc gttattcagt aagtaatcca attcggctaa gcggctgtct aagctattcg 2460
tatagggaca atccgatatg tcgatggagt gaaagagcct gatgcactcc gcatacagct 2520
cgataatctt ttcagggctt tgttcatctt catactcttc cgagcaaagg acgccatcgg 2580
cctcactcat gagcagattg ctccagccat catgccgttc aaagtgcagg acctttggaa 2640
caggcagctt tccttccagc catagcatca tgtccttttc ccgttccaca tcataggtgg 2700
tccctttata ccggctgtcc gtcattttta aatataggtt ttcattttct cccaccagct 2760
tatatacctt agcaggagac attccttccg tatcttttac gcagcggtat ttttcgatca 2820
gttttttcaa ttccggtgat attctcattt tagccattta ttatttcctt cctcttttct 2880
acagtattta aagatacccc aagaagctaa ttataacaag acgaactcca attcactgtt 2940
ccttgcattc taaaacctta aataccagaa aacagctttt tcaaagttgt tttcaaagtt 3000
ggcgtataac atagtatcga cggagccgat tttgaaaccg cggtgatcac aggcagcaac 3060
gctctgtcat cgttacaatc aacatgctac cctccgcgag atcatccgtg tttcaaaccc 3120
ggcagcttag ttgccgttct tccgaatagc atcggtaaca tgagcaaagt ctgccgcctt 3180
acaacggctc tcccgctgac gccgtcccgg actgatgggc tgcctgtatc gagtggtgat 3240
tttgtgccga gctgccggtc ggggagctgt tggctggctg gtggcaggat atattgtggt 3300
gtaaacaaat tgacgcttag acaacttaat aacacattgc ggacgttttt aatgtactga 3360
attaacgccg aattaattcg ggggatctgg attttagtac tggattttgg ttttaggaat 3420
tagaaatttt attgatagaa gtattttaca aatacaaata catactaagg gtttcttata 3480
tgctcaacac atgagcgaaa ccctatagga accctaattc ccttatctgg gaactactca 3540
cacattatta tggagaaact cgagtcaaat ctcggtgacg ggcaggaccg gacggggcgg 3600
taccggcagg ctgaagtcca gctgccagaa acccacgtca tgccagttcc cgtgcttgaa 3660
gccggccgcc cgcagcatgc cgcggggggc atatccgagc gcctcgtgca tgcgcacgct 3720
cgggtcgttg ggcagcccga tgacagcgac cacgctcttg aagccctgtg cctccaggga 3780
cttcagcagg tgggtgtaga gcgtggagcc cagtcccgtc cgctggtggc ggggggagac 3840
gtacacggtc gactcggccg tccagtcgta ggcgttgcgt gccttccagg ggcccgcgta 3900
ggcgatgccg gcgacctcgc cgtccacctc ggcgacgagc cagggatagc gctcccgcag 3960
acggacgagg tcgtccgtcc actcctgcgg ttcctgcggc tcggtacgga agttgaccgt 4020
gcttgtctcg atgtagtggt tgacgatggt gcagaccgcc ggcatgtccg cctcggtggc 4080
acggcggatg tcggccgggc gtcgttctgg gctcatggta gactcgagag agatagattt 4140
gtagagagag actggtgatt tcagcgtgtc ctctccaaat gaaatgaact tccttatata 4200
gaggaaggtc ttgcgaagga tagtgggatt gtgcgtcatc ccttacgtca gtggagatat 4260
cacatcaatc cacttgcttt gaagacgtgg ttggaacgtc ttctttttcc acgatgctcc 4320
tcgtgggtgg gggtccatct ttgggaccac tgtcggcaga ggcatcttga acgatagcct 4380
ttcctttatc gcaatgatgg catttgtagg tgccaccttc cttttctact gtccttttga 4440
tgaagtgaca gatagctggg caatggaatc cgaggaggtt tcccgatatt accctttgtt 4500
gaaaagtctc aatagccctt tggtcttctg agactgtatc tttgatattc ttggagtaga 4560
cgagagtgtc gtgctccacc atgttatcac atcaatccac ttgctttgaa gacgtggttg 4620
gaacgtcttc tttttccacg atgctcctcg tgggtggggg tccatctttg ggaccactgt 4680
cggcagaggc atcttgaacg atagcctttc ctttatcgca atgatggcat ttgtaggtgc 4740
caccttcctt ttctactgtc cttttgatga agtgacagat agctgggcaa tggaatccga 4800
ggaggtttcc cgatattacc ctttgttgaa aagtctcaat agccctttgg tcttctgaga 4860
ctgtatcttt gatattcttg gagtagacga gagtgtcgtg ctccaccatg ttggcaagct 4920
gctctagcca atacgcaaac cgcctctccc cgcgcgttgg ccgattcatt aatgcagctg 4980

gcacgacagg tttcccgact ggaaagcggg cagtgagcgc aacgcaatta atgtgagtta 5040
gctcactcat taggcacccc aggctttaca ctttatgctt ccggctcgta tgttgtgtgg 5100
aattgtgagc ggataacaat ttcacacagg aaacagctat gaccatgatt acgaattcga 5160
gccttgacta gagggtcgac ggtatacaga catgataaga tacattgatg agtttggaca 5220
aaccacaact agaatgcagt gaaaaaaatg ctttatttgt gaaatttgtg atgctattgc 5280
tttatttgta accattataa gctgcaataa acaagttggg gtgggcgaag aactccagca 5340
tgagatcccc gcgctggagg atcatccagc cggcgtcccg gaaaacgatt ccgaagccca 5400
acctttcata gaaggcggcg gtggaatcga aatctcgtag cacgtgtcag tcctgctcct 5460
cggccacgaa gtgcacgcag ttgccggccg ggtcgcgcag ggcgaactcc cgcccccacg 5520
gctgctcgcc gatctcggtc atggccggcc cggaggcgtc ccggaagttc gtggacacga 5580
cctccgacca ctcggcgtac agctcgtcca ggccgcgcac ccacacccag gccagggtgt 5640
tgtccggcac cacctggtcc tggaccgcgc tgatgaacag ggtcacgtcg tcccggacca 5700
caccggcgaa gtcgtcctcc acgaagtccc gggagaaccc gagccggtcg gtccagaact 5760
cgaccgctcc ggcgacgtcg cgcgcggtga gcaccggaac ggcactggtc aacttggcca 5820
tggatccaga tttcgctcaa gttagtataa aaaagcaggc ttcaatcctg caggaattcg 5880
atcgacactc tcgtctactc caagaatatc aaagatacag tctcagaaga ccaaagggct 5940
attgagactt ttcaacaaag ggtaatatcg ggaaacctcc tcggattcca ttgcccagct 6000
atctgtcact tcatcaaaag gacagtagaa aaggaaggtg gcacctacaa atgccatcat 6060
tgcgataaag gaaaggctat cgttcaagat gcctctgccg acagtggtcc caaagatgga 6120
cccccaccca cgaggagcat cgtggaaaaa gaagacgttc caaccacgtc ttcaaagcaa 6180
gtggattgat gtgataacat ggtggagcac gacactctcg tctactccaa gaatatcaaa 6240
gatacagtct cagaagacca aagggctatt gagacttttc aacaaagggt aatatcggga 6300
aacctcctcg gattccattg cccagctatc tgtcacttca tcaaaaggac agtagaaaag 6360
gaaggtggca cctacaaatg ccatcattgc gataaaggaa aggctatcgt tcaagatgcc 6420
tctgccgaca gtggtcccaa agatggaccc ccacccacga ggagcatcgt ggaaaaagaa 6480
gacgttccaa ccacgtcttc aaagcaagtg gattgatgtg atatctccac tgacgtaagg 6540
gatgacgcac aatcccacta tccttcgcaa gaccttcctc tatataagga agttcatttc 6600
atttggagag gacacgctga aatcaccagt ctctctctac aaatctatct ctctcgagct 6660
ttcgcagatc cggggggcaa tgagatatga aaaagcctga actcaccgcg acgtctgtcg 6720
agaagtttct gatcgaaaag ttcgacagcg tctccgacct gatgcagctc tcggagggcg 6780
aagaatctcg tgctttcagc ttcgatgtag gagggcgtgg atatgtcctg cgggtaaata 6840
gctgcgccga tggtttctac aaagatcgtt atgtttatcg gcactttgca tcggccgcgc 6900
tcccgattcc ggaagtgctt gacattgggg agtttagcga gagcctgacc tattgcatct 6960
cccgccgtgc acagggtgtc acgttgcaag acctgcctga aaccgaactg cccgctgttc 7020
tacaaccggt cgcggaggct atggatgcga tcgctgcggc cgatcttagc cagacgagcg 7080
ggttcggccc attcggaccg caaggaatcg gtcaatacac tacatggcgt gatttcatat 7140
gcgcgattgc tgatccccat gtgtatcact ggcaaactgt gatggacgac accgtcagtg 7200
cgtccgtcgc gcaggctctc gatgagctga tgctttgggc cgaggactgc cccgaagtcc 7260
ggcacctcgt gcacgcggat ttcggctcca acaatgtcct gacggacaat ggccgcataa 7320
cagcggtcat tgactggagc gaggcgatgt tcggggattc ccaatacgag gtcgccaaca 7380
tcttcttctg gaggccgtgg ttggcttgta tggagcagca gacgcgctac ttcgagcgga 7440
ggcatccgga gcttgcagga tcgccacgac tccgggcgta tatgctccgc attggtcttg 7500
accaactcta tcagagcttg gttgacggca atttcgatga tgcagcttgg gcgcagggtc 7560
gatgcgacgc aatcgtccga tccggagccg ggactgtcgg gcgtacacaa atcgcccgca 7620
gaagcgcggc cgtctggacc gatggctgtg tagaagtact cgccgatagt ggaaaccgac 7680
gccccagcac tcgtccgagg gcaaagaaat agagtagatg ccgaccggat ctgtcgatcg 7740
acaagctcga gtttctccat aataatgtgt gagtagttcc cagataaggg aattagggtt 7800
cctatagggt ttcgctcatg tgttgagcat ataagaaacc cttagtatgt atttgtattt 7860
gtaaaatact tctatcaata aaatttctaa ttcctaaaac caaaatccag tactaaaatc 7920
cagatccccc gaattaattc ggcgttaatt cagatcaagc ttggcactgg ccgtcgtttt 7980
acaacgtcgt gactgggaaa accctggcgt tacccaactt aatcgccttg cagcacatcc 8040
ccctttcgcc agctggcgta atagcgaaga ggcccgcacc gatcgccctt cccaacagtt 8100
gcgcagcctg aatggcgaat gctagagcag cttgagcttg gatcagattg tcgtttcccg 8160
ccttcagttt ggggatcctc tagactgaag gcgggaaacg acaatctgat catgagcgga 8220
gaattaaggg agtcacgtta tgacccccgc cgatgacgcg ggacaagccg ttttacgttt 8280
ggaactgaca gaaccgcaac gttgaaggag ccactcagcc gcgggtttct ggagtttaat 8340
gagctaagca catacgtcag aaaccattat tgcgcgttca aaagtcgcct aaggtcacta 8400
tcagctagca aatatttctt gtcaaaaatg ctccactgac gttccataaa ttcccctcgg 8460
tatccaatta gagtctcata ttcactctca atccaaataa tctgcaccgg atctcgagaa 8520
tcgaattccc gcggccgcca tggtagatct gactagtaaa ggagaagaac ttttcactgg 8580
agttgtccca attcttgttg aattagatgg tgatgttaat gggcacaaat tttctgtcag 8640

gtggagaggg tgaaggtgat gcaacatacg gaaaacttac ccttaaattt atttgcacta 8700
ctggaaaact acctgttccg tggccaacac ttgtcactac tttctcttat ggtgttcaat 8760
gcttttcaag atacccagat catatgaagc ggcacgactt cttcaagagc gccatgcctg 8820
agggatacgt gcaggagagg accatcttct tcaaggacga cgggaactac aagacacgtg 8880
ctgaagtcaa gtttgaggga gacaccctcg tcaacaggat cgagcttaag ggaatcgatt 8940
tcaaggagga cggaaacatc ctcggccaca agttggaata caactacaac tcccacaacg 9000
tatacatcat ggccgacaag caaaagaacg gcatcaaagc caacttcaag acccgccaca 9060
acatcgaaga cggcggcgtg caactcgctg atcattatca acaaaatact ccaattggcg 9120
atggccctgt ccttttacca gacaaccatt acctgtccac acaatctgcc ctttcgaaag 9180
atcccaacga aaagagagac cacatggtcc ttcttgagtt tgtaacagct gctgggatta 9240
cacatggcat ggatgaacta tacaaagcta gccaccacca ccaccaccac gtgtgaattg 9300
gtgaccagct cgaatttccc cgatcgttca aacatttggc aataaagttt cttaagattg 9360
aatcctgttg ccggtcttgc gatgattatc atataatttc tgttgaatta cgttaagcat 9420
gtaataatta acatgtaatg catgacgtta tttatgagat gggtttttat gattagagtc 9480
ccgcaattat acatttaata cgcgatagaa aacaaaatat agcgcgcaaa ctaggataaa 9540
ttatcgcgcg cggtgtcatc tatgttacta gatcgggaat taaactatca gtgtttgaca 9600
ggatatattg gcgggtaaac ctaagagaaa agagcgttta ttagaataac ggatatttaa 9660
aagggcgtga aaaggtttat ccgttcgtcc atttgtatgt gcatgccaac cacagggttc 9720
ccctcgggat caaagtactt tgatccaacc cctccgctgc tatagtgcag tcggcttctg 9780
acgttcagtg cagccgtctt ctgaaaacga catgtcgcac aagtcctaag ttacgcgaca 9840
ggctgccgcc ctgccctttt cctggcgttt tcttgtcgcg tgttttagtc gcataaagta 9900
gaatacttgc gactagaacc ggagacatta cgccatgaac aagagcgccg ccgctggcct 9960
gctgggctat gcccgcgtca gcaccgacga ccaggacttg accaaccaac gggccgaact 10020
gcacgcggcc ggctgcacca agctgttttc cgagaagatc accggcacca ggcgcgaccg 10080
cccggagctg gccaggatgc ttgaccacct acgccctggc gacgttgtga cagtgaccag 10140
gctagaccgc ctggcccgca gcacccgcga cctactggac attgccgagc gcatccagga 10200
ggccggcgcg ggcctgcgta gcctggcaga gccgtgggcc gacaccacca cgccggccgg 10260
ccgcatggtg ttgaccgtgt tcgccggcat tgccgagttc gagcgttccc taatcatcga 10320
ccgcacccgg agcgggcgcg aggccgccaa ggcccgaggc gtgaagtttg gcccccgccc 10380
taccctcacc ccggcacaga tcgcgcacgc ccgcgagctg atcgaccagg aaggccgcac 10440
cgtgaaagag gcggctgcac tgcttggcgt gcatcgctcg accctgtacc gcgcacttga 10500
gcgcagcgag gaagtgacgc ccaccgaggc caggcggcgc ggtgccttcc gtgaggacgc 10560
attgaccgag gccgacgccc tggcggccgc cgagaatgaa cgccaagagg aacaagcatg 10620
aaaccgcacc aggacggcca ggacgaaccg tttttcatta ccgaagagat cgaggcggag 10680
atgatcgcgg ccgggtacgt gttcgagccg cccgcgcacg tctcaaccgt gcggctgcat 10740
gaaatcctgg ccggtttgtc tgatgccaag ctggcggcct ggccggccag cttggccgct 10800
gaagaaaccg agcgccgccg tctaaaaagg tgatgtgtat ttgagtaaaa cagcttgcgt 10860
catgcggtcg ctgcgtatat gatgcgatga gtaaataaac aaatacgcaa ggggaacgca 10920
tgaaggttat cgctgtactt aaccagaaag gcgggtcagg caagacgacc atcgcaaccc 10980
atctagcccg cgccctgcaa ctcgccgggg ccgatgttct gttagtcgat tccgatcccc 11040
agggcagtgc ccgcgattgg gcggccgtgc gggaagatca accgctaacc gttgtcggca 11100
tcgaccgccc gacgattgac cgcgacgtga aggccatcgg ccggcgcgac ttcgtagtga 11160
tcgacggagc gccccaggcg gcggacttgg ctgtgtccgc gatcaaggca gccgacttcg 11220
tgctgattcc ggtgcagcca agcccttacg acatatgggc caccgccgac ctggtggagc 11280
tggttaagca gcgcattgag gtcacggatg gaaggctaca agcggccttt gtcgtgtcgc 11340
gggcgatcaa aggcacgcgc atcggcggtg aggttgccga ggcgctggcc gggtacgagc 11400
tgcccattct tgagtcccgt atcacgcagc gcgtgagcta cccaggcact gccgccgccg 11460
gcacaaccgt tcttgaatca gaacccgagg gcgacgctgc ccgcgaggtc caggcgctgg 11520
ccgctgaaat taaatcaaaa ctcatttgag ttaatgaggt aaagagaaaa tgagcaaaag 11580
cacaaacacg ctaagtgccg gccgtccgag cgcacgcagc agcaaggctg caacgttggc 11640
cagcctggca gacacgccag ccatgaagcg ggtcaacttt cagttgccgg cggaggatca 11700
caccaagctg aagatgtacg cggtacgcca aggcaagacc attaccgagc tgctatctga 11760
atacatcgcg cagctaccag agtaaatgag caaatgaata aatgagtaga tgaattttag 11820
cggctaaagg aggcggcatg gaaaatcaag aacaaccagg caccgacgcc gtggaatgcc 11880
ccatgtgtgg aggaacgggc ggttggccag gcgtaagcgg ctgggttgtc tgccggccct 11940
gcaatggcac tggaaccccc aagcccgagg aatcggcgtg acggtcgcaa accatccggc 12000
ccggtacaaa tcggcgcggc gctgggtgat gacctggtgg agaagttgaa ggccgcgcag 12060
gccgcccagc ggcaacgcat cgaggcagaa gcacgccccg gtgaatcgtg gcaagcggcc 12120
gctgatcgaa tccgcaaaga atcccggcaa ccgccggcag ccggtgcgcc gtcgattagg 12180
aagccgccca agggcgacga gcaaccagat tttttcgttc cgatgctcta tgacgtgggc 12240
acccgcgata gtcgcagcat catggacgtg gccgttttcc gtctgtcgaa gcgtgaccga 12300

cgagctggcg aggtgatccg ctacgagctt ccagacgggc acgtagaggt ttccgcaggg 12360
ccggccggca tggccagtgt gtgggattac gacctggtac tgatggcggt ttcccatcta 12420
accgaatcca tgaaccgata ccgggaaggg aagggagaca agcccggccg cgtgttccgt 12480
ccacacgttg cggacgtact caagttctgc cggcgagccg atggcggaaa gcagaaagac 12540
gacctggtag aaacctgcat tcggttaaac accacgcacg ttgccatgca gc 12592

<210> 7
<211> 3357
<212> DNA
<213> Artificial Sequence

<220>
<223> pGEMEasyNOS Plasmid

<400> 7
tatcactagt gaattcgcgg ccgcctgcag gtcgaccata tgggagagct cccaacgcgt 60
tggatgcata gcttgagtat tctatagtgt cacctaaata gcttggcgta atcatggtca 120
tagctgtttc ctgtgtgaaa ttgttatccg ctcacaattc cacacaacat acgagccgga 180
agcataaagt gtaaagcctg gggtgcctaa tgagtgagct aactcacatt aattgcgttg 240
cgctcactgc ccgctttcca gtcgggaaac ctgtcgtgcc agctgcatta atgaatcggc 300
caacgcgcgg ggagaggcgg tttgcgtatt gggcgctctt ccgcttcctc gctcactgac 360
tcgctgcgct cggtcgttcg gctgcggcga gcggtatcag ctcactcaaa ggcggtaata 420
cggttatcca cagaatcagg ggataacgca ggaaagaaca tgtgagcaaa aggccagcaa 480
aaggccagga accgtaaaaa ggccgcgttg ctggcgtttt tccataggct ccgcccccct 540
gacgagcatc acaaaaatcg acgctcaagt cagaggtggc gaaacccgac aggactataa 600
agataccagg cgtttccccc tggaagctcc ctcgtgcgct ctcctgttcc gaccctgccg 660
cttaccggat acctgtccgc ctttctccct tcgggaagcg tggcgctttc tcatagctca 720
cgctgtaggt atctcagttc ggtgtaggtc gttcgctcca agctgggctg tgtgcacgaa 780
ccccccgttc agcccgaccg ctgcgcctta tccggtaact atcgtcttga gtccaacccg 840
gtaagacacg acttatcgcc actggcagca gccactggta acaggattag cagagcgagg 900
tatgtaggcg gtgctacaga gttcttgaag tggtggccta actacggcta cactagaaga 960
acagtatttg gtatctgcgc tctgctgaag ccagttacct tcggaaaaag agttggtagc 1020
tcttgatccg gcaaacaaac caccgctggt agcggtggtt tttttgtttg caagcagcag 1080
attacgcgca gaaaaaaagg atctcaagaa gatcctttga tcttttctac ggggtctgac 1140
gctcagtgga acgaaaactc acgttaaggg attttggtca tgagattatc aaaaaggatc 1200
ttcacctaga tccttttaaa ttaaaaatga agttttaaat caatctaaag tatatatgag 1260
taaacttggt ctgacagtta ccaatgctta atcagtgagg cacctatctc agcgatctgt 1320
ctatttcgtt catccatagt tgcctgactc cccgtcgtgt agataactac gatacgggag 1380
ggcttaccat ctggccccag tgctgcaatg ataccgcgag acccacgctc accggctcca 1440
gatttatcag caataaacca gccagccgga agggccgagc gcagaagtgg tcctgcaact 1500
ttatccgcct ccatccagtc tattaattgt tgccgggaag ctagagtaag tagttcgcca 1560
gttaatagtt tgcgcaacgt tgttgccatt gctacaggca tcgtggtgtc acgctcgtcg 1620
tttggtatgg cttcattcag ctccggttcc caacgatcaa ggcgagttac atgatccccc 1680
atgttgtgca aaaaagcggt tagctccttc ggtcctccga tcgttgtcag aagtaagttg 1740
gccgcagtgt tatcactcat ggttatggca gcactgcata attctcttac tgtcatgcca 1800
tccgtaagat gcttttctgt gactggtgag tactcaacca agtcattctg agaatagtgt 1860
atgcggcgac cgagttgctc ttgcccggcg tcaatacggg ataataccgc gccacatagc 1920
agaactttaa aagtgctcat cattggaaaa cgttcttcgg ggcgaaaact ctcaaggatc 1980
ttaccgctgt tgagatccag ttcgatgtaa cccactcgtg cacccaactg atcttcagca 2040
tcttttactt tcaccagcgt ttctgggtga gcaaaaacag gaaggcaaaa tgccgcaaaa 2100
aagggaataa gggcgacacg gaaatgttga atactcatac tcttcctttt tcaatattat 2160
tgaagcattt atcagggtta ttgtctcatg agcggataca tatttgaatg tatttagaaa 2220
aataaacaaa taggggttcc gcgcacattt ccccgaaaag tgccacctga tgcggtgtga 2280
aataccgcac agatgcgtaa ggagaaaata ccgcatcagg aaattgtaag cgttaatatt 2340
ttgttaaaat tcgcgttaaa tttttgttaa atcagctcat tttttaacca ataggccgaa 2400
atcggcaaaa tcccttataa atcaaaagaa tagaccgaga tagggttgag tgttgttcca 2460
gtttggaaca agagtccact attaaagaac gtggactcca acgtcaaagg gcgaaaaacc 2520
gtctatcagg gcgatggccc actacgtgaa ccatcaccct aatcaagttt tttggggtcg 2580
aggtgccgta aagcactaaa tcggaaccct aaagggagcc cccgatttag agcttgacgg 2640
ggaaagccgg cgaacgtggc gagaaaggaa gggaagaaag cgaaaggagc gggcgctagg 2700
gcgctggcaa gtgtagcggt cacgctgcgc gtaaccacca cacccgccgc gcttaatgcg 2760

ccgctacagg gcgcgtccat tcgccattca ggctgcgcaa ctgttgggaa gggcgatcgg 2820
tgcgggcctc ttcgctatta cgccagctgg cgaaaggggg atgtgctgca aggcgattaa 2880
gttgggtaac gccagggttt tcccagtcac gacgttgtaa aacgacggcc agtgaattgt 2940
aatacgactc actatagggc gaattgggcc cgacgtcgca tgctcccggc cgccatggcg 3000
gccgcgggaa ttcgattctc gagatccggt gcagattatt tggattgaga gtgaatatga 3060
gactctaatt ggataccgag gggaatttat ggaacgtcag tggagcattt ttgacaagaa 3120
atatttgcta gctgatagtg accttaggcg acttttgaac gcgcaataat ggtttctgac 3180
gtatgtgctt agctcattaa actccagaaa cccgcggctg agtggctcct tcaacgttgc 3240
ggttctgtca gttccaaacg taaaacggct tgtcccgcgt catcggcggg ggtcataacg 3300
tgactccctt aattctccgc tcatgatcag attgtcgttt cccgccttca gtctaga 3357

<210> 8
<211> 10122
<212> DNA
<213> Artificial Sequence

<220>
<223> p1302NOS Plasmid

<400> 8
catggtagat ctgactagta aaggagaaga acttttcact ggagttgtcc caattcttgt 60
tgaattagat ggtgatgtta atgggcacaa attttctgtc agtggagagg gtgaaggtga 120
tgcaacatac ggaaaactta cccttaaatt tatttgcact actggaaaac tacctgttcc 180
gtggccaaca cttgtcacta ctttctctta tggtgttcaa tgcttttcaa gatacccaga 240
tcatatgaag cggcacgact tcttcaagag cgccatgcct gagggatacg tgcaggagag 300
gaccatcttc ttcaaggacg acgggaacta caagacacgt gctgaagtca agtttgaggg 360
agacaccctc gtcaacagga tcgagcttaa gggaatcgat ttcaaggagg acggaaacat 420
cctcggccac aagttggaat acaactacaa ctcccacaac gtatacatca tggccgacaa 480
gcaaaagaac ggcatcaaag ccaacttcaa gacccgccac aacatcgaag acggcggcgt 540
gcaactcgct gatcattatc aacaaaatac tccaattggc gatggccctg tccttttacc 600
agacaaccat tacctgtcca cacaatctgc cctttcgaaa gatcccaacg aaaagagaga 660
ccacatggtc cttcttgagt ttgtaacagc tgctgggatt acacatggca tggatgaact 720
atacaaagct agccaccacc accaccacca cgtgtgaatt ggtgaccagc tcgaatttcc 780
ccgatcgttc aaacatttgg caataaagtt tcttaagatt gaatcctgtt gccggtcttg 840
cgatgattat catataattt ctgttgaatt acgttaagca tgtaataatt aacatgtaat 900
gcatgacgtt atttatgaga tgggttttta tgattagagt cccgcaatta tacatttaat 960
acgcgataga aaacaaaata tagcgcgcaa actaggataa attatcgcgc gcggtgtcat 1020
ctatgttact agatcgggaa ttaaactatc agtgtttgac aggatatatt ggcgggtaaa 1080
cctaagagaa aagagcgttt attagaataa cggatattta aaagggcgtg aaaaggttta 1140
tccgttcgtc catttgtatg tgcatgccaa ccacagggtt cccctcggga tcaaagtact 1200
ttgatccaac ccctccgctg ctatagtgca gtcggcttct gacgttcagt gcagccgtct 1260
tctgaaaacg acatgtcgca caagtcctaa gttacgcgac aggctgccgc cctgcccttt 1320
tcctggcgtt ttcttgtcgc gtgttttagt cgcataaagt agaatacttg cgactagaac 1380
cggagacatt acgccatgaa caagagcgcc gccgctggcc tgctgggcta tgcccgcgtc 1440
agcaccgacg accaggactt gaccaaccaa cgggccgaac tgcacgcggc cggctgcacc 1500
aagctgtttt ccgagaagat caccggcacc aggcgcgacc gcccggagct ggccaggatg 1560
cttgaccacc tacgccctgg cgacgttgtg acagtgacca ggctagaccg cctggcccgc 1620
agcacccgcg acctactgga cattgccgag cgcatccagg aggccggcgc gggcctgcgt 1680
agcctggcag agccgtgggc cgacaccacc acgccggccg gccgcatggt gttgaccgtg 1740
ttcgccggca ttgccgagtt cgagcgttcc ctaatcatcg accgcacccg gagcgggcgc 1800
gaggccgcca aggcccgagg cgtgaagttt ggcccccgcc ctaccctcac cccggcacag 1860
atcgcgcacg cccgcgagct gatcgaccag gaaggccgca ccgtgaaaga ggcggctgca 1920
ctgcttggcg tgcatcgctc gaccctgtac cgcgcacttg agcgcagcga ggaagtgacg 1980
cccaccgagg ccaggcggcg cggtgccttc cgtgaggacg cattgaccga ggccgacgcc 2040
ctggcggccg ccgagaatga acgccaagag gaacaagcat gaaaccgcac caggacggcc 2100
aggacgaacc gtttttcatt accgaagaga tcgaggcgga gatgatcgcg gccgggtacg 2160
tgttcgagcc gcccgcgcac gtctcaaccg tgcggctgca tgaaatcctg gccggtttgt 2220
ctgatgccaa gctggcggcc tggccggcca gcttggccgc tgaagaaacc gagcgccgcc 2280
gtctaaaaag gtgatgtgta tttgagtaaa acagcttgcg tcatgcggtc gctgcgtata 2340
tgatgcgatg agtaaataaa caaatacgca aggggaacgc atgaaggtta tcgctgtact 2400
taaccagaaa ggcgggtcag gcaagacgac catcgcaacc catctagccc gcgccctgca 2460

actcgccggg gccgatgttc tgttagtcga ttccgatccc cagggcagtg cccgcgattg 2520
ggcggccgtg cgggaagatc aaccgctaac cgttgtcggc atcgaccgcc cgacgattga 2580
ccgcgacgtg aaggccatcg gccggcgcga cttcgtagtg atcgacggag cgccccaggc 2640
ggcggacttg gctgtgtccg cgatcaaggc agccgacttc gtgctgattc cggtgcagcc 2700
aagcccttac gacatatggg ccaccgccga cctggtggag ctggttaagc agcgcattga 2760
ggtcacggat ggaaggctac aagcggcctt tgtcgtgtcg cgggcgatca aaggcacgcg 2820
catcggcggt gaggttgccg aggcgctggc cgggtacgag ctgcccattc ttgagtcccg 2880
tatcacgcag cgcgtgagct acccaggcac tgccgccgcc ggcacaaccg ttcttgaatc 2940
agaacccgag ggcgacgctg cccgcgaggt ccaggcgctg gccgctgaaa ttaaatcaaa 3000
actcatttga gttaatgagg taaagagaaa atgagcaaaa gcacaaacac gctaagtgcc 3060
ggccgtccga gcgcacgcag cagcaaggct gcaacgttgg ccagcctggc agacacgcca 3120
gccatgaagc gggtcaactt tcagttgccg gcggaggatc acaccaagct gaagatgtac 3180
gcggtacgcc aaggcaagac cattaccgag ctgctatctg aatacatcgc gcagctacca 3240
gagtaaatga gcaaatgaat aaatgagtag atgaatttta gcggctaaag gaggcggcat 3300
ggaaaatcaa gaacaaccag gcaccgacgc cgtggaatgc cccatgtgtg gaggaacggg 3360
cggttggcca ggcgtaagcg gctgggttgt ctgccggccc tgcaatggca ctggaacccc 3420
caagcccgag gaatcggcgt gacggtcgca aaccatccgg cccggtacaa atcggcgcgg 3480
cgctgggtga tgacctggtg gagaagttga aggccgcgca ggccgcccag cggcaacgca 3540
tcgaggcaga agcacgcccc ggtgaatcgt ggcaagcggc cgctgatcga atccgcaaag 3600
aatcccggca accgccggca gccggtgcgc cgtcgattag gaagccgccc aagggcgacg 3660
agcaaccaga ttttttcgtt ccgatgctct atgacgtggg cacccgcgat agtcgcagca 3720
tcatggacgt ggccgttttc cgtctgtcga agcgtgaccg acgagctggc gaggtgatcc 3780
gctacgagct tccagacggg cacgtagagg tttccgcagg gccggccggc atggccagtg 3840
tgtgggatta cgacctggta ctgatggcgg tttcccatct aaccgaatcc atgaaccgat 3900
accgggaagg gaagggagac aagcccggcc gcgtgttccg tccacacgtt gcggacgtac 3960
tcaagttctg ccggcgagcc gatggcggaa agcagaaaga cgacctggta gaaacctgca 4020
ttcggttaaa caccacgcac gttgccatgc agcgtacgaa gaaggccaag aacggccgcc 4080
tggtgacggt atccgagggt gaagccttga ttagccgcta caagatcgta aagagcgaaa 4140
ccgggcggcc ggagtacatc gagatcgagc tagctgattg gatgtaccgc gagatcacag 4200
aaggcaagaa cccggacgtg ctgacggttc accccgatta ctttttgatc gatcccggca 4260
tcggccgttt tctctaccgc ctggcacgcc gcgccgcagg caaggcagaa gccagatggt 4320
tgttcaagac gatctacgaa cgcagtggca gcgccggaga gttcaagaag ttctgtttca 4380
ccgtgcgcaa gctgatcggg tcaaatgacc tgccggagta cgatttgaag gaggaggcgg 4440
ggcaggctgg cccgatccta gtcatgcgct accgcaacct gatcgagggc gaagcatccg 4500
ccggttccta atgtacggag cagatgctag ggcaaattgc cctagcaggg gaaaaaggtc 4560
gaaaaggtct ctttcctgtg gatagcacgt acattgggaa cccaaagccg tacattggga 4620
accggaaccc gtacattggg aacccaaagc cgtacattgg gaaccggtca cacatgtaag 4680
tgactgatat aaaagagaaa aaaggcgatt tttccgccta aaactcttta aaacttatta 4740
aaactcttaa aacccgcctg gcctgtgcat aactgtctgg ccagcgcaca gccgaagagc 4800
tgcaaaaagc gcctaccctt cggtcgctgc gctccctacg ccccgccgct tcgcgtcggc 4860
ctatcgcggc cgctggccgc tcaaaaatgg ctggcctacg gccaggcaat ctaccagggc 4920
gcggacaagc cgcgccgtcg ccactcgacc gccggcgccc acatcaaggc accctgcctc 4980
gcgcgtttcg gtgatgacgg tgaaaacctc tgacacatgc agctcccgga gacggtcaca 5040
gcttgtctgt aagcggatgc cgggagcaga caagcccgtc agggcgcgtc agcgggtgtt 5100
ggcgggtgtc ggggcgcagc catgacccag tcacgtagcg atagcggagt gtatactggc 5160
ttaactatgc ggcatcagag cagattgtac tgagagtgca ccatatgcgg tgtgaaatac 5220
cgcacagatg cgtaaggaga aaataccgca tcaggcgctc ttccgcttcc tcgctcactg 5280
actcgctgcg ctcggtcgtt cggctgcggc gagcggtatc agctcactca aaggcggtaa 5340
tacggttatc cacagaatca ggggataacg caggaaagaa catgtgagca aaaggccagc 5400
aaaaggccag gaaccgtaaa aaggccgcgt tgctggcgtt tttccatagg ctccgccccc 5460
ctgacgagca tcacaaaaat cgacgctcaa gtcagaggtg gcgaaacccg acaggactat 5520
aaagatacca ggcgtttccc cctggaagct ccctcgtgcg ctctcctgtt ccgaccctgc 5580
cgcttaccgg atacctgtcc gcctttctcc cttcgggaag cgtggcgctt tctcatagct 5640
cacgctgtag gtatctcagt tcggtgtagg tcgttcgctc caagctgggc tgtgtgcacg 5700
aaccccccgt tcagcccgac cgctgcgcct tatccggtaa ctatcgtctt gagtccaacc 5760
cggtaagaca cgacttatcg ccactggcag cagccactgg taacaggatt agcagagcga 5820
ggtatgtagg cggtgctaca gagttcttga agtggtggcc taactacggc tacactagaa 5880
ggacagtatt tggtatctgc gctctgctga agccagttac cttcggaaaa agagttggta 5940
gctcttgatc cggcaaacaa accaccgctg gtagcggtgg tttttttgtt tgcaagcagc 6000
agattacgcg cagaaaaaaa ggatctcaag aagatccttt gatcttttct acggggtctg 6060
acgctcagtg gaacgaaaac tcacgttaag ggattttggt catgcattct aggtactaaa 6120

acaattcatc cagtaaaata taatatttta ttttctccca atcaggcttg atccccagta 6180
agtcaaaaaa tagctcgaca tactgttctt ccccgatatc ctccctgatc gaccggacgc 6240
agaaggcaat gtcataccac ttgtccgccc tgccgcttct cccaagatca ataaagccac 6300
ttactttgcc atctttcaca aagatgttgc tgtctcccag gtcgccgtgg gaaaagacaa 6360
gttcctcttc gggcttttcc gtctttaaaa aatcatacag ctcgcgcgga tctttaaatg 6420
gagtgtcttc ttcccagttt tcgcaatcca catcggccag atcgttattc agtaagtaat 6480
ccaattcggc taagcggctg tctaagctat tcgtataggg acaatccgat atgtcgatgg 6540
agtgaaagag cctgatgcac tccgcataca gctcgataat cttttcaggg ctttgttcat 6600
cttcatactc ttccgagcaa aggacgccat cggcctcact catgagcaga ttgctccagc 6660
catcatgccg ttcaaagtgc aggacctttg gaacaggcag ctttccttcc agccatagca 6720
tcatgtcctt ttcccgttcc acatcatagg tggtcccttt ataccggctg tccgtcattt 6780
ttaaatatag gttttcattt tctcccacca gcttatatac cttagcagga gacattcctt 6840
ccgtatcttt tacgcagcgg tatttttcga tcagtttttt caattccggt gatattctca 6900
ttttagccat ttattatttc cttcctcttt tctacagtat ttaaagatac cccaagaagc 6960
taattataac aagacgaact ccaattcact gttccttgca ttctaaaacc ttaaatacca 7020
gaaaacagct ttttcaaagt tgttttcaaa gttggcgtat aacatagtat cgacggagcc 7080
gattttgaaa ccgcggtgat cacaggcagc aacgctctgt catcgttaca atcaacatgc 7140
taccctccgc gagatcatcc gtgtttcaaa cccggcagct tagttgccgt tcttccgaat 7200
agcatcggta acatgagcaa agtctgccgc cttacaacgg ctctcccgct gacgccgtcc 7260
cggactgatg ggctgcctgt atcgagtggt gattttgtgc cgagctgccg gtcggggagc 7320
tgttggctgg ctggtggcag gatatattgt ggtgtaaaca aattgacgct tagacaactt 7380
aataacacat tgcggacgtt tttaatgtac tgaattaacg ccgaattaat tcgggggatc 7440
tggattttag tactggattt tggttttagg aattagaaat tttattgata gaagtatttt 7500
acaaatacaa atacatacta agggtttctt atatgctcaa cacatgagcg aaaccctata 7560
ggaaccctaa ttcccttatc tgggaactac tcacacatta ttatggagaa actcgagctt 7620
gtcgatcgac agatccggtc ggcatctact ctatttcttt gccctcggac gagtgctggg 7680
gcgtcggttt ccactatcgg cgagtacttc tacacagcca tcggtccaga cggccgcgct 7740
tctgcgggcg atttgtgtac gcccgacagt cccggctccg gatcggacga ttgcgtcgca 7800
tcgaccctgc gcccaagctg catcatcgaa attgccgtca accaagctct gatagagttg 7860
gtcaagacca atgcggagca tatacgcccg gagtcgtggc gatcctgcaa gctccggatg 7920
cctccgctcg aagtagcgcg tctgctgctc catacaagcc aaccacggcc tccagaagaa 7980
gatgttggcg acctcgtatt gggaatcccc gaacatcgcc tcgctccagt caatgaccgc 8040
tgttatgcgg ccattgtccg tcaggacatt gttggagccg aaatccgcgt gcacgaggtg 8100
ccggacttcg gggcagtcct cggcccaaag catcagctca tcgagagcct gcgcgacgga 8160
cgcactgacg gtgtcgtcca tcacagtttg ccagtgatac acatggggat cagcaatcgc 8220
gcatatgaaa tcacgccatg tagtgtattg accgattcct tgcggtccga atgggccgaa 8280
cccgctcgtc tggctaagat cggccgcagc gatcgcatcc atagcctccg cgaccggttg 8340
tagaacagcg ggcagttcgg tttcaggcag gtcttgcaac gtgacaccct gtgcacggcg 8400
ggagatgcaa taggtcaggc tctcgctaaa ctccccaatg tcaagcactt ccggaatcgg 8460
gagcgcggcc gatgcaaagt gccgataaac ataacgatct ttgtagaaac catcggcgca 8520
gctatttacc cgcaggacat atccacgccc tcctacatcg aagctgaaag cacgagattc 8580
ttcgccctcc gagagctgca tcaggtcgga gacgctgtcg aacttttcga tcagaaactt 8640
ctcgacagac gtcgcggtga gttcaggctt tttcatatct cattgccccc ccggatctgc 8700
gaaagctcga gagagataga tttgtagaga gagactggtg atttcagcgt gtcctctcca 8760
aatgaaatga acttccttat atagaggaag gtcttgcgaa ggatagtggg attgtgcgtc 8820
atcccttacg tcagtggaga tatcacatca atccacttgc tttgaagacg tggttggaac 8880
gtcttctttt tccacgatgc tcctcgtggg tgggggtcca tctttgggac cactgtcggc 8940
agaggcatct tgaacgatag cctttccttt atcgcaatga tggcatttgt aggtgccacc 9000
ttccttttct actgtccttt tgatgaagtg acagatagct gggcaatgga atccgaggag 9060
gtttcccgat attacccttt gttgaaaagt ctcaatagcc ctttggtctt ctgagactgt 9120
atctttgata ttcttggagt agacgagagt gtcgtgctcc accatgttat cacatcaatc 9180
cacttgcttt gaagacgtgg ttggaacgtc ttctttttcc acgatgctcc tcgtgggtgg 9240
gggtccatct ttgggaccac tgtcggcaga ggcatcttga acgatagcct ttcctttatc 9300
gcaatgatgg catttgtagg tgccaccttc cttttctact gtccttttga tgaagtgaca 9360
gatagctggg caatggaatc cgaggaggtt tcccgatatt accctttgtt gaaaagtctc 9420
aatagccctt tggtcttctg agactgtatc tttgatattc ttggagtaga cgagagtgtc 9480
gtgctccacc atgttggcaa gctgctctag ccaatacgca aaccgcctct ccccgcgcgt 9540
tggccgattc attaatgcag ctggcacgac aggtttcccg actggaaagc gggcagtgag 9600
cgcaacgcaa ttaatgtgag ttagctcact cattaggcac cccaggcttt acactttatg 9660
cttccggctc gtatgttgtg tggaattgtg agcggataac aatttcacac aggaaacagc 9720
tatgaccatg attacgaatt cgagctcggt acccggggat cctctagact gaaggcggga 9780

aacgacaatc tgatcatgag cggagaatta agggagtcac gttatgaccc ccgccgatga 9840
cgcgggacaa gccgttttac gtttggaact gacagaaccg caacgttgaa ggagccactc 9900
agccgcgggt ttctggagtt taatgagcta agcacatacg tcagaaacca ttattgcgcg 9960
ttcaaaagtc gcctaaggtc actatcagct agcaaatatt tcttgtcaaa aatgctccac 10020
tgacgttcca taaattcccc tcggtatcca attagagtct catattcact ctcaatccaa 10080
ataatctgca ccggatctcg agaatcgaat tcccgcggcc gc 10122

<210> 9
<211> 621
<212> DNA
<213> Artificial Sequence

<220>
<223> N. tabacum rDNA intergnic spacer (IGS) sequence

<300>
<308> Genbank #Y08422
<309> 1997-10-31

<400> 9
gtgctagcca atgtttaaca agatgtcaag cacaatgaat gttggtggtt ggtggtcgtg 60
gctggcggtg gtggaaaatt gcggtggttc gagcggtagt gatcggcgat ggttggtgtt 120
tgcagcggtg tttgatatcg gaatcactta tggtggttgt cacaatggag gtgcgtcatg 180
gttattggtg gttggtcatc tatatatttt tataataata ttaagtattt tacctatttt 240
ttacatattt tttattaaat ttatgcattg tttgtatttt taaatagttt ttatcgtact 300
tgttttataa aatattttat tattttatgt gttatattat tacttgatgt attggaaatt 360
ttctccattg ttttttctat atttataata attttcttat ttttttttgt tttattatgt 420
attttttcgt tttataataa atatttatta aaaaaaatat tatttttgta aaatatatca 480
tttacaatgt ttaaaagtca tttgtgaata tattagctaa gttgtacttc tttttgtgca 540
tttggtgttg tacatgtcta ttatgattct ctggccaaaa catgtctact cctgtcactt 600
gggttttttt ttttaagaca t 621

<210> 10
<211> 25
<212> DNA
<213> Artificial Sequence

<220>
<223> PCR Primer NTIGS-F1

<400> 10
gtgctagcca atgtttaaca agatg 25

<210> 11
<211> 28
<212> DNA
<213> Artificial Sequence

<220>
<223> PCR Primer NTIGS-RI

<400> 11
atgtcttaaa aaaaaaaacc caagtgac 28

<210> 12
<211> 233
<212> DNA
<213> Mus musculus

<300>
<308> Genbank #V00846

<309> 1989-07-06

<400> 12
gacctggaat atggcgagaa aactgaaaat cacggaaaat gagaaataca cactttagga 60
cgtgaaatat ggcgaggaaa actgaaaaag gtggaaaatt tagaaatgtc cactgtagga 120
cgtggaatat ggcaagaaaa ctgaaaatca tggaaaatga gaaacatcca cttgacgact 180
tgaaaaatga cgaaatcact aaaaaacgtg aaaaatgaga aatgcacact gaa 233

<210> 13
<211> 31
<212> DNA
<213> Artificial Sequence

<220>
<223> Primer MSAT-F1

<400> 13
aataccgcgg aagcttgacc tggaatatcg c 31

<210> 14
<211> 27
<212> DNA
<213> Artificial Sequence

<220>
<223> Primer MSAT-RI

<400> 14
ataaccgcgg agtccttcag tgtgcat 27

<210> 15
<211> 277
<212> DNA
<213> Artificial Sequence

<220>
<223> Nopaline Synthase Promoter Fragment

<300>
<308> Genebank #U09365
<309> 1997-10-17

<400> 15
gagctcgaat ttccccgatc gttcaaacat ttggcaataa agtttcttaa gattgaatcc 60
tgttgccggt cttgcgatga ttatcatata atttctgttg aattacgtta agcatgtaat 120
aattaacatg taatgcatga cgttatttat gagatgggtt tttatgatta gagtcccgca 180
attatacatt taatacgcga tagaaaacaa aatatagcgc gcaaactagg ataaattatc 240
gcgcgcggtg tcatctatgt tactagatcg ggaattc 277

<210> 16
<211> 1812
<212> DNA
<213> Escherichia coli

<220>
<221> CDS
<222> (1)...(1812)
<223> Beta-glucuronidase

<300>
<308> Genbank #S69414

<309> 1994-09-23

<400> 16
atg tta cgt cct gta gaa acc cca acc cgt gaa atc aaa aaa ctc gac     48
Met Leu Arg Pro Val Glu Thr Pro Thr Arg Glu Ile Lys Lys Leu Asp
1               5                   10                  15

ggc ctg tgg gca ttc agt ctg gat cgc gaa aac tgt gga att gat cag     96
Gly Leu Trp Ala Phe Ser Leu Asp Arg Glu Asn Cys Gly Ile Asp Gln
            20                  25                  30

cgt tgg tgg gaa agc gcg tta caa gaa agc cgg gca att gct gtg cca     144
Arg Trp Trp Glu Ser Ala Leu Gln Glu Ser Arg Ala Ile Ala Val Pro
        35                  40                  45

ggc agt ttt aac gat cag ttc gcc gat gca gat att cgt aat tat gcg     192
Gly Ser Phe Asn Asp Gln Phe Ala Asp Ala Asp Ile Arg Asn Tyr Ala
    50                  55                  60

ggc aac gtc tgg tat cag cgc gaa gtc ttt ata ccg aaa ggt tgg gca     240
Gly Asn Val Trp Tyr Gln Arg Glu Val Phe Ile Pro Lys Gly Trp Ala
65                  70                  75                  80

ggc cag cgt atc gtg ctg cgt ttc gat gcg gtc act cat tac ggc aaa     288
Gly Gln Arg Ile Val Leu Arg Phe Asp Ala Val Thr His Tyr Gly Lys
                85                  90                  95

gtg tgg gtc aat aat cag gaa gtg atg gag cat cag ggc ggc tat acg     336
Val Trp Val Asn Asn Gln Glu Val Met Glu His Gln Gly Gly Tyr Thr
            100                 105                 110

cca ttt gaa gcc gat gtc acg ccg tat gtt att gcc ggg aaa agt gta     384
Pro Phe Glu Ala Asp Val Thr Pro Tyr Val Ile Ala Gly Lys Ser Val
        115                 120                 125

cgt atc acc gtt tgt gtg aac aac gaa ctg aac tgg cag act atc ccg     432
Arg Ile Thr Val Cys Val Asn Asn Glu Leu Asn Trp Gln Thr Ile Pro
    130                 135                 140

ccg gga atg gtg att acc gac gaa aac ggc aag aaa aag cag tct tac     480
Pro Gly Met Val Ile Thr Asp Glu Asn Gly Lys Lys Lys Gln Ser Tyr
145                 150                 155                 160

ttc cat gat ttc ttt aac tat gcc gga atc cat cgc agc gta atg ctc     528
Phe His Asp Phe Phe Asn Tyr Ala Gly Ile His Arg Ser Val Met Leu
                165                 170                 175

tac acc acg ccg aac acc tgg gtg gac gat atc acc gtg gtg acg cat     576
Tyr Thr Thr Pro Asn Thr Trp Val Asp Asp Ile Thr Val Val Thr His
            180                 185                 190

gtc gcg caa gac tgt aac cac gcg tct gtt gac tgg cag gtg gtg gcc     624
Val Ala Gln Asp Cys Asn His Ala Ser Val Asp Trp Gln Val Val Ala
        195                 200                 205

aat ggt gat gtc agc gtt gaa ctg cgt gat gcg gat caa cag gtg gtt     672
Asn Gly Asp Val Ser Val Glu Leu Arg Asp Ala Asp Gln Gln Val Val
    210                 215                 220

gca act gga caa ggc act agc ggg act ttg caa gtg gtg aat ccg cac     720
Ala Thr Gly Gln Gly Thr Ser Gly Thr Leu Gln Val Val Asn Pro His

225                 230                 235                 240

ctc tgg caa ccg ggt gaa ggt tat ctc tat gaa ctg tgc gtc aca gcc     768
Leu Trp Gln Pro Gly Glu Gly Tyr Leu Tyr Glu Leu Cys Val Thr Ala
                245                 250                 255

aaa agc cag aca gag tgt gat atc tac ccg ctt cgc gtc ggc atc cgg     816
Lys Ser Gln Thr Glu Cys Asp Ile Tyr Pro Leu Arg Val Gly Ile Arg
            260                 265                 270

tca gtg gca gtg aag ggc gaa cag ttc ctg att aac cac aaa ccg ttc     864
Ser Val Ala Val Lys Gly Glu Gln Phe Leu Ile Asn His Lys Pro Phe
        275                 280                 285

tac ttt act ggc ttt ggt cgt cat gaa gat gcg gac ttg cgt ggc aaa     912
Tyr Phe Thr Gly Phe Gly Arg His Glu Asp Ala Asp Leu Arg Gly Lys
    290                 295                 300

gga ttc gat aac gtg ctg atg gtg cac gac cac gca tta atg gac tgg     960
Gly Phe Asp Asn Val Leu Met Val His Asp His Ala Leu Met Asp Trp
305                 310                 315                 320

att ggg gcc aac tcc tac cgt acc tcg cat tac cct tac gct gaa gag     1008
Ile Gly Ala Asn Ser Tyr Arg Thr Ser His Tyr Pro Tyr Ala Glu Glu
                325                 330                 335

atg ctc gac tgg gca gat gaa cat ggc atc gtg gtg att gat gaa act     1056
Met Leu Asp Trp Ala Asp Glu His Gly Ile Val Val Ile Asp Glu Thr
            340                 345                 350

gct gct gtc ggc ttt aac ctc tct tta ggc att ggt ttc gaa gcg ggc     1104
Ala Ala Val Gly Phe Asn Leu Ser Leu Gly Ile Gly Phe Glu Ala Gly
        355                 360                 365

aac aag ccg aaa gaa ctg tac agc gaa gag gca gtc aac ggg gaa act     1152
Asn Lys Pro Lys Glu Leu Tyr Ser Glu Glu Ala Val Asn Gly Glu Thr
    370                 375                 380

cag caa gcg cac tta cag gcg att aaa gag ctg ata gcg cgt gac aaa     1200
Gln Gln Ala His Leu Gln Ala Ile Lys Glu Leu Ile Ala Arg Asp Lys
385                 390                 395                 400

aac cac cca agc gtg gtg atg tgg agt att gcc aac gaa ccg gat acc     1248
Asn His Pro Ser Val Val Met Trp Ser Ile Ala Asn Glu Pro Asp Thr
                405                 410                 415

cgt ccg caa ggt gca cgg gaa tat ttc gcg cca ctg gcg gaa gca acg     1296
Arg Pro Gln Gly Ala Arg Glu Tyr Phe Ala Pro Leu Ala Glu Ala Thr
            420                 425                 430

cgt aaa ctc gac ccg acg cgt ccg atc acc tgc gtc aat gta atg ttc     1344
Arg Lys Leu Asp Pro Thr Arg Pro Ile Thr Cys Val Asn Val Met Phe
        435                 440                 445

tgc gac gct cac acc gat acc atc agc gat ctc ttt gat gtg ctg tgc     1392
Cys Asp Ala His Thr Asp Thr Ile Ser Asp Leu Phe Asp Val Leu Cys
    450                 455                 460

ctg aac cgt tat tac gga tgg tat gtc caa agc ggc gat ttg gaa acg     1440
Leu Asn Arg Tyr Tyr Gly Trp Tyr Val Gln Ser Gly Asp Leu Glu Thr
465                 470                 475                 480

gca gag aag gta ctg gaa aaa gaa ctt ctg gcc tgg cag gag aaa ctg 1488
Ala Glu Lys Val Leu Glu Lys Glu Leu Leu Ala Trp Gln Glu Lys Leu
                485                 490                 495

cat cag ccg att atc atc acc gaa tac ggc gtg gat acg tta gcc ggg 1536
His Gln Pro Ile Ile Ile Thr Glu Tyr Gly Val Asp Thr Leu Ala Gly
            500                 505                 510

ctg cac tca atg tac acc gac atg tgg agt gaa gag tat cag tgt gca 1584
Leu His Ser Met Tyr Thr Asp Met Trp Ser Glu Glu Tyr Gln Cys Ala
        515                 520                 525

tgg ctg gat atg tat cac cgc gtc ttt gat cgc gtc agc gcc gtc gtc 1632
Trp Leu Asp Met Tyr His Arg Val Phe Asp Arg Val Ser Ala Val Val
    530                 535                 540

ggt gaa cag gta tgg aat ttc gcc gat ttt gcg acc tcg caa ggc ata 1680
Gly Glu Gln Val Trp Asn Phe Ala Asp Phe Ala Thr Ser Gln Gly Ile
545                 550                 555                 560

ttg cgc gtt ggc ggt aac aag aaa ggg atc ttc act cgc gac cgc aaa 1728
Leu Arg Val Gly Gly Asn Lys Lys Gly Ile Phe Thr Arg Asp Arg Lys
                565                 570                 575

ccg aag tcg gcg gct ttt ctg ctg caa aaa cgc tgg act ggc atg aac 1776
Pro Lys Ser Ala Ala Phe Leu Leu Gln Lys Arg Trp Thr Gly Met Asn
            580                 585                 590

ttc ggt gaa aaa ccg cag cag gga ggc aaa caa tga 1812
Phe Gly Glu Lys Pro Gln Gln Gly Gly Lys Gln *
        595                 600

<210> 17
<211> 603
<212> PRT
<213> Escherichia coli

<300>
<308> Genbank #S69414
<309> 1994-09-23

<400> 17
Met Leu Arg Pro Val Glu Thr Pro Thr Arg Glu Ile Lys Lys Leu Asp
1               5                   10                  15
Gly Leu Trp Ala Phe Ser Leu Asp Arg Glu Asn Cys Gly Ile Asp Gln
            20                  25                  30
Arg Trp Trp Glu Ser Ala Leu Gln Glu Ser Arg Ala Ile Ala Val Pro
        35                  40                  45
Gly Ser Phe Asn Asp Gln Phe Ala Asp Ala Asp Ile Arg Asn Tyr Ala
    50                  55                  60
Gly Asn Val Trp Tyr Gln Arg Glu Val Phe Ile Pro Lys Gly Trp Ala
65                  70                  75                  80
Gly Gln Arg Ile Val Leu Arg Phe Asp Ala Val Thr His Tyr Gly Lys
                85                  90                  95
Val Trp Val Asn Asn Gln Glu Val Met Glu His Gln Gly Gly Tyr Thr
            100                 105                 110
Pro Phe Glu Ala Asp Val Thr Pro Tyr Val Ile Ala Gly Lys Ser Val
        115                 120                 125
Arg Ile Thr Val Cys Val Asn Asn Glu Leu Asn Trp Gln Thr Ile Pro

130                 135                 140
Pro Gly Met Val Ile Thr Asp Glu Asn Gly Lys Lys Lys Gln Ser Tyr
145                 150                 155                 160
Phe His Asp Phe Phe Asn Tyr Ala Gly Ile His Arg Ser Val Met Leu
                165                 170                 175
Tyr Thr Thr Pro Asn Thr Trp Val Asp Asp Ile Thr Val Val Thr His
            180                 185                 190
Val Ala Gln Asp Cys Asn His Ala Ser Val Asp Trp Gln Val Val Ala
        195                 200                 205
Asn Gly Asp Val Ser Val Glu Leu Arg Asp Ala Asp Gln Gln Val Val
    210                 215                 220
Ala Thr Gly Gln Gly Thr Ser Gly Thr Leu Gln Val Val Asn Pro His
225                 230                 235                 240
Leu Trp Gln Pro Gly Glu Gly Tyr Leu Tyr Glu Leu Cys Val Thr Ala
                245                 250                 255
Lys Ser Gln Thr Glu Cys Asp Ile Tyr Pro Leu Arg Val Gly Ile Arg
            260                 265                 270
Ser Val Ala Val Lys Gly Glu Gln Phe Leu Ile Asn His Lys Pro Phe
        275                 280                 285
Tyr Phe Thr Gly Phe Gly Arg His Glu Asp Ala Asp Leu Arg Gly Lys
    290                 295                 300
Gly Phe Asp Asn Val Leu Met Val His Asp His Ala Leu Met Asp Trp
305                 310                 315                 320
Ile Gly Ala Asn Ser Tyr Arg Thr Ser His Tyr Pro Tyr Ala Glu Glu
                325                 330                 335
Met Leu Asp Trp Ala Asp Glu His Gly Ile Val Val Ile Asp Glu Thr
            340                 345                 350
Ala Ala Val Gly Phe Asn Leu Ser Leu Gly Ile Gly Phe Glu Ala Gly
        355                 360                 365
Asn Lys Pro Lys Glu Leu Tyr Ser Glu Glu Ala Val Asn Gly Glu Thr
    370                 375                 380
Gln Gln Ala His Leu Gln Ala Ile Lys Glu Leu Ile Ala Arg Asp Lys
385                 390                 395                 400
Asn His Pro Ser Val Val Met Trp Ser Ile Ala Asn Glu Pro Asp Thr
                405                 410                 415
Arg Pro Gln Gly Ala Arg Glu Tyr Phe Ala Pro Leu Ala Glu Ala Thr
            420                 425                 430
Arg Lys Leu Asp Pro Thr Arg Pro Ile Thr Cys Val Asn Val Met Phe
        435                 440                 445
Cys Asp Ala His Thr Asp Thr Ile Ser Asp Leu Phe Asp Val Leu Cys
    450                 455                 460
Leu Asn Arg Tyr Tyr Gly Trp Tyr Val Gln Ser Gly Asp Leu Glu Thr
465                 470                 475                 480
Ala Glu Lys Val Leu Glu Lys Glu Leu Leu Ala Trp Gln Glu Lys Leu
                485                 490                 495
His Gln Pro Ile Ile Ile Thr Glu Tyr Gly Val Asp Thr Leu Ala Gly
            500                 505                 510
Leu His Ser Met Tyr Thr Asp Met Trp Ser Glu Glu Tyr Gln Cys Ala
        515                 520                 525
Trp Leu Asp Met Tyr His Arg Val Phe Asp Arg Val Ser Ala Val Val
    530                 535                 540
Gly Glu Gln Val Trp Asn Phe Ala Asp Phe Ala Thr Ser Gln Gly Ile
545                 550                 555                 560
Leu Arg Val Gly Gly Asn Lys Lys Gly Ile Phe Thr Arg Asp Arg Lys
                565                 570                 575
Pro Lys Ser Ala Ala Phe Leu Leu Gln Lys Arg Trp Thr Gly Met Asn
            580                 585                 590
Phe Gly Glu Lys Pro Gln Gln Gly Gly Lys Gln
        595                 600

<210> 18

<211> 277
<212> DNA
<213> Artificial Sequence

<220>
<223> Nopaline Synthase Terminator Sequence

<300>
<308> Genbank #U09365
<309> 1995-10-17

<400> 18
gagctcgaat ttccccgatc gttcaaacat ttggcaataa agtttcttaa gattgaatcc 60
tgttgccggt cttgcgatga ttatcatata atttctgttg aattacgtta agcatgtaat 120
aattaacatg taatgcatga cgttatttat gagatgggtt tttatgatta gagtcccgca 180
attatacatt taatacgcga tagaaaacaa aatatagcgc gcaaactagg ataaattatc 240
gcgcgcggtg tcatctatgt tactagatcg ggaattc 277

<210> 19
<211> 3438
<212> DNA
<213> Artificial Sequence

<220>
<223> pLIT38attBZeo Plasmid

<400> 19
tcgaccctct agtcaaggcc ttaagtgagt cgtattacgg actggccgtc gttttacaac 60
gtcgtgactg ggaaaaccct ggcgttaccc aacttaatcg ccttgcagca catccccctt 120
tcgccagctg gcgtaatagc gaagaggccc gcaccgatcg cccttcccaa cagttgcgca 180
gcctgaatgg cgaatggcgc ttcgcttggt aataaagccc gcttcggcgg gctttttttt 240
gttaactacg tcaggtggca cttttcgggg aaatgtgcgc ggaaccccta tttgtttatt 300
tttctaaata cattcaaata tgtatccgct catgagacaa taaccctgat aaatgcttca 360
ataatattga aaaaggaaga gtatgagtat tcaacatttc cgtgtcgccc ttattccctt 420
ttttgcggca ttttgccttc ctgtttttgc tcacccagaa acgctggtga aagtaaaaga 480
tgctgaagat cagttgggtg cacgagtggg ttacatcgaa ctggatctca acagcggtaa 540
gatccttgag agttttcgcc ccgaagaacg ttttccaatg atgagcactt ttaaagttct 600
gctatgtggc gcggtattat cccgtgttga cgccgggcaa gagcaactcg gtcgccgcat 660
acactattct cagaatgact tggttgagta ctcaccagtc acagaaaagc atcttacgga 720
tggcatgaca gtaagagaat tatgcagtgc tgccataacc atgagtgata acactgcggc 780
caacttactt ctgacaacga tcggaggacc gaaggagcta accgcttttt tgcacaacat 840
gggggatcat gtaactcgcc ttgatcgttg ggaaccggag ctgaatgaag ccataccaaa 900
cgacgagcgt gacaccacga tgcctgtagc aatggcaaca acgttgcgca aactattaac 960
tggcgaacta cttactctag cttcccggca acaattaata gactggatgg aggcggataa 1020
agttgcagga ccacttctgc gctcggccct tccggctggc tggtttattg ctgataaatc 1080
tggagccggt gagcgtgggt ctcgcggtat cattgcagca ctggggccag atggtaagcc 1140
ctcccgtatc gtagttatct acacgacggg gagtcaggca actatggatg aacgaaatag 1200
acagatcgct gagataggtg cctcactgat taagcattgg taactgtcag accaagttta 1260
ctcatatata ctttagattg atttaccccg gttgataatc agaaaagccc caaaaacagg 1320
aagattgtat aagcaaatat ttaaattgta aacgttaata ttttgttaaa attcgcgtta 1380
aatttttgtt aaatcagctc attttttaac caataggccg aaatcggcaa aatcccttat 1440
aaatcaaaag aatagcccga gatagggttg agtgttgttc cagtttggaa caagagtcca 1500
ctattaaaga acgtggactc caacgtcaaa gggcgaaaaa ccgtctatca gggcgatggc 1560
ccactacgtg aaccatcacc caaatcaagt tttttggggt cgaggtgccg taaagcacta 1620
aatcggaacc ctaaagggag cccccgattt agagcttgac ggggaaagcg aacgtggcga 1680
gaaaggaagg gaagaaagcg aaaggagcgg gcgctagggc gctggcaagt gtagcggtca 1740
cgctgcgcgt aaccaccaca cccgccgcgc ttaatgcgcc gctacagggc gcgtaaaagg 1800
atctaggtga agatcctttt tgataatctc atgaccaaaa tcccttaacg tgagttttcg 1860
ttccactgag cgtcagaccc cgtagaaaag atcaaaggat cttcttgaga tccttttttt 1920
ctgcgcgtaa tctgctgctt gcaaacaaaa aaaccaccgc taccagcggt ggtttgtttg 1980
ccggatcaag agctaccaac tctttttccg aaggtaactg gcttcagcag agcgcagata 2040

ccaaatactg ttcttctagt gtagccgtag ttaggccacc acttcaagaa ctctgtagca 2100
ccgcctacat acctcgctct gctaatcctg ttaccagtgg ctgctgccag tggcgataag 2160
tcgtgtctta ccgggttgga ctcaagacga tagttaccgg ataaggcgca gcggtcgggc 2220
tgaacggggg gttcgtgcac acagcccagc ttggagcgaa cgacctacac cgaactgaga 2280
tacctacagc gtgagctatg agaaagcgcc acgcttcccg aagggagaaa ggcggacagg 2340
tatccggtaa gcggcagggt cggaacagga gagcgcacga gggagcttcc agggggaaac 2400
gcctggtatc tttatagtcc tgtcgggttt cgccacctct gacttgagcg tcgatttttg 2460
tgatgctcgt caggggggcg gagcctatgg aaaaacgcca gcaacgcggc ctttttacgg 2520
ttcctggcct tttgctggcc ttttgctcac atgtaatgtg agttagctca ctcattaggc 2580
accccaggct ttacacttta tgcttccggc tcgtatgttg tgtggaattg tgagcggata 2640
acaatttcac acaggaaaca gctatgacca tgattacgcc aagctacgta atacgactca 2700
ctagtggggc ccgtgcaatt gaagccggct ggcgccaagc ttctctgcag gattgaagcc 2760
tgctttttta tactaacttg agcgaaatct ggatccatgg ccaagttgac cagtgccgtt 2820
ccggtgctca ccgcgcgcga cgtcgccgga gcggtcgagt tctggaccga ccggctcggg 2880
ttctcccggg acttcgtgga ggacgacttc gccggtgtgg tccgggacga cgtgaccctg 2940
ttcatcagcg cggtccagga ccaggtggtg ccggacaaca ccctggcctg ggtgtgggtg 3000
cgcggcctgg acgagctgta cgccgagtgg tcggaggtcg tgtccacgaa cttccgggac 3060
gcctccgggc cggccatgac cgagatcggc gagcagccgt gggggcggga gttcgccctg 3120
cgcgacccgg ccggcaactg cgtgcacttc gtggccgagg agcaggactg acacgtgcta 3180
cgagatttcg attccaccgc cgccttctat gaaaggttgg gcttcggaat cgttttccgg 3240
gacgccggct ggatgatcct ccagcgcggg gatctcatgc tggagttctt cgcccacccc 3300
aacttgttta ttgcagctta taatggttac aaataaagca atagcatcac aaatttcaca 3360
aataaagcat ttttttcact gcattctagt tgtggtttgt ccaaactcat caatgtatct 3420
tatcatgtct gtataccg 3438

<210> 20
<211> 3451
<212> DNA
<213> Artificial Sequence

<220>
<223> HindIII Fragment containing the beta-glucuronidase
      coding sequence, the rDNA intergenic spacer, and
      the Mast1 sequence

<400> 20
aagcttgacc tggaatatcg cgagtaaact gaaaatcacg gaaaatgaga aatacacact 60
ttaggacgtg aaatatggcg aggaaaactg aaaaaggtgg aaaatttaga aatgtccact 120
gtaggacgtg gaatatggca agaaaactga aaatcatgga aaatgagaaa catccacttg 180
acgacttgaa aaatgacgaa atcactaaaa aacgtgaaaa atgagaaatg cacactgaag 240
gactccgcgg gaattcgatt gtgctagcca atgtttaaca agatgtcaag cacaatgaat 300
gttggtggtt ggtggtcgtg gctggcggtg gtggaaaatt gcggtggttc gagcggtagt 360
gatcggcgat ggttggtgtt tgcagcggtg tttgatatcg gaatcactta tggtggttgt 420
cacaatggag gtgcgtcatg gttattggtg gttggtcatc tatatatttt tataataata 480
ttaagtattt tacctatttt ttacatattt tttattaaat ttatgcattg tttgtatttt 540
taaatagttt ttatcgtact tgttttataa aatattttat tattttatgt gttatattat 600
tacttgatgt attggaaatt ttctccattg ttttttctat atttataata attttcttat 660
ttttttttgt tttattatgt attttttcgt tttataataa atatttatta aaaaaaatat 720
tatttttgta aaatatatca tttacaatgt ttaaaagtca tttgtgaata tattagctaa 780
gttgtacttc tttttgtgca tttggtgttg tacatgtcta ttatgattct ctggccaaaa 840
catgtctact cctgtcactt gggttttttt ttttaagaca taatcactag tgattatatc 900
tagactgaag gcgggaaacg acaatctgat catgagcgga gaattaaggg agtcacgtta 960
tgacccccgc cgatgacgcg ggacaagccg ttttacgttt ggaactgaca gaaccgcaac 1020
gttgaaggag ccactcagcc gcgggtttct ggagtttaat gagctaagca catacgtcag 1080
aaaccattat tgcgcgttca aaagtcgcct aaggtcacta tcagctagca aatatttctt 1140
gtcaaaaatg ctccactgac gttccataaa ttcccctcgg tatccaatta gagtctcata 1200
ttcactctca atccaaataa tctgcaccgg atctcgagat cgaattcccg cggccgcgaa 1260
ttcactagtg gatccccggg tacggtcagt cccttatgtt acgtcctgta gaaaccccaa 1320
cccgtgaaat caaaaaactc gacggcctgt gggcattcag tctggatcgc gaaaactgtg 1380
gaattgagca gcgttggtgg gaaagcgcgt tacaagaaag ccgggcaatt gctgtgccag 1440
gcagttttaa cgatcagttc gccgatgcag atattcgtaa ttatgtgggc aacgtctggt 1500

atcagcgcga agtctttata ccgaaaggtt gggcaggcca gcgtatcgtg ctgcgtttcg 1560
atgcggtcac tcattacggc aaagtgtggg tcaataatca ggaagtgatg gagcatcagg 1620
gcggctatac gccatttgaa gccgatgtca cgccgtatgt tattgccggg aaaagtgtac 1680
gtatcacagt ttgtgtgaac aacgaactga actggcagac tatcccgccg ggaatggtga 1740
ttaccgacga aaacggcaag aaaaagcagt cttacttcca tgatttcttt aactacgccg 1800
ggatccatcg cagcgtaatg ctctacacca cgccgaacac ctgggtggac gatatcaccg 1860
tggtgacgca tgtcgcgcaa gactgtaacc acgcgtctgt tgactggcag gtggtggcca 1920
atggtgatgt cagcgttgaa ctgcgtgatg cggatcaaca ggtggttgca actggacaag 1980
gcaccagcgg gactttgcaa gtggtgaatc cgcacctctg gcaaccgggt gaaggttatc 2040
tctatgaact gtacgtcaca gccaaaagcc agacagagtg tgatatctac ccgctgcgcg 2100
tcggcatccg gtcagtggca gtgaagggcg aacagttcct gatcaaccac aaaccgttct 2160
actttactgg ctttggccgt catgaagatg cggatttgcg cggcaaagga ttcgataacg 2220
tgctgatggt gcacgatcac gcattaatgg actggattgg ggccaactcc taccgtacct 2280
cgcattaccc ttacgctgaa gagatgctcg actgggcaga tgaacatggc atcgtggtga 2340
ttgatgaaac tgcagctgtc ggctttaacc tctctttagg cattggtttc gaagcgggca 2400
acaagccgaa agaactgtac agcgaagagg cagtcaacgg ggaaactcag caggcgcact 2460
tacaggcgat taaagagctg atagcgcgtg acaaaaacca cccaagcgtg gtgatgtgga 2520
gtattgccaa cgaaccggat acccgtccgc aaggtgcacg ggaatatttc gcgccactgg 2580
cggaagcaac gcgtaaactc gatccgacgc gtccgatcac ctgcgtcaat gtaatgttct 2640
gcgacgctca caccgatacc atcagcgatc tctttgatgt gctgtgcctg aaccgttatt 2700
acggttggta tgtccaaagc ggcgatttgg aaacggcaga gaaggtactg gaaaaagaac 2760
ttctggcctg gcaggagaaa ctgcatcagc cgattatcat caccgaatac ggcgtggata 2820
cgttagccgg gctgcactca atgtacaccg acatgtggag tgaagagtat cagtgtgcat 2880
ggctggatat gtatcaccgc gtctttgatc gcgtcagcgc cgtcgtcggt gaacaggtat 2940
ggaatttcgc cgattttgcg acctcgcaag gcatattgcg cgttggcggt aacaagaagg 3000
ggatcttcac ccgcgaccgc aaaccgaagt cggcggcttt tctgctgcaa aaacgctgga 3060
ctggcatgaa cttcggtgaa aaaccgcagc agggaggcaa acaatgaatc aacaactctc 3120
ctggcgcacc atcgtcggct acagcctcgg gaattgcgta ccgagctcga atttccccga 3180
tcgttcaaac atttggcaat aaagtttctt aagattgaat cctgttgccg gtcttgcgat 3240
gattatcata taatttctgt tgaattacgt taagcatgta ataattaaca tgtaatgcat 3300
gacgttattt atgagatggg tttttatgat tagagtcccg caattataca tttaatacgc 3360
gatagaaaac aaaatatagc gcgcaaacta ggataaatta tcgcgcgcgg tgtcatctat 3420
gttactagat cgggaattcg atatcaagct t 3451

<210> 21
<211> 14627
<212> DNA
<213> Artificial Sequence

<220>
<223> pAg11a Plasmid

<400> 21
   catgccaacc acagggttcc cctcgggatc aaagtacttt gatccaaccc ctccgctgct 60
   atagtgcagt cggcttctga cgttcagtgc agccgtcttc tgaaaacgac atgtcgcaca 120
   agtcctaagt tacgcgacag gctgccgccc tgcccttttc ctggcgtttt cttgtcgcgt 180
   gttttagtcg cataaagtag aatacttgcg actagaaccg gagacattac gccatgaaca 240
   agagcgccgc cgctggcctg ctgggctatg cccgcgtcag caccgacgac caggacttga 300
   ccaaccaacg ggccgaactg cacgcggccg gctgcaccaa gctgttttcc gagaagatca 360
   ccggcaccag gcgcgaccgc ccggagctgg ccaggatgct tgaccaccta cgccctggcg 420
   acgttgtgac agtgaccagg ctagaccgcc tggcccgcag cacccgcgac ctactggaca 480
   ttgccgagcg catccaggag gccggcgcgg gcctgcgtag cctggcagag ccgtgggccg 540
   acaccaccac gccggccggc cgcatggtgt tgaccgtgtt cgccggcatt gccgagttcg 600
   agcgttccct aatcatcgac cgcacccgga gcgggcgcga ggccgccaag gcccgaggcg 660
   tgaagtttgg cccccgccct accctcaccc cggcacagat cgcgcacgcc cgcgagctga 720
   tcgaccagga aggccgcacc gtgaaagagg cggctgcact gcttggcgtg catcgctcga 780
   ccctgtaccg cgcacttgag cgcagcgagg aagtgacgcc caccgaggcc aggcggcgcg 840
   gtgccttccg tgaggacgca ttgaccgagg ccgacgccct ggcggccgcc gagaatgaac 900
   gccaagagga acaagcatga aaccgcacca ggacggccag gacgaaccgt ttttcattac 960
   cgaagagatc gaggcggaga tgatcgcggc cgggtacgtg ttcgagccgc ccgcgcacgt 1020
   ctcaaccgtg cggctgcatg aaatcctggc cggtttgtct gatgccaagc tggcggcctg 1080
   gccggccagc ttggccgctg aagaaaccga gcgccgccgt ctaaaaaggt gatgtgtatt 1140
   tgagtaaaac agcttgcgtc atgcggtcgc tgcgtatatg atgcgatgag taaataaaca 1200
   aatacgcaag gggaacgcat gaaggttatc gctgtactta accagaaagg cgggtcaggc 1260
   aagacgacca tcgcaaccca tctagcccgc gccctgcaac tcgccggggc cgatgttctg 1320
   ttagtcgatt ccgatcccca gggcagtgcc cgcgattggg cggccgtgcg ggaagatcaa 1380
   ccgctaaccg ttgtcggcat cgaccgcccg acgattgacc gcgacgtgaa ggccatcggc 1440
   cggcgcgact tcgtagtgat cgacggagcg ccccaggcgg cggacttggc tgtgtccgcg 1500
   atcaaggcag ccgacttcgt gctgattccg gtgcagccaa gcccttacga catatgggcc 1560
   accgccgacc tggtggagct ggttaagcag cgcattgagg tcacggatgg aaggctacaa 1620
   gcggcctttg tcgtgtcgcg ggcgatcaaa ggcacgcgca tcggcggtga ggttgccgag 1680
   gcgctggccg ggtacgagct gcccattctt gagtcccgta tcacgcagcg cgtgagctac 1740
   ccaggcactg ccgccgccgg cacaaccgtt cttgaatcag aacccgaggg cgacgctgcc 1800
   cgcgaggtcc aggcgctggc cgctgaaatt aaatcaaaac tcatttgagt taatgaggta 1860
   aagagaaaat gagcaaaagc acaaacacgc taagtgccgg ccgtccgagc gcacgcagca 1920
   gcaaggctgc aacgttggcc agcctggcag acacgccagc catgaagcgg gtcaactttc 1980
   agttgccggc ggaggatcac accaagctga agatgtacgc ggtacgccaa ggcaagacca 2040
   ttaccgagct gctatctgaa tacatcgcgc agctaccaga gtaaatgagc aaatgaataa 2100
   atgagtagat gaattttagc ggctaaagga ggcggcatgg aaaatcaaga acaaccaggc 2160
   accgacgccg tggaatgccc catgtgtgga ggaacgggcg gttggccagg cgtaagcggc 2220
   tgggttgtct gccggccctg caatggcact ggaaccccca agcccgagga atcggcgtga 2280
   cggtcgcaaa ccatccggcc cggtacaaat cggcgcggcg ctgggtgatg acctggtgga 2340
   gaagttgaag gccgcgcagg ccgcccagcg gcaacgcatc gaggcagaag cacgccccgg 2400
   tgaatcgtgg caagcggccg ctgatcgaat ccgcaaagaa tcccggcaac cgccggcagc 2460
   cggtgcgccg tcgattagga agccgcccaa gggcgacgag caaccagatt ttttcgttcc 2520
   gatgctctat gacgtgggca cccgcgatag tcgcagcatc atggacgtgg ccgttttccg 2580
   tctgtcgaag cgtgaccgac gagctggcga ggtgatccgc tacgagcttc cagacgggca 2640
   cgtagaggtt tccgcagggc cggccggcat ggccagtgtg tgggattacg acctggtact 2700
   gatggcggtt tcccatctaa ccgaatccat gaaccgatac cgggaaggga agggagacaa 2760
   gcccggccgc gtgttccgtc cacacgttgc ggacgtactc aagttctgcc ggcgagccga 2820
   tggcggaaag cagaaagacg acctggtaga aacctgcatt cggttaaaca ccacgcacgt 2880
   tgccatgcag cgtacgaaga aggccaagaa cggccgcctg gtgacggtat ccgagggtga 2940
   agccttgatt agccgctaca agatcgtaaa gagcgaaacc gggcggccgg agtacatcga 3000
   gatcgagcta gctgattgga tgtaccgcga gatcacagaa ggcaagaacc cggacgtgct 3060
   gacggttcac cccgattact ttttgatcga tcccggcatc ggccgttttc tctaccgcct 3120
   ggcacgccgc gccgcaggca aggcagaagc cagatggttg ttcaagacga tctacgaacg 3180
   cagtggcagc gccggagagt tcaagaagtt ctgtttcacc gtgcgcaagc tgatcgggtc 3240
   aaatgacctg ccggagtacg atttgaagga ggaggcgggg caggctggcc cgatcctagt 3300
   catgcgctac cgcaacctga tcgagggcga agcatccgcc ggttcctaat gtacggagca 3360
   gatgctaggg caaattgccc tagcagggga aaaaggtcga aaaggtctct ttcctgtgga 3420
   tagcacgtac attgggaacc caaagccgta cattgggaac cggaacccgt acattgggaa 3480
   cccaaagccg tacattggga accggtcaca catgtaagtg actgatataa aagagaaaaa 3540
   aggcgatttt tccgcctaaa actctttaaa acttattaaa actcttaaaa cccgcctggc 3600
   ctgtgcataa ctgtctggcc agcgcacagc cgaagagctg caaaaagcgc ctacccttcg 3660
   gtcgctgcgc tccctacgcc ccgccgcttc gcgtcggcct atcgcggccg ctggccgctc 3720
   aaaaatggct ggcctacggc caggcaatct accagggcgc ggacaagccg cgccgtcgcc 3780
   actcgaccgc cggcgcccac atcaaggcac cctgcctcgc gcgtttcggt gatgacggtg 3840
   aaaacctctg acacatgcag ctcccggaga cggtcacagc ttgtctgtaa gcggatgccg 3900
   ggagcagaca agcccgtcag ggcgcgtcag cgggtgttgg cgggtgtcgg ggcgcagcca 3960
   tgacccagtc acgtagcgat agcggagtgt atactggctt aactatgcgg catcagagca 4020
   gattgtactg agagtgcacc atatgcggtg tgaaataccg cacagatgcg taaggagaaa 4080
   ataccgcatc aggcgctctt ccgcttcctc gctcactgac tcgctgcgct cggtcgttcg 4140
   gctgcggcga gcggtatcag ctcactcaaa ggcggtaata cggttatcca cagaatcagg 4200
   ggataacgca ggaaagaaca tgtgagcaaa aggccagcaa aaggccagga accgtaaaaa 4260
   ggccgcgttg ctggcgtttt tccataggct ccgcccccct gacgagcatc acaaaaatcg 4320
   acgctcaagt cagaggtggc gaaacccgac aggactataa agataccagg cgtttccccc 4380
   tggaagctcc ctcgtgcgct ctcctgttcc gaccctgccg cttaccggat acctgtccgc 4440
   ctttctccct tcgggaagcg tggcgctttc tcatagctca cgctgtaggt atctcagttc 4500
   ggtgtaggtc gttcgctcca agctgggctg tgtgcacgaa ccccccgttc agcccgaccg 4560
   ctgcgcctta tccggtaact atcgtcttga gtccaacccg gtaagacacg acttatcgcc 4620
   actggcagca gccactggta acaggattag cagagcgagg tatgtaggcg gtgctacaga 4680
   gttcttgaag tggtggccta actacggcta cactagaagg acagtatttg gtatctgcgc 4740
   tctgctgaag ccagttacct tcggaaaaag agttggtagc tcttgatccg gcaaacaaac 4800
   caccgctggt agcggtggtt tttttgtttg caagcagcag attacgcgca gaaaaaaagg 4860
   atctcaagaa gatcctttga tcttttctac ggggtctgac gctcagtgga acgaaaactc 4920
   acgttaaggg attttggtca tgcattctag gtactaaaac aattcatcca gtaaaatata 4980
   atattttatt ttctcccaat caggcttgat ccccagtaag tcaaaaaata gctcgacata 5040
   ctgttcttcc ccgatatcct ccctgatcga ccggacgcag aaggcaatgt cataccactt 5100
   gtccgccctg ccgcttctcc caagatcaat aaagccactt actttgccat ctttcacaaa 5160
   gatgttgctg tctcccaggt cgccgtggga aaagacaagt tcctcttcgg gcttttccgt 5220
   ctttaaaaaa tcatacagct cgcgcggatc tttaaatgga gtgtcttctt cccagttttc 5280
   gcaatccaca tcggccagat cgttattcag taagtaatcc aattcggcta agcggctgtc 5340
   taagctattc gtatagggac aatccgatat gtcgatggag tgaaagagcc tgatgcactc 5400
   cgcatacagc tcgataatct tttcagggct ttgttcatct tcatactctt ccgagcaaag 5460
   gacgccatcg gcctcactca tgagcagatt gctccagcca tcatgccgtt caaagtgcag 5520
   gacctttgga acaggcagct ttccttccag ccatagcatc atgtcctttt cccgttccac 5580
   atcataggtg gtccctttat accggctgtc cgtcattttt aaatataggt tttcattttc 5640
   tcccaccagc ttatatacct tagcaggaga cattccttcc gtatctttta cgcagcggta 5700
   tttttcgatc agttttttca attccggtga tattctcatt ttagccattt attatttcct 5760
   tcctcttttc tacagtattt aaagataccc caagaagcta attataacaa gacgaactcc 5820
   aattcactgt tccttgcatt ctaaaacctt aaataccaga aaacagcttt ttcaaagttg 5880
   ttttcaaagt tggcgtataa catagtatcg acggagccga ttttgaaacc gcggtgatca 5940
   caggcagcaa cgctctgtca tcgttacaat caacatgcta ccctccgcga gatcatccgt 6000
   gtttcaaacc cggcagctta gttgccgttc ttccgaatag catcggtaac atgagcaaag 6060
   tctgccgcct tacaacggct ctcccgctga cgccgtcccg gactgatggg ctgcctgtat 6120
   cgagtggtga ttttgtgccg agctgccggt cggggagctg ttggctggct ggtggcagga 6180
   tatattgtgg tgtaaacaaa ttgacgctta gacaacttaa taacacattg cggacgtttt 6240
   taatgtactg aattaacgcc gaattaattc gggggatctg gattttagta ctggattttg 6300
   gttttaggaa ttagaaattt tattgataga agtattttac aaatacaaat acatactaag 6360
   ggtttcttat atgctcaaca catgagcgaa accctatagg aaccctaatt cccttatctg 6420
   ggaactactc acacattatt atggagaaac tcgagtcaaa tctcggtgac gggcaggacc 6480
   ggacggggcg gtaccggcag gctgaagtcc agctgccaga aacccacgtc atgccagttc 6540
   ccgtgcttga agccggccgc ccgcagcatg ccgcgggggg catatccgag cgcctcgtgc 6600
   atgcgcacgc tcgggtcgtt gggcagcccg atgacagcga ccacgctctt gaagccctgt 6660
   gcctccaggg acttcagcag gtgggtgtag agcgtggagc ccagtcccgt ccgctggtgg 6720
   cggggggaga cgtacacggt cgactcggcc gtccagtcgt aggcgttgcg tgccttccag 6780
   gggcccgcgt aggcgatgcc ggcgacctcg ccgtccacct cggcgacgag ccagggatag 6840
   cgctcccgca gacggacgag gtcgtccgtc cactcctgcg gttcctgcgg ctcggtacgg 6900
   aagttgaccg tgcttgtctc gatgtagtgg ttgacgatgg tgcagaccgc cggcatgtcc 6960
   gcctcggtgg cacggcggat gtcggccggg cgtcgttctg ggctcatggt agactcgaga 7020
   gagatagatt tgtagagaga gactggtgat ttcagcgtgt cctctccaaa tgaaatgaac 7080
   ttccttatat agaggaaggt cttgcgaagg atagtgggat tgtgcgtcat cccttacgtc 7140
   agtggagata tcacatcaat ccacttgctt tgaagacgtg gttggaacgt cttctttttc 7200
   cacgatgctc ctcgtgggtg ggggtccatc tttgggacca ctgtcggcag aggcatcttg 7260
   aacgatagcc tttcctttat cgcaatgatg gcatttgtag gtgccacctt ccttttctac 7320
   tgtccttttg atgaagtgac agatagctgg gcaatggaat ccgaggaggt ttcccgatat 7380
   taccctttgt tgaaaagtct caatagccct ttggtcttct gagactgtat ctttgatatt 7440
   cttggagtag acgagagtgt cgtgctccac catgttatca catcaatcca cttgctttga 7500
   agacgtggtt ggaacgtctt ctttttccac gatgctcctc gtgggtgggg gtccatcttt 7560
   gggaccactg tcggcagagg catcttgaac gatagccttt cctttatcgc aatgatggca 7620
   tttgtaggtg ccaccttcct tttctactgt ccttttgatg aagtgacaga tagctgggca 7680
   atggaatccg aggaggtttc ccgatattac cctttgttga aaagtctcaa tagccctttg 7740
   gtcttctgag actgtatctt tgatattctt ggagtagacg agagtgtcgt gctccaccat 7800
   gttggcaagc tgctctagcc aatacgcaaa ccgcctctcc ccgcgcgttg gccgattcat 7860
   taatgcagct ggcacgacag gtttcccgac tggaaagcgg gcagtgagcg caacgcaatt 7920
   aatgtgagtt agctcactca ttaggcaccc caggctttac actttatgct tccggctcgt 7980
   atgttgtgtg gaattgtgag cggataacaa tttcacacag gaaacagcta tgaccatgat 8040
   tacgaattcg agccttgact agagggtcga cggtatacag acatgataag atacattgat 8100
   gagtttggac aaaccacaac tagaatgcag tgaaaaaaat gctttatttg tgaaatttgt 8160
   gatgctattg ctttatttgt aaccattata agctgcaata aacaagttgg ggtgggcgaa 8220
   gaactccagc atgagatccc cgcgctggag gatcatccag ccggcgtccc ggaaaacgat 8280
   tccgaagccc aacctttcat agaaggcggc ggtggaatcg aaatctcgta gcacgtgtca 8340
   gtcctgctcc tcggccacga agtgcacgca gttgccggcc gggtcgcgca gggcgaactc 8400
   ccgcccccac ggctgctcgc cgatctcggt catggccggc ccggaggcgt cccggaagtt 8460
   cgtggacacg acctccgacc actcggcgta cagctcgtcc aggccgcgca cccacaccca 8520
   ggccagggtg ttgtccggca ccacctggtc ctggaccgcg ctgatgaaca gggtcacgtc 8580
   gtcccggacc acaccggcga agtcgtcctc cacgaagtcc cgggagaacc cgagccggtc 8640
   ggtccagaac tcgaccgctc cggcgacgtc gcgcgcggtg agcaccggaa cggcactggt 8700
   caacttggcc atggatccag atttcgctca agttagtata aaaaagcagg cttcaatcct 8760
   gcaggaattc gatcgacact ctcgtctact ccaagaatat caaagataca gtctcagaag 8820
   accaaagggc tattgagact tttcaacaaa gggtaatatc gggaaacctc ctcggattcc 8880
   attgcccagc tatctgtcac ttcatcaaaa ggacagtaga aaaggaaggt ggcacctaca 8940
   aatgccatca ttgcgataaa ggaaaggcta tcgttcaaga tgcctctgcc gacagtggtc 9000
   ccaaagatgg acccccaccc acgaggagca tcgtggaaaa agaagacgtt ccaaccacgt 9060
   cttcaaagca agtggattga tgtgataaca tggtggagca cgacactctc gtctactcca 9120
   agaatatcaa agatacagtc tcagaagacc aaagggctat tgagactttt caacaaaggg 9180
   taatatcggg aaacctcctc ggattccatt gcccagctat ctgtcacttc atcaaaagga 9240
   cagtagaaaa ggaaggtggc acctacaaat gccatcattg cgataaagga aaggctatcg 9300
   ttcaagatgc ctctgccgac agtggtccca aagatggacc cccacccacg aggagcatcg 9360
   tggaaaaaga agacgttcca accacgtctt caaagcaagt ggattgatgt gatatctcca 9420
   ctgacgtaag ggatgacgca caatcccact atccttcgca agacccttcc ctatataagg 9480
   aagttcattt catttggaga ggacacgctg aaatcaccag tctctctcta caaatctatc 9540
   tctctcgagc tttcgcagat ccgggggggc aatgagatat gaaaaagcct gaactcaccg 9600
   cgacgtctgt cgagaagttt ctgatcgaaa agttcgacag cgtctccgac ctgatgcagc 9660
   tctcggaggg cgaagaatct cgtgctttca gcttcgatgt aggagggcgt ggatatgtcc 9720
   tgcgggtaaa tagctgcgcc gatggtttct acaaagatcg ttatgtttat cggcactttg 9780
   catcggccgc gctcccgatt ccggaagtgc ttgacattgg ggagtttagc gagagcctga 9840
   cctattgcat ctcccgccgt gcacagggtg tcacgttgca agacctgcct gaaaccgaac 9900
   tgcccgctgt tctacaaccg gtcgcggagg ctatggatgc gatcgctgcg gccgatctta 9960
   gccagacgag cgggttcggc ccattcggac cgcaaggaat cggtcaatac actacatggc 10020
   gtgatttcat atgcgcgatt gctgatcccc atgtgtatca ctggcaaact gtgatggacg 10080
   acaccgtcag tgcgtccgtc gcgcaggctc tcgatgagct gatgctttgg gccgaggact 10140
   gccccgaagt ccggcacctc gtgcacgcgg atttcggctc caacaatgtc ctgacggaca 10200
   atggccgcat aacagcggtc attgactgga gcgaggcgat gttcggggat tcccaatacg 10260
   aggtcgccaa catcttcttc tggaggccgt ggttggcttg tatggagcag cagacgcgct 10320
   acttcgagcg gaggcatccg gagcttgcag gatcgccacg actccgggcg tatatgctcc 10380
   gcattggtct tgaccaactc tatcagagct tggttgacgg caatttcgat gatgcagctt 10440
   gggcgcaggg tcgatgcgac gcaatcgtcc gatccggagc cgggactgtc gggcgtacac 10500
   aaatcgcccg cagaagcgcg gccgtctgga ccgatggctg tgtagaagta ctcgccgata 10560
   gtggaaaccg acgccccagc actcgtccga gggcaaagaa atagagtaga tgccgaccgg 10620
   atctgtcgat cgacaagctc gagtttctcc ataataatgt gtgagtagtt cccagataag 10680
   ggaattaggg ttcctatagg gtttcgctca tgtgttgagc atataagaaa cccttagtat 10740
   gtatttgtat ttgtaaaata cttctatcaa taaaatttct aattcctaaa accaaaatcc 10800
   agtactaaaa tccagatccc ccgaattaat tcggcgttaa ttcagatcaa gcttgacctg 10860
   gaatatcgcg agtaaactga aaatcacgga aaatgagaaa tacacacttt aggacgtgaa 10920
   atatggcgag gaaaactgaa aaaggtggaa aatttagaaa tgtccactgt aggacgtgga 10980
   atatggcaag aaaactgaaa atcatggaaa atgagaaaca tccacttgac gacttgaaaa 11040
   atgacgaaat cactaaaaaa cgtgaaaaat gagaaatgca cactgaagga ctccgcggga 11100
   attcgattgt gctagccaat gtttaacaag atgtcaagca caatgaatgt tggtggttgg 11160
   tggtcgtggc tggcggtggt ggaaaattgc ggtggttcga gcggtagtga tcggcgatgg 11220
   ttggtgtttg cagcggtgtt tgatatcgga atcacttatg gtggttgtca caatggaggt 11280
   gcgtcatggt tattggtggt tggtcatcta tatattttta taataatatt aagtatttta 11340
   cctatttttt acatattttt tattaaattt atgcattgtt tgtattttta aatagttttt 11400
   atcgtacttg ttttataaaa tattttatta ttttatgtgt tatattatta cttgatgtat 11460
   tggaaatttt ctccattgtt ttttctatat ttataataat tttcttattt ttttttgttt 11520
   tattatgtat tttttcgttt tataataaat atttattaaa aaaaatatta tttttgtaaa 11580
   atatatcatt tacaatgttt aaaagtcatt tgtgaatata ttagctaagt tgtacttctt 11640
   tttgtgcatt tggtgttgta catgtctatt atgattctct ggccaaaaca tgtctactcc 11700
   tgtcacttgg gttttttttt ttaagacata atcactagtg attatatcta gactgaaggc 11760
   gggaaacgac aatctgatca tgagcggaga attaagggag tcacgttatg acccccgccg 11820
   atgacgcggg acaagccgtt ttacgtttgg aactgacaga accgcaacgt tgaaggagcc 11880
   actcagccgc gggtttctgg agtttaatga gctaagcaca tacgtcagaa accattattg 11940
   cgcgttcaaa agtcgcctaa ggtcactatc agctagcaaa tatttcttgt caaaaatgct 12000
   ccactgacgt tccataaatt cccctcggta tccaattaga gtctcatatt cactctcaat 12060
   ccaaataatc tgcaccggat ctcgagatcg aattcccgcg gccgcgaatt cactagtgga 12120
   tccccgggta cggtcagtcc cttatgttac gtcctgtaga aaccccaacc cgtgaaatca 12180
   aaaaactcga cggcctgtgg gcattcagtc tggatcgcga aaactgtgga attgagcagc 12240
   gttggtggga aagcgcgtta caagaaagcc gggcaattgc tgtgccaggc agttttaacg 12300
   atcagttcgc cgatgcagat attcgtaatt atgtgggcaa cgtctggtat cagcgcgaag 12360
   tctttatacc gaaaggttgg gcaggccagc gtatcgtgct gcgtttcgat gcggtcactc 12420
   attacggcaa agtgtgggtc aataatcagg aagtgatgga gcatcagggc ggctatacgc 12480
   catttgaagc cgatgtcacg ccgtatgtta ttgccgggaa aagtgtacgt atcacagttt 12540
   gtgtgaacaa cgaactgaac tggcagacta tcccgccggg aatggtgatt accgacgaaa 12600
   acggcaagaa aaagcagtct tacttccatg atttctttaa ctacgccggg atccatcgca 12660
   gcgtaatgct ctacaccacg ccgaacacct gggtggacga tatcaccgtg gtgacgcatg 12720
   tcgcgcaaga ctgtaaccac gcgtctgttg actggcaggt ggtggccaat ggtgatgtca 12780
   gcgttgaact gcgtgatgcg gatcaacagg tggttgcaac tggacaaggc accagcggga 12840
   ctttgcaagt ggtgaatccg cacctctggc aaccgggtga aggttatctc tatgaactgt 12900
   acgtcacagc caaaagccag acagagtgtg atatctaccc gctgcgcgtc ggcatccggt 12960
   cagtggcagt gaagggcgaa cagttcctga tcaaccacaa accgttctac tttactggct 13020
   ttggccgtca tgaagatgcg gatttgcgcg gcaaaggatt cgataacgtg ctgatggtgc 13080
   acgatcacgc attaatggac tggattgggg ccaactccta ccgtacctcg cattaccctt 13140
   acgctgaaga gatgctcgac tgggcagatg aacatggcat cgtggtgatt gatgaaactg 13200
   cagctgtcgg ctttaacctc tctttaggca ttggtttcga agcgggcaac aagccgaaag 13260
   aactgtacag cgaagaggca gtcaacgggg aaactcagca ggcgcactta caggcgatta 13320
   aagagctgat agcgcgtgac aaaaaccacc caagcgtggt gatgtggagt attgccaacg 13380
   aaccggatac ccgtccgcaa ggtgcacggg aatatttcgc gccactggcg gaagcaacgc 13440
   gtaaactcga tccgacgcgt ccgatcacct gcgtcaatgt aatgttctgc gacgctcaca 13500
   ccgataccat cagcgatctc tttgatgtgc tgtgcctgaa ccgttattac ggttggtatg 13560
   tccaaagcgg cgatttggaa acggcagaga aggtactgga aaaagaactt ctggcctggc 13620
   aggagaaact gcatcagccg attatcatca ccgaatacgg cgtggatacg ttagccgggc 13680
   tgcactcaat gtacaccgac atgtggagtg aagagtatca gtgtgcatgg ctggatatgt 13740
   atcaccgcgt ctttgatcgc gtcagcgccg tcgtcggtga acaggtatgg aatttcgccg 13800
   attttgcgac ctcgcaaggc atattgcgcg ttggcggtaa caagaagggg atcttcaccc 13860
   gcgaccgcaa accgaagtcg gcggcttttc tgctgcaaaa acgctggact ggcatgaact 13920
   tcggtgaaaa accgcagcag ggaggcaaac aatgaatcaa caactctcct ggcgcaccat 13980
   cgtcggctac agcctcggga attgcgtacc gagctcgaat ttccccgatc gttcaaacat 14040
   ttggcaataa agtttcttaa gattgaatcc tgttgccggt cttgcgatga ttatcatata 14100
   atttctgttg aattacgtta agcatgtaat aattaacatg taatgcatga cgttatttat 14160
   gagatgggtt tttatgatta gagtcccgca attatacatt taatacgcga tagaaaacaa 14220
   aatatagcgc gcaaactagg ataaattatc gcgcgcggtg tcatctatgt tactagatcg 14280
   ggaattcgat atcaagcttg gcactggccg tcgttttaca acgtcgtgac tgggaaaacc 14340
   ctggcgttac ccaacttaat cgccttgcag cacatccccc tttcgccagc tggcgtaata 14400
   gcgaagaggc ccgcaccgat cgcccttccc aacagttgcg cagcctgaat ggcgaatgct 14460
   agagcagctt gagcttggat cagattgtcg tttcccgcct tcagtttaaa ctatcagtgt 14520
   ttgacaggat atattggcgg gtaaacctaa gagaaaagag cgtttattag aataacggat 14580
   atttaaaagg gcgtgaaaag gtttatccgt tcgtccattt gtatgtg 14627

<210> 22
<211> 4257
<212> DNA
<213> Artificial Sequence

<220>
<223> pPUR Plasmid

<400> 22
ctgtggaatg tgtgtcagtt agggtgtgga aagtccccag gctccccagc aggcagaagt 60
atgcaaagca tgcatctcaa ttagtcagca accaggtgtg gaaagtcccc aggctcccca 120
gcaggcagaa gtatgcaaag catgcatctc aattagtcag caaccatagt cccgccccta 180
actccgccca tcccgcccct aactccgccc agttccgccc attctccgcc ccatggctga 240
ctaatttttt ttatttatgc agaggccgag gccgcctcgg cctctgagct attccagaag 300
tagtgaggag gcttttttgg aggcctaggc ttttgcaaaa agcttgcatg cctgcaggtc 360
ggccgccacg accggtgccg ccaccatccc ctgacccacg cccctgaccc ctcacaagga 420
gacgaccttc catgaccgag tacaagccca cggtgcgcct cgccacccgc gacgacgtcc 480

cccgggccgt acgcaccctc gccgccgcgt tcgccgacta ccccgccacg cgccacaccg 540
tcgacccgga ccgccacatc gagcgggtca ccgagctgca agaactcttc ctcacgcgcg 600
tcgggctcga catcggcaag gtgtgggtcg cggacgacgg cgccgcggtg gcggtctgga 660
ccacgccgga gagcgtcgaa gcgggggcgg tgttcgccga gatcggcccg cgcatggccg 720
agttgagcgg ttcccggctg gccgcgcagc aacagatgga aggcctcctg gcgccgcacc 780
ggcccaagga gcccgcgtgg ttcctggcca ccgtcggcgt ctcgcccgac caccagggca 840
agggtctggg cagcgccgtc gtgctccccg gagtggaggc ggccgagcgc gccggggtgc 900
ccgccttcct ggagacctcc gcgccccgca acctcccctt ctacgagcgg ctcggcttca 960
ccgtcaccgc cgacgtcgag gtgcccgaag gaccgcgcac ctggtgcatg acccgcaagc 1020
ccggtgcctg acgcccgccc cacgacccgc agcgcccgac cgaaaggagc gcacgacccc 1080
atggctccga ccgaagccga cccgggcggc cccgccgacc ccgcacccgc ccccgaggcc 1140
caccgactct agaggatcat aatcagccat accacatttg tagaggtttt acttgcttta 1200
aaaaacctcc cacacctccc cctgaacctg aaacataaaa tgaatgcaat tgttgttgtt 1260
aacttgttta ttgcagctta taatggttac aaataaagca atagcatcac aaatttcaca 1320
aataaagcat ttttttcact gcattctagt tgtggtttgt ccaaactcat caatgtatct 1380
tatcatgtct ggatccccag gaagctcctc tgtgtcctca taaaccctaa cctcctctac 1440
ttgagaggac attccaatca taggctgccc atccaccctc tgtgtcctcc tgttaattag 1500
gtcacttaac aaaaaggaaa ttgggtaggg gtttttcaca gaccgctttc taagggtaat 1560
tttaaaatat ctgggaagtc ccttccactg ctgtgttcca gaagtgttgg taaacagccc 1620
acaaatgtca acagcagaaa catacaagct gtcagctttg cacaagggcc caacaccctg 1680
ctcatcaaga agcactgtgg ttgctgtgtt agtaatgtgc aaaacaggag gcacattttc 1740
cccacctgtg taggttccaa aatatctagt gttttcattt ttacttggat caggaaccca 1800
gcactccact ggataagcat tatccttatc caaaacagcc ttgtggtcag tgttcatctg 1860
ctgactgtca actgtagcat tttttggggt tacagtttga gcaggatatt tggtcctgta 1920
gtttgctaac acaccctgca gctccaaagg ttccccacca acagcaaaaa aatgaaaatt 1980
tgacccttga atgggttttc cagcaccatt ttcatgagtt ttttgtgtcc ctgaatgcaa 2040
gtttaacata gcagttaccc caataacctc agttttaaca gtaacagctt cccacatcaa 2100
aatatttcca caggttaagt cctcatttaa attaggcaaa ggaattcttg aagacgaaag 2160
ggcctcgtga tacgcctatt tttataggtt aatgtcatga taataatggt ttcttagacg 2220
tcaggtggca cttttcgggg aaatgtgcgc ggaaccccta tttgtttatt tttctaaata 2280
cattcaaata tgtatccgct catgagacaa taaccctgat aaatgcttca ataatattga 2340
aaaaggaaga gtatgagtat tcaacatttc cgtgtcgccc ttattccctt ttttgcggca 2400
ttttgccttc ctgtttttgc tcacccagaa acgctggtga aagtaaaaga tgctgaagat 2460
cagttgggtg cacgagtggg ttacatcgaa ctggatctca acagcggtaa gatccttgag 2520
agttttcgcc ccgaagaacg ttttccaatg atgagcactt ttaaagttct gctatgtggc 2580
gcggtattat cccgtgttga cgccgggcaa gagcaactcg gtcgccgcat acactattct 2640
cagaatgact tggttgagta ctcaccagtc acagaaaagc atcttacgga tggcatgaca 2700
gtaagagaat tatgcagtgc tgccataacc atgagtgata acactgcggc caacttactt 2760
ctgacaacga tcggaggacc gaaggagcta accgcttttt tgcacaacat gggggatcat 2820
gtaactcgcc ttgatcgttg ggaaccggag ctgaatgaag ccataccaaa cgacgagcgt 2880
gacaccacga tgcctgcagc aatggcaaca acgttgcgca aactattaac tggcgaacta 2940
cttactctag cttcccggca acaattaata gactggatgg aggcggataa agttgcagga 3000
ccacttctgc gctcggccct tccggctggc tggtttattg ctgataaatc tggagccggt 3060
gagcgtgggt ctcgcggtat cattgcagca ctggggccag atggtaagcc ctcccgtatc 3120
gtagttatct acacgacggg gagtcaggca actatggatg aacgaaatag acagatcgct 3180
gagataggtg cctcactgat taagcattgg taactgtcag accaagttta ctcatatata 3240
ctttagattg atttaaaact tcatttttaa tttaaaagga tctaggtgaa gatccttttt 3300
gataatctca tgaccaaaat cccttaacgt gagttttcgt tccactgagc gtcagacccc 3360
gtagaaaaga tcaaaggatc ttcttgagat cctttttttc tgcgcgtaat ctgctgcttg 3420
caaacaaaaa aaccaccgct accagcggtg gtttgtttgc cggatcaaga gctaccaact 3480
ctttttccga aggtaactgg cttcagcaga gcgcagatac caaatactgt ccttctagtg 3540
tagccgtagt taggccacca cttcaagaac tctgtagcac cgcctacata cctcgctctg 3600
ctaatcctgt taccagtggc tgctgccagt ggcgataagt cgtgtcttac cgggttggac 3660
tcaagacgat agttaccgga taaggcgcag cggtcgggct gaacgggggg ttcgtgcaca 3720
cagcccagct tggagcgaac gacctacacc gaactgagat acctacagcg tgagctatga 3780
gaaagcgcca cgcttcccga agggagaaag gcggacaggt atccggtaag cggcagggtc 3840
ggaacaggag agcgcacgag ggagcttcca gggggaaacg cctggtatct ttatagtcct 3900
gtcgggtttc gccacctctg acttgagcgt cgatttttgt gatgctcgtc aggggggcgg 3960
agcctatgga aaaacgccag caacgcggcc tttttacggt tcctggcctt ttgctggcct 4020
tttgctcaca tgttctttcc tgcgttatcc cctgattctg tggataaccg tattaccgcc 4080
tttgagtgag ctgataccgc tcgccgcagc cgaacgaccg agcgcagcga gtcagtgagc 4140

gaggaagcgg aagagcgcct gatgcggtat tttctcctta cgcatctgtg cggtatttca 4200
caccgcatat ggtgcactct cagtacaatc tgctctgatg ccgcatagtt aagccag 4257

<210> 23
<211> 2713
<212> DNA
<213> Artificial Sequence

<220>
<223> pNEB193 Plasmid

<400> 23
tcgcgcgttt cggtgatgac ggtgaaaacc tctgacacat gcagctcccg gagacggtca 60
cagcttgtct gtaagcggat gccgggagca gacaagcccg tcagggcgcg tcagcgggtg 120
ttggcgggtg tcggggctgg cttaactatg cggcatcaga gcagattgta ctgagagtgc 180
accatatgcg gtgtgaaata ccgcacagat gcgtaaggag aaaataccgc atcaggcgcc 240
attcgccatt caggctgcgc aactgttggg aagggcgatc ggtgcgggcc tcttcgctat 300
tacgccagct ggcgaaaggg ggatgtgctg caaggcgatt aagttgggta acgccagggt 360
tttcccagtc acgacgttgt aaaacgacgg ccagtgaatt cgagctcggt acccgggggc 420
gcgccggatc cttaattaag tctagagtcg actgtttaaa cctgcaggca tgcaagcttg 480
gcgtaatcat ggtcatagct gtttcctgtg tgaaattgtt atccgctcac aattccacac 540
aacatacgag ccggaagcat aaagtgtaaa gcctggggtg cctaatgagt gagctaactc 600
acattaattg cgttgcgctc actgcccgct ttccagtcgg gaaacctgtc gtgccagctg 660
cattaatgaa tcggccaacg cgcggggaga ggcggtttgc gtattgggcg ctcttccgct 720
tcctcgctca ctgactcgct gcgctcggtc gttcggctgc ggcgagcggt atcagctcac 780
tcaaaggcgg taatacggtt atccacagaa tcaggggata acgcaggaaa gaacatgtga 840
gcaaaaggcc agcaaaaggc caggaaccgt aaaaaggccg cgttgctggc gtttttccat 900
aggctccgcc cccctgacga gcatcacaaa aatcgacgct caagtcagag gtggcgaaac 960
ccgacaggac tataaagata ccaggcgttt ccccctggaa gctccctcgt gcgctctcct 1020
gttccgaccc tgccgcttac cggatacctg tccgcctttc tcccttcggg aagcgtggcg 1080
ctttctcata gctcacgctg taggtatctc agttcggtgt aggtcgttcg ctccaagctg 1140
ggctgtgtgc acgaaccccc cgttcagccc gaccgctgcg ccttatccgg taactatcgt 1200
cttgagtcca acccggtaag acacgactta tcgccactgg cagcagccac tggtaacagg 1260
attagcagag cgaggtatgt aggcggtgct acagagttct tgaagtggtg gcctaactac 1320
ggctacacta gaaggacagt atttggtatc tgcgctctgc tgaagccagt taccttcgga 1380
aaaagagttg gtagctcttg atccggcaaa caaaccaccg ctggtagcgg tggttttttt 1440
gtttgcaagc agcagattac gcgcagaaaa aaaggatctc aagaagatcc tttgatcttt 1500
tctacggggt ctgacgctca gtggaacgaa aactcacgtt aagggatttt ggtcatgaga 1560
ttatcaaaaa ggatcttcac ctagatcctt ttaaattaaa aatgaagttt taaatcaatc 1620
taaagtatat atgagtaaac ttggtctgac agttaccaat gcttaatcag tgaggcacct 1680
atctcagcga tctgtctatt tcgttcatcc atagttgcct gactccccgt cgtgtagata 1740
actacgatac gggagggctt accatctggc cccagtgctg caatgatacc gcgagaccca 1800
cgctcaccgg ctccagattt atcagcaata aaccagccag ccggaagggc cgagcgcaga 1860
agtggtcctg caactttatc cgcctccatc cagtctatta attgttgccg ggaagctaga 1920
gtaagtagtt cgccagttaa tagtttgcgc aacgttgttg ccattgctac aggcatcgtg 1980
gtgtcacgct cgtcgtttgg tatggcttca ttcagctccg gttcccaacg atcaaggcga 2040
gttacatgat cccccatgtt gtgcaaaaaa gcggttagct ccttcggtcc tccgatcgtt 2100
gtcagaagta agttggccgc agtgttatca ctcatggtta tggcagcact gcataattct 2160
cttactgtca tgccatccgt aagatgcttt tctgtgactg gtgagtactc aaccaagtca 2220
ttctgagaat agtgtatgcg gcgaccgagt tgctcttgcc cggcgtcaat acgggataat 2280
accgcgccac atagcagaac tttaaaagtg ctcatcattg gaaaacgttc ttcggggcga 2340
aaactctcaa ggatcttacc gctgttgaga tccagttcga tgtaacccac tcgtgcaccc 2400
aactgatctt cagcatcttt tactttcacc agcgtttctg ggtgagcaaa aacaggaagg 2460
caaaatgccg caaaaaaggg aataagggcg acacggaaat gttgaatact catactcttc 2520
ctttttcaat attattgaag catttatcag ggttattgtc tcatgagcgg atacatattt 2580
gaatgtattt agaaaaataa acaaataggg gttccgcgca catttccccg aaaagtgcca 2640
cctgacgtct aagaaaccat tattatcatg acattaacct ataaaaatag gcgtatcacg 2700
aggccctttc gtc
2713

<210> 24
<211> 25

<212> DNA
<213> Artificial Sequence

<220>
<223> attPUP Primer

<400> 24
ccttgcgcta atgctctgtt acagg 25

<210> 25
<211> 26
<212> DNA
<213> Artificial Sequence

<220>
<223> attPDWN Primer

<400> 25
cagaggcagg gagtgggaca aaattg 26

<210> 26
<211> 4346
<212> DNA
<213> Artificial Sequence

<220>
<223> pSV40193attPsensePUR Plasmid

<400> 26
ccggtgccgc caccatcccc tgacccacgc ccctgacccc tcacaaggag acgaccttcc 60
atgaccgagt acaagcccac ggtgcgcctc gccacccgcg acgacgtccc ccgggccgta 120
cgcaccctcg ccgccgcgtt cgccgactac cccgccacgc gccacaccgt cgacccggac 180
cgccacatcg agcgggtcac cgagctgcaa gaactcttcc tcacgcgcgt cgggctcgac 240
atcggcaagg tgtgggtcgc ggacgacggc gccgcggtgg cggtctggac cacgccggag 300
agcgtcgaag cgggggcggt gttcgccgag atcggcccgc gcatggccga gttgagcggt 360
tcccggctgg ccgcgcagca acagatggaa ggcctcctgg cgccgcaccg gcccaaggag 420
cccgcgtggt tcctggccac cgtcggcgtc tcgcccgacc accagggcaa gggtctgggc 480
agcgccgtcg tgctccccgg agtggaggcg gccgagcgcg ccggggtgcc cgccttcctg 540
gagacctccg cgccccgcaa cctccccttc tacgagcggc tcggcttcac cgtcaccgcc 600
gacgtcgagg tgcccgaagg accgcgcacc tggtgcatga cccgcaagcc cggtgcctga 660
cgcccgcccc acgacccgca gcgcccgacc gaaaggagcg cacgacccca tggctccgac 720
cgaagccgac ccgggcggcc ccgccgaccc cgcacccgcc cccgaggccc accgactcta 780
gaggatcata atcagccata ccacatttgt agaggtttta cttgctttaa aaaacctccc 840
acacctcccc ctgaacctga aacataaaat gaatgcaatt gttgttgtta acttgtttat 900
tgcagcttat aatggttaca aataaagcaa tagcatcaca aatttcacaa ataaagcatt 960
tttttcactg cattctagtt gtggtttgtc caaactcatc aatgtatctt atcatgtctg 1020
gatccgcgcc ggatccttaa ttaagtctag agtcgactgt ttaaacctgc aggcatgcaa 1080
gcttggcgta atcatggtca tagctgtttc ctgtgtgaaa ttgttatccg ctcacaattc 1140
cacacaacat acgagccgga agcataaagt gtaaagcctg gggtgcctaa tgagtgagct 1200
aactcacatt aattgcgttg cgctcactgc ccgctttcca gtcgggaaac ctgtcgtgcc 1260
agctgcatta atgaatcggc caacgcgcgg ggagaggcgg tttgcgtatt gggcgctctt 1320
ccgcttcctc gctcactgac tcgctgcgct cggtcgttcg gctgcggcga gcggtatcag 1380
ctcactcaaa ggcggtaata cggttatcca cagaatcagg ggataacgca ggaaagaaca 1440
tgtgagcaaa aggccagcaa aaggccagga accgtaaaaa ggccgcgttg ctggcgtttt 1500
tccataggct ccgcccccct gacgagcatc acaaaaatcg acgctcaagt cagaggtggc 1560
gaaacccgac aggactataa agataccagg cgtttccccc tggaagctcc ctcgtgcgct 1620
ctcctgttcc gaccctgccg cttaccggat acctgtccgc ctttctccct tcgggaagcg 1680
tggcgctttc tcatagctca cgctgtaggt atctcagttc ggtgtaggtc gttcgctcca 1740
agctgggctg tgtgcacgaa ccccccgttc agcccgaccg ctgcgcctta tccggtaact 1800
atcgtcttga gtccaacccg gtaagacacg acttatcgcc actggcagca gccactggta 1860
acaggattag cagagcgagg tatgtaggcg gtgctacaga gttcttgaag tggtggccta 1920

actacggcta cactagaagg acagtatttg gtatctgcgc tctgctgaag ccagttacct 1980
tcggaaaaag agttggtagc tcttgatccg gcaaacaaac caccgctggt agcggtggtt 2040
tttttgtttg caagcagcag attacgcgca gaaaaaaagg atctcaagaa gatcctttga 2100
tcttttctac ggggtctgac gctcagtgga acgaaaactc acgttaaggg attttggtca 2160
tgagattatc aaaaaggatc ttcacctaga tccttttaaa ttaaaaatga agttttaaat 2220
caatctaaag tatatatgag taaacttggt ctgacagtta ccaatgctta atcagtgagg 2280
cacctatctc agcgatctgt ctatttcgtt catccatagt tgcctgactc cccgtcgtgt 2340
agataactac gatacgggag ggcttaccat ctggccccag tgctgcaatg ataccgcgag 2400
acccacgctc accggctcca gatttatcag caataaacca gccagccgga agggccgagc 2460
gcagaagtgg tcctgcaact ttatccgcct ccatccagtc tattaattgt tgccgggaag 2520
ctagagtaag tagttcgcca gttaatagtt tgcgcaacgt tgttgccatt gctacaggca 2580
tcgtggtgtc acgctcgtcg tttggtatgg cttcattcag ctccggttcc caacgatcaa 2640
ggcgagttac atgatccccc atgttgtgca aaaaagcggt tagctccttc ggtcctccga 2700
tcgttgtcag aagtaagttg gccgcagtgt tatcactcat ggttatggca gcactgcata 2760
attctcttac tgtcatgcca tccgtaagat gcttttctgt gactggtgag tactcaacca 2820
agtcattctg agaatagtgt atgcggcgac cgagttgctc ttgcccggcg tcaatacggg 2880
ataataccgc gccacatagc agaactttaa aagtgctcat cattggaaaa cgttcttcgg 2940
ggcgaaaact ctcaaggatc ttaccgctgt tgagatccag ttcgatgtaa cccactcgtg 3000
cacccaactg atcttcagca tcttttactt tcaccagcgt ttctgggtga gcaaaaacag 3060
gaaggcaaaa tgccgcaaaa aagggaataa gggcgacacg gaaatgttga atactcatac 3120
tcttcctttt tcaatattat tgaagcattt atcagggtta ttgtctcatg agcggataca 3180
tatttgaatg tatttagaaa aataaacaaa taggggttcc gcgcacattt ccccgaaaag 3240
tgccacctga cgtctaagaa accattatta tcatgacatt aacctataaa aataggcgta 3300
tcacgaggcc ctttcgtctc gcgcgtttcg gtgatgacgg tgaaaacctc tgacacatgc 3360
agctcccgga gacggtcaca gcttgtctgt aagcggatgc cgggagcaga caagcccgtc 3420
agggcgcgtc agcgggtgtt ggcgggtgtc ggggctggct taactatgcg gcatcagagc 3480
agattgtact gagagtgcac catatgcggt gtgaaatacc gcacagatgc gtaaggagaa 3540
aataccgcat caggcgccat tcgccattca ggctgcgcaa ctgttgggaa gggcgatcgg 3600
tgcgggcctc ttcgctatta cgccagctgg cgaaaggggg atgtgctgca aggcgattaa 3660
gttgggtaac gccagggttt tcccagtcac gacgttgtaa aacgacggcc agtgaattcg 3720
agctgtggaa tgtgtgtcag ttagggtgtg gaaagtcccc aggctcccca gcaggcagaa 3780
gtatgcaaag catgcatctc aattagtcag caaccaggtg tggaaagtcc ccaggctccc 3840
cagcaggcag aagtatgcaa agcatgcatc tcaattagtc agcaaccata gtcccgcccc 3900
taactccgcc catcccgccc ctaactccgc ccagttccgc ccattctccg ccccatggct 3960
gactaatttt ttttatttat gcagaggccg aggccgcctc ggcctctgag ctattccaga 4020
agtagtgagg aggctttttt ggaggctcgg tacccccttg cgctaatgct ctgttacagg 4080
tcactaatac catctaagta gttgattcat agtgactgca tatgttgtgt tttacagtat 4140
tatgtagtct gttttttatg caaaatctaa tttaatatat tgatatttat atcattttac 4200
gtttctcgtt cagctttttt atactaagtt ggcattataa aaaagcattg cttatcaatt 4260
tgttgcaacg aacaggtcac tatcagtcaa aataaaatca ttatttgatt tcaattttgt 4320
cccactccct gcctctgggg ggcgcg 4346

<210> 27
<211> 5855
<212> DNA
<213> Artificial Sequence

<220>
<223> pCXLamIntR Plasmid

<400> 27
gtcgacattg attattgact agttattaat agtaatcaat tacggggtca ttagttcata 60
gcccatatat ggagttccgc gttacataac ttacggtaaa tggcccgcct ggctgaccgc 120
ccaacgaccc ccgcccattg acgtcaataa tgacgtatgt tcccatagta acgccaatag 180
ggactttcca ttgacgtcaa tgggtggact atttacggta aactgcccac ttggcagtac 240
atcaagtgta tcatatgcca agtacgcccc ctattgacgt caatgacggt aaatggcccg 300
cctggcatta tgcccagtac atgaccttat gggactttcc tacttggcag tacatctacg 360
tattagtcat cgctattacc atgggtcgag gtgagcccca cgttctgctt cactctcccc 420
atctcccccc cctccccacc cccaattttg tatttattta ttttttaatt attttgtgca 480
gcgatggggg cggggggggg gggggcgcgc gccaggcggg gcggggcggg gcgaggggcg 540
gggcggggcg aggcggagag gtgcggcggc agccaatcag agcggcgcgc tccgaaagtt 600

tccttttatg gcgaggcggc ggcggcggcg gccctataaa aagcgaagcg cgcggcgggc 660
gggagtcgct gcgttgcctt cgccccgtgc cccgctccgc gccgcctcgc gccgcccgcc 720
ccggctctga ctgaccgcgt tactcccaca ggtgagcggg cgggacggcc cttctcctcc 780
gggctgtaat tagcgcttgg tttaatgacg gctcgtttct tttctgtggc tgcgtgaaag 840
ccttaaaggg ctccgggagg gccctttgtg cgggggggag cggctcgggg ggtgcgtgcg 900
tgtgtgtgtg cgtggggagc gccgcgtgcg gcccgcgctg cccggcggct gtgagcgctg 960
cgggcgcggc gcggggcttt gtgcgctccg cgtgtgcgcg aggggagcgc ggccgggggc 1020
ggtgccccgc ggtgcggggg ggctgcgagg ggaacaaagg ctgcgtgcgg ggtgtgtgcg 1080
tgggggggtg agcagggggt gtgggcgcgg cggtcgggct gtaacccccc cctgcacccc 1140
cctccccgag ttgctgagca cggcccggct tcgggtgcgg ggctccgtgc ggggcgtggc 1200
gcggggctcg ccgtgccggg cggggggtgg cggcaggtgg gggtgccggg cggggcgggg 1260
ccgcctcggg ccggggaggg ctcgggggag gggcgcggcg gccccggagc gccggcggct 1320
gtcgaggcgc ggcgagccgc agccattgcc ttttatggta atcgtgcgag agggcgcagg 1380
gacttccttt gtcccaaatc tggcggagcc gaaatctggg aggcgccgcc gcaccccctc 1440
tagcgggcgc gggcgaagcg gtgcggcgcc ggcaggaagg aaatgggcgg ggagggcctt 1500
cgtgcgtcgc cgcgccgccg tccccttctc catctccagc ctcggggctg ccgcaggggg 1560
acggctgcct tcggggggga cggggcaggg cggggttcgg cttctggcgt gtgaccggcg 1620
gctctagagc ctctgctaac catgttcatg ccttcttctt tttcctacag ctcctgggca 1680
acgtgctggt tgttgtgctg tctcatcatt ttggcaaaga attcatggga agaaggcgaa 1740
gtcatgagcg ccgggattta ccccctaacc tttatataag aaacaatgga tattactgct 1800
acagggaccc aaggacgggt aaagagtttg gattaggcag agacaggcga atcgcaatca 1860
ctgaagctat acaggccaac attgagttat tttcaggaca caaacacaag cctctgacag 1920
cgagaatcaa cagtgataat tccgttacgt tacattcatg gcttgatcgc tacgaaaaaa 1980
tcctggccag cagaggaatc aagcagaaga cactcataaa ttacatgagc aaaattaaag 2040
caataaggag gggtctgcct gatgctccac ttgaagacat caccacaaaa gaaattgcgg 2100
caatgctcaa tggatacata gacgagggca aggcggcgtc agccaagtta atcagatcaa 2160
cactgagcga tgcattccga gaggcaatag ctgaaggcca tataacaaca aaccatgtcg 2220
ctgccactcg cgcagcaaaa tctagagtaa ggagatcaag acttacggct gacgaatacc 2280
tgaaaattta tcaagcagca gaatcatcac catgttggct cagacttgca atggaactgg 2340
ctgttgttac cgggcaacga gttggtgatt tatgcgaaat gaagtggtct gatatcgtag 2400
atggatatct ttatgtcgag caaagcaaaa caggcgtaaa aattgccatc ccaacagcat 2460
tgcatattga tgctctcgga atatcaatga aggaaacact tgataaatgc aaagagattc 2520
ttggcggaga aaccataatt gcatctactc gtcgcgaacc gctttcatcc ggcacagtat 2580
caaggtattt tatgcgcgca cgaaaagcat caggtctttc cttcgaaggg gatccgccta 2640
cctttcacga gttgcgcagt ttgtctgcaa gactctatga gaagcagata agcgataagt 2700
ttgctcaaca tcttctcggg cataagtcgg acaccatggc atcacagtat cgtgatgaca 2760
gaggcaggga gtgggacaaa attgaaatca aataagaatt cactcctcag gtgcaggctg 2820
cctatcagaa ggtggtggct ggtgtggcca atgccctggc tcacaaatac cactgagatc 2880
tttttccctc tgccaaaaat tatggggaca tcatgaagcc ccttgagcat ctgacttctg 2940
gctaataaag gaaatttatt ttcattgcaa tagtgtgttg gaattttttg tgtctctcac 3000
tcggaaggac atatgggagg gcaaatcatt taaaacatca gaatgagtat ttggtttaga 3060
gtttggcaac atatgccata tgctggctgc catgaacaaa ggtggctata aagaggtcat 3120
cagtatatga aacagccccc tgctgtccat tccttattcc atagaaaagc cttgacttga 3180
ggttagattt tttttatatt ttgttttgtg ttattttttt ctttaacatc cctaaaattt 3240
tccttacatg ttttactagc cagatttttc ctcctctcct gactactccc agtcatagct 3300
gtccctcttc tcttatgaag atccctcgac ctgcagccca agcttggcgt aatcatggtc 3360
atagctgttt cctgtgtgaa attgttatcc gctcacaatt ccacacaaca tacgagccgg 3420
aagcataaag tgtaaagcct ggggtgccta atgagtgagc taactcacat taattgcgtt 3480
gcgctcactg cccgctttcc agtcgggaaa cctgtcgtgc cagcggatcc gcatctcaat 3540
tagtcagcaa ccatagtccc gcccctaact ccgcccatcc cgcccctaac tccgcccagt 3600
tccgcccatt ctccgcccca tggctgacta atttttttta tttatgcaga ggccgaggcc 3660
gcctcggcct ctgagctatt ccagaagtag tgaggaggct tttttggagg cctaggcttt 3720
tgcaaaaagc taacttgttt attgcagctt ataatggtta caaataaagc aatagcatca 3780
caaatttcac aaataaagca tttttttcac tgcattctag ttgtggtttg tccaaactca 3840
tcaatgtatc ttatcatgtc tggatccgct gcattaatga atcggccaac gcgcggggag 3900
aggcggtttg cgtattgggc gctcttccgc ttcctcgctc actgactcgc tgcgctcggt 3960
cgttcggctg cggcgagcgg tatcagctca ctcaaaggcg gtaatacggt tatccacaga 4020
atcaggggat aacgcaggaa agaacatgtg agcaaaaggc cagcaaaagg ccaggaaccg 4080
taaaaaggcc gcgttgctgg cgtttttcca taggctccgc ccccctgacg agcatcacaa 4140
aaatcgacgc tcaagtcaga ggtggcgaaa cccgacagga ctataaagat accaggcgtt 4200
tccccctgga agctccctcg tgcgctctcc tgttccgacc ctgccgctta ccggatacct 4260

gtccgccttt ctcccttcgg gaagcgtggc gctttctcaa tgctcacgct gtaggtatct 4320
cagttcggtg taggtcgttc gctccaagct gggctgtgtg cacgaacccc ccgttcagcc 4380
cgaccgctgc gccttatccg gtaactatcg tcttgagtcc aacccggtaa gacacgactt 4440
atcgccactg gcagcagcca ctggtaacag gattagcaga gcgaggtatg taggcggtgc 4500
tacagagttc ttgaagtggt ggcctaacta cggctacact agaaggacag tatttggtat 4560
ctgcgctctg ctgaagccag ttaccttcgg aaaaagagtt ggtagctctt gatccggcaa 4620
acaaaccacc gctggtagcg gtggtttttt tgtttgcaag cagcagatta cgcgcagaaa 4680
aaaaggatct caagaagatc ctttgatctt ttctacgggg tctgacgctc agtggaacga 4740
aaactcacgt taagggattt tggtcatgag attatcaaaa aggatcttca cctagatcct 4800
tttaaattaa aaatgaagtt ttaaatcaat ctaaagtata tatgagtaaa cttggtctga 4860
cagttaccaa tgcttaatca gtgaggcacc tatctcagcg atctgtctat ttcgttcatc 4920
catagttgcc tgactccccg tcgtgtagat aactacgata cgggagggct taccatctgg 4980
ccccagtgct gcaatgatac cgcgagaccc acgctcaccg gctccagatt tatcagcaat 5040
aaaccagcca gccggaaggg ccgagcgcag aagtggtcct gcaactttat ccgcctccat 5100
ccagtctatt aattgttgcc gggaagctag agtaagtagt tcgccagtta atagtttgcg 5160
caacgttgtt gccattgcta caggcatcgt ggtgtcacgc tcgtcgtttg gtatggcttc 5220
attcagctcc ggttcccaac gatcaaggcg agttacatga tcccccatgt tgtgcaaaaa 5280
agcggttagc tccttcggtc ctccgatcgt tgtcagaagt aagttggccg cagtgttatc 5340
actcatggtt atggcagcac tgcataattc tcttactgtc atgccatccg taagatgctt 5400
ttctgtgact ggtgagtact caaccaagtc attctgagaa tagtgtatgc ggcgaccgag 5460
ttgctcttgc ccggcgtcaa tacgggataa taccgcgcca catagcagaa ctttaaaagt 5520
gctcatcatt ggaaaacgtt cttcggggcg aaaactctca aggatcttac cgctgttgag 5580
atccagttcg atgtaaccca ctcgtgcacc caactgatct tcagcatctt ttactttcac 5640
cagcgtttct gggtgagcaa aaacaggaag gcaaaatgcc gcaaaaaagg gaataagggc 5700
gacacggaaa tgttgaatac tcatactctt cctttttcaa tattattgaa gcatttatca 5760
gggttattgt ctcatgagcg gatacatatt tgaatgtatt tagaaaaata aacaaatagg 5820
ggttccgcgc acatttcccc gaaaagtgcc acctg 5855

<210> 28
<211> 37
<212> DNA
<213> Artificial Sequence

<220>
<223> 5PacSV40 Primer

<400> 28
ctgttaatta actgtggaat gtgtgtcagt tagggtg 37

<210> 29
<211> 20
<212> DNA
<213> Artificial Sequence

<220>
<223> Antisense Zeo Primer

<400> 29
tgaacagggt cacgtcgtcc 20

<210> 30
<211> 1032
<212> DNA
<213> Escherichia Coli

<220>
<221> CDS
<222> (1)...(1032)
<223> nucleotide sequence encoding Cre recombinase

<400> 30

atg tcc aat tta ctg acc gta cac caa aat ttg cct gca tta ccg gtc 48
Met Ser Asn Leu Leu Thr Val His Gln Asn Leu Pro Ala Leu Pro Val
1               5                   10                  15

gat gca acg agt gat gag gtt cgc aag aac ctg atg gac atg ttc agg 96
Asp Ala Thr Ser Asp Glu Val Arg Lys Asn Leu Met Asp Met Phe Arg
            20                  25                  30

gat cgc cag gcg ttt tct gag cat acc tgg aaa atg ctt ctg tcc gtt 144
Asp Arg Gln Ala Phe Ser Glu His Thr Trp Lys Met Leu Leu Ser Val
        35                  40                  45

tgc cgg tcg tgg gcg gca tgg tgc aag ttg aat aac cgg aaa tgg ttt 192
Cys Arg Ser Trp Ala Ala Trp Cys Lys Leu Asn Asn Arg Lys Trp Phe
    50                  55                  60

ccc gca gaa cct gaa gat gtt cgc gat tat ctt cta tat ctt cag gcg 240
Pro Ala Glu Pro Glu Asp Val Arg Asp Tyr Leu Leu Tyr Leu Gln Ala
65                  70                  75                  80

cgc ggt ctg gca gta aaa act atc cag caa cat ttg ggc cag cta aac 288
Arg Gly Leu Ala Val Lys Thr Ile Gln Gln His Leu Gly Gln Leu Asn
                85                  90                  95

atg ctt cat cgt cgg tcc ggg ctg cca cga cca agt gac agc aat gct 336
Met Leu His Arg Arg Ser Gly Leu Pro Arg Pro Ser Asp Ser Asn Ala
            100                 105                 110

gtt tca ctg gtt atg cgg cgg atc cga aaa gaa aac gtt gat gcc ggt 384
Val Ser Leu Val Met Arg Arg Ile Arg Lys Glu Asn Val Asp Ala Gly
        115                 120                 125

gaa cgt gca aaa cag gct cta gcg ttc gaa cgc act gat ttc gac cag 432
Glu Arg Ala Lys Gln Ala Leu Ala Phe Glu Arg Thr Asp Phe Asp Gln
    130                 135                 140

gtt cgt tca ctc atg gaa aat agc gat cgc tgc cag gat ata cgt aat 480
Val Arg Ser Leu Met Glu Asn Ser Asp Arg Cys Gln Asp Ile Arg Asn
145                 150                 155                 160

ctg gca ttt ctg ggg att gct tat aac acc ctg tta cgt ata gcc gaa 528
Leu Ala Phe Leu Gly Ile Ala Tyr Asn Thr Leu Leu Arg Ile Ala Glu
                165                 170                 175

att gcc agg atc agg gtt aaa gat atc tca cgt act gac ggt ggg aga 576
Ile Ala Arg Ile Arg Val Lys Asp Ile Ser Arg Thr Asp Gly Gly Arg
            180                 185                 190

atg tta atc cat att ggc aga acg aaa acg ctg gtt agc acc gca ggt 624
Met Leu Ile His Ile Gly Arg Thr Lys Thr Leu Val Ser Thr Ala Gly
        195                 200                 205

gta gag aag gca ctt agc ctg ggg gta act aaa ctg gtc gag cga tgg 672
Val Glu Lys Ala Leu Ser Leu Gly Val Thr Lys Leu Val Glu Arg Trp
    210                 215                 220

att tcc gtc tct ggt gta gct gat gat ccg aat aac tac ctg ttt tgc 720
Ile Ser Val Ser Gly Val Ala Asp Asp Pro Asn Asn Tyr Leu Phe Cys
225                 230                 235                 240

cgg gtc aga aaa aat ggt gtt gcc gcg cca tct gcc acc agc cag cta 768

Arg Val Arg Lys Asn Gly Val Ala Ala Pro Ser Ala Thr Ser Gln Leu
                245                 250                 255

tca act cgc gcc ctg gaa ggg att ttt gaa gca act cat cga ttg att     816
Ser Thr Arg Ala Leu Glu Gly Ile Phe Glu Ala Thr His Arg Leu Ile
            260                 265                 270

tac ggc gct aag gat gac tct ggt cag aga tac ctg gcc tgg tct gga     864
Tyr Gly Ala Lys Asp Asp Ser Gly Gln Arg Tyr Leu Ala Trp Ser Gly
        275                 280                 285

cac agt gcc cgt gtc gga gcc gcg cga gat atg gcc cgc gct gga gtt     912
His Ser Ala Arg Val Gly Ala Ala Arg Asp Met Ala Arg Ala Gly Val
    290                 295                 300

tca ata ccg gag atc atg caa gct ggt ggc tgg acc aat gta aat att     960
Ser Ile Pro Glu Ile Met Gln Ala Gly Gly Trp Thr Asn Val Asn Ile
305                 310                 315                 320

gtc atg aac tat atc cgt aac ctg gat agt gaa aca ggg gca atg gtg     1008
Val Met Asn Tyr Ile Arg Asn Leu Asp Ser Glu Thr Gly Ala Met Val
                325                 330                 335

cgc ctg ctg gaa gat ggc gat tag                                     1032
Arg Leu Leu Glu Asp Gly Asp  *
            340

<210> 31
<211> 343
<212> PRT
<213> Escherichia Coli

<400> 31
Met Ser Asn Leu Leu Thr Val His Gln Asn Leu Pro Ala Leu Pro Val
1               5                   10                  15
Asp Ala Thr Ser Asp Glu Val Arg Lys Asn Leu Met Asp Met Phe Arg
            20                  25                  30
Asp Arg Gln Ala Phe Ser Glu His Thr Trp Lys Met Leu Leu Ser Val
        35                  40                  45
Cys Arg Ser Trp Ala Ala Trp Cys Lys Leu Asn Asn Arg Lys Trp Phe
    50                  55                  60
Pro Ala Glu Pro Glu Asp Val Arg Asp Tyr Leu Leu Tyr Leu Gln Ala
65                  70                  75                  80
Arg Gly Leu Ala Val Lys Thr Ile Gln Gln His Leu Gly Gln Leu Asn
                85                  90                  95
Met Leu His Arg Arg Ser Gly Leu Pro Arg Pro Ser Asp Ser Asn Ala
            100                 105                 110
Val Ser Leu Val Met Arg Arg Ile Arg Lys Glu Asn Val Asp Ala Gly
        115                 120                 125
Glu Arg Ala Lys Gln Ala Leu Ala Phe Glu Arg Thr Asp Phe Asp Gln
    130                 135                 140
Val Arg Ser Leu Met Glu Asn Ser Asp Arg Cys Gln Asp Ile Arg Asn
145                 150                 155                 160
Leu Ala Phe Leu Gly Ile Ala Tyr Asn Thr Leu Leu Arg Ile Ala Glu
                165                 170                 175
Ile Ala Arg Ile Arg Val Lys Asp Ile Ser Arg Thr Asp Gly Gly Arg
            180                 185                 190
Met Leu Ile His Ile Gly Arg Thr Lys Thr Leu Val Ser Thr Ala Gly
        195                 200                 205
Val Glu Lys Ala Leu Ser Leu Gly Val Thr Lys Leu Val Glu Arg Trp
    210                 215                 220

Ile Ser Val Ser Gly Val Ala Asp Asp Pro Asn Asn Tyr Leu Phe Cys
225                 230                 235                 240
Arg Val Arg Lys Asn Gly Val Ala Ala Pro Ser Ala Thr Ser Gln Leu
                245                 250                 255
Ser Thr Arg Ala Leu Glu Gly Ile Phe Glu Ala Thr His Arg Leu Ile
            260                 265                 270
Tyr Gly Ala Lys Asp Asp Ser Gly Gln Arg Tyr Leu Ala Trp Ser Gly
        275                 280                 285
His Ser Ala Arg Val Gly Ala Ala Arg Asp Met Ala Arg Ala Gly Val
    290                 295                 300
Ser Ile Pro Glu Ile Met Gln Ala Gly Gly Trp Thr Asn Val Asn Ile
305                 310                 315                 320
Val Met Asn Tyr Ile Arg Asn Leu Asp Ser Glu Thr Gly Ala Met Val
                325                 330                 335
Arg Leu Leu Glu Asp Gly Asp
            340

<210> 32
<211> 33
<212> DNA
<213> Artificial Sequence

<220>
<223> attB1 recognition sequence

<400> 32
tgaagcctgc ttttttatac taacttgagc gaa                                33

<210> 33
<211> 25
<212> DNA
<213> Artificial Sequence

<220>
<223> m-att recognition sequence

<221> misc_difference
<222> 18
<223> n is a or g or c or t/u

<400> 33
rkycwgcttt yktrtacnaa stsgb                                        25

<210> 34
<211> 25
<212> DNA
<213> Artificial Sequence

<220>
<223> m-attB recognition sequence

<221> misc_difference
<222> 18
<223> n is a or c or g or t/u

<400> 34
agccwgcttt yktrtacnaa ctsgb                                        25

<210> 35
<211> 25
<212> DNA

<213> Artificial Sequence

<220>
<223> m-attR recognition sequence

<221> misc_difference
<222> 18
<223> n is a or g or c or t/u

<400> 35
gttcagcttt cktrtacnaa ctsgb                                          25

<210> 36
<211> 25
<212> DNA
<213> Artificial Sequence

<220>
<223> m-attL recognition sequence

<221> misc_difference
<222> 18
<223> n is a or g or c or t/u

<400> 36
agccwgcttt cktrtacnaa gtsgb                                          25

<210> 37
<211> 25
<212> DNA
<213> Artificial Sequence

<220>
<223> m-attP1 recognition sequence

<221> misc_difference
<222> 18
<223> n is a or g or c or t/u

<400> 37
gttcagcttt yktrtacnaa gtsgb                                          25

<210> 38
<211> 25
<212> DNA
<213> Artificial Sequence

<220>
<223> attB2 recognition sequence

<400> 38
agcctgcttt cttgtacaaa cttgt                                          25

<210> 39
<211> 25
<212> DNA
<213> Artificial Sequence

<220>
<223> attB3 recognition sequence

<400> 39
acccagcttt cttgtacaaa cttgt                                          25

<210> 40
<211> 25
<212> DNA
<213> Artificial Sequence

<220>
<223> attR1 recognition sequence

<400> 40
gttcagcttt tttgtacaaa cttgt                                          25

<210> 41
<211> 25
<212> DNA
<213> Artificial Sequence

<220>
<223> attR2 recognition sequence

<400> 41
gttcagcttt cttgtacaaa cttgt                                          25

<210> 42
<211> 25
<212> DNA
<213> Artificial Sequence

<220>
<223> attR3 recognition sequence

<400> 42
gttcagcttt cttgtacaaa gttgg                                          25

<210> 43
<211> 25
<212> DNA
<213> Artificial Sequence

<220>
<223> attL1 recognition sequence

<400> 43
agcctgcttt tttgtacaaa gttgg                                          25

<210> 44
<211> 25
<212> DNA
<213> Artificial Sequence

<220>
<223> attL2 recognition sequence

<400> 44
agcctgcttt cttgtacaaa gttgg                                          25

<210> 45
<211> 25
<212> DNA

<213> Artificial Sequence

<220>
<223> attL3 recognition sequence

<400> 45
acccagcttt cttgtacaaa gttgg                                          25

<210> 46
<211> 25
<212> DNA
<213> Artificial Sequence

<220>
<223> attP1 recognition sequence

<400> 46
gttcagcttt tttgtacaaa gttgg                                          25

<210> 47
<211> 25
<212> DNA
<213> Artificial Sequence

<220>
<223> attP2,P3 recognition sequence

<400> 47
gttcagcttt cttgtacaaa gttgg                                          25

<210> 48
<211> 282
<212> DNA
<213> Artificial Sequence

<220>
<223> attP recognition sequence

<400> 48
ccttgcgcta atgctctgtt acaggtcact aataccatct aagtagttga ttcatagtga 60
ctgcatatgt tgtgttttac agtattatgt agtctgtttt ttatgcaaaa tctaatttaa 120
tatattgata tttatatcat tttacgtttc tcgttcagct tttttatact aagttggcat 180
tataaaaaag cattgcttat caatttgttg caacgaacag gtcactatca gtcaaaataa 240
aatcattatt tgatttcaat tttgtcccac tccctgcctc tg                       282

<210> 49
<211> 1071
<212> DNA
<213> Artificial Sequence

<220>
<223> nucleotide sequence encoding Integrase E174R

<221> CDS
<222> (1)...(1071)
<223> Integrase E174R

<400> 49
atg gga aga agg cga agt cat gag cgc cgg gat tta ccc cct aac ctt   48
Met Gly Arg Arg Arg Ser His Glu Arg Arg Asp Leu Pro Pro Asn Leu
 1               5                  10                  15

tat ata aga aac aat gga tat tac tgc tac agg gac cca agg acg ggt    96
Tyr Ile Arg Asn Asn Gly Tyr Tyr Cys Tyr Arg Asp Pro Arg Thr Gly
            20                  25                  30

aaa gag ttt gga tta ggc aga gac agg cga atc gca atc act gaa gct    144
Lys Glu Phe Gly Leu Gly Arg Asp Arg Arg Ile Ala Ile Thr Glu Ala
        35                  40                  45

ata cag gcc aac att gag tta ttt tca gga cac aaa cac aag cct ctg    192
Ile Gln Ala Asn Ile Glu Leu Phe Ser Gly His Lys His Lys Pro Leu
    50                  55                  60

aca gcg aga atc aac agt gat aat tcc gtt acg tta cat tca tgg ctt    240
Thr Ala Arg Ile Asn Ser Asp Asn Ser Val Thr Leu His Ser Trp Leu
65                  70                  75                  80

gat cgc tac gaa aaa atc ctg gcc agc aga gga atc aag cag aag aca    288
Asp Arg Tyr Glu Lys Ile Leu Ala Ser Arg Gly Ile Lys Gln Lys Thr
                85                  90                  95

ctc ata aat tac atg agc aaa att aaa gca ata agg agg ggt ctg cct    336
Leu Ile Asn Tyr Met Ser Lys Ile Lys Ala Ile Arg Arg Gly Leu Pro
            100                 105                 110

gat gct cca ctt gaa gac atc acc aca aaa gaa att gcg gca atg ctc    384
Asp Ala Pro Leu Glu Asp Ile Thr Thr Lys Glu Ile Ala Ala Met Leu
        115                 120                 125

aat gga tac ata gac gag ggc aag gcg gcg tca gcc aag tta atc aga    432
Asn Gly Tyr Ile Asp Glu Gly Lys Ala Ala Ser Ala Lys Leu Ile Arg
    130                 135                 140

tca aca ctg agc gat gca ttc cga gag gca ata gct gaa ggc cat ata    480
Ser Thr Leu Ser Asp Ala Phe Arg Glu Ala Ile Ala Glu Gly His Ile
145                 150                 155                 160

aca aca aac cat gtc gct gcc act cgc gca gca aaa tct aga gta agg    528
Thr Thr Asn His Val Ala Ala Thr Arg Ala Ala Lys Ser Arg Val Arg
                165                 170                 175

aga tca aga ctt acg gct gac gaa tac ctg aaa att tat caa gca gca    576
Arg Ser Arg Leu Thr Ala Asp Glu Tyr Leu Lys Ile Tyr Gln Ala Ala
            180                 185                 190

gaa tca tca cca tgt tgg ctc aga ctt gca atg gaa ctg gct gtt gtt    624
Glu Ser Ser Pro Cys Trp Leu Arg Leu Ala Met Glu Leu Ala Val Val
        195                 200                 205

acc ggg caa cga gtt ggt gat tta tgc gaa atg aag tgg tct gat atc    672
Thr Gly Gln Arg Val Gly Asp Leu Cys Glu Met Lys Trp Ser Asp Ile
    210                 215                 220

gta gat gga tat ctt tat gtc gag caa agc aaa aca ggc gta aaa att    720
Val Asp Gly Tyr Leu Tyr Val Glu Gln Ser Lys Thr Gly Val Lys Ile
225                 230                 235                 240

gcc atc cca aca gca ttg cat att gat gct ctc gga ata tca atg aag    768
Ala Ile Pro Thr Ala Leu His Ile Asp Ala Leu Gly Ile Ser Met Lys
                245                 250                 255

gaa aca ctt gat aaa tgc aaa gag att ctt ggc gga gaa acc ata att     816
Glu Thr Leu Asp Lys Cys Lys Glu Ile Leu Gly Gly Glu Thr Ile Ile
            260                 265                 270

gca tct act cgt cgc gaa ccg ctt tca tcc ggc aca gta tca agg tat     864
Ala Ser Thr Arg Arg Glu Pro Leu Ser Ser Gly Thr Val Ser Arg Tyr
        275                 280                 285

ttt atg cgc gca cga aaa gca tca ggt ctt tcc ttc gaa ggg gat ccg     912
Phe Met Arg Ala Arg Lys Ala Ser Gly Leu Ser Phe Glu Gly Asp Pro
    290                 295                 300

cct acc ttt cac gag ttg cgc agt ttg tct gca aga ctc tat gag aag     960
Pro Thr Phe His Glu Leu Arg Ser Leu Ser Ala Arg Leu Tyr Glu Lys
305                 310                 315                 320

cag ata agc gat aag ttt gct caa cat ctt ctc ggg cat aag tcg gac     1008
Gln Ile Ser Asp Lys Phe Ala Gln His Leu Leu Gly His Lys Ser Asp
                325                 330                 335

acc atg gca tca cag tat cgt gat gac aga ggc agg gag tgg gac aaa     1056
Thr Met Ala Ser Gln Tyr Arg Asp Asp Arg Gly Arg Glu Trp Asp Lys
            340                 345                 350

att gaa atc aaa taa                                                 1071
Ile Glu Ile Lys  *
        355

<210> 50
<211> 356
<212> PRT
<213> Artificial Sequence

<220>
<223> Integrase E174R

<400> 50
Met Gly Arg Arg Arg Ser His Glu Arg Arg Asp Leu Pro Pro Asn Leu
1               5                   10                  15
Tyr Ile Arg Asn Asn Gly Tyr Tyr Cys Tyr Arg Asp Pro Arg Thr Gly
            20                  25                  30
Lys Glu Phe Gly Leu Gly Arg Asp Arg Arg Ile Ala Ile Thr Glu Ala
        35                  40                  45
Ile Gln Ala Asn Ile Glu Leu Phe Ser Gly His Lys His Lys Pro Leu
    50                  55                  60
Thr Ala Arg Ile Asn Ser Asp Asn Ser Val Thr Leu His Ser Trp Leu
65                  70                  75                  80
Asp Arg Tyr Glu Lys Ile Leu Ala Ser Arg Gly Ile Lys Gln Lys Thr
                85                  90                  95
Leu Ile Asn Tyr Met Ser Lys Ile Lys Ala Ile Arg Arg Gly Leu Pro
            100                 105                 110
Asp Ala Pro Leu Glu Asp Ile Thr Thr Lys Glu Ile Ala Ala Met Leu
        115                 120                 125
Asn Gly Tyr Ile Asp Glu Gly Lys Ala Ala Ser Ala Lys Leu Ile Arg
    130                 135                 140
Ser Thr Leu Ser Asp Ala Phe Arg Glu Ala Ile Ala Glu Gly His Ile
145                 150                 155                 160
Thr Thr Asn His Val Ala Ala Thr Arg Ala Ala Lys Ser Arg Val Arg
                165                 170                 175
Arg Ser Arg Leu Thr Ala Asp Glu Tyr Leu Lys Ile Tyr Gln Ala Ala
            180                 185                 190

Glu Ser Ser Pro Cys Trp Leu Arg Leu Ala Met Glu Leu Ala Val Val
        195                 200                 205
Thr Gly Gln Arg Val Gly Asp Leu Cys Glu Met Lys Trp Ser Asp Ile
    210                 215                 220
Val Asp Gly Tyr Leu Tyr Val Glu Gln Ser Lys Thr Gly Val Lys Ile
225                 230                 235                 240
Ala Ile Pro Thr Ala Leu His Ile Asp Ala Leu Gly Ile Ser Met Lys
                245                 250                 255
Glu Thr Leu Asp Lys Cys Lys Glu Ile Leu Gly Gly Glu Thr Ile Ile
            260                 265                 270
Ala Ser Thr Arg Arg Glu Pro Leu Ser Ser Gly Thr Val Ser Arg Tyr
        275                 280                 285
Phe Met Arg Ala Arg Lys Ala Ser Gly Leu Ser Phe Glu Gly Asp Pro
    290                 295                 300
Pro Thr Phe His Glu Leu Arg Ser Leu Ser Ala Arg Leu Tyr Glu Lys
305                 310                 315                 320
Gln Ile Ser Asp Lys Phe Ala Gln His Leu Leu Gly His Lys Ser Asp
                325                 330                 335
Thr Met Ala Ser Gln Tyr Arg Asp Asp Arg Gly Arg Glu Trp Asp Lys
            340                 345                 350
Ile Glu Ile Lys
        355

<210> 51
<211> 34
<212> DNA
<213> Artificial Sequence

<220>
<223> Lox P Site

<400> 51
ataacttcgt ataatgtatg ctatacgaag ttat                                34